# EUROPEAN PATENT SPECIFICATION

(11) **EP 0 577 712 B1**
(45) Date of publication and mention of the grant of the patent: **29.07.1998**
(21) Application number: 92908770.8
(22) Date of filing: 26.02.1992
(51) Int. Cl.: C07D 263/46, C07D 263/48, A01N 43/76, C07F 9/653, C07D 263/52, C07D 498/10, C07D 403/04, C07D 403/12

(54) **FUNGICIDAL 4-THIOXOOXAZOLIDIN-2-ONES AND 4-IMINOOXAZOLINDIN-2-ONES**
FUNGIZIDE 4-THIOXOOXAZOLIDIN-2-ONE UND 4-IMINOOXAZOLIDIN-2-ONE
4-THIOXOOXAZOLIDINE-2-ONES ET 4-IMINOOXAZOLIDINE-2-ONES FONGICIDES

(30) Priority: 15.03.1991 US 670539
(43) Date of publication of application: 12.01.1994
(73) Proprietor: E.I. DU PONT DE NEMOURS AND COMPANY, Wilmington Delaware 19898 (US)
(72) Inventor: STERNBERG, Jeffrey, Arthur, Newark, DE 19713 (US); ADAMS, John, Benjamin, Jr., Hockessin, DE 19707 (US)
(74) Representative: Woodman, Derek
(86) International application number: US9201224
(87) International publication number: WO9216515

(56) References cited:
- EP-A- 0 393 911
- DE-A- 3 607 068
- US-A- 3 671 535

## Description

### CROSS REFERENCE TO RELATED APPLICATION

This is a continuation-in-part of application U.S.S.N 07/670,539, filed March 15, 1991.

### BACKGROUND OF THE INVENTION

New fungicides for controlling fungus growth on vegetation are in constant demand. In the most common situation, such compounds are sought to selectively control fungus growth on such useful crops as cotton, rice, corn, wheat and soybeans. Unchecked fungus growth in such crops can cause significant losses, reducing profit to the grower and increasing costs to the consumer.

A number of fungicides have been developed and employed. For example, WO 90/12791 pertains to the use of compounds of Formula i as fungicides, certain novel compounds of Formula i, compositions containing those compounds, and processes for preparing them: 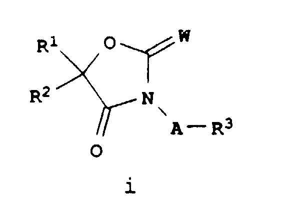 wherein:
A is O and NR⁴; and
W is O and S.

The compounds of this invention differ from those of the current invention in the nature of R² and the lack of the A = S or N-J moiety shown in Formula I below.

The above reference does not specifically disclose the compounds of the instant invention or their particular fungicidal utility.
DE-A-3607068 also discloses oxazolidine derivatives with fungicidal properties.

The compounds of the prior art, although useful as fungicides, tend to have drawbacks including poor efficacy, toxicity to non-target organisms and/or unacceptable environmental effects.

### SUMMARY OF THE INVENTION

This invention pertains to compounds of Formula I including all geometric and stereoisomers, agriculturally suitable salts thereof, agricultural compositions containing them and their use as fungicides: 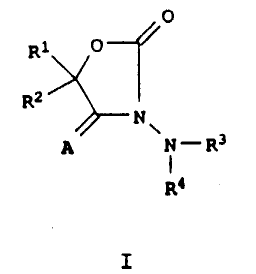 wherein:
- A: is S or N-J;
- J: is R¹⁵; C(=O)R¹⁶; C(=O)OR¹⁷; C(=O)SR¹⁸; C(=O)NR¹⁹R²⁰; P(=O)(C₁-C₄ alkyl)₂; or OG;
- G: is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
- R¹: is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
- R²: is C₁-C₂₀ alkyl optionally substituted with R²²; C₂-C₂₀ alkoxyalkyl optionally substituted with R³⁵; C₂-C₂₀ alkenyl optionally substituted with R⁴²; C₂-C₂₀ alkynyl optionally substituted with R⁴¹; (CH₂CH₂OCH₂CH₂)CH-; (CH₂CH₂SCH₂CH₂)CH-; (CH₂CH₂SO₂CH₂CH₂)CH-; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷; or
- R¹ and R²: can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 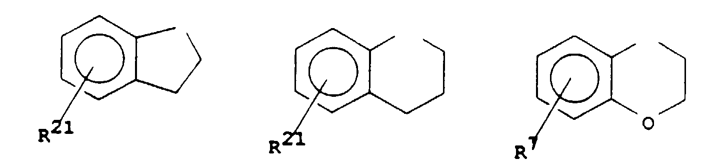
- R³: is phenyl, pyridyl, or pyrimidinyl each optionally substituted with R¹⁰; or phenylmethyl;
- R⁴: is H or methyl;
- R⁵: is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
- R⁶, R⁷, R¹², R¹³, R²⁴, R²⁶ and R³⁴: are independently 1-2 halogen; nitro; C₁-C₄ alkyl; trifluoromethyl; methylthio; or C₁-C₄ alkoxy;
- R⁸, R¹⁴, R²⁰, R⁴⁰ and R³⁸: are independently H or C₁-C₄ alkyl;
- R⁹: is C₁-C₁₈ alkyl; or phenyl optionally substituted with R⁷;
- R¹⁰, R²⁵ and R³³: are independently 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
- R¹¹ and R³⁶: are independently C₁-C₆ alkyl; or phenyl optionally substituted with R¹²;
- R¹⁵: is H; C₁-C₈ alkyl optionally substituted with C₁-C₂ alkoxy; C₃-C₆ cycloalkyl; C₃-C₈ alkenyl; C₃-C₈ alkynyl; phenyl optionally substituted with R¹³; benzyl optionally substituted with R¹³ on the phenyl ring and with R²⁰ on the benzylic carbon; or pyridyl optionally substituted with R¹³;
- R¹⁶: is H; C₁-C₁₇ alkyl optionally substituted with R³¹; C₂-C₁₇ alkenyl optionally substituted with R³²; C₂-C₇ alkynyl; C₃-C₈ cycloalkyl; C₅-C₆ cycloalkenyl; C₆-C₇ alkylcycloalkyl; C₄-C₈ cycloalkylalkyl; phenyl optionally substituted with R³³; naphthalenyl, furanyl, thienyl, benzoyl, or pyridyl each optionally substituted with R³⁴; or C₂-C₅ alkoxycarbonyl;
- R¹⁷ and R¹⁸: are independently C₁-C₁₈ alkyl optionally substituted with R²³; C₂-C₁₀ alkenyl optionally substituted with R³²; C₃-C₈ alkynyl; C₃-C₁₂ cycloalkyl; C₅-C₆ cycloalkenyl; C₆-C₇ alkylcycloalkyl; C₆-C₇ cycloalkylalkyl; or phenyl, naphthalenyl, or thienyl each optionally substituted with R³⁴;
- R¹⁹: is H; C₁-C₁₀ alkyl; C₅-C₆ cycloalkyl; or phenyl optionally substituted with R³⁴; or
- R¹⁹ and R²⁰: can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, and -CH₂CH(Me)OCH (Me)CH₂-;
- R²¹: is 1-2 halogen; nitro; C₁-C₄ alkyl; trifluoromethyl; methylthio; or C₁-C₄ alkoxy; or phenoxy optionally substituted with R²⁶;
- R²²: is cyano; nitro; C₁-C₁₉ alkylthio; C₁-C₁₉ alkylsulfinyl; C₁-C₁₉ haloalkoxy; C₅-C₆ cycloalkyloxy; C₃-C₁₉ alkenyloxy; C₃-C₁₉ alkynyloxy; C₁-C₁₉ alkylsulfonyl; C₂-C₁₉ alkoxycarbonyl; hydroxyl; hydroxycarbonyl; R²⁸C(=O)O; R²⁸OC(=O)O; R²⁸R⁴⁰NC(=O)O; R³⁹R⁴⁰N; (C₁-C₄ alkoxy)₂P(=E)O; R¹¹SO₃; R⁴⁰R¹⁴R³⁸N⁺; phenyl, phenylthio, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; C₃-C₆ cycloalkyl; 2-tetrahydropyranyloxy; or C(=Q)R⁴⁰;
- E: is O or S;
- Q: is O or N-T-W;
- T: is O; NR³⁷; or a direct bond;
- W: is H; C₁-C₈ alkyl, C₃-C₈ alkenyl; phenylmethyl optionally substituted with R⁷ on the phenyl ring and R¹⁴ on the benzylic carbon; phenyl or pyridyl each optionally substituted with R⁷; C(=O)R²⁸; C(=O)OR²⁸; or C(=O)NR²⁸R¹⁴
- R²³: is 1-3 halogen; C₁-C₁₂ alkoxy; C₁-C₁₂ alkylthio; phenyl or naphthalenyl each optionally substituted with R³⁴; or phenoxymethyl optionally substituted with R³⁴ on the phenyl ring;
- R²⁷: is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
- R²⁸: is C₁-C₈ alkyl; or phenyl or pyridyl each optionally substituted with R³⁰;
- R³⁰: is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
- R³¹: is 1-3 halogen; C₁-C₁₈ alkoxy; allyloxy; C₁-C₁₈ alkylthio; phenyl, phenoxy, benzyloxy, or phenylthio each optionally substituted with R³⁴ on the phenyl ring; acetyl; or C₂-C₅ alkoxycarbonyl;
- R³²: is 1-3 halogen; or C₁-C₄ alkoxy;
- R³⁵: is cyano; nitro; C₁-C₁₇ alkylthio; C₁-C₁₇ alkylsulfinyl; C₁-C₁₇ haloalkoxy; C₅-C₆ cycloalkyloxy; C₂-C₁₇ haloalkenyl; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; C₂-C₁₇ alkoxyalkoxy; C₃-C₁₇ alkynyl; C₃-C₆ cycloalkyl; or C₂-C₁₇ haloalkoxyalkoxy;
- R³⁷: is H; C₁-C₆ alkyl; or phenyl optionally substituted with R⁷;
- R³⁹: is C₁-C₁₉ alkyl; C₂-C₁₉ alkylcarbonyl; C₂-C₁₉ alkoxycarbonyl; (R⁹R⁴⁰N)C=O; phenyl optionally substituted with R²⁵; or phenoxycarbonyl optionally substituted with R⁷;
- R⁴¹: is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)2P(=E)O; R¹¹SO₃; R⁴⁰R¹⁴R³⁸N⁺; phenyl, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; or C₃-C₆ cycloalkyl;
- R⁴²: is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl;
2-tetrahydropyranyloxy; C₁-C₁₇ alkoxy; 1-3 halogen; C₂-C₁₇ alkoxyalkoxy; C₃-C₁₇ alkynyl; or C₃-C₆ cycloalkyl;
provided that
i) when R² is a substituted phenyl or heterocyclic ring, then only H or F can be substituted on the carbon atom(s) of the phenyl or heterocyclic ring adjacent to the carbon bearing the oxazolidinone ring;
ii) when R³ is a phenyl or heterocyclic ring disubstituted with two alkyl or alkoxy groups, or one alkyl and one alkoxy group, then at least one of the alkyl and alkoxy groups is methyl or methoxy; and
iii) the total number of carbons in R², R¹⁶, R¹⁷, and R¹⁸ is each less than or equal to 20.

A further embodiment of the invention pertains to a fungicidally active compound of Formula I comprising 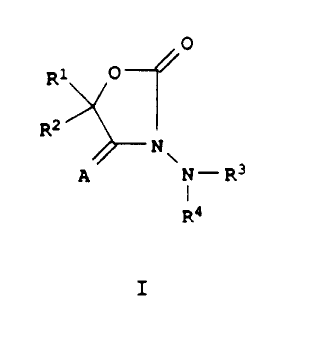 wherein:
- A: is S or N-OG;
- G: is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
- R¹: is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
- R²: is C₁-C₆ alkyl; C₅-C₇ cycloalkyl; C₂-C₆ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁷; or phenoxy or phenylthio each optionally substituted with R⁷; or
- R¹ and R²: can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 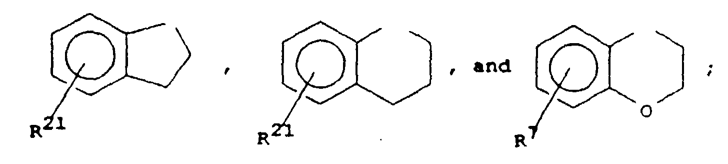
- R³: is phenyl, pyridyl, or pyrimidinyl each optionally substituted with R¹⁰; or phenylmethyl;
- R⁴: is H or methyl;
- R⁵: is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; and C₂-C₆ alkoxycarbonyl;
- R⁶, R⁷, R²⁴, R²⁶ and R³⁴: are independently 1-2 halogen; nitro; C₁-C₄ alkyl; trifluoromethyl; methylthio; or C₁-C₄ alkoxy;
- R⁸: is H or C₁-C₄ alkvl:
- R¹⁰: is 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
- R²¹: is halogen; nitro; C₁-C₄ alkyl; trifluoromethyl; methylthio; or C₁-C₄ alkoxy; or phenoxy optionally substituted with R²⁶;
- R²⁷: is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; C₂-C₄ alkoxycarbonyl; or phenoxy; and
- R³⁶: is C₁-C₆ alkyl; or phenyl optionally substituted with R¹².

A still further embodiment of the invention pertains to a fungicidally active composition comprising the compounds of Formula I.

Another embodiment of the invention pertains to a method of controlling disease in plants comprising applying to said plants a fungicidally active compound of Formula I.

### DETAILED DESCRIPTION OF THE INVENTION

In the above recitations, the term "alkyl", used either alone or in compound words such as "alkylthio" or "haloalkyl" denotes straight-chain or branched alkyl, e.g. methyl, ethyl, *n*-propyl, *i*-propyl, or the different butyl, pentyl or hexyl isomers.

"Alkenyl" denotes straight-chain or branched alkenes, e.g. 1-propenyl, 2-propenyl, 3-propenyl and the different butenyl, pentenyl and hexenyl isomers. "Alkenyl" also denotes polyenes such as 1,3-hexadiene and 2,4,6-heptatriene,

"Alkenyloxy" denotes straight-chain or branched alkenyloxy moieties. Examples of alkenyloxy include H₂C=CHCH₂O, (CH₃)₂C=CHCH₂O, (CH₃)CH=CHCH₂O, (CH₃)CH=C(CH₃)CH₂O and CH₂=CHCH₂CH₂O.

"Alkynyl" denotes straight-chain or branched alkynes, e.g., ethynyl, 1-propynyl, 3-propynyl and the different butynyl, pentynyl and hexynyl isomers. "Alkynyl" can also denote moieties comprised of multiple triple bonds, e.g. 2,7-octadiyne and 2,5,8-decatriyne.

"Alkynyloxy" denotes straight-chain or branched alkynyloxy moieties. Examples include HC≡CCH₂O, CH₃C≡CCH₂O and CH₃C≡CCH₂CH₂O.

"Alkylthio" denotes branched or straight-chain alkylthio moieties, e.g. methylthio, ethylthio, and the different propylthio, butylthio, pentylthio and hexylthio isomers.

Examples of "alkylsulfonyl" include CH₃SO₂, CH₃CH₂SO₂, CH₃CH₂CH₂SO₂, (CH₃)₂CHSO₂ and the different butylsulfonyl, pentylsulfonyl and hexylsulfonyl isomers.

"Alkylsulfinyl" denotes both enantiomers of an alkylsulfinyl group. For example, CH₃S(O), CH₃CH₂S(O), CH₃CH₂CH₂S(O), (CH₃)₂CHS(O) and the different butylsulfinyl, pentylsulfinyl and hexylsufinyl isomers.

"Alkoxy" denotes, for example, methoxy, ethoxy, n-propyloxy, isopropyloxy and the different butoxy, pentoxy and hexyloxy isomers.

Examples of "alkoxyalkoxyalkoxy" include CH₃OCH₂CH₂OCH₂O, CH₃CH₂OCH₂CH₂OCH₂O, and (CH₃)₂CHOCH₂CH₂OCH₂O.

"Cycloalkyl" denotes, for example, cyclopropyl, cyclobutyl, cyclopentyl, and cyclohexyl. The term "cycloalkyloxy" denotes the same groups linked through an oxygen atom such as cyclopentyloxy and cyclohexyloxy. "Cycloalkenyl" denotes groups such as cyclopentenyl and cyclohexenyl.

Examples of "cycloalkylalkyl" include cyclopropylmethyl, cyclohexylethyl, and other cycloalkyl moieties bonded to straight-chain or branched alkyl groups. "Alkylcycloalkyl" denotes alkyl substitution on a cycloalkyl moiety. Examples include 4-methylcyclohexyl and 3-isopropylcyclopentyl.

The term "halogen", either alone or in compound words such as "haloalkyl", denotes fluorine, chlorine, bromine or iodine. Further, when used in compound words such as "haloalkyl", said alkyl may be partially or fully substituted with halogen atoms which may be the same or different. Examples of "haloalkyl" include F₃C, ClCH₂, CF₃CH₂ and CF₃CF₂. Examples of "haloalkenyl" include (Cl)₂C=CHCH₂ and CF₃CH₂CH=CHCH₂. Examples of "haloalkynyl" include HC≡CCHCl, CF₃C≡C, CCl₃C≡C and FCH₂C≡CCH₂. Examples of "haloalkoxy" include CF₃O, CCl₃CH₂O, CF₂HCH₂CH₂O and CF₃CH₂O. Examples of "haloalkylthio" include CCl₃S, CF₃S, CCl₃CH₂S and CH₂ClCH₂CH₂S. Examples of "haloalkylsulfonyl" include CF₃SO₂, CCl₃SO₂, CF₃CH₂SO₂ and CF₃CF₂SO₂. Examples of "haloalkoxyalkoxy" include CF₃OCH₂O, ClCH₂CH₂OCH₂CH₂O, Cl₃CH₂OCH₂O as well as branched alkyl derivatives.

The total number of carbon atoms in a substituent group is indicated by the "Cᵢ-Cⱼ" prefix where i and j are numbers from 1 to 8. For example, C₁-C₃ alkylsulfonyl designates methylsulfonyl through propylsulfonyl; C₂ alkoxyalkoxy designates CH₃OCH₂O; C₃ alkoxyalkoxy designates, for example, CH₃OCH₂CH₂O or CH₃CH₂OCH₂O; and C₄ alkoxyalkoxy designates the various isomers of an alkoxy group substituted with a second alkoxy group containing a total of 4 carbon atoms, examples including CH₃CH₂CH₂OCH₂O, and CH₃CH₂OCH₂CH₂O. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. Examples of "alkoxycarbonyl" include CH₃OC(=O), CH₃CH₂OC(=O), CH₃CH₂CH₂OC(=O), (CH₃)₂CHOC(=O) and the different butoxy-, pentoxy- or hexyloxycarbonyl isomers.

In the above recitations, when a compound of Formula I comprises one or more pyridyl or pyrimidinyl rings, all bonds to these heterocycles are made through the carbon atom(s) of the moieties.

Preferred for greatest fungicidal activity and/or ease of synthesis are the compounds of Group 1 that are the compounds of Formula I wherein:
- J: is H; OH; C(=O)R¹⁶; C(=O)OR¹⁷; C(=0)NHR¹⁹;
- R¹: is C₁-C₄ alkyl; C₁-C₄ haloalkyl; or vinyl;
- R²: is C₁-C₂₀ alkyl; C₂-C₂₀ alkoxyalkyl; C₂-C₂₀ haloalkyl; C₃-C₈ alkyl substituted with phenoxy or phenylthio each optionally substituted with R³⁰; C₅-C₇ cycloalkyl; C₂-C₂₀ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
- R³: is phenyl optionally substituted with R¹⁰;
- R¹⁶: is H; C₁-C₆ alkyl; or phenyl optionally substituted with R³⁴
- R¹⁷: is C₁-C₆ alkyl;
- R¹⁹: is C₁-C₆ alkyl; or phenyl optionally substituted with R³⁴;
provided that when R² is phenyl and R⁵ is not F, then
- R⁵: is attached to the para-position relative to the oxazolidinone ring.

More preferred are the compounds of Group 2 that are the compounds of Group 1 wherein:
- J: is H; C(=O)R¹⁶;
- R¹: is C₁-C₂ alkyl or vinyl;
- R²: is C₁-C₁₂ alkyl; C₅-C₇ cycloalkyl; phenyl optionally substituted with R⁵ and R^{7;} thienyl optionally substituted with R⁷; or pyridyl optionally substituted with R⁵ and R⁷;
- R³: is phenyl optionally substituted with F; Cl; or methyl;
- R⁴: is H;
- R⁵: is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring; or -OC(=O)R²⁸;
- R⁷ and R²⁴: are independently F; C₁-C₂ alkyl; methylthio; or C₁-C₂ alkoxy;
- R¹⁶: is C₁-C₆ alkyl;
- R¹⁹: is phenyl optionally substituted with R³⁴;
- R²⁷: is 1-2 halogen; cyano; C₁-C₄ alkyl;
trifluoromethyl; C₁-C₄ alkoxy; C₁-C₄ haloalkoxy;
C₁-C₄ alkylthio; C₅-C₆ cycloalkyloxy; or allyl;
- R³⁰: is 1-2 halogen; cyano; C₁-C₄ alkyl; trifluoromethyl; C₁-C₄ alkoxy; or trifluoromethoxy;
- R³⁴: is 1-2 halogen; nitro; C₁-C₂ alkyl; or C₁-C₂ alkoxy.

Even more preferred are the compounds of Group 3 that are the compounds of Group 2 wherein:
- J: is H;
- R¹: is methyl;
- R²: is C₁-C₁₂ alkyl; phenyl optionally substituted with R⁵ and R⁷; pyridyl optionally substituted with R⁵ and R⁷;
- R⁵: is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸;

Specifically preferred for greatest fungicidal activity and/or ease of synthesis are the compounds of Formula I that are:
5-(2,4-Difluorophenyl)-5-methyl-3-(phenylamino)-4-thioxo-2-oxazolidinone;
4-Imino-5-methyl-5-(4-phenoxyphenyl)-3-(phenylamino)-2-oxazolidinone;
4-Imino-5-methyl-5-(4-phenoxyphenyl)-3-(phenylamino)-2-oxazolidinone hydrobromide; and
5-(2,4-Difluorophenyl)-4-imino-5-methyl-3-(phenylamino)-2-oxazolidinone.

### Synthesis

When R¹ and R² of the compounds of Formula I are different, the compounds of Formula I possess a chiral center. This invention therefore includes racemic mixtures as well as pure enantiomers. Although one enantiomer can have superior fungicidal activity for a given compound of Formula I, the other enantiomer is neither devoid of activity nor interferes with the activity of the more potent enantiomer. One also will notice that compounds of Formula I can exist as a mixture of E- and Z-imine isomers when A = N-J. This invention therefore pertains also to mixtures of geometric isomers as well as the individual isomers.

For clarity in discussion of their preparation, the compounds of Formula I can be subdivided into compounds of Formulas Ia, Ia.HX, Ib, Ic, Id and Ie: 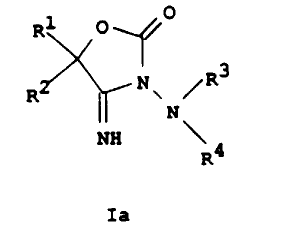 Compound I wherein A = N-J and J = H. 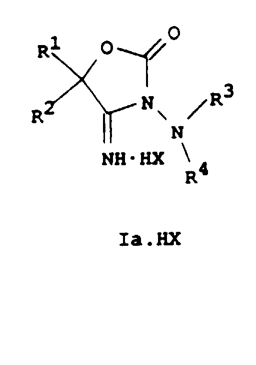 Salts of compounds Ia with acids HX, wherein HX is a strong acid (such as HBr, HCl, HI, HNO₃, H₂SO₄, H₃PO₄, as well as organic acids such as alkyl- and arylsulfonic acids). 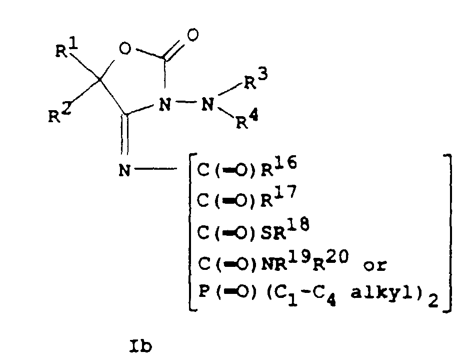 Compounds I wherein A is N-J, and J is a shown in brackets. 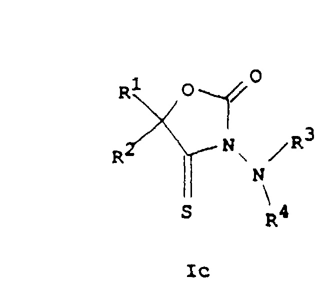 Compounds I wherein A is S. 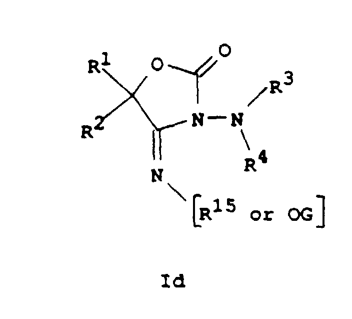 Compound I wherein A is N-J, J is R¹⁵ or OG, and G is H; C₁-C₄ alkyl; or phenylmethyl optionally substituted with R³⁴ on the phenyl ring. 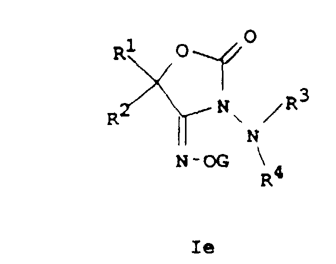 Compounds I wherein A is N-J, J is OG, and G is C(=O) (C₁-C₄ alkyl); C(=O) (C₁-C₄ alkoxy); or C(=O)NHR³⁶.

Preparation of the compounds Ia, Ia.HX, Ib, Ic, Id and Ie, all within structure I, is described schematically (Schematics 1 through 3) and in writing below.

Structure Ia: 4-Imino-2-oxazolidinones Ia can be prepared by sequential conversion of cyanohydrins II to their respective chloroformates III or
*O*-(imidazolylcarbonyl) derivatives IV, treatment of a compound III or IV with a substituted hydrazine which produces the carbazates V, then cyclization of a carbazate V to the product Ia. This sequence is schematically illustrated in Schematic 1.

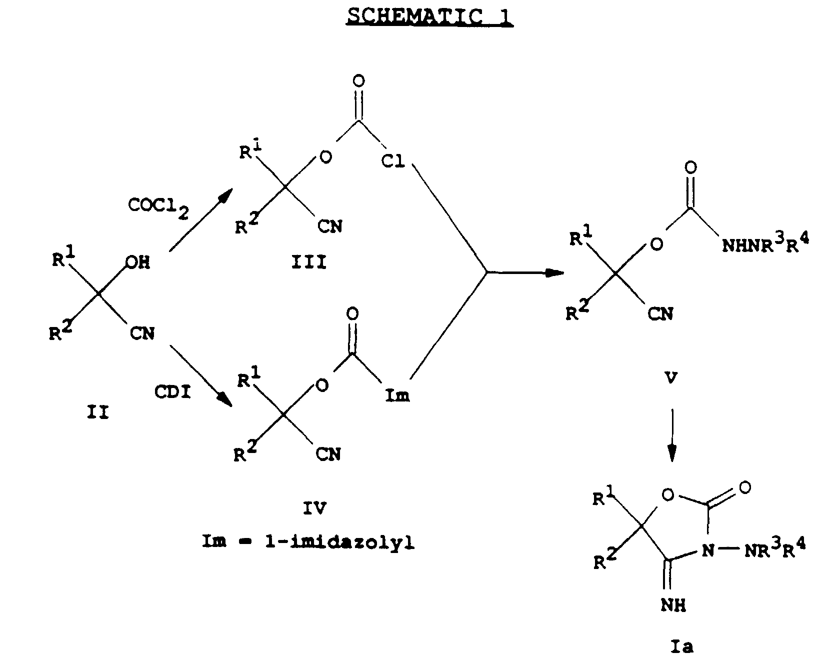

The *O*-(imidazolylcarbonyl) derivatives IV will be the intermediates of choice over the chloroformates III for those cyanohydrins II which fail to react, or react only poorly, with phosgene. The cyanohydrins which react poorly with phosgene tend to be those derived from electron-rich-aryl alkyl ketones. Methodology for preparation of ketone cyanohydrins is well known to those skilled in the art. See, for example, *Org. Syntheses,* Coll. Vol. 4, 58, (1968) which teaches the preparation of acetophenone cyanohydrin, and Gassman et al. in *Tetrahedron Lett.* **1978**, 3773, which teaches a general method for preparation of trimethylsilyl cyanohydrins.

Conversion of cyanohydrins to chloroformates is generally accomplished by mixing the cyanohydrin with phosgene and an acid acceptor in an inert solvent. Typical acid acceptors include the tertiary-amine bases (such as pyridine, *N*,*N*-dimethylaniline, *N*,*N*-diethylaniline). Typical inert solvents include the aromatic hydrocarbons (such as benzene, toluene, xylenes), ethers (such as THF, dioxane, diethyl ether), and chlorinated hydrocarbons (such as methylene chloride, chloroform). Temperatures are typically -20° to +40°. The chloroformates can be used directly in solution or be isolated by filtration of the reaction mixture and evaporation of the solvent from the filtrate; further purification can be accomplished by dissolution of the chloroformate in an inert, water-immiscible solvent (such as ether, benzene, toluene, xylenes, ethyl acetate, methylene chloride, chloroform, 1-chlorobutane), washing the solution with cold, dilute mineral acid, cold water, drying the solution, and evaporation of the solvent. Conversion of cyanohydrins to their chloroformates is known in the literature (e.g., V. Kondratenko et al., *Probl. Poluch. Poluprod. Prom. Org. Sin., Akad, Nauk SSSR, Otd. Obshch. Tekh. Khim.* **1967**, 60-63; *Chem. Abs.,* **1968**, *68,* 12441m).

Conversion of cyanohydrins to their *O*-imidazolyl carbonates can be accomplished by mixing the cyanohydrin with 1,1'-carbonyldiimidazole (CDI) in an inert solvent until the reaction is substantially complete [optionally the CDI can be generated in situ from phosgene and imidazole as described by Staab and Wendel, Org. Synthesis Coll. Vol. 5, 201, (1973)]. The progress of the reaction can be conveniently followed by thin-layer chromatography, or by noting evolution of CO₂ (or lack thereof) on treatment of a sample of the reaction mixture with water, which reacts quickly with any unreacted CDI. Depending on the particular substrates, temperature, and concentration of reactants in solution, the reaction is generally over within a few minutes to a few days; typical times are about 2 h to an overnight period. Suitable inert solvents include those mentioned above for preparation of the chloroformates. Reaction temperatures can vary from about -20° up to the boiling point of the solvent, with ambient temperatures preferred for convenience. The reaction mixtures can be used directly for the next step (reaction with a substituted hydrazine), or the intermediate *O*-(imidazolylcarbonyl) compounds can be purified and even isolated prior to use. For purification and isolation the solvent in the reaction mixture is evaporated (if water-miscible), and the residue is treated with cold water and water-immiscible solvent (e.g., diethyl ether, benzene, toluene, 1-chlorobutane, methylene chloride, chloroform, ethyl acetate); the layers are separated, and the organic layer is dried and evaporated to the *O*-(imidazolylcarbonyl) compound.

Conversion of the cyanohydrin chloroformates to the carbazates comprises mixing the chloroformate with a substituted hydrazine in an inert solvent, with either some of a substituted hydrazine or an added tertiary-amine base [e.g., pyridine, *N*,*N*-diethylaniline, *N*,*N*-dimethylaniline, triethylamine, *(N,N-*diisopropyl)ethylamine] acting as an acid acceptor. Suitable solvents include those suitable for preparation of the chloroformate. The reaction is generally rapid and complete within a few minutes in the usual temperature range (about -20° to ambient). If desired, the intermediate carbazate can be isolated by evaporating the solvent in vacuo (if water-miscible) and washing a solution of the residue in a water-immiscible solvent [such as mentioned for the preparation of an O-(imidazolylcarbonyl) compound] with dilute mineral acid and water, drying, and evaporation of the solvent. If the carbazate does not spontaneously cyclize to the 4-imino-2-oxazolidinone within an acceptable time, cyclization can be effected by treatment of a solution of the carbazate in an inert solvent (e.g., toluene, benzene, 1-chlorobutane, THF, chloroform) with imidazole or a tertiary-amine base [e.g., triethylamine, (*N*,*N*-diisopropyl)ethylamine, pyridine] and optionally heating the mixture up to the boiling point of the solvent; for example, cyclization is typically effected by boiling a toluene solution of the carbazate in the presence of triethylamine at reflux for 2 h.

Conversion of an *O*-(imidazolylcarbonyl) derivative to a carbazate (which generally cyclizes spontaneously to the 4-imino-2-oxazolidinone under the influence of the liberated imidazole) is effected by mixing an *O*-(imidazolylcarbonyl) derivative with a substituted hydrazine in an inert solvent (such as those mentioned for preparation of the chloroformate). Reaction is generally complete within a few minutes to an hour. Purification of the products can be effected by washing the reaction mixtures (if a water-immiscible solvent was used) with water, drying, and evaporation of the solvent. If cyclization of a carbazate to a 4-imino-2-oxazolidinone is incomplete, it can be effected as described above. If cyclization is substantially complete, then the product has been obtained. As noted in the Examples, the infrared spectrum of the carbazate shows absorption around 1738 cm⁻¹.; for the 4-imino-2-oxazolidinone the corresponding figure is around 1800 cm⁻¹.

Substituted hydrazines like those illustrated in Schematic 1 can be prepared by literature methods. For example, see J. Timberlake; J. Stowell; *The Chemistry of the Hydrazo, Azo, and Azoxy Groups* (S. Patai, Ed.) John Wiley and Sons, Ltd., London (1975), p 69; and Demers, J. P.; Klaubert, D. J.; *Tetrahedron Lett.* 1987, 4933

Structure Ia.HX: Ease of isolation of the imino compound Ia can sometimes be enhanced by conversion of it to a salt, for example by addition of a strong acid HX (as earlier described) to a solution of compound Ia in an inert organic solvent (such as those mentioned for preparation of the chloroformate above). Crystallization of the salt Ia·HX can then often be effected from the same or different organic solvent, or the salt can be recovered by evaporation of the solvent. Some of the imine salts offer advantage of formulation or enhanced fungicidal activity over the free-base forms.

Additionally, compounds Ia·HX can be converted to the corresponding 2,4-oxazolidinedione ia by reaction with water; typically the salt (or Ia plus an acid) is mixed with water, optionally in the presence of a cosolvent (e.g., acetone, butanone, THF) and optionally heated until conversion to the 4-oxo compound ia is substantially complete. The 4-oxo compound can be isolated in the usual ways, such as by filtration or evaporation of the reaction mixture, or by extraction of the product into an organic solvent and evaporation of that solvent. Schematically:

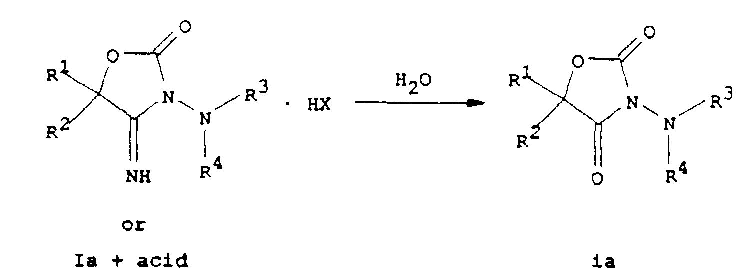

Structure Ib: Schematic 2 depicts preparation of compounds of Structure Ib. 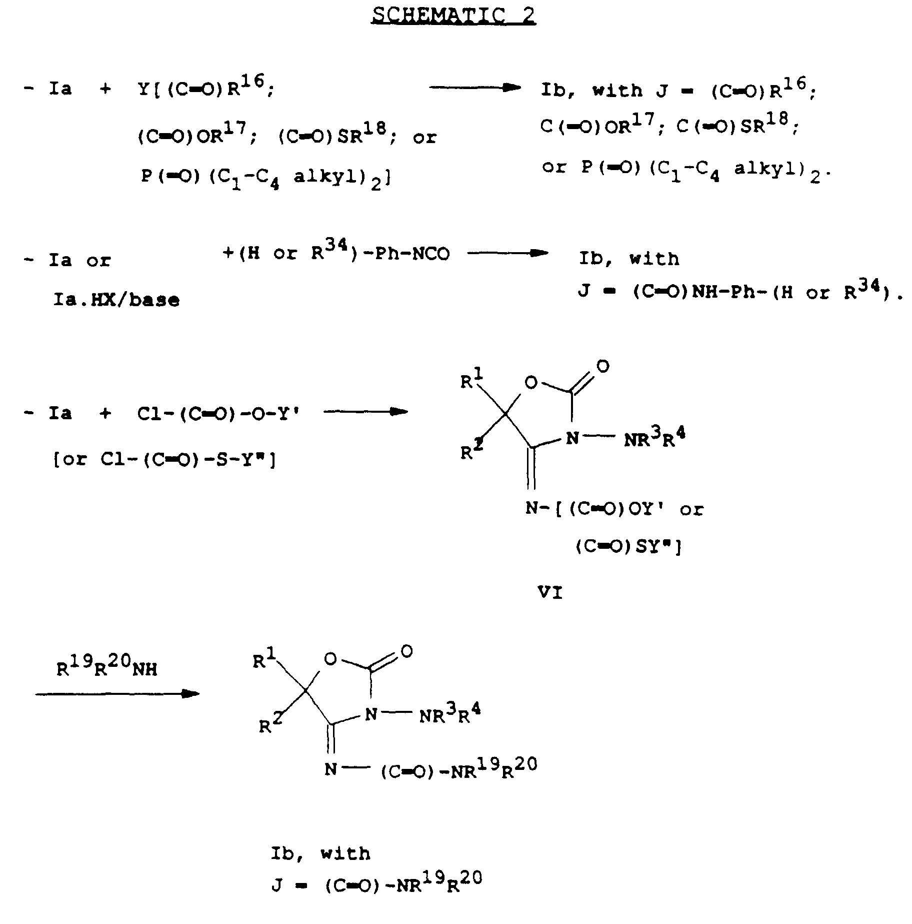 The *N*-acyl compounds Ib wherein J is (C=O)R¹⁶, (C=O)OR¹⁷, (C=O)SR¹⁸, or P(=O) (C₁-C₄ alkyl)₂ can be prepared by reaction of compound Ia (or Ia·HX) with an appropriate acylating agent Y-[(C=O)R¹⁶; (C=O)OR¹⁷; (C=O)SR¹⁸; or P(=O)(C₁-C₄ alkyl)₂], wherein Y is a leaving group such as chloride, bromide, acetate, alkyloxycarbonyloxy (such as isobutoxycarbonyloxy), or other moiety commonly used as a leaving group in such acylations. When acylating agents with the most common leaving group (i.e., chloride) are used, the agents are acid chlorides, chloroformates, chlorothiolformates, and dialkylphosphinic chlorides. Operationally, the compound Ia (or Ia·HX) is mixed with an inert organic solvent (e.g., THF, ether, ethyl acetate, methylene chloride) and treated with a tertiary-amine base [such as *N,N*-diethylaniline, *N,N*-dimethylaniline, pyridine, triethylamine, (*N,N*-diisopropyl)ethylamine; with weak-acid-forming leaving groups such as acetate and alkoxycarbonyloxy, the tertiary-amine base can be omitted when the free base Ia is used] and the acylating agent. The reaction is allowed to proceed at about -20° to ambient temperature (for convenience) until it is substantially complete, which may require a few minutes to several days. The solvent (if water-miscible) is evaporated from the reaction mixture and treated with a water-immiscible solvent and water. The organic solution is washed with dilute mineral acid, dried, and evaporated to the product, which can be further purified, if desired, by recrystallization or chromatography.

For preparation of compounds Ib wherein J is C(=O)NR¹⁹R²⁰ -- with R¹⁹ as phenyl optionally substituted with R³⁴, and R²⁰ as H -- the compound Ia (as such, or free-based by a tertiary-amine base in situ from Ia·HX, as mentioned above) is allowed to react with an optionally-substituted-phenyl isocyanate until the reaction is substantially complete. Isolation and purification of product can be accomplished as described above for the other N-acyl compounds.

For preparation of compounds Ib wherein J is C(=O)NR¹⁹R²⁰, the compounds Ia are allowed to react sequentially with a chloroformate Cl-C(=O)O-Y' or chlorothiolformate Cl-C(=O)S-Y" in substantially the same way as with the similar acylating agents above, followed by reaction of the intermediate VI with the appropriate amine HNR¹⁹R²⁰. In these reactions,
- Y'O: is a labile leaving group less labile than halide; e.g., 4-nitrophenoxy, in which case the chloroformate is 4-nitrophenyl chloroformate, and
- Y": is, e.g. C₁-C₄ alkyl, phenyl, nitrophenyl or chlorophenyl in which case a chlorothiolformate could be methyl chlorothiolformate.

Structure Ic: The 4-thioxooxazolidin-2-ones Ic can be prepared by two Methods (Method 1 and Method 2) shown in Schematic 3 and discussed below. 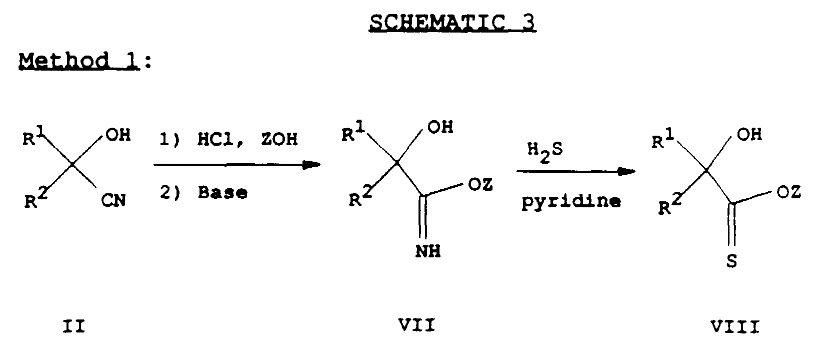 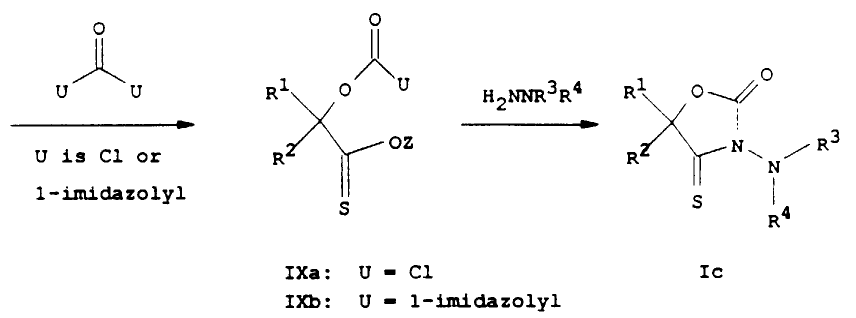 Z in ZOH and VII-IX is C₁-C₄ alkyl, C₃-C₄ alkenyl, C₅-C₆ cycloalkyl, or PhCH₂.

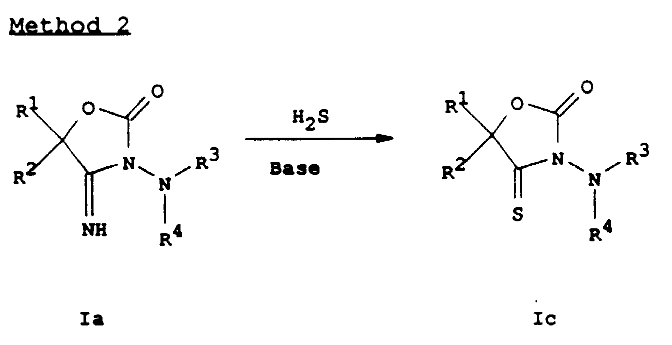

Preparations of 2-hydroxy thionocarboxylic acid esters like VIII are known in the literature. It is known that cyanohydrins II or TMS cyanohydrins (previously referenced) can be converted to the 2-hydroxy iminoethers VII by treatment with HCl gas in an alcohol (or alcohol-containing) solvent; preferred for ease of synthesis, lower expense, or greater utility are the ethers in which Z is C₁-C₄ alkyl. This is followed by alkalinization. This two-step procedure is well known; see for example Schnur, R. C. et al., *J. Med. Chem.* 1982, *25*, 1451.

The imino ethers VII are converted to 2-hydroxy thionocarboxylic esters VIII with dry hydrogen sulfide in pyridine as taught, for example, by Janssen, M.J., "The Chemistry of Carboxylic Acids and Esters," Patai, S., Ed., Chap. 15, Interscience, New York, 1969; and Geffken, D., et al., *Arch. Pharm. (Weinheim),* 1988, 321, 45.

The 2-hydroxy thionocarboxylic esters VIII are dissolved in an inert solvent such as methylene chloride or 1-chlorobutane, and treated with a tertiary-amine base such as triethylamine, pyridine, and (*N*,*N*-diisopropyl)ethylamine, at a temperature of about -60° to +30°; to this mixture is added phosgene to provide products of Formula IXa. The phosgene can be added as a gas or liquid or dissolved in an inert solvent such as toluene and added in solution. When the reaction is complete, the resulting mixture is poured into a water-immiscible solvent and washed with mineral acid, water, and brine. The organic-liquid phase is dried and evaporated to yield products of Formula IXa.

The compounds IXb can be prepared with 1,1'-carbonyldiimidazole (CDI) and the hydroxy compounds VIII. The hydroxy compound VIII is dissolved in an inert solvent in which the CDI has sufficient solubility at the reaction temperature. Methylene chloride, 1-chlorobutane and toluene are three of many suitable inert solvents. The CDI is added as a solid or as a solution in an inert solvent at temperatures from 0° to 100°. When the synthesis reaction is complete, the resulting mixture is poured into a water-immiscible solvent and washed successively with dilute mineral acid, water, and brine. The organic-liquid phase of the mixture is separated, dried, and evaporated to isolate the product.

In some cases, isolation of the compounds IXa and IXb is unnecessary. For example, after their formation is complete, the compounds can be treated directly with the substituted hydrazine H₂N-NR³R⁴. Further, the use of pure, isolated CDI is unnecessary in the synthesis of compounds IXb. The CDI can be first prepared, for example, by treatment of a solution of imidazole in an inert solvent with phosgene as previously noted, and then treated directly with the hydroxy compounds VIII.

Compounds of Formula IXa or IXb can be dissolved in an inert solvent such as methylene chloride, 1-chlorobutane, or THF and treated with a substituted hydrazine H₂N-NR³R⁴ at a temperature from 0° to 80°. When compound IXa (U = C1) is used, about one equivalent of a tertiary-amine base such as triethylamine, *N*,*N*-diethylaniline, (*N*,*N*-diisopropyl)ethylamine, or a second equivalent of the substituted hydrazine can be added. When compound IXb (U = 1-imidazolyl) is used, about one equivalent of a carboxylic acid can be added to accelerate the reaction; suitable carboxylic acids include acetic, pivalic, and benzoic acids. Upon completion of the reaction of IXa or IXb with the substituted hydrazine, the product of Formula Ic can be isolated by evaporation of the aforementioned inert solvent, and purified by dissolving the residue in a water-immiscible solvent such as ether or methylene chloride, washing with mineral acid, aqueous base, and water, drying, and evaporating the extraction solvent. Crystallization or chromatography can be utilized for additional purification if desired.

Preferred for reasons of higher yields and lower expense is the use of CDI [compound U-(C=O)-U with U = 1-imidazolyl] for preparation of compounds Ic.

As illustrated in Method 2 above, the 4-thiooxazolidin-2-ones Ic can be prepared by reaction of 4-iminooxazolidin-2-ones Ia (or Ia·HX) with hydrogen sulfide under basic conditions. For example, a solution of the imino compound Ia in pyridine can be treated with H₂S (added as a gas or liquid) and kept at about -20° to +40° until the conversion is substantially complete. Or, a solution of the imino compound in an alcohol (e.g., methanol, ethanol) can be treated with a tertiary-amine base (e.g., triethylamine, triethanolamine) and H2S and kept at about the same temperature until reaction is substantially complete. The product Ic can be isolated, and further purified as noted above.

Structure Id: These compounds can be prepared by alkylation of the 4-sulfur atom of compounds Ic with iodomethane, dimethyl sulfate, trimethyloxonium tetrafluoroborate, or other alkylating agent and treatment of the resulting thionium salt with either the amine H₂NR¹⁵ or hydroxylamine derivative H₂N-OG (with G defined as noted alongside of Formula Id). Reactions of this type have been described by Hunig, S., et al. (*Liebigs Ann. Chem.* **1962**, *651*, 89).

The amines and hydroxylamines employed in Method 2 above can be purchased from commercial sources or prepared by literature methods. The general preparation of hydroxylamines can also be accomplished by methods described in Castellino, A. J., et al. *J. Org. Chem.* **1984**, 49, 1348; Murray, R. W., et al. *Synthetic Comm.* **1989**, *19,* 3509 and references cited therein; and Baldol, C., et al. *Synthesis* **1988**, 344.

Structure Ie: These compounds can be prepared from those compounds of Id wherein OG is OH, through reaction with acylating agents Y-[C(=O)(C₁-C₄ alkyl) or C(=O)(C₁-C₄ alkoxy)] or isocyanates R³⁶⁻NCO. The nature of Group Y, reactions with the acylating agents and isocyanates, and isolation of the products are substantially as discussed earlier for preparation of the compounds Ib.

In addition to those disclosed above, other derivatives may be expected from reaction of imines of Formula Ia and 4-thioxo-2-oxazolidinones of Formula Ic with various nucleophiles and electrophiles.

Specific compounds of Formula I that can be made by the process of this invention are described in the Examples and Tables which follow. These are intended to be only exemplary, and are not inclusive.

The following are a list of abbreviations and their definitions used in the Tables and Examples which follow. Unless indicated otherwise, alkyl, alkenyl, and alkynyl radicals are the normal isomers. For example, "pentyl" implies the *n*-pentyl isomer. The numbering system for heterocyclic groups is defined in the following way. First, hetero atoms contained in the ring are given the lowest possible numbers using the accepted rules of heterocycle numbering. Then, the point of attachment of the radical is the position assigned the highest priority. For example, the attached group at C-3 in 6-MeOC(=O)-3-pyridyl is higher priority that the MeOC(=O) substituent. Double bonds contained in compounds listed in the Tables are of the (E)-configuration unless indicated otherwise.
Ac = acetyl
Me = methyl
Et = ethyl
Pr = n-propyl
Bu = n-butyl
Bzl = benzyl
BzlO = benzyloxy
*c* = cyclo
OAc = acetate
CN = cyano
NO₂ = nitro
SO₂ = sulfonyl
S(O) = sulfinyl
PhO = phenoxy
BuO = n-butoxy
PrO = *n*-propoxy

### EXAMPLE 1

### Preparation of O-Methyl 2-(2,4-difluorophenyl)thiolactate

A 10 g (64 mmol) portion of commercially-available 2,4-difluoroacetophenone was dissolved in dry methylene chloride (100 mL) and treated at room temperature with 10.2 mL (7.6 g, 77 mmol) trimethylsilyl cyanide and then 0.61 g (2 mmol) of zinc iodide. The mixture was stirred at room temperature overnight (14 h). The resulting yellow solution was then washed with saturated aq. NaHCO₃ (2 x 100 mL) and brine (100 mL), and each aqueous wash was back-extracted with fresh methylene chloride. The organic phases were combined, dried (MgSO₄), and evaporated to give a yellow oil. The resulting, crude trimethylsilyl cyanohydrin (TMSC) was used immediately in the following step.

All but three grams of the crude TMSC were dissolved in a mixture of ether (360 mL) and methanol (90 mL) and cooled to -78°. Hydrogen chloride gas was introduced while maintaining a temperature of -30°. When the solution became saturated as evidenced by a drop in temperature, the gas flow was halted. The solution was warmed to 0° and allowed to stand at 0° overnight (14 h). The reaction mixture was evaporated to a white solid, and the solid was washed with ether and dried. The free base of the iminoether hydrochloride was obtained by dissolving the solid in saturated aq. NaHCO₃ (150 mL) and extracting with ether (2 x 150 mL). The ether layers were washed with brine (100 mL), combined, dried (MgSO₄), and evaporated. The resulting iminoether (8.29 g) was obtained as a viscous yellow oil, and was used without further purification in the following step.

The iminoether from above was dissolved in pyridine (100 mL) and cooled to 0°. Hydrogen sulfide gas was introduced for 1 h, and then the reaction mixture was gradually warmed to room temperature and outgassed for 1 h. The reaction mixture was poured into ice-cold 3N aqueous HCl (100 mL) and extracted with ether (2X). The ether phases were washed with 1N aq. HCl and brine, dried (MgSO₄), and evaporated to provide the crude product. Traces of pyridine that remained in the crude product were removed by redissolving the crude product in ether and washing again with 1N aq. HCl (2X), and brine. The ether extract was dried (MgSO₄) and evaporated. The crude product was further purified by bulb-to-bulb distillation to give 6.84 g of the title compound of this example as a yellow oil. ¹H NMR (CDCl₃, 200 MHz): δ 7.53 (m, 1H), 6.80 (m, 2H), 4.59 (s, 1H, OH), 4.13 (s, 3H), 1.84 (s, 3H).

### EXAMPLE 2

### Preparation of 5-(2,4-difluorophenyl)-5-methyl-3-(phenylamino)-4-thioxo-2-oxazalidinone

Ethyl 2-(2,4-difluorophenyl)thiolactate (3 g, 12.9 mmol) was dissolved in 1-chlorobutane (25 mL), treated with 1,1'-carbonyldiimidazole (3.15 g, 19.4 mmol), and heated at reflux for 6 h. After standing at room temperature overnight (14 h), the resulting mixture was poured into ether and washed with water and brine. The aqueous washes were reextracted with ether and then both ether layers were combined, dried (MgSO₄), and evaporated. The resulting crude product (4.5 g), was purified by chromatography over silica gel eluting with 3:1 hexanes:EtOAc to give 1.3 g of acyl imidazole. The acyl imidazole material was dissolved in 1-chlorobutane (75 mL) and treated sequentially with phenylhydrazine (0.435 mL, 4.4 mmol) and HOAc (0.275 mL, 4.8 mmol). The mixture was stirred overnight (14 h) at room temperature and then poured into ether and washed with 1N aq. HCl, water, saturated aq. NaHCO₃, and brine. The aqueous layers were extracted with fresh ether and the ether layers were combined, dried (MgSO₄), and evaporated to give 0.81 g of an orange oil. Chromatography over silica gel eluting with 5:1 hexanes:EtOAc afforded 0.57 mg of a yellow-brown oil. Further purification of the yellow-brown oil by recrystallization from 1-chlorobutane/hexanes provided the title compound of this example as a light green solid. ¹H NMR (CDCl₃, 200 MHz): δ 7.53 (m, 1H), 7.32 (m, 2H), 7.07 (m, 1H), 6.96 (m, 4H), 6.60 (s, 1H, NH), 2.14 (s, 3H); IR (mineral oil): 3352, 1783 cm⁻¹; mp 123-124°.

### EXAMPLE 3

### Preparation of 5-methyl-5-octyl-3-(phenylamino)-4-thioxo-2-oxazolidinone

A solution of 4-imino-5-methyl-5-octyl-3-(phenylamino)-2-oxazolidinone hydrobromide (0.92 g, 0.0023 mol) in pyridine (25 mL) was treated with liquid H₂S (3 mL, 0.085 mol) with cooling in an ice bath, then stirred overnight at room temperature. The mixture was stripped to a yellow oil/solid residue, which was treated with 1-chlorobutane and water. The organic solution was washed with 1N HCl, water, and saturated brine, dried (MgSO₄), and stripped to a yellow oil comprising the title product of this example. IR (neat): 1806 cm⁻¹; TLC (CHCl₃): R_{f} ∼0.7 (major), trace component of slightly higher R_{f}; Anal. Calcd for C₁₈H₂₆N₂O₂S: C, 64.63; H, 7.84; N, 8.38; S, 9.59; Found: C, 62.93, 63.02; H, 7.67, 7.68; N, 8.01, 7.99; S, 12.35, 12.34.

### EXAMPLE 4

### Preparation of 4-imino-5-methyl-5-octyl-3-(phenylamino)-2-oxazolidinone hydrobromide

A solution of 2-decanone (16.62 g, 0.1064 mol) in methylene chloride was treated with trimethylsilyl cyanide (15.2 mL, 0.112 mol, 98% reagent) and a catalytic amount of zinc iodide. After 3.5 h, the solution was heated briefly to boiling. The cooled solution was washed with ice-cold water and saturated brine, dried (MgSO₄), and stripped to an oil (25.81 g), which was 2-decanone trimethylsilylcyanohydrin. The IR spectrum showed no evidence of ketone. The above oil dissolved in THF (150 mL) was treated with conc. HCl (16.7 mL, 0.2 mol), and the THF stripped off. The residue was extracted with ether (2X). The combined extracts were washed with water (2X) and saturated brine, dried (MgSO₄), and stripped to an oil (22.69 g) which contained 2-decanone cyanohydrin.

The above oil dissolved in ether (200 mL) and cooled to -10 +/-2°, was treated with liquid phosgene (10.4 mL, 0.15 mol), followed by addition during a 4-min period of a solution of pyridine (5 drops) in *N*,*N*-diethylaniline (17 mL, 0.107 mol). The mixture was stirred in the cold for 5 min, then for 2.5 h as the temperature was allowed to rise to 12°, and finally for 1 h without cooling. Filtration removed a white water-soluble solid, and the filtrate was stripped to an oil. A solution of the oil in ether (200 mL) was washed with cold 1N HCl (2X) and ice-water, dried (MgSO₄), and stripped to an orange oil. The oil was then dissolved in benzene and reevaporated, leaving an oil (26.55 g), which contained the chloroformate of 2-decanone cyanohydrin. IR (neat): 1786 cm⁻¹.

Into a stirred, ice-cooled solution of the above oil (9.96 g, 0.0405 mol assuming pure chloroformate) was pipetted phenylhydrazine (4.1 mL, 0.0404 mol), followed by pyridine (3.3 mL, 0.0410 mol). After 1.5 h without cooling the mixture was filtered. The filtrate was stripped to a yellow oil, and the oil was dissolved in ether. The ether solution was washed with 1N HCl (2X), water and saturated brine, dried (MgSO₄) and stripped to a light-orange oil (9.49 g), which contained the carbamate (*n*-octyl)(Me)C(CN)OC(=O)NHNHPh. IR (neat): 1738 cm⁻¹.

A solution of the above carbamate in toluene (100 mL), treated with triethylamine (3.5 mL), was heated at reflux for 2 h. The yellow supernatant solution was decanted from a little brown material, and stripped to a yellow oil, which contained the title product of this example in the free-base form. IR (neat): 1801 cm⁻¹.

The free base, dissolved in ether, was treated with a solution of 30% HBr in HOAc. The resulting solution was stripped of solvent, then dissolved in toluene and restripped (2X). The residual oil hardened to a soft solid under a nitrogen stream. The solid was stirred with ether, and the title product of this example was filtered off as a white solid, 2 g; m.p. 183-6°, dec.; IR (mineral oil): 1840 cm⁻¹; Anal. Calcd for C₁₈H₂₈BrN₃O₂: C, 54.27; H, 7.08; N, 10.55; Br, 20.06; Found: C, 53.82, 53.76; H, 7.05, 7.09; N, 10.52, 10.52; Br, 20.07, 20.16.

### EXAMPLE 5

### Preparation of 4-imino-5-methyl-5-(4-phenoxyphenyl)-3-(phenylamino)-2-oxazolidinone hydrobromide

To a solution of 4-phenoxyacetophenone trimethylsilylcyanohydrin (5.10 g, 0.0164 mol, prepared as described in Example 4 for 2-decanone trimethylsilylcyanohydrin) in THF (25 mL) was added conc. HCl (2.5 mL, 0.03 mol). The solution was stripped of THF, and the residue was extracted with ether. The ether solution was washed with water (2X) and saturated brine, dried (MgSO₄), and evaporated to an orange oil (4.20 g). The oil contained 4-phenoxyacetophenone cyanohydrin. TLC (silica gel, CHCl₃): R_{f}∼0.4.

To a solution of the above cyanohydrin in THF (25 mL) was added 1,1'-carbonyldiimidazole (CDI, 3.20 g of 90% reagent; 0.0178 mol). Not all of the CDI dissolved. After 68 h, the clear, orange solution was stripped to an oil. The oil was dissolved in 1-chlorobutane, and the solution was washed with water (3X), dried (MgSO₄), and stripped to an orange oil (6.14 g). The oil contained the *O*-(imidazolylcarbonyl) derivative of 4-phenoxyacetophenone cyanohydrin. IR (neat): 1777 (s), 1679 (w) cm⁻¹; TLC (CHCl₃, R_{f}): ∼0.27 (major), and 3 minor spots (2 lower- and 1 higher-R_{f}).

To a solution of the above oil in 1-chlorobutane (25 mL) was added phenylhydrazine (1.7 mL, ∼0.0164 mol). After 20 h the IR spectrum of a sample, after evaporation to an oil, showed a strong absorption at ∼1797 cm⁻¹ showing the formation of the oxazolidinone ring. The TLC (CHCl₃) of the reaction mixture, the same after 20 h as after 2.5 h, showed 2 major spots (R_{f}'s=0.20, 0.60) and a minor spot (R_{f}=0.006). The reaction solution was stripped to a red oil, and then dissolved in chloroform. The chloroform solution was filtered on silica gel which filled a Buchner funnel. Elution with chloroform provided a fraction that was substantially homogeneous by TLC (9:1, CHCl₃/EtOAC, v/v; R_{f} 0.4); evaporation of this fraction provided a red oil (3.87 g). The oil was the substantially pure 2-oxazolidinone of this example title, in the free-base form. IR (neat): ∼1796 (s) cm-1.

To an ether solution of the above oil, cooled in an ice bath, was added a solution of 30% HBr in HOAc. A tacky off-white solid precipitated. The supernatant solution was decanted, and the solid was rinsed with ether and recrystallized from acetonitrile-ether to give 2.51 g of the title product of this example as a white solid. IR (mineral oil): ∼1834 (s) cm⁻¹; Anal. Calcd for C₂₂H₂₀BrN₃O₃: C, 58.16; H, 4.44; N, 9.25; Br, 17.59; Found: C, 57.82; H, 4.35; N, 9.19; Br, 17.66; mp 161°, dec.

### EXAMPLE 6

### Preparation of 5-methyl-5-(4-phenoxyphenyl)-3-(phenylamino)-2,4-oxazolidinedione.

A sample of 4-imino-5-methyl-5-(4-phenoxyphenyl)-3-(phenylamino)-2-oxazolidinone hydrobromide, prepared as described in Example 5, was dissolved in aq. acetone. The solution was heated on a steam bath for 5 min. Evaporation of the mixture and extraction of the residue with 1-chlorobutane provided, on evaporation of the dried solution, a white solid identified as the title compound of this example. The IR spectrum and TLC behavior were identical to an authentic sample of 5-methyl-5-(4-phenoxyphenyl)-3-(phenylamino)-2,4-oxazolidinedione prepared as described in WO 90/12791.

### EXAMPLE 7

### Preparation of 4-imino-5-methyl-5-(4-phenoxyphenyl)-3-(phenylamino)-2-oxazolidinone from the hydrobromide

4-Imino-5-methyl-5-(4-phenoxyphenyl)-3-(phenylamino)-2-oxazolidinone hydrobromide, a saturated NaHCO₃ solution, and EtOAc were mixed well for 5 min, providing 2 clear layers. The EtOAc solution was dried (MgSO₄) and evaporated to a foamy grease which slowly solidified. Crystallization was effected from 1-chlorobutane/hexane, providing the title compound of this example as a white solid. mp 137-139°; Anal. Calcd for C₂₂H₁₉N₃O₃: C, 70.76; H, 5.13; N, 11.25; Found: C, 69.69; H, 5.24; N, 11.15.

### EXAMPLE 8

### Preparation of N-[5-methyl-2-oxo-5-(4-phenoxyphenyl)-3-(phenylamino)-4-oxazolidinylidene]acetamide

A solution of 4-imino-5-methyl-5-(4-phenoxyphenyl)-3-(phenylamino)-2-oxazolidinone (0.84 g, 0.0022 mol) in THF (25 mL) was treated with acetyl chloride (0.16 mL, 0.0022 mol), followed by *N*,*N*-diethylaniline (0.35 mL, 0.0022 mol) and pyridine (1 drop). After 3 h, the mixture was stripped to a clear grease. The grease was dissolved in 1-chlorobutane/water, and the organic layer washed with 1N HCl, water, and saturated brine, dried, and evaporated until the precipitation of white solid. The solid, the title compound of this example, was isolated by filtration. mp 160-162°; Anal. Calcd for C₂₄H₃₁N₃O₄: C, 69.38; H, 5.10; N, 10.11; Found: C, 69.32; H, 5.01; N, 9.96.

### EXAMPLE 9

### Preparation of N-[5-methyl-5-(4-phenoxyphenyl)-3-phenylamino-4-oxazolidinylidene]-N'-phenylurea

A solution of 4-imino-5-methyl-5-(4-phenoxyphenyl)-3-(phenylamino)-2-oxazolidinone (0.5 g, 0.0013 mol) in THF (25 mL) was treated with phenyl isocyanate (0.14 mL, 0.0013 mol). After stirring overnight, the mixture was stripped to an oil. The oil was crystallized from 1-chlorobutane/hexane, and then again from 1-chlorobutane to provide the title compound of this example as a white solid. mp 104-106°, dec.; Anal. Calcd for C₂₉H₂₄N₄O₄: C, 70.72; H, 4.91; N, 11.28; Found: C, 70.54; H, 5.04; N, 11.29.

### EXAMPLE 10

### Preparation of methyl N-[5-methyl-2-oxo-5-(4-phenoxyphanyl)-3-(phenylamino)-4-oxazolidinylidene]carbamate

A solution of 4-imino-5-methyl-5-(4-phenoxyphenyl)-3-(phenylamino)-2-oxazolidinone (0.64 g, 0.0017 mol) in THF (25 mL) was treated with methyl chloroformate (0.13 mL, 0.0017 mol), followed by *N*,*N*-diethylaniline (0.27 mL, 0.0017 mol) and 1 drop of pyridine. After stirring overnight, the mixture was stripped to a clear grease, which was treated with water and 1-chlorobutane. The layers were separated and the organic layer was washed with 1N HCl and saturated brine, dried (MgSO₄), and evaporated until crystallization occurred. The title product of this example was isolated as a white solid. mp 147-149°, dec.; Anal. Calcd for C₂₄H₂₁N₃O₅: C, 66.81; H, 4.91; N, 9.74; Found: C, 66.71; H, 5.00; N, 9.54.

From the teaching above, one can prepare the compounds of this invention such as those exemplified in Tables 1-23.

Compounds referred to in Tables below:

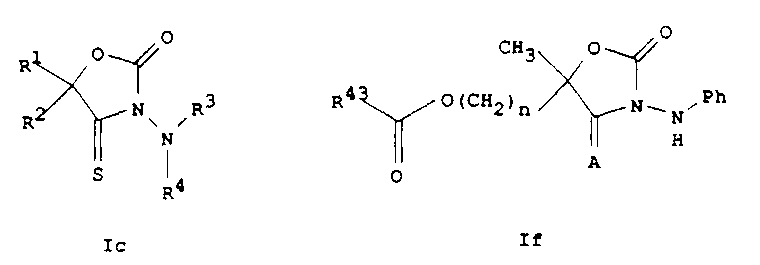

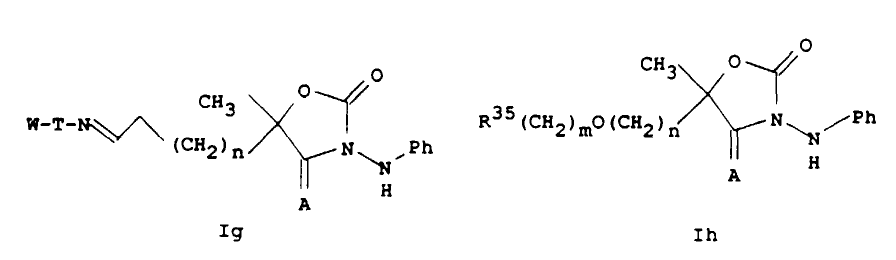

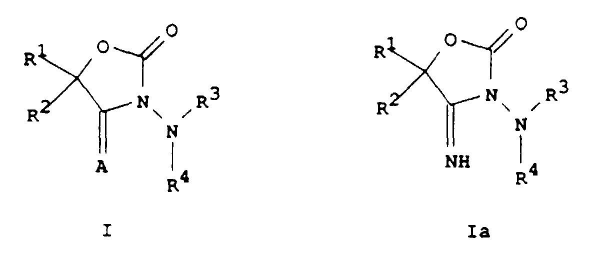

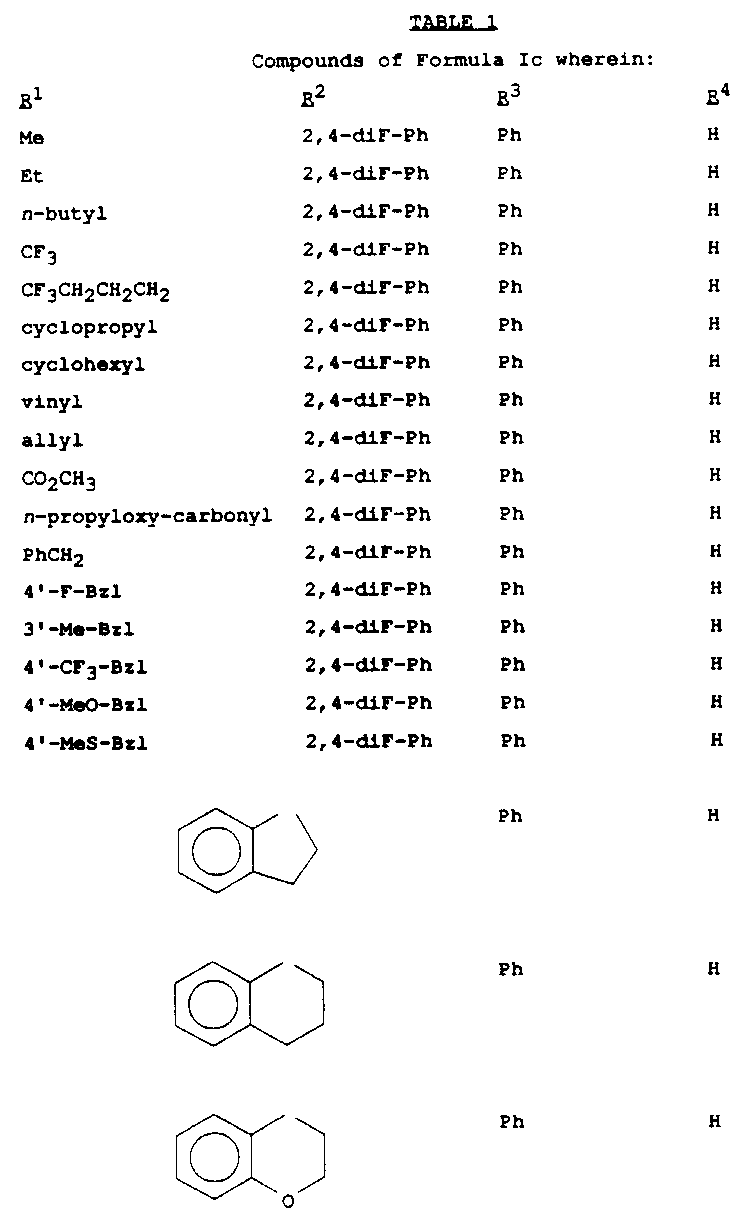

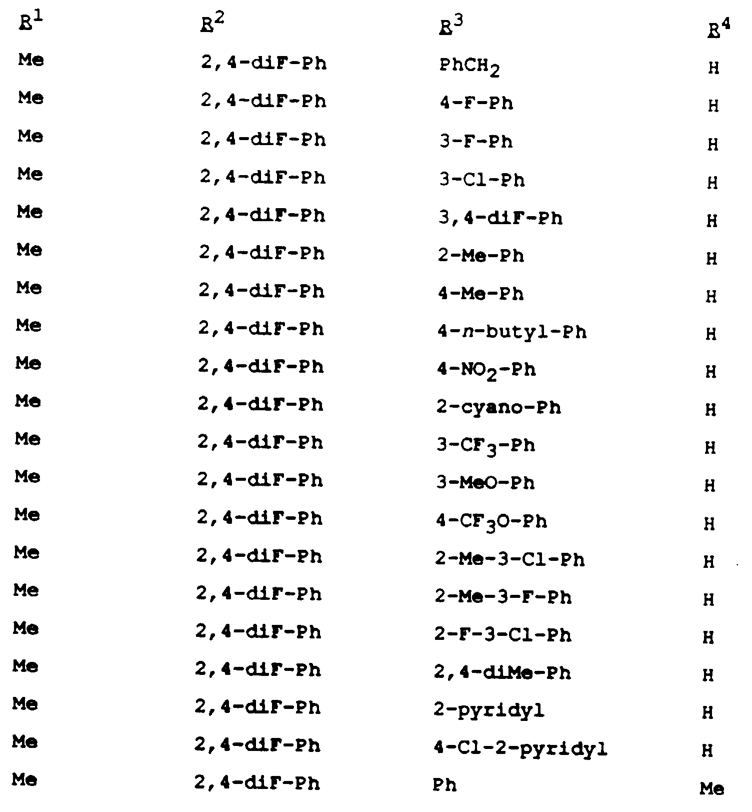

**TABLE 2**

| Compounds of Formula Ic wherein R¹=Me, R³=Ph, R⁴=H and: | |
|---|---|
| R² | R² |
| Et | 3-hydroxypropyl |
| heptyl | HO₂C(CH₂)₇ |
| octyl | (MeO)₂P(=O)OCH₂(CH₂)₃CH₂ |
| decyl | (BuO)₂P(=S)OCH₂(CH₂)₃CH₂ |
| undecyl | 8-(MeSO₃)octyl |
| eicosanyl | 5-(BuSO₃)pentyl |
| 3-Me-Bu | (PhSO₃)CH₂CH₂ |
| 3-Me-hexyl | 3-(4-F-PhSO₃)propyl |
| 2-Me-pentyl | (4-Et-PhSO₃)Bu(CH₂)₄, |
| 9,9,9-triF-nonyl | (Me₃N⁺)(CH₂)₄ I⁻ |
| 2,4-diCl-octyl | (H₃N⁺)(CH₂)₄ Cl⁻ |
| 4-(*c*-propyl)Bu | MeC(=O)NMe(CH₂)₄ |
| 8-(*c*-hexyl)octyl | 3-(PhSO₂)propyl |
| 6-Ph-octyl | (2,6-diMe-PhSO₂)(CH₂)₄ |
| 4-(3-CF₃-Ph)Bu | 6-(2-EtO-PhSO₂)hexyl |
| 8-(4-F₃CO-Ph)octyl | 8-(PhS(O))octyl |
| 10-(2,4-diF-Ph)decyl | 7-(4-F-PhS(O))heptyl |
| 4-(4-Cl-Ph)Bu | (3-Me-PhS(O))CH₂CH₂ |
| 5-(4-Et-Ph)pentyl | 5-(3-BuO-PhS(O))pentyl |
| 8-(3-propyloxy-Ph)octyl | (4-pyridyl)CH₂ |
| 6-(4-(4-F-PhO)Ph)hexyl | Me₂N(CH₂)₆ |
| 7-(3-(4-Et-PhO)Ph)heptyl | 8-(2,6-diMe-4-pyridyl)octyl |
| 10-(4-(3-MeS-PhO)Ph)decyl | 10-(6-Cl-3-pyridyl)decyl |
| 2-CN-propyl | 6-(4-pyridyloxy)hexyl |
| 3-NO₂-propyl | 3-(2,6-diCl-4-pyridyloxy)propyl |
| 2-MeS-hexyl | (4-MeO-2-pyridyloxy)(CH₂)₄ |
| 2-(MeS(O))Et | 3-(2-thienyl)propyl |
| 2-(EtSO₂)Et | 6-(3-Me-2-thienyl)hexyl |
| EtOC(=O)(CH₂)₄ | 8-(2-pyrimidinyl)octyl |
| 7-(4-MeO-2-pyrimidinyl)heptyl | 3-*c*-pentenyl |
| 10-(2-furanyl)decyl | (CH₂CH₂OCH₂CH₂)CH |
| (5-Et-2-furanyl)(CH₂)₄ | (CH₂CH₂SCH₂CH₂)CH |
| 3-(1-naphthalenyl)propyl | (CH₂CH₂SO₂CH₂CH₂)CH |
| 6-(6-F-2-naphthalenyl)hexyl | PhOCH₂CH₂CH₂ |
| 5-(2-pyrimidinyloxy)pentyl | PhO(CH₂)₄ |
| 8-(1-naphthalenyloxy)octyl | PhO(CH₂)₅ |
| 3-(2-tetrahydropyranyl)propyl | PhO(CH₂)₈ |
| 8-(2-tetrahydropyranyloxy) octyl | (3-CF₃-PhO)(CH₂)₃ |
| 2-octenyl | (3,5-diCl-PhO)(CH₂)₆ |
| 2-decenyl | (2-F-PhO)(CH₂)₃ |
| 4-octenyl | (2-Me-PhO)(CH₂)₃ |
| 3-EtS-2-propenyl | (3,5-diMeO-PhO)(CH₂)₈ |
| 4-*c*-hexyloxy-2-butenyl | (4-(4-F-PhO)PhO)(CH₂)₃ |
| 5-*c*-pentylozy-3-pentenyl | (3-(3,5-diMe-PhO)PhO)(CH₂)₆ |
| 6-hydroxy-4-hexenyl | PhS(CH₂)₃ |
| 3-(MeC(=O)O)-2-propenyl | (3-CF₃-PhS)(CH₂)₇ |
| 3-(4-pyridyl)-2-propenyl | (3-F₃CO-PhS)(CH₂)₆ |
| 5-EtO-2-pentenyl | (2,4-diF-PhS)(CH₂)₅ |
| 3-CF₃-2-propenyl | (4-Et-PhS)(CH₂)₅ |
| 3-Ph-2-propenyl | (2-EtO-PhS)(CH₂)₁₀ |
| 4-PhO-2-butenyl | (3-(4-Cl-PhO)PhS)(CH₂)₇ |
| (Z)-(4-PhO-2-butenyl) | (3-(2,4-diMe-PhO)PhS)(CH₂)₃ |
| 7-octynyl | (4-(4-EtO-PhO)PhS)CH₂)₇ |
| 2-hydroxy-4-butynyl | (3-(2-MeS-PhO)PhS)(CH₂)₃ |
| 2-EtO-6-heptynyl | FCH₂CH₂O(CH₂)₆ |
| 2,4-diF-7-octynyl | (*c*-hexyloxy)(CH₂)₆ |
| 3-*c*-hexenyl | (*c*-pentyloxy)(CH₂)₈ |
| (CH₂=CHCH₂O)(CH₂)₇ | 2,5-diF-4-Me-Ph |
| CH=CCH₂OCH₂CH₂ | 2-F-Ph |
| CH(=O)CH₂CH₂ | 4-Me-Ph |
| MeC(=O)CH₂CH₂CH₂ | 4-NO₂-Ph |
| BuO(=O)(OH₂)₇ | 4-hexyl-Ph |
| MeC(=NMe)CH₂ | 4-(cyclohexyl)Ph |
| MeC(=NOMe)CH₂CH₂CH₂ | 4-CF₃-Ph |
| MeC(=NNHPh)CH₂CH₂CH₂ | 4-MeS-Ph |
| hexyl | 4-(hexylthio)Ph |
| cyclopentyl | 3-MeO-Ph |
| cyclohexyl | 4-(pentyloxy)Ph |
| vinyl | 4-CF₃O-Ph |
| 3-hexenyl | 4-(cyclohexyloxy)Ph |
| 2-cyclohexenyl | 4-(BuOCH₂)Ph |
| 4-F-Ph | 4-MeOCH₂-Ph |
| 4-Cl-Ph | 4-(2-propenyl)Ph |
| 3-Cl-Ph | 4-(3-pentenyl)Ph |
| 3-F-Ph | 4-(Cl₂C=CHCH₂)Ph |
| 4-Br-Ph | 4-(2-propenyloxy)Ph |
| 3,4-diCl-Ph | 4-(2-hexenyloxy)Ph |
| 2,3-diF-Ph | 4-(propargyl)Ph |
| Ph | 4-(2-butynyloxy)Ph |
| 2,5-diF-Ph | 4-BuSO₂-Ph |
| 3,5-diF-Ph | 4-PhO-Ph |
| 2,6-diF-Ph | 4-(4-Cl-PhO)Ph |
| 3,4-diF-Ph | 4-(3-Cl-PhO)Ph |
| 5-Cl-2-F-Ph | 4-(2,4-diF-PhO)Ph |
| 2-F-5-Me-Ph | 4-(4-NO₂-PhO)Ph |
| 4-(4-CN-PhO)Ph | 4-(2-MeS-Ph)CH=CH-Ph |
| 4-(4-Me-PhO)Ph | 4-(4-MeO-Ph)CH=CH-Ph |
| 4-(3-hexyl-PhO)Ph | 4-(4-BuO-Ph)CH=CH-Ph |
| 4-(3-cyclohexyl-PhO)Ph | 4-PhCH₂CH₂-Ph |
| 4-(3-CF₃-PhO)Ph | 4-(3-Br-Ph)CH₂CH₂-Ph |
| 4-(4-MeS-PhO)Ph | 4-(2-F-Ph)CH₂CH₂-Ph |
| 4-(4-MeSO₂-PhO)Ph | 4-(3-Cl-Ph)CH₂CH₂-Ph |
| 4-(3-BuSO₂-PhO)Ph | 4-(2,4-diF-Ph)CH₂CH₂-Ph |
| 4-(3-MeO-PhO)Ph | 4-(4-NO₂-Ph)CH₂CH₂-Ph |
| 4-(3-hexyloxy-PhO)Ph | 4-(3-Me-Ph)CH₂CH₂-Ph |
| 4-(4-CF₃O-PhO)Ph | 4-(3-Bu-Ph)CH₂CH₂-Ph |
| 4-(4-cyclohexyloxy-PhO)Ph | 4-(4-CF₃-Ph)CH₂CH₂-Ph |
| 4-(4-MeOC(=O)-PhO)Ph | 4-(2-MeS-Ph)CH₂CH₂-Ph |
| 4-(4-allyl-PhO)Ph | 4-(4-MeO-Ph)CH₂CH₂-Ph |
| 4-(4-propargyl-PhO)Ph | 4-(4-BuO-Ph)CH₂CH₂-Ph |
| 4-((3-PhO)PhO)Ph | 4-PhOCH₂-Ph |
| 4-(3-(2-Cl-PhO)PhO)Ph | 4-(3-Br-Ph)OCH₂-Ph |
| 4-(3-(4-Me-PhO)PhO)Ph | 4-(2-F-Ph)OCH₂-Ph |
| 4-(3-(3-MeO-PhO)PhO)Ph | 4-(3-Cl-Ph)OCH₂-Ph |
| 4-PhCH=CH-Ph | 4-(2,4-diF-Ph)OCH₂-Ph |
| 4-(3-Br-Ph)CH=CH-Ph | 4-(4-NO₂-Ph)OCH₂-Ph |
| 4-(2-F-Ph)CH=CH-Ph | 4-(3-Me-Ph)OCH₂-Ph |
| 4-(3-Cl-Ph)CH=CH-Ph | 4-(3-Bu-Ph)OCH₂-Ph |
| 4-(2,4-diF-Ph)CH=CH-Ph | 4-(4-CF₃-Ph)OCH₂-Ph |
| 4-(4-NO₂-Ph)CH-CH-Ph | 4-(2-MeS-Ph)OCH₂-Ph |
| 4-(3-Me-Ph)CH-CH-Ph | 4-(4-MeO-Ph)OCH₂-ph |
| 4-(3-Bu-Ph)CH=CH-Ph | 4-(4-BuO-Ph)OCH₂-Ph |
| 4-(4-CF₃-Ph)CH=CH-Ph | 4-PhCH₂-Ph |
| 4-(3-Br-Ph)CH₂-Ph | 4-(4-NO₂-Ph)Ph |
| 4-(2-F-Ph)CH₂-Ph | 4-(3-Me-Ph)Ph |
| 4-(3-Cl-Ph)CH₂-Ph | 4-(3-Bu-Ph)Ph |
| 4-(2,4-diF-Ph)CH₂-Ph | 4-(4-CF₃-Ph)Ph |
| 4-(4-NO₂-Ph)CH₂-Ph | 4-(2-MeS-Ph)Ph |
| 4-(3-Ne-Ph)CH₂-Ph | 4-(4-MeO-Ph)Ph |
| 4-(3-Bu-Ph)CH₂-Ph | 4-(4-BuO-Ph)Ph |
| 4-(4-CF₃-Ph)CH₂-Ph | 4-PhCH₂O-Ph |
| 4-(2-MeS-Pb)CH₂-Pb | 4-(3-Br-Ph)CH₂O-Ph |
| 4-(4-MeO-Ph)CH₂-Ph | 4-(2-F-Ph)CH₂O-Ph |
| 4-(4-BuO-Ph)CH₂-Ph | 4-(3-Cl-Ph)CH₂O-Ph |
| 4-PhS-Ph | 4-(2,4-diF-Ph)CH₂O-Ph |
| 4-(3-Br-Ph)S-Ph | 4-(4-NO₂-Ph)CH₂O-Ph |
| 4-(2-F-Ph)S-Ph | 4-(3-Me-Ph)CH₂O-Ph |
| 4-(3-Cl-Ph)S-Ph | 4-(3-Bu-Pb)CH₂O-Ph |
| 4-(2,4-diF-Ph)S-Ph | 4-(4-CF₃-Ph)CH₂O-Ph |
| 4-(4-NO₂-Ph)S-Ph | 4-(2-MeS-Ph)CH₂O-Pb |
| 4-(3-Me-Ph)S-Ph | 4-(4-MeO-Ph)CH₂O-Ph |
| 4-(3-Bu-Ph)S-Ph | 4-(4-BuO-Ph)CH₂O-Ph |
| 4-(4-CF₃-Ph)S-Ph | 4-(4-CN-Ph)CH₂O-Ph |
| 4-(2-MeS-Ph)S-Ph | 4-(3-PhO-Ph)CH₂O-Ph |
| 4-(4-NeO-Ph)S-Ph | 4-[3-(3-F-PhO)Ph]CH₂O-Ph |
| 4-(4-BuO-Ph)S-Ph | 4-[4-(2-NO₂-PhO)Ph]CH₂O-Ph |
| 4-Ph-Ph | 4-[3-(4-Me-PhO)Ph]CH₂O-Ph |
| 4-(3-Br-Ph)Ph | 4-[4-(4-Bu-PhO)Ph]CH₂O-Ph |
| 4-(2-F-Ph)Ph | 4-[3-(3-CF₃-PhO)Ph]CH₂O-Ph |
| 4-(3-Cl-Ph)Ph | 4-(2-(3-MeS-PhO)Ph]CH₂O-Ph |
| 4-(2,4-diF-Ph)Ph | 4-[4-(4-MeO-PhO)Ph)CH₂O-Ph |
| 4-[3-(3-BuO-PhO)Ph]CH₂O-Ph | 3-[(2-CF₃-4-Et-Ph)NHC(=O)O]Ph |
| 4-(2-F-4-MeO-Ph)CH₂O-Ph | 4-[(3-pyridyl)NHC(=O)O]Ph |
| 3-(2-CF₃-4-Et-Ph)CH₂O-Ph | 4-[(3-Br-2-pyridyl)NHC(=O)O]Ph |
| 4-[MeNHC(=O)O]Ph | 4-[(2-F-3-pyridyl)NHC(=O)O]Ph |
| 4-[octyl-NHC(=O)O]Ph | 4-[(3-Cl-4-pyridyl)NHC(=O)O]Ph |
| 4-[PhNHC(=O)O]Ph | 4-[(2,4-diF-3-pyridyl)NHC(=O)O]Ph |
| 4-[(3-Br-Ph)NHC(=O)O]Ph | 4-[(4-NO₂-2-pyridyl)NHC(=O)O]Ph |
| 4-[(2-F-Ph)NHC(=O)O]Ph | 4-[(3-Me-2-pyridyl)NHC(=O)O]Ph |
| 4-[(3-Cl-Ph)NHC(=O)O]Ph | 4-[(3-Bu-2-pyridyl)NHC(=O)O]Ph |
| 4-[(2,4-diF-Ph)NHC(=O)O]Ph | 4-[(4-CF₃-3-pyridyl)NHC(=O)O]Ph |
| 4-[(4-NO₂-Ph)NHC(=O)O]Ph | 4-[(2-MeS-4-pyridyl)NHC(=O)O]Ph |
| 4-[(3-Me-Ph)NHC(=O)O]Ph | 4-[(4-MeO-3-pyridyl)NHC(=O)O]Ph |
| 4-[(3-Bu-Ph)NHC(=O)O]Ph | 4-[(4-BuO-3-pyridyl)NHC(=O)O]Ph |
| 4-[(4-CF₃-Ph)NHC(=O)O]Ph | 4-[(4-CN-2-pyridyl)NHC(=O)O]Ph |
| 4-[(2-MeS-Ph)NHC(=O)O]Ph | 4-[(3-PhO-2-pyridyl)NHC(=O)O]Ph |
| 4-[(4-MeO-Ph)NHC(=O)O]Ph | 4-[3-(3-F-PhO)-2-pyridyl-NHC(=O)O]Ph |
| 4-[(4-BuO-Ph)NHC(=O)O]Ph | 4-(4-(2-NO₂-PhO)-3-pyridyl-NHC(=O)O]Ph |
| 4-[(4-CN-Ph)NHC(=O)O]Ph | 4-[2-(4-Me-PhO)-4-pyridyl-NHC(=O)O]Ph |
| 4-[(3-PhO-Ph)NHC(=O)O]Ph | 4-[4-(4-Bu-PhO)-3-pyridyl-NHC(=O)O]Ph |
| 4-[3-(3-F-PhO)Ph-NHC(=O)O]Ph | 4-[4-(3-CF₃-PhO)-2-pyridyl-NHC(=O)O]Ph |
| 4-[4-(2-NO₂-PhO)Ph-NHO(=O)O]Ph | 4-(4-(3-MeS-PhO)-3-pyridyl-NHC(=O)O]Ph |
| 4-[2-(4-Me-PhO)Ph-NHC(=O)O]Ph | 4-[3-(4-MeO-PhO)-2-pyridyl-NHC(=O)O]Ph |
| 4-[4-(4-Bu-PhO)Ph-NHC(=O)O]Ph | 4-[3-(3-BuO-PhO)-2-pyridyl-NHC(=O)O]Ph |
| 4-[4-(3-CF₃-PhO)Ph-NHC(=O)O]Ph | 4-[(2-F-4-MeO-3-pyridyl)NHC(=O)O]Ph |
| 4-[4-(3-MeS-PhO)Ph-NHO(=O)O]Ph | 3-[(2-CF₃-4-Et-3-pyridyl)NHC(=O)O]Ph |
| 4-(3-(4-MeO-PhO)Ph-NHC(=O)O]Ph | 4-[MeO-C(=O)]Ph |
| 4-[3-(3-BuO-PhO)Ph-NHC(-O)O]Ph | 4-[octyloxy-C(=O)]Ph |
| 4-[(2-F-4-MeO-Ph)NHC(=O)O]Ph | 4-[PhO-C(=O)]Ph |

| R² | |
|---|---|
| 4-[3-Br-PhO-C(=O)]Ph | |
| 4-[2-F-PhO-C(=O)]Ph | |
| 4-[3-Cl-PhO-C(=O)]Ph | |
| 4-[2,4-diF-PhO-C(=O)]Ph | |
| 4-(4-NO₂-PhO-(=O)]Ph | |
| 4-[3-Me-PhO-C(=O)]Ph | |
| 4-[3-Bu-PhO-C(=O)]Ph | |
| 4-[4-CF₃-PhO-C(=O)]Ph | |
| 4-[2-MeS-PhO-C(=O)]Ph | |
| 4-[4-MeO-PhO-C(=O)]Ph | |
| 4-(4-BuO-PhO-C(=O)]Ph | |
| 4-[4-CN-PhO-C(=O)]Ph | |
| 4-[3-PhO-PhO-C(=O)]Ph | |
| 4-[3-(3-F-PhO)PhO-C(=O)]Ph | |
| 4-[4-(2-NO₂-PhO)PhO-C(=O)]Ph | |
| 4-[2-(4-Me-PhO)PhO-C(=O)]Ph | |
| 4-[4-(4-Bu-PhO)PhO-C(=O)]Ph | |
| 4-[4-(3-CF₃-PhO)PhO-C(=O))Ph | |
| 4-[4-(3-MeS-PhO)PhO-C(=O)]Ph | |
| 4-[3-(4-MeO-PhO)PhO-C(=O)]Ph | |
| 4-[3-(3-BuO-PhO)PhO-C(=O)]Ph | |
| 4-[(2-F-4-MeO-PhO-C(=O)]Ph | |
| 3-[(2-CF₃-4-Et-PhO-C(=O)]Ph | |
| 4-[(3-pyridyloxy)C(=O)]Ph | |
| 4-[(3-Br-2-pyridyloxy)C(=O)]Ph | |
| 4-[(2-F-3-pyridyloxy)C(=O)]Ph | |
| 4-[(3-Cl-4-pyridyloxy)C(=O)]Ph | |
| 4-[(2,4-diF-3-pyridyloxy)C(=O)]Ph | |

| R² | R² |
|---|---|
| 4-[(4-NO₂-2-pyridyloxy)C(=O)]Ph | 4-[3-Bu-PhC(=O)O]Ph |
| 4-[(3-Me-2-pyridyloxy)C(=O)]Ph | 4-[4-CF₃-PhC(=O)O]Ph |
| 4-[(3-Bu-2-pyridyloxy)C(=O)]Ph | 4-[2-MeS-PhC(=O)O]Ph |
| 4-[(4-CF₃-3-pyridyloxy)C(=O)]Ph | 4-[4-MeO-PhC(=O)O]Ph |
| 4-[(2-MeS-4-pyridyloxy)C(=O)]Ph | 4-[4-BuO-PhC(=O)O]Ph |
| 4-[(4-MeO-3-pyridyloxy)C(=O)]Ph | 4-[4-CN-PbC(=O)O]Ph |
| 4-[(4-BuO-3-pyridyloxy)C(=O)]Ph | 4-[3-PbO-PhC(=O)O]Ph |
| 4-[(4-CN-2-pyridyloxy)C(=O)]Ph | 4-[3-(3-F-PhO)PhC(=O)O]Ph |
| 4-[(3-PhO-2-pyridyloxy)C(=O)]Ph | 4-[4-(2-NO₂-PhO)PhC(=O)O]Ph |
| 4-[3-(3-F-PhO)-2-pyridyloxy-C(=O)]Ph | 4-[2-(4-Me-PhO)PhC(=O)O]Ph |
| 4-(4-(2-NO₂-PhO)-3-pyridyloxy-C(=O)]Ph | 4-[4-(4-Bu-PhO)PhC(=O)O]Ph |
| 4-[2-(4-Me-PhO)-4-pyridyloxy-C(=O)]Ph | 4-(4-(3-CF₃-PhO)PhC(=O)O]Ph |
| 4-[4-(4-Bu-PhO)-3-pyridyloxy-C(=O)]Ph | 4-(4-(3-MeS-PhO)PhC(=O)O]Ph |
| 4-[4-(3-CF₃-PhO)-2-pyridyloxy-C(=O)]Ph | 4-[3-(4-MeO-PhO)PbC(=O)O]Ph |
| 4-(4-(3-MeS-PhO)-3-pyridyloxy-C(=O)]Ph | 4-[3-(3-BuO-PhO)PhC(=O)O]Ph |
| 4-[3-(4-MeO-PhO)-2-pyridyloxy-C(=O)]Ph | 4-[(2-F-4-MbO-Ph)C(=O)O]Ph |
| 4-[3-(3-BuO-PhO)-2-pyridyloxy-C(=O)]Ph | 3-[(2-CF₃-4-Et-Ph)C(=O)O]Ph |
| 4-[(2-F-4-MeO-3-pyridyloxy)C(=O)]Ph | 4-[(3-pyridyl)C(=O)O]Ph |
| 3-[(2-CF₃-4-Et-3-pyridyloxy)C(=O)]Ph | 4-[(3-Br-2-pyridyl)C(=O)O]Ph |
| 4-[MeC(=O)O]Ph | 4-[(2-F-3-pyridyl)C(=O)O]Ph |
| 4-[octyl-C(=O)O]Ph | 4-[(3-Cl-4-pyridyl)C(=O)O]Ph |
| 4-[PhC(=O)O]Ph | 4-[(2,4-diF-3-pyridyl)C(=O)O]Ph |
| 4-[3-Br-PhC(=O)O]Ph | 4-[(4-NO₂-2-pyridyl)C(=O)O]Ph |
| 4-[2-F-PhC(=O)O]Ph | 4-[(3-Me-2-pyridyl)C(=O)O]Ph |
| 4-[3-Cl-PhC(=O)O]Ph | 4-[(3-Bu-2-pyridyl)C(=O)O]Ph |
| 4-[2,4-diF-PhC(=O)O]Ph | 4-[(4-CF₃-3-pyridyl)C(=O)O]Ph |
| 4-[4-NO₂-Ph(=O)O]Ph | 4-((2-MeS-4-pyridyl)C(=O)O]Ph |
| 4-[3-Me-PhC(=O)O]Ph | 4-[(4-MeO-3-pyridyl)C(=O)O]Ph |
| 4-[(4-BuO-3-pyridyl)C(=O)O]Ph | 2-thienyl |
| 4-[(4-CN-2-pyridyl)C(=O)O]Ph | 3-thienyl |
| 4-[(3-PhO-2-pyridyl)C(=O)O]Ph | 2,5-diF-3-thienyl |
| 4-[3-(3-F-PhO)-2-pyridyl-C(=O)O]Ph | 3,5-diF-2-thienyl |
| 4-[4-(2-NO₂-PhO)-3-pyridyl-C(=O)O]Ph | 3,4-diF-2-thienyl |
| 4-[2-(4-Me-PhO)-4-pyridyl-C(=O)O]Ph | 5-Cl-2-F-3-thienyl |
| 4-[4-(4-Bu-PhO)-3-pyridyl-C(=O)O]Ph | 2-F-5-Me-3-thienyl |
| 4-[4-(3-CF₃-PhO)-2-pyridyl-C(=O)O]Ph | 2,5-diF-4-Me-3-thienyl |
| 4-[4-(3-MeS-PhO)-3-pyridyl-C(=O)O]Ph | 2-F-3-thienyl |
| 4-[3-(4-MeO-PhO)-2-pyridyl-C(-O)O]Ph | 4-Me-2-thienyl |
| 4-[3-(3-BuO-PhO)-2-pyridyl-C(=O)O]Ph | 4-NO₂-2-thienyl |
| 4-[(2-F-4-MeO-3-pyridyl)C(=O)O]Ph | 4-hexyl-2-thienyl |
| 3-[(2-CF₃-4-Et-3-pyridyl)C(=O)O]Ph | 5-cyclohexyl-3-thienyl |
| 2-F-4-PhCH₂CH₂-Ph | 5-CF₃-3-thienyl |
| 3-Cl-4-(2-F-Ph)OCH₂-Ph | 4-MeS-2-thienyl |
| 3-Me-4-(4-MeO-Ph)CH₂-Ph | 4-hexylthio-2-thienyl |
| 2-F-4-PhS-Ph | 5-MeO-3-thienyl |
| 3-BuO-4-Ph-Ph | 4-pentyloxy-2-thienyl |
| 3-MeS-4-PhO-Ph | 4-CF₃O-2-thienyl |
| 2-F-4-PhCH₂O-Ph | 5-cyclohezyloxy-3-thienyl |
| 2-F-4-[octyl-NHC(=O)O]Ph | 4-BuOCH₂-2-thienyl |
| 4-F-3-thienyl | 4-MeOCH₂-2-thienyl |
| 4-Cl-2-thienyl | 5-(2-propenyl)-3-thienyl |
| 5-Cl-2-thienyl | 5-(3-pentenyl)-3-thienyl |
| 3-F-2-thienyl | 4-(Cl₂C=CHCH₂)-2-thienyl |
| 4-Br-2-thienyl | 4-(2-propenyloxy)-2-thienyl |
| 3,4-diCl-2-thienyl | 4-(2-hexenyloxy)-2-thienyl |
| 2,4-diF-3-thienyl | 5-(propargyl)-3-thienyl |
| 4-(2-butynyloxy)-2-thienyl | 4-(2-F-Ph)CH=CH-2-thienyl |
| 5-BuSO₂-3-thienyl | 4-(3-Cl-Ph)CH=CH-2-thienyl |
| 5-PhO-3-thienyl | 5-(2,4-diF-Ph)CH=CH-3-thienyl |
| 4-(4-Cl-PhO)-2-thienyl | 5-(4-NO₂-Ph)CH=CH-3-thienyl |
| 4-(3-Cl-PhO)-2-thienyl | 5-(3-Me-Ph)CH=CH-3-thienyl |
| 5-(2,4-diF-PhO)-3-thienyl | 5-(3-Bu-Ph)CH=CH-3-thienyl |
| 5-(4-NO₂-PhO)-3-thienyl | 4-(4-CF₃-Ph)CH=CH-2-thienyl |
| 5-(4-CN-PhO)-3-thienyl | 4-(2-MeS-Ph)CH=CH-2-thienyl |
| 5-(4-Me-PhO)-3-thienyl | 4-(4-MeO-Ph)CH=CH-2-thienyl |
| 4-(3-hexyl-PhO)-2-thienyl | 4-(4-BuO-Ph)CH=CH-2-thienyl |
| 4-(3-cyclohexyl-PhO)-2-thienyl | 5-PhCH₂CH₂-3-thienyl |
| 4-(3-CF₃-PhO)-2-thienyl | 5-(3-Br-Ph)CH₂CH₂-3-thienyl |
| 4-(4-MeS-PhO)-2-thienyl | 4-(2-F-Ph)CH₂CH₂-2-thienyl |
| 5-(4-MeSO₂-PhO)-3-thienyl | 4-(3-Cl-Ph)CH₂CH₂-2-thienyl |
| 5-(3-BuSO₂-PhO)-3-thienyl | 4-(2,4-diF-Ph)CH₂CH₂-2-thienyl |
| 5-(3-MeO-PhO)-3-thienyl | 5-(4-NO₂-Ph)CH₂CH₂-3-thienyl |
| 5-(3-hexyloxy-PhO)-3-thienyl | 5-(3-Me-Ph)CH₂CH₂-3-thienyl |
| 5-(4-CF₃O-PhO)-3-thienyl | 4-(3-Bu-Ph)CH₂CH₂-2-thienyl |
| 5-(4-cyclohexyloxy-PhO)-3-thienyl | 5-(4-CF₃-Ph)CH₂CH₂-3-thienyl |
| 4-(4-MeOC(=O)-PhO)-2-thienyl | 4-(2-MeS-Ph)CH₂CH₂-2-thienyl |
| 4-(4-allyl-PhO)-2-thienyl | 5-(4-MeO-Ph)CH₂CH₂-3-thienyl |
| 4-(4-propargyl-PhO)-2-thienyl | 4-(4-BuO-Ph)CH₂CH₂-2-thienyl |
| 5-((3-PhO)PhO)-3-thienyl | 4-PhOCH₂-2-thienyl |
| 5-(3-(2-Cl-PhO)PhO)-3-thienyl | 5-(3-Br-Ph)OCH₂-3-thienyl |
| 5-(3-(4-Me-PhO)PhO)-3-thienyl | 5-(2-F-Ph)OCH₂-3-thienyl |
| 5-(3-(3-MeO-PhO)PhO)-3-thienyl | 4-(3-Cl-Ph)OCH₂-2-thienyl |
| 4-PhCH-CH-2-thienyl | 5-(2,4-diF-Ph)OCH₂-3-thienyl |
| 4-(3-Br-Ph)CH=CH-2-thienyl | 5-(4-NO₂-Ph)OCH₂-3-thienyl |
| 4-(3-Me-Ph)OCH₂-2-thienyl | 5-(4-MeO-Ph)S-3-thienyl |
| 5-(3-Bu-Ph)OCH₂-3-thienyl | 5-(4-BuO-Ph)S-3-thienyl |
| 5-(4-CF₃-Ph)OCH₂-3-thienyl | 4-Ph-2-thienyl |
| 4-(2-MeS-Ph)OCH₂-2-thienyl | 4-(3-Br-Ph)-2-thienyl |
| 5-(4-MeO-Ph)OCH₂-3-thienyl | 4-(2-F-Ph)-2-thienyl |
| 4-(4-BuO-Ph)OCH₂-2-thienyl | 5-(3-Cl-Ph)-3-thienyl |
| 5-PhCH₂-3-thienyl | 5-(2,4-diF-Ph)-3-thienyl |
| 5-(3-Br-Ph)CH₂-3-thienyl | 5-(4-NO₂-Ph)-3-thienyl |
| 5-(2-F-Ph)CH₂-3-thienyl | 4-(3-Me-Ph)-2-thienyl |
| 5-(3-Cl-Ph)CH₂-3-thienyl | 5-(3-Bu-Ph)-3-thienyl |
| 4-(2,4-diF-Ph)CH₂-2-thienyl | 4-(4-CF₃-Ph)-2-thienyl |
| 4-(4-NO₂-Ph)CH₂-2-thienyl | 5-(2-MeS-Ph)-3-thienyl |
| 5-(3-Me-Ph)CH₂-3-thienylPh | 5-(4-MeO-Ph)-3-thienyl |
| 4-(3-Bu-Ph)CH₂-2-thienyl | 4-(4-BuO-Ph)-2-thienyl |
| 4-(4-CF₃-Ph)CH₂-2-thienyl | 5-PhCH₂O-3-thienyl |
| 5-(2-MeS-Ph)CH₂-3-thienyl | 4-(3-Br-Ph)CH₂O-2-thienyl |
| 4-(4-MeO-Ph)CH₂-2-thienyl | 5-(2-F-Ph)CH₂O-3-thienyl |
| 4-(4-BuO-Ph)CH₂-2-thienyl | 5-(3-Cl-Ph)CH₂O-3-thienyl |
| 5-PhS-3-thienyl | 5-(2,4-diF-Ph)CH₂O-3-thienyl |
| 4-(3-Br-Ph)S-2-thienyl | 4-(4-NO₂-Ph)CH₂O-2-thienyl |
| 5-(2-F-Ph)S-3-thienyl | 5-(3-Me-Ph)CH₂O-3-thienyl |
| 4-(3-Cl-Ph)S-2-thienyl | 4-(3-Bu-Ph)CH₂O-2-thienyl |
| 5-(2,4-diF-Ph)S-3-thienyl | 4-(4-CF₃-Ph)CH₂O-2-thienyl |
| 4-(4-NO₂-Ph)S-2-thienyl | 5-(2-MeS-Ph)CH₂O-3-thienyl |
| 5-(3-Me-Ph)S-3-thienyl | 4-(4-MeO-Ph)CH₂O-2-thienyl |
| 4-(3-Bu-Ph)S-2-thienyl | 5-(4-BuO-Ph)CH₂O-3-thienyl |
| 5-(4-CF₃-Ph)S-3-thienyl | 4-(4-CN-Ph)CH₂O-2-thienyl |
| 4-(2-MeS-Ph)S-2-thienyl | 5-(3-PhO-Ph)CH₂O-3-thienyl |

| R² | |
|---|---|
| 5-[3-(3-F-PhO)Ph]CH₂O-3-thienyl | |
| 4-(4-(2-NO₂-PhO)Ph]CH₂O-2-thienyl | |
| 4-[3-(4-Me-PhO)Ph]CH₂O-2-thienyl | |
| 4-[4-(4-Bu-PhO)Ph]CH₂O-2-thienyl | |
| 5-[3-[3-CF₃-PhO)Ph]CH₂O-3-thienyl | |
| 5-[2-(3-MeS-PhO)Ph]CH₂O-3-thienyl | |
| 4-[4-(4-MeO-PhO)Ph]CH₂O-2-thienyl | |
| 5-[3-(3-BuO-PhO)Ph]CH₂O-3-thienyl | |
| 4-(2-F-4-MeO-Ph)CH₂O-2-thienyl | |
| 5-(2-CF₃-4-Et-Ph)CH₂O-3-thienyl | |
| 4-[MeNHC(=O)O]-2-thienyl | |
| 4-[octyl-NHC(=O)O]-2-thienyl | |
| 5-[PhNHC(=O)O]-3-thienyl | |
| 5-[(3-Br-Ph)NHC(=O)O]-3-thienyl | |
| 4-[(2-F-Ph)NHC(=O)O]-2-thienyl | |
| 5-[(3-Cl-Ph)NHC(=O)O]-3-thienyl | |
| 5-[(2,4-diF-Ph)NHC(=O)O]-3-thienyl | |
| 5-[(4-NO₂-Ph)NHC(=O)O]-3-thienyl | |
| 4-[(3-Me-Ph)NHC(=O)O]-2-thienyl | |
| 5-[(3-Bu-Ph)NHC(=O)O]-3-thienyl | |
| 5-[(4-CF₃-Ph)NHC(=O)O]-3-thienyl | |
| 4-[(2-MeS-Ph)NHC(=O)O]-2-thienyl | |
| 4-[(4-MeO-Ph)NHC(=O)O]-2-thienyl | |
| 5-[(4-BuO-Ph)NHC(=O)O]-3-thienyl | |
| 5-[(4-CN-Ph)NHC(=O)O]-3-thienyl | |
| 4-[(3-PhO-Pb)NHC(=O)O]-2-thienyl | |
| 4-[3-(3-F-PhO)Ph-NHC(=O)O]-2-thienyl | |
| 4-[4-(2-NO₂-PhO)Ph-NHC(=O)O]-2-thienyl | |
| 4-[2-(4-Me-PhO)Ph-NHC(=O)O]-2-thienyl | |
| 5-[4-(4-Bu-PhO)Ph-NHC(=O)O]-3-thienyl | |
| 5-(4-(3-CF₃-PhO)Ph-NHC(=O)O]-3-thienyl | |
| 5-[4-(3-MeS-PhO)Ph-NHC(=O)O]-3-thienyl | |
| 4-[3-(4-MeO-PhO)Ph-NHC(=O)O)-2-thienyl | |
| 5-[3-(3-BuO-PhO)Ph-NHC(=O)O]-3-thienyl | |
| 4-[(2-F-4-MeO-Ph)NHC(=O)O]-2-thienyl | |
| 5-[(2-CF₃-4-Et-Ph)NHC(=O)O]-3-thienyl | |
| 5-[(3-pyridyl)NHC(=O)O]-3-thienyl | |
| 4-[(3-Br-2-pyridyl)NHC(=O)O]-2-thienyl | |
| 5-[(2-F-3-pyridyl)NHC(=O)O]-3-thienyl | |
| 5-[(3-Cl-4-pyridyl)NHC(=O)O]-3-thienyl | |
| 5-[(2,4-diF-3-pyridyl)NHC(=O)O]-3-thienyl | |
| 4-[(4-NO₂-2-pyridyl)NHC(=O)O]-2-thienyl | |
| 4-[(3-Me-2-pyridyl)NHC(=O)O]-2-thienyl | |
| 5-[(3-Bu-2-pyridyl)NHC(=O)O]-3-thienyl | |
| 5-[(4-CF₃-3-pyridyl)NHC(=O)O]-3-thienyl | |
| 4-[(2-MeS-4-pyridyl)NHC(=O)O]-2-thienyl | |
| 5-[(4-MeO-3-pyridyl)NHC(=O)O]-3-thienyl | |
| 4-[(4-BuO-3-pyridyl)NHC(=O)O]-2-thienyl | |
| 5-[(4-CN-2-pyridyl)NHC(=O)O]-3-thienyl | |
| 4-[(3-PhO-2-pyridyl)NHC(=O)O]-2-thienyl | |
| 4-[3-(3-F-PhO)-2-pyridyl-NHC(=O)O]-2-thienyl | |
| 5-[4-(2-NO₂-PhO)-3-pyridyl-NHC(=O)O]-3-thienyl | |
| 4-[2-(4-Me-PhO)-4-pyridyl-NHC(=O)O]-2-thienyl | |
| 5-[4-(4-Bu-PhO)-3-pyridyl-NHC(=O)O]-3-thienyl | |
| 4-(4-(3-CF₃-PhO)-2-pyridyl-NHC(=O)O]-2-thienyl | |
| 5-[4-(3-MeS-PhO)-3-pyridyl-NHC(=O)O)-3-thienyl | |
| 4-[3-(4-MeO-PhO)-2-pyridyl-NHC(=O)O]-2-thienyl | |
| 5-[3-(3-BuO-PhO)-2-pyridyl-NHC(=O)O]-3-thienyl | |
| 5-[(2-F-4-MeO-3-pyridyl)NHC(=O)O]-3-thienyl | |
| 4-[(2-CF₃-4-Et-3-pyridyl)NHC(=O)O]-2-thienyl | |
| 4-[MeO-C(=O)]-2-thienyl | |
| 4-[octyloxy-C(=O)]-2-thienyl | |
| 5-[PhO-C(=O)]-3-thienyl | |
| 5-[3-Br-PhO-C(=O)]-3-thienyl | |
| 4-[2-F-PhO-C(=O)]-2-thienyl | |
| 4-[3-Cl-PhO-C(=O)]-2-thienyl | |
| 5-[2,4-diF-PhO-C(=O)]-3-thienyl | |
| 5-(4-NO₂-PhO-(=O)]-3-thienyl | |
| 4-[3-Me-PhO-C(=O)]-2-thienyl | |
| 4-[3-Bu-PhO-C(=O)]-2-thienyl | |
| 5-(4-CF₃-PhO-C(=O)]-3-thienyl | |
| 5-[2-MeS-PhO-C(=O)]-3-thienyl | |
| 4-[4-MeO-PhO-C(=O)]-2-thienyl | |
| 4-[4-BuO-PhO-C(=O)]-2-thienyl | |
| 5-[4-CN-PhO-C(=O)]-3-thienyl | |
| 5-[3-PhO-PhO-C(=O)]-3-thienyl | |
| 4-[3-(3-F-PhO)PhO-C(=O)]-2-thienyl | |
| 5-[4-(2-NO₂-PhO)PhO-C(=O)]-3-thienyl | |
| 4-[2-(4-Me-PhO)PhO-C(=O)]-2-thienyl | |
| 5-[4-(4-Bu-PhO)PhO-C(=O)]-3-thienyl | |
| 4-[4-(3-CF₃-PhO)PhO-C(=O)]-2-thienyl | |
| 5-[4-(3-MeS-PhO)PhO-C(=O)]-3-thienyl | |
| 4-[3-(4-MeO-PhO)PhO-C(=O)]-2-thienyl | |
| 5-[3-(3-BuO-PhO)PhO-C(=O)]-3-thienyl | |
| 4-[(2-F-4-MeO-PhO-C(=O)]-2-thienyl | |
| 5-[(2-CF₃-4-Et-PhO-C(=O)]-3-thienyl | |
| 5-[(3-pyridyloxy)C(=O)]-3-thienyl | |
| 4-[(3-Br-2-pyridyloxy)C(=O)]-2-thienyl | |
| 5-[(2-F-3-pyridyloxy)C(=O)]-3-thienyl | |
| 5-[(3-Cl-4-pyridyloxy)C(=O)]-3-thienyl | |
| 4-[(2,4-diF-3-pyridyloxy)C(=O)]-2-thienyl | |
| 4-[(4-NO₂-2-pyridyloxy)C(=O)]-2-thienyl | |
| 5-[(3-Me-2-pyridyloxy)C(=O)]-3-thienyl | |
| 5-[(3-Bu-2-pyridyloxy)C(=O)]-3-thienyl | |
| 5-[(4-CF₃-3-pyridyloxy)C(=O)]-3-thienyl | |
| 5-[(2-MeS-4-pyridyloxy)C(=O)]-3-thienyl | |
| 5-[(4-MeO-3-pyridyloxy)C(=O)]-3-thienyl | |
| 4-[(4-BuO-3-pyridyloxy)C(=O)]-2-thienyl | |
| 4-[(4-CN-2-pyridyloxy)C(=O)]-2-thienyl | |
| 4-[(3-PhO-2-pyridyloxy)C(=O)]-2-thienyl | |
| 5-[3-(3-F-PhO)-2-pyridyloxy-C(=O)]-3-thienyl | |
| 5-[4-(2-NO₂-PhO)-3-pyridyloxy-C(=O)]-3-thienyl | |
| 5-[2-(4-Me-PhO)-4-pyridyloxy-C(=O)]-3-thienyl | |
| 5-[4-(4-Bu-PhO)-3-pyridyloxy-C(=O)]-3-thienyl | |
| 4-[4-(3-CF₃-PhO)-2-pyridyloxy-C(=O)]-2-thienyl | |
| 4-[4-(3-MeS-PhO)-3-pyridyloxy-C(=O))-2-thienyl | |
| 4-[3-(4-MeO-PhO)-2-pyridyloxy-C(=O)]-2-thienylPh | |
| 5-[3-(3-BuO-PhO)-2-pyridyloxy-C(=O)]-3-thienyl | |
| 4-[(2-F-4-Meo-3-pyridyloxy)C(=O)]-2-thienyl | |
| 4-[(2-CF₃-4-Et-3-pyridyloxy)C(=O)]-2-thienyl | |
| 4-[MeC(=O)O]-2-thienyl | |
| 4-[octyl-C(=O)O]-2-thienyl | |
| 5-[PhC(=O)O]-3-thienyl | |
| 4-[3-Br-PhC(=O)O]-2-thienyl | |
| 5-[2-F-PhC(=O)O]-3-thienyl | |
| 5-[3-Cl-PhC(=O)O]-3-thienyl | |
| 4-[2,4-diF-PhC(=O)O]-2-thienyl | |
| 5-[4-NO₂-Ph(=O)O]-3-thienyl | |
| 4-[3-Me-PhC(=O)O]-2-thienyl | |
| 5-[3-Bu-PhC(=O)O]-3-thienyl | |
| 4-[4-CF₃-PhC(=O)O]-2-thienyl | |
| 4-[2-MeS-PhC(=O)O]-2-thienyl | |
| 5-[4-MeO-PhC(=O)O]-3-thienyl | |
| 5-[4-BuO-PhC(=O)O]-3-thienyl | |
| 4-[4-CN-PhC(=O)O]-2-thienyl | |
| 4-[3-PhO-PhC(=O)O]-2-thienyl | |
| 5-[3-(3-F-PhO)PhC(=O)O]-3-thienyl | |
| 4-[4-(2-NO₂-PhO)PhC(=O)O]-2-thienyl | |
| 5-[2-(4-Me-PhO)PhC(=O)O]-3-thienyl | |
| 4-[4-(4-Bu-PhO)PhC(=O)O]-2-thienyl | |
| 5-[4-(3-CF₃-PhO)PhC(=O)O]-3-thienyl | |
| 4-[4-(3-MeS-PhO)PhC(=O)O]-2-thienyl | |
| 5-[3-(4-MeO-PhO)PhC(=O)O]-3-thienyl | |
| 5-[3-(3-BuO-PhO)PhC(=O)O]-3-thienyl | |
| 4-[(2-F-4-MeO-Ph)C(=O)O]-2-thienyl | |
| 5-[(2-CF₃-4-Et-Ph)C(=O)O]-3-thienyl | |
| 5-[(3-pyridyl)C(=O)O]-3-thienyl | |
| 4-[(3-Br-2-pyridyl)C(=O)O]-2-thienyl | |
| 4-[(2-F-3-pyridyl)C(=O)O]-2-thienyl | |
| 5-[(3-Cl-4-pyridyl)C(=O)O]-3-thienyl | |
| 5-[(2,4-diF-3-pyridyl)C(=O)O]-3-thienyl | |
| 5-[(4-NO₂-2-pyridyl)C(=O)O]-3-thienyl | |
| 5-[(3-Me-2-pyridyl)C(=O)O)-3-thienyl | |
| 5-[(3-Bu-2-pyridyl)C(=O)O]-3-thienyl | |
| 4-[(4-CF₃-3-pyridyl)C(=O)O]-2-thienyl | |
| 5-[(2-MeS-4-pyridyl)C(=O)O]-3-thienyl | |
| 5-[(4-MeO-3-pyridyl)C(=O)O]-3-thienyl | |
| 4-[(4-BuO-3-pyridyl)C(=O)O]-2-thienyl | |
| 4-[(4-CN-2-pyridyl)C(=O)O]-2-thienyl | |
| 5-[(3-PhO-2-pyridyl)C(=O)O]-3-thienyl | |
| 5-[3-(3-F-PhO)-2-pyridyl-C(=O)O)-3-thienyl | |
| 5-[4-(2-NO₂-PhO)-3-pyridyl-C(=O)O]-3-thienyl | |
| 4-[2-(4-Me-PhO)-4-pyridyl-C(=O)O]-2-thienyl | |
| 5-[4-(4-Bu-PhO)-3-pyridyl-C(=O)O]-3-thienyl | |
| 5-[4-(3-CF₃-PhO)-2-pyridyl-C(=O)O)-3-thienyl | |
| 5-[4-(3-MeS-PhO)-3-pyridyl-C(=O)O]-3-thienyl | |
| 4-[3-(4-MeO-PhO)-2-pyridyl-C(=O)O]-2-thienyl | |
| 4-[3-(3-BuO-PhO)-2-pyridyl-C(=O)O]-2-thienyl | |
| 5-[(2-F-4-MeO-3-pyridyl)C(=O)O]-3-thienyl | |
| 4-[(2-CF₃-4-Et-3-pyridyl)C(=O)O]-2-thienyl | |
| 3-F-4-PhCH₂CH₂-2-thienyl | |
| 3-F-4-(2-F-Ph)OCH₂-2-thienyl | |
| 4-F-5-(4-MeO-Ph)CH₂-3-thienyl | |
| 2-F-5-PhS-3-thienyl | |
| 5-MeS-4-PhO-2-thienyl | |
| 2-F-5-PhCH₂O-3-thienyl | |
| 2-F-5-[octyl-NHC(=O)O]-3-thienyl | |

| R² | R² |
|---|---|
| 4-F-3-pyridyl | 6-(2-propenyl)-3-pyridyl |
| 4-Cl-3-pyridyl | 5-(3-pentenyl)-2-pyridyl |
| 3-F-2-pyridyl | 4-(Cl₂C=CHCH₂)-2-pyridyl |
| 4-Br-3-pyridyl | 4-(2-propenyloxy)-2-pyridyl |
| 2,3-diF-4-pyridyl | 6-(2-hexenyloxy)-3-pyridyl |
| 2-pyridyl | 6-(propargyl)-3-pyridyl |
| 3-pyridyl | 2-(2-butynyloxy)-4-pyridyl |
| 4-pyridyl | 4-BuSO₂-2-pyridyl |
| 2,5-diF-3-pyridyl | 6-PhO-3-pyridyl |
| 3,5-diF-4-pyridyl | 6-(4-Cl-PhO)-3-pyridyl |
| 2,6-diF-3-pyridyl | 6-(3-Cl-PhO)-3-pyridyl |
| 3,4-diF-2-pyridyl | 5-(2,4-diF-PhO)-2-pyridyl |
| 5-Cl-2-F-3-pyridyl | 5-(4-NO₂-PhO)-3-pyridyl |
| 2-F-5-Me-3-pyridyl | 2-(4-CN-PhO)-4-pyridyl |
| 2-F-4-pyridyl | 5-(4-Me-PhO)-2-pyridyl |
| 4-Me-2-pyridyl | 6-(3-hexyl-PhO)-3-pyridyl |
| 4-NO₂-2-pyridyl | 6-(3-cyclohexyl-PhO)-3-pyridyl |
| 5-hexyl-3-pyridyl | 4-(3-CF₃-PhO)-2-pyridyl |
| 5-(cyclohexyl)-3-pyridyl | 4-(4-MeS-PhO)-2-pyridyl |
| 5-CF₃-3-pyridyl | 5-(4-MeSO₂-PhO)-2-pyridyl |
| 2-MeS-4-pyridyl | 6-(3-BuSO₂-PhO)-3-pyridyl |
| 4-hexylthio-2-pyridyl | 6-(3-MeO-PhO)-3-pyridyl |
| 5-MeO-4-pyridyl | 6-(3-hexyloxy-PhO)-3-pyridyl |
| 6-pentyloxy-3-pyridyl | 6-(4-CF₃O-PhO)-3-pyridyl |
| 6-CF₃O-3-pyridyl | 5-(4-cyclohexyloxy-PhO)-2-pyridyl |
| 6-cyclohexyloxy-3-pyridyl | 5- (4-MeOC(=O)-PhO)-2-pyridyl |
| 6-(BuOCH₂)-3-pyridyl | 5-(4-allyl-PhO)-3-pyridyl |
| 5-MeOCH₂-2-pyridyl | 6-(4-propargyl-PhO)-3-pyridyl |
| 6-((3-PhO)PhO)-3-pyridyl | 6-PhOCH₂-3-pyridyl |
| 2-(3-(2-Cl-PhO)PhO)-4-pyridyl | 6-(3-Br-Ph)OCH₂-3-pyridyl |
| 4-(3-(4-Me-PhO)PhO)-2-pyridyl | 5-(2-F-Ph)OCH₂-2-pyridyl |
| 5-(3-(3-MeO-PhO)PhO)-2-pyridyl | 2-(3-Cl-Ph)OCH₂-4-pyridyl |
| 6-PhCH=CH-3-pyridyl | 5-(2,4-diF-Ph)OCH₂-3-pyridyl |
| 6-(3-Br-Ph)CH=CH-3-pyridyl | 6-(4-NO₂-Ph)OCH₂-3-pyridyl |
| 5-(2-F-Ph)CH=CH-2-pyridyl | 4-(3-Me-Ph)OCH₂-2-pyridyl |
| 2-(3-Cl-Ph)CH=CH-4-pyridyl | 5-(3-Bu-Ph)OCH₂-2-pyridyl |
| 5-(2,4-diF-Ph)CH=CH-3-pyridyl | 6-(4-CF₃-Ph)OCH₂-3-pyridyl |
| 6-(4-NO₂-Ph)CH-CH-3-pyridyl | 6-(2-MeS-Ph)OCH₂-3-pyridyl |
| 4-(3-Me-Ph)CH=CH-2-pyridyl | 5-(4-MeO-Ph)OCH₂-2-pyridyl |
| 5-(3-Bu-Ph)CH=CH-2-pyridyl | 6-(4-BuO-Ph)OCH₂-3-pyridyl |
| 6-(4-CF₃-Ph)CH=CH-3-pyridyl | 6-PhCH₂-3-pyridyl |
| 6-(2-MeS-Ph)CH=CH-3-pyridyl | 6-(3-Br-Ph)CH₂-3-pyridyl |
| 5-(4-MeO-Ph)CH=CH-2-pyridyl | 5-(2-F-Ph)CH₂-2-pyridyl |
| 6-(4-BuO-Ph)CH=CH-3-pyridyl | 2-(3-Cl-Ph)CH₂-4-pyridyl |
| 6-PhCH₂CH₂-3-pyridyl | 5-(2,4-diF-Ph)CH₂-3-pyridyl |
| 6-(3-Br-Ph)CH₂CH₂-3-pyridyl | 6-(4-NO₂-Ph)CH₂-3-pyridyl |
| 5-(2-F-Ph)CH₂CH₂-2-pyridyl | 4-(3-Me-Ph)CH₂-2-pyridyl |
| 2-(3-Cl-Ph)CH₂CH₂-4-pyridyl | 5-(3-Bu-Ph)CH₂-2-pyridyl |
| 5-(2,4-diF-Ph)CH₂CH₂-3-pyridyl | 6-(4-CF₃-Ph)CH₂-3-pyridyl |
| 6-(4-NO₂-Ph)CH₂CH₂-3-pyridyl | 6-(2-MeS-Ph)CH₂-3-pyridyl |
| 4-(3-Me-Ph)CH₂CH₂-2-pyridyl | 5-(4-MeO-Ph)CH₂-2-pyridyl |
| 5-(3-Bu-Ph)CH₂CH₂-2-pyridyl | 6-(4-BuO-Ph)CH₂-3-pyridyl |
| 6-(4-CF₃-Ph)CH₂CH₂-3-pyridyl | 6-PhS-3-pyridyl |
| 6-(2-MeS-Ph)CH₂CH₂-3-pyridyl | 6-(3-Br-Ph)S-3-pyridyl |
| 5-(4-MeO-Ph)CH₂CH₂-2-pyridyl | 5-(2-F-Ph)S-2-pyridyl |
| 6-(4-BuO-Ph)CH₂CH₂-3-pyridyl | 2-(3-Cl-Ph)S-4-pyridyl |
| 5-(2,4-diF-Ph)S-3-pyridyl | 2-(4-CF₃-Ph)CH₂O-4-pyridyl |
| 6-(4-NO₂-Ph)S-3-pyridyl | 5-(2-MeS-Ph)CH₂O-3-pyridyl |
| 4-(3-Me-Ph)S-2-pyridyl | 5-(4-MeO-Ph)CH₂O-2-pyridyl |
| 5-(3-Bu-Ph)S-2-pyridyl | 6-(4-BuO-Ph)CH₂O-3-pyridyl |
| 6-(4-CF₃-Ph)S-3-pyridyl | 5-(4-CN-Ph)CH₂O-2-pyridyl |
| 6-(2-MeS-Ph)S-3-pyridyl | 6-(3-PhO-Ph)CH₂O-3-pyridyl |
| 5-(4-MeO-Ph)S-2-pyridyl | 6-[3-(3-F-PhO)Ph]CH₂O-3-pyridyl |
| 6-(4-BuO-Ph)S-3-pyridyl | 6-[4-(2-NO₂-PhO)Ph]CH₂O-3-pyridyl |
| 6-Ph-3-pyridyl | 5-[3-(4-Me-PhO)Ph]CH₂O-2-pyridyl |
| 6-(3-Br-Ph)-3-pyridyl | 5-[4-(4-Bu-PhO)Ph]CH₂O-2-pyridyl |
| 5-(2-F-Ph)-2-pyridyl | 6-[3-(3-CF₃-PhO)Ph]CH₂O-3-pyridyl |
| 2-(3-Cl-Ph)-4-pyridyl | 6-[2-(3-MeS-PhO)Ph]CH₂O-3-pyridyl |
| 5-(2,4-diF-Ph)-3-pyridyl | 6-[4-(4-MeO-PhO)Ph]CH₂O-3-pyridyl |
| 6-(4-NO₂-Ph)-3-pyridyl | 5-[3-(3-BuO-PhO)Ph]CH₂O-2-pyridyl |
| 4-(3-Me-Ph)-2-pyridyl | 6-(2-F-4-MeO-Ph)CH₂O-3-pyridyl |
| 5-(3-Bu-Ph)-2-pyridyl | 6-(2-CF₃-4-Et-Ph)CH₂O-3-pyridyl |
| 6-(4-CF₃-Ph)-3-pyridyl | 6-[(3-pyridyl)NHC(=O)O]-3-pyridyl |
| 6-(2-MeS-Ph)-3-pyridyl | 5-[(3-Br-2-pyridyl)NHC(=O)O]-2-pyridyl |
| 5-(4-MeO-Ph)-2-pyridyl | 6-[(2-F-3-pyridyl)NHC(=O)O]-3-pyridyl |
| 6-(4-BuO-Ph)-3-pyridyl | 4-[(3-Cl-4-pyridyl)NHC(=O)O]-2-pyridyl |
| 6-PhCH₂O-3-pyridyl | 2-[(2,4-diF-3-pyridyl)NHC(=O)O]-4-pyridyl |
| 5-(3-Br-Ph)CH₂O-2-pyridyl | 6-[(4-NO₂-2-pyridyl)NHC(=O)O]-3-pyridyl |
| 6-(2-F-Ph)CH₂O-3-pyridyl | 6-[(3-Me-2-pyridyl)NHC(=O)O]-3-pyridyl |
| 4-(3-Cl-Ph)CH₂O-2-pyridyl | 5-[(3-Bu-2-pyridyl)NHC(=O)O]-2-pyridyl |
| 2-(2,4-diF-Ph)CH₂O-4-pyridyl | 2-((4-CF₃-3-pyridyl)NHC(=O)O]-4-pyridyl |
| 6-(4-NO₂-Ph)CH₂O-3-pyridyl | 5-[(2-MeS-4-pyridyl)NHC(=O)O]-3-pyridyl |
| 6-(3-Me-Ph)CH₂O-3-pyridyl | 5-[(4-MeO-3-pyridyl)NHC(=O)O]-2-pyridyl |
| 5-(3-Bu-Ph)CH₂O-2-pyridyl | 2-[(4-BuO-3-pyridyl)NHC(=O)O]-4-pyridyl |

| R² | |
|---|---|
| 5-[(4-CN-2-pyridyl)NHC(=O)O]-3-pyridyl | |
| 5-[(3-PhO-2-pyridyl)NHC(=O)O]-2-pyridyl | |
| 6-[3-(3-F-PhO)-2-pyridyl-NHC(=O)O]-3-pyridyl | |
| 5-(4-(2-NO₂-PhO)-3-pyridyl-NHC(=O)O]-2-pyridyl | |
| 6-[2-(4-Me-PhO)-4-pyridyl-NHC(=O)O]-3-pyridyl | |
| 6-[4-(4-Bu-PhO)-3-pyridyl-NHC(=O)O]-3-pyridyl | |
| 6-[4-(3-CF₃-PhO)-2-pyridyl-NHC(=O)O]-3-pyridyl | |
| 5-[4-(3-MeS-PhO)-3-pyridyl-NHC(=O)O]-2-pyridyl | |
| 5-[3-(4-MeO-PhO)-2-pyridyl-NHC(=O)O]-2-pyridyl | |
| 6-[3-(3-Buo-PhO)-2-pyridyl-NHC(=O)O]-3-pyridyl | |
| 6-[(2-F-4-MeO-3-pyridyl)NHC(=O)O]-3-pyridyl | |
| 6-[(2-CF₃-4-Et-3-pyridyl)NHC(=O)O]-3-pyridyl | |
| 6-[(3-pyridyloxy)C(=O)]-3-pyridyl | |
| 5-[(3-Br-2-pyridyloxy)C(=O)]-2-pyridyl | |
| 6-[(2-F-3-pyridyloxy)C(=O)]-3-pyridyl | |
| 4-[(3-Cl-4-pyridyloxy)C(=O)]-2-pyridyl | |
| 2-[(2,4-diF-3-pyridyloxy)C(=O)]-4-pyridyl | |
| 6-[(4-NO₂-2-pyridyloxy)C(=O)]-3-pyridyl | |
| 6-[(3-Me-2-pyridyloxy)C(=O)]-3-pyridyl | |
| 5-[(3-Bu-2-pyridyloxy)C(=O)]-2-pyridyl | |
| 2-[(4-CF₃-3-pyridyloxy)C(=O)]-4-pyridyl | |
| 5-[(2-MeS-4-pyridyloxy)C(=O)]-3-pyridyl | |
| 5-[(4-MeO-3-pyridyloxy)C(=O)]-2-pyridyl | |
| 4-[(4-BuO-3-pyridyloxy)C(=O)]-3-pyridyl | |
| 2-[(4-CN-2-pyridyloxy)C(=O)]-4-pyridyl | |
| 5-[(3-PhO-2-pyridyloxy)C(=O)]-2-pyridyl | |
| 5-[3-(3-F-PhO)-2-pyridyloxy-C(=O)]-3-pyridyl | |
| 6-[4-(2-NO₂-PhO)-3-pyridyloxy-C(=O)]-3-pyridyl | |
| 5-[2-(4-Me-PhO)-4-pyridyloxy-C(=O)]-2-pyridyl | |
| 6-(4-(4-Bu-PhO)-3-pyridyloxy-C(=O)]-3-pyridyl | |
| 6-[4-(3-CF₃-PhO)-2-pyridyloxy-C(=O)]-3-pyridyl | |
| 6-(4-(3-MeS-PhO)-3-pyridyloxy-C(=O)]-3-pyridyl | |
| 5-[3-(4-MeO-PhO)-2-pyridyloxy-C(=O)]-2-pyridyl | |
| 5-[3-(3-BuO-PhO)-2-pyridyloxy-C(=O)]-2-pyridyl | |
| 6-[(2-F-4-MeO-3-pyridyloxy)C(=O)]-3-pyridyl | |
| 6-[(2-CF₃-4-Et-3-pyridyloxy)C(=O)]-3-pyridyl | |
| 6-[(3-pyridyl)C(=O)O]-3-pyridyl | |
| 5-[(3-Br-2-pyridyl)C(=O)O]-2-pyridyl | |
| 6-[(2-F-3-pyridyl)C(=O)O]-3-pyridyl | |
| 4-[(3-Cl-4-pyridyl)C(=O)O]-2-pyridyl | |
| 2-[(2,4-diF-3-pyridyl)C(=O)O]-4-pyridyl | |
| 6-[(4-NO₂-2-pyridyl)C(=O)O]-3-pyridyl | |
| 6-[(3-Me-2-pyridyl)C(=O)O]-3-pyridyl | |
| 5-[(3-Bu-2-pyridyl)C(=O)O]-2-pyridyl | |
| 2-[(4-CF₃-3-pyridyl)C(=O)O]-4-pyridyl | |
| 5-[(2-MeS-4-pyridyl)O(=O)O]-3-pyridyl | |
| 5-[(4-MeO-3-pyridyl)C(=O)O]-2-pyridyl | |
| 2-[(4-BuO-3-pyridyl)C(=O)O]-4-pyridyl | |
| 5-[(4-CN-2-pyridyl)C(=O)O]-3-pyridyl | |
| 5-[(3-PhO-2-pyridyl)C(=O)O]-2-pyridyl | |
| 6-[3-(3-F-PhO)-2-pyridyl-C(=O)O]-3-pyridyl | |
| 5-(4-(2-NO₂-PhO)-3-pyridyl-C(=O)O]-2-pyridyl | |
| 6-[2-(4-Me-PhO)-4-pyridyl-C(=O)O]-3-pyridyl | |
| 6-[4-(4-Bu-PhO)-3-pyridyl-C(=O)O]-3-pyridyl | |
| 6-(4-(3-CF₃-PhO)-2-pyridyl-C(=O)O]-3-pyridyl | |
| 5-[4-(3-MeS-PhO)-3-pyridyl-C(=O)O]-2-pyridyl | |
| 5-[3-(4-MeO-PhO)-2-pyridyl-C(=O)O]-2-pyridyl | |
| 6-[3-(3-BuO-PhO)-2-pyridyl-C(=O)O]-3-pyridyl | |
| 6-[(2-F-4-MeO-3-pyridyl)C(=O)O]-3-pyridyl | |
| 6-[(2-CF₃-4-Et-3-pyridyl)C(=O)O]-3-pyridyl | |
| 5-(MeNHC(=O)O]-2-pyridyl | |
| 2-F-6-[octyl-NHC(=O)O]-3-pyridyl | |
| 5-[PhNHC(=O)O]-3-pyridyl | |
| 2-[(3-Br-Ph)NHC(=O)O]-4-pyridyl | |
| 5-[(2-F-Ph)NHC(=O)O]-3-pyridyl | |
| 2-F-6-[(3-Cl-Ph)NHC(=O)O]-3-pyridyl | |
| 6-[(2,4-diF-Ph)NHC(=O)O]-3-pyridyl | |
| 3-F-2-[(4-NO₂-Ph)NHC(=O)O]-4-pyridyl | |
| 6-[(3-Me-Ph)NHC(=O)O]-3-pyridyl | |
| 5-[(3-Bu-Ph)NHC(=O)O]-2-pyridyl | |
| 6-[(4-CF₃-Ph)NHC(=O)O]-3-pyridyl | |
| 2-F-6-[(2-MeS-Ph)NHC(=O)O]-3-pyridyl | |
| 5-[(4-MeO-Ph)NHC(=O)O]-2-pyridyl | |
| 4-[(4-BuO-Ph)NHC(=O)O]-2-pyridyl | |
| 6-[(4-CN-Ph)NHC(=O)O]-3-pyridyl | |
| 2-F-6-[(3-PhO-Ph)NHC(=O)O]-3-pyridyl | |
| 2-[3-(3-F-PhO)Ph-NHC(=O)O]-4-pyridyl | |
| 5-[4-(2-NO₂-PhO)Ph-NHC(=O)O]-2-pyridyl | |
| 5-[2-(4-Me-PhO)Ph-NHC(=O)O]-3-pyridyl | |
| 5-[4-(4-Bu-PhO)Ph-NHC(=O)O]-2-pyridyl | |
| 6-[4-(3-CF₃-PhO)Ph-NHC(=O)O]-3-pyridyl | |
| 6-[4-(3-MeS-PhO)Ph-NHC(=O)O]-3-pyridyl | |
| 6-[3-(4-MeO-PhO)Ph-NHC(=O)O]-3-pyridyl | |
| 2-F-6-[3-(3-BuO-PhO)Ph-NHC(=O)O]-3-pyridyl | |
| 5-[(2-F-4-MeO-Ph)NHC(=O)O]-2-pyridyl | |
| 6-[(2-CF₃-4-Et-Ph)NHC(=O)O]-2-pyridyl | |
| 5-[Me)C(=O)]-2-pyridyl | |
| 2-F-6-[octyloxy-C(=O)]-3-pyridyl | |
| 5-[PhOC(=O)]-3-pyridyl | |
| 2-[(3-Br-PhO)C(=O)]-4-pyridyl | |
| 5-[(2-F-PhO)C(=O)]-3-pyridyl | |
| 2-F-6-[(3-Cl-PhO)C(=O)]-3-pyridyl | |
| 6-[(2,4-diF-PhO)C(=O)]-3-pyridyl | |
| 3-F-2-[(4-NO₂-PhO)C(=O)]-4-pyridyl | |
| 6-[(3-Me-PhO)C(=O)]-3-pyridyl | |
| 5-[(3-Bu-PhO)C(=O)]-2-pyridyl | |
| 6-[(4-CF₃-PhO)C(=O)]-3-pyridyl | |
| 2-F-6-[(2-MeS-PhO)C(=O)]-3-pyridyl | |
| 5-[(4-MeO-PhO)C(=O)]-2-pyridyl | |
| 4-[(4-BuO-PhO)C(=O)]-2-pyridyl | |
| 6-[(4-CN-PhO)C(=O)]-3-pyridyl | |
| 2-F-6-[(3-PhO-PhO)C(=O)]-3-pyridyl | |
| 2-[3-(3-F-PhO)PhO-C(=O)]-4-pyridyl | |
| 5-[4-(2-NO₂-PhO)PhO-C(=O)]-2-pyridyl | |
| 5-[2-(4-Me-PhO)PhO-C(=O)]-3-pyridyl | |
| 5-[4-(4-Bu-PhO)PhO-C(=O)]-2-pyridyl | |
| 6-(4-(3-CF₃-PhO)PhO-C(=O)]-3-pyridyl | |
| 6-(4-(3-MeS-PhO)PhO-C(=O)]-3-pyridyl | |
| 6-[3-(4-MeO-PhO)PhO-C(=O)]-3-pyridyl | |
| 2-F-6-[3-(3-BuO-PhO)PhO-C(=O)]-3-pyridyl | |
| 5-[(2-F-4-MeO-PhO)C(=O)]-2-pyridyl | |
| 6-[(2-CF₃-4-Et-PhO)C(=O)]-2-pyridyl | |

| R² | R² |
|---|---|
| 5-[MeC(=O)O]-2-pyridyl | 2-furanyl |
| 2-F-6-[octyl-C(=O)O]-3-pyridyl | 3-furanyl |
| 5-[PhC(=O)O]-3-pyridyl | 2-F-3-furanyl |
| 2-[(3-Br-Ph)C(=O)O]-4-pyridyl | 5-Cl-3-furanyl |
| 5-[(2-F-Ph)C(=O)O]-3-pyridyl | 5-NO₂-3-furanyl |
| 2-F-6-[(3-Cl-Ph)C(=O)O]-3-pyridyl | 3,4-diF-2-furanyl |
| 6-[(2,4-diF-Ph)C(=O)O]-3-pyridyl | 4-Me-2-furanyl |
| 3-F-2-[(4-NO₂-Ph)C(=O)O]-4-pyridyl | 5-Bu-3-furanyl |
| 6-[(3-Me-Ph)C(=O)O]-3-pyridyl | 4-CF₃-2-furanyl |
| 5-[(3-Bu-Ph)C(=O)O]-2-pyridyl | 5-MeS-2-furanyl |
| 6-[(4-CF₃-Ph)C(=O)O]-3-pyridyl | 5-MeO-3-furanyl |
| 2-F-6-[(2-MeS-Ph)C(=O)O]-3-pyridyl | 4-BuO-2-furanyl |
| 5-[(4-MeO-Ph)C(=O)O]-2-pyridyl | |
| 4-[(4-BuO-Ph)C(=O)O]-2-pyridyl | |
| 6-[(4-CN-Ph)C(=O)O]-3-pyridyl | |
| 2-F-6-[(3-PhO-Ph)C(=O)O]-3-pyridyl | |
| 2-[3-(3-F-PhO)Ph-C(=O)O]-4-pyridyl | |
| 5-(4-(2-NO₂-PhO)Ph-C(=O)O]-2-pyridyl | |
| 5-[2-(4-Me-PhO)Ph-C(=O)O]-3-pyridyl | |
| 5-[4-(4-Bu-PhO)Ph-C(=O)O]-2-pyridyl | |
| 6-[4-(3-CF₃-PhO)Ph-C(=O)O]-3-pyridyl | |
| 6-[4-(3-MeS-PhO)Ph-C(=O)O]-3-pyridyl | |
| 6-[3-(4-MeO-PhO)Ph-C(=O)O]-3-pyridyl | |
| 2-F-6-[3-(3-BuO-Ph)PhO-C(=O)O]-3-pyridyl | |
| 5-[(2-F-4-MeO-Ph)C(=O)O]-2-pyridyl | |
| 6-[(2-CF₃-4-Et-Ph)C(=O)O]-2-pyridyl | |

**TABLE 3**

| Compounds of Formula If wherein A=S and: | | | |
|---|---|---|---|
| n | R⁴³ | n | R⁴³ |
| 5 | Me | 6 | 4-F-PhO |
| 6 | Ph | 8 | 4-Et-PhO |
| 8 | 4-CF₃-Ph | 2 | 4-EtO-PhO |
| 9 | 3-F₃CO-Ph | 1 | 4-(4-F-PhO)PhO |
| 5 | 2,4-diCl-Ph | 2 | 4-(3-Et-PhO)PhO |
| 1 | 4-F-Ph | 3 | 4-(3-Bu-PhO)PhO |
| 2 | 4-Et-Ph | 3 | MeNH |
| 3 | 3-EtO-Ph | 4 | Me₂N |
| 4 | 4-(4-F-PhO)Ph | 1 | PhNH |
| 3 | 4-(3-Et-PhO)Ph | 5 | PhN(Me) |
| 1 | 4-(3-BuO-PhO)Ph | 6 | 4-CF₃-PhNH |
| 5 | 3-(4-CF₃-PhO)Ph | 2 | 3-F₃CO-PhNH |
| 8 | MeO | 3 | 2,4-diCl-PhNH |
| 6 | PhO | 8 | 4-Et-PhNH |
| 2 | 4-F₃CO-PhO | 4 | 4-(4-F-PhO)PhNH |
| 4 | 3,5-diCl-PhO | 3 | 4-(4-CF₃-PhO)PhNH |

**TABLE 4**

| Compounds of Formula Ig wherein A=S and: | | | | | |
|---|---|---|---|---|---|
| n | T | W | n | T | W |
| 1 | O | H | 1 | O | 2,6-diEt-4-pyridyl |
| 4 | O | Et | 4 | O | MeC(=O) |
| 2 | O | Bzl | 2 | O | PhC(=O) |
| 3 | O | 4-Cl-Bzl | 1 | O | 3,5-diF-PhC(=O) |
| 5 | O | Ph | 3 | O | 3,5-MeO-PhC(=O) |
| 1 | O | 2,6-diF-Ph | 6 | O | 4-pyridyl-C(=O) |
| 6 | O | 4-F-Ph | 7 | O | 3,5-diCl-4-pyridyl-C(=O) |
| 7 | O | 3,5-diMe-Ph | 4 | O | BuOC(=O) |
| 8 | O | 4-pyridyl | 2 | O | PhOC(=O) |
| 5 | O | (3,5-diF-Ph)OC(=O) | 4 | NH | PhC(=O) |
| 3 | O | (4-EtO-Ph)OC(=O) | 5 | NH | (4-pyridyl)C(=O) |
| 6 | O | (4-pyridyl)OC(=O) | 6 | NH | MeOC(=O) |
| 2 | O | NHMeC(=O) | 3 | NH | PhOC(=O) |
| 5 | O | NHPhC(=O) | 1 | NH | (3-pyridyl)OC(=O) |
| 1 | O | NHpyridylC(=O) | 1 | NH | MeNHC(=O) |
| 4 | NH | Me | 2 | N-Me | Me |
| 1 | NH | Bzl | 3 | N-Me | Ph |
| 8 | NH | Ph | 4 | N-Ph | Ph |
| 3 | NH | 3, 5-diF-Ph | 5 | --- | H |
| 2 | NH | 4-pyridyl | 1 | --- | Me |
| 1 | NH | EtC(=O) | 2 | --- | Ph |

**TABLE 5**

| Compounds of Formula Ih wherein A=S and: | | | | | |
|---|---|---|---|---|---|
| n | m | R³⁵ | n | m | R³⁵ |
| 2 | 1 | MeS | 2 | 2 | 2-pyrimidyl |
| 3 | 1 | FCH₂CH₂O | 2 | 1 | *c*-hexyl |
| 1 | 1 | *c*-hexyloxy | 1 | 2 | *c*-pentyl |
| 1 | 1 | EtOC (=O) | 1 | 2 | EtO |
| 1 | 2 | OH | 1 | 1 | Ph |
| 3 | 1 | MeC(=O)O | 2 | 2 | PhO |
| 2 | 2 | PhOC(=O)O | 2 | 6 | H |
| 1 | 3 | MeNHC(=O)O | 2 | 8 | H |
| 2 | 1 | PhNH | 1 | 7 | EtS |
| 2 | 3 | (MeO)₂P(=O)O | 3 | 5 | CF₃O |
| 1 | 2 | MeSO₃ | 3 | 5 | *c*-hexyl |
| 3 | 2 | 4-pyridyl | 1 | 8 | Ph |
| 1 | 3 | 4-pyridyloxy | | | |

**TABLE 6**

| Compounds of Formula I wherein | |
|---|---|
| R¹ = Me, R² = 4-PhO-Ph, R³ = Ph, R⁴ = H, A = N-R¹⁵ and: | |
| R¹⁵ | R¹⁵ |
| H | Ph |
| H (HBr salt) | 4-Cl-Ph |
| H (HCl salt) | 3,5-diCl-Ph |
| H (HI salt) | 3-Br-Ph |
| H (H₂SO₄ salt) | 2-F-Ph |
| H (HNO₃ salt) | 4-I-Ph |
| H (CF₃CO₂H salt) | 3-NO₂-Ph |
| H (MeSO₃H salt) | 4-Me-Ph |
| H (PhSO₃H salt) | 4-Bu-Ph |
| H (4-TsOH salt) | 3-CF₃-Ph |
| H [(4-dodecyl-Ph)SO₃H salt] | 4-MeS-Ph |
| H (H₃PO₄ salt) | 4-Me-O-Ph |
| H (camphor sulfonic acid salt) | 4-BuO-Ph |
| Me | Bzl |
| Me (HBr salt) | Bzl (HBr salt) |
| Et | 4-Cl-Bzl |
| Pr | 2,4-diCl-Bzl |
| Bu | 4-Br-Bzl |
| octyl | 4-F-Bzl |
| MeOCH₂CH₂ | 4-I-Bzl |
| EtOCH₂CH₂ | 4-NO₂-Bzl |
| cyclopropyl | 4-Me-Bzl |
| cyclohexyl | 4-Bu-Bzl |
| allyl | 4-CF₃-Bzl |
| 2-octenyl | 4-MeS-Bzl |
| propargyl | 4-MeO-Bzl |
| 2-octynyl | |
| 4-BuO-Bzl | 6-F-2-pyridyl |
| Ph-(CH)Me | 4-I-2-pyridyl |
| Ph-(CH)Bu | 5-NO₂-2-pyridyl |
| 2-pyridyl | 6-Me-2-pyridyl |
| 3-pyridyl | 6-Bu-2-pyridyl |
| 4-pyridyl | 5-CF₃-3-pyridyl |
| 5-Cl-2-pyridyl | 2-MeS-3-pyridyl |
| 3,5-diCl-2-pyridyl | 2-MeO-3-pyridyl |
| 5-Br-2-pyridyl | 2-BuO-3-pyridyl |

**TABLE 7**

| Compounds of Formula I wherein | |
|---|---|
| R¹ = Me, R² = 4-PhO-Ph, R³ = Ph, R⁴ = H, A = N-C(=O)R¹⁶ and: | |
| R¹⁶ | R¹⁶ |
| H | Cl₂CH |
| Me | BrCH₂ |
| heptadecyl | F(CH₂)₂ |
| Et | ICH₂ |
| Pr | CF₃ |
| *i*-Pr | MeOCH₂ |
| Bu | CH₃(CH₂)₇OCH₂ |
| pentyl | CH₂=CHCH₂OCH₂ |
| Me₃C | MeSCH₂ |
| ClCH₂ | CH₃(CH₂)₇SCH₂ |
| ClCH₂C(Me)₂ | Bzl |
| Ph-(CH)Me | (4-BuO-Ph)OCH₂ |
| PhOCH₂ | 4-Cl-BzlO |
| PhO(CH)Me | 2,4-diCl-BzlO |
| BzlOCH₂ | 4-Br-BzlO |
| PhSCH₂ | 4-F-BzlO |
| 4-Cl-Bzl | 4-I-BzlO |
| 2,4-diCl-Bzl | 4-NO₂-BzlO |
| 4-Br-Bzl | 4-Me-BzlO |
| 4-F-Bzl | 4-Bu-BzlO |
| 4-I-Bzl | 4-CF₃-BzlO |
| 4-NO₂-Bzl | 4-MeS-BzlO |
| 4-Me-Bzl | 4-MeO-BzlO |
| 4-Bu-Bzl | 4-Bu-BzlO |
| 4-CF₃-Bzl | (4-Cl-Ph)SCH₂ |
| 4-MeS-Bzl | (2,4-diCl-Ph)SCH₂ |
| 4-MeO-Bzl | (4-Br-Ph)SCH₂ |
| 4-BuO-Bzl | (4-F-Ph)SCH₂ |
| (4-Cl-Ph)OCH₂ | (4-I-Pb)SCH₂ |
| (2,4-diCl-Ph)OCH₂ | (4-NO₂-Ph)SCH₂ |
| (4-Br-Ph)OCH₂ | (4-Me-Ph)SCH₂ |
| (4-F-Ph)OCH₂ | (4-Bu-Ph)SCH₂ |
| (4-I-Ph)OCH₂ | (4-CF₃-Ph)SCH₂ |
| (4-NO₂-Ph)OCH₂ | (4-MeS-Ph)SCH₂ |
| (4-Me-Ph)OCH₂ | (4-MeO-Ph)SCH₂ |
| (4-Bu-Ph)OCH₂ | (4-BuO-Ph)SCH₂ |
| (4-CF₃-Ph)OCH₂ | CH₃C(=O)CH₂ |
| (4-MeS-Ph)OCH₂ | MeOC(=O)CH₂ |
| (4-MeO-Ph)OCH₂ | EtOC(=O)CH₂ |
| BuOC(=O)CH₂ | 4-F-Ph |
| vinyl | 4-I-Ph |
| 1-propen-2-yl | 4-NO₂-Ph |
| 8-heptadecenyl | 4-CN-Ph |
| ClCH₂CH=CH | 4-Me-Ph |
| Cl₃CCH-CH | 4-Bu-Ph |
| BrCH₂CH=CH | 4-MeO-Ph |
| FCH₂CH=CH | 4-BuO-Ph |
| ICH₂CH=CH | 4-MeS-Ph |
| MeOCH₂CH=OH | 4-BuS-Ph |
| BuOCH₂CH=CH | 4-CF₃-Ph |
| 1-heptynyl | 1-naphthalenyl |
| cyclopropyl | 2-naphthalenyl |
| cyclopentyl | 2-furanyl |
| cyclohexyl | 3-furanyl |
| cycloheptyl | 2-thienyl |
| cyclooctyl | 3-thienyl |
| 1-cyclopentenyl | benzoyl |
| 1-cyclobexenyl | 2-pyridinyl |
| 2-Me-cyclopentyl | 3-pyridinyl |
| 2-Me-cyclohexyl | 4-pyridinyl |
| cyclopropylmethyl | 1-Cl-2-naphthalenyl |
| cyclopentylmethyl | 1,6-di-Cl-2-naphthalenyl |
| cyclohexylmethyl | 1-Br-2-naphthalenyl |
| Ph | 1-F-2-naphthalenyl |
| 4-Cl-Ph | 1-I-2-naphthalenyl |
| 3-Cl-Ph | 4-NO₂-2-naphthalenyl |
| 2-Cl-Ph | 1-Me-2-naphthalenyl |
| 2,4-diCl-Ph | 3-Bu-2-naphthalenyl |
| 4-Br-Ph | 3-CF₃-2-naphthalanyl |
| 1-MeS-2-naphthalenyl | 4-Cl-PhC(=O) |
| 1-MeO-2-naphthalenyl | 2,4-diCl-PhC(=O) |
| 1-BuO-2-naphthalenyl | 4-Br-PhC(=O) |
| 5-Cl-2-furanyl | 4-F-PhC(=O) |
| 4,5-diCl-2-furanyl | 4-I-PhC(=O) |
| 5-Br-2-furanyl | 3-NO₂-PhC(=O) |
| 5-F-2-furanyl | 4-Me-PhC(=O) |
| 5-I-2-furanyl | 4-Bu-PhC(=O) |
| 5-NO₂-2-furanyl | 4-CF₃-PhC(=O) |
| 5-Me-2-furanyl | 4-MeS-PhC(=O) |
| 5-Br-2-furanyl | 4-BuO-PhC(=O) |
| 5-CF₃-3-furanyl | 2-Cl-3-pyridyl |
| 5-MeS-2-furanyl | 6-Cl-3-pyridyl |
| 5-MeO-2-furanyl | 5-Br-3-pyridyl |
| 5-BuO-2-furanyl | 2-F-3-pyridyl |
| 5-Cl-2-thienyl | 6-I-3-pyridyl |
| 4,5-diCl-2-thienyl | 5-NO₂-3-pyridyl |
| 5-Br-2-thienyl | 6-Me-3-pyridyl |
| 5-r-2-thienyl | 6-Bu-3-pyridyl |
| 5-I-2-thienyl | 5-CF₃-2-pyridyl |
| 5-NO₂-2-thienyl | 2-MeS-3-pyridyl |
| 5-Me-2-thienyl | 2-MeO-3-pyridyl |
| 5-Bu-2-thienyl | 2-BuO-3-pyridyl |
| 5-CF₃-2-thienyl | MeOC(=O) |
| 5-MeS-2-thienyl | EtOC(=O) |
| 5-MeO-2-thienyl | BuOC(=O) |
| 5-BuO-2-thienyl | |

**TABLE 8**

| Compounds of Formula I wherein | |
|---|---|
| R¹ = Me, R² = 4-PhO-Ph, R³ = Ph, R⁴ = H, A = N-C(=O)OR¹⁷ and: | |
| R¹⁷ | R¹⁷ |
| Me | 4-Br-Bzl |
| octadecyl | 4-F-Bzl |
| Et | 4-I-Bzl |
| Pr | 4-NO₂-Bzl |
| *i*-Pr | 4-Me-Bzl |
| Bu | 4-Bu-Bzl |
| *i*-Bu | 4-CF₃-Bzl |
| *sec*-Bu | 4-MeS-Bzl |
| ClCH₂CH₂ | 4-MeO-Bzl |
| Cl₂CHCH₂ | 4-BuO-Bzl |
| Cl₃CCH₂ | 1-Cl-2-naphthalenylmethyl |
| BrCH₂CH₂ | 1,4-diCl-naphthalenylmethyl |
| F(CH₂)₃ | 1-Br-2-naphthalenylmethyl |
| CF₃CH₂ | 1-F-2-naphthalenylmethyl |
| ICH₂CH₂ | 1-I-2-naphthalanylmethyl |
| MeOCH₂CH₂ | 1-NO₂-2-naphthalenylmethyl |
| CH₂(CH₂)₁₁OCH₂CH₂ | 1-Me-2-naphthalenylmethyl |
| EtOCH₂CH₂ | 1-Bu-2-naphthalenylmethyl |
| MeSCH₂CH₂ | 1-CF₃-2-naphthalenylmethyl |
| CH₃(CH₂)₁₁SCH₂CH₂ | 1-MeS-2-naphthalenylmethyl |
| Bzyl | 1-MeO-2-naphthalenylmethyl |
| Ph-(CH)Me | 1-BuO-2-naphthalenylmethyl |
| 1-naphthalenylmethyl | (4-Cl-Ph)OCH₂CH₂ |
| 2-naphthalenylmethyl | (2,4-diCl-Ph)OCH₂CH₂ |
| 2-(2-naphthalenyl)ethyl | (4-F-Ph)OCH₂CH₂ |
| PhOCH₂CH₂ | (4-I-Ph)OCH₂CH₂ |
| 4-Cl-Bzl | (4-NO₂-Ph)OCH₂CH₂ |
| 2,4-diCl-Bzl | (4-Me-Ph)OCH₂CH₂ |
| (4-Bu-Ph)OCH₂CH₂ | 2-naphthalenyl |
| (4-CF₃-Ph)OCH₂CH₂ | 2-thienyl |
| (4-MeS-Ph)OCH₂CH₂ | 3-thienyl |
| (4-MeO-Ph)OCH₂CH₂ | 4-Cl-Ph |
| (4-BuO-Ph)OCH₂CH₂ | 2,4-diCl-Ph |
| CH₂=CH | 4-Br-Ph |
| CH₃(CH₂)₆CH=CHCH₂ | 4-F-Ph |
| CH₂=CHCH₂ | 4-I-Ph |
| ClCH₂CH=CHCH₂ | 4-NO₂-Ph |
| Cl₃CCH=CHCH₂ | 4-Me-Ph |
| BrCH₂CH=CHCH₂ | 4-Bu-Ph |
| FCH₂CH=CHCH₂ | 4-CF₃-Ph |
| ICH₂CH=CHCH₂ | 4-MeS-Ph |
| MeOCH₂CH=CHCH₂ | 4-MeO-Ph |
| BuOCH₂CH=CHCH₂ | 4-BuO-Ph |
| HC≡CCH₂ | 1-Cl-2-naphthalenyl |
| CH₃(CH₂)₄C≡CCH₂ | 1,3-diCl-2-naphthalenyl |
| cyclopropyl | 1-Br-2-naphthalenyl |
| cyclopentyl | 1-F-2-naphthalenyl |
| cyclohexyl | 1-I-2-naphthalenyl |
| cycloheptyl | 1-NO₂-2-naphthalenyl |
| cyclododecyl | 1-Me-2-naphthalenyl |
| 2-cyclopentenyl | 1-Bu-2-naphthalenyl |
| 2-cyclohexenyl | 1-CF₃-2-naphthalenyl |
| 2-Me-cyclopentyl | 1-MeS-2-naphthalenyl |
| 2-Me-cyclohexyl | 1-MeO-2-naphthalenyl |
| cyclopentylmethyl | 1-BuO-2-naphthalenyl |
| cyclohexylmethyl | 5-Cl-2-thienyl |
| 1-naphthalenyl | 4,5-diCl-thienyl |
| 5-Br-2-thienyl | 5-Bu-2-thienyl |
| 5-F-2-thienyl | 5-CF₃-2-thienyl |
| 5-I-2-thienyl | 5-MeS-2-thienyl |
| 5-NO₂-2-thienyl | 5-MeO-2-thienyl |
| 5-Me-2-thienyl | 5-BuO-2-thienyl |

**TABLE 9**

| Compounds of Formula I wherein | |
|---|---|
| R¹ = Me, R² = 4-PhO-Ph, R³ = Ph, R⁴ = H, A = N-C(=O)SR¹⁸ and: | |
| R¹⁸ | R¹⁸ |
| Me | CH₂(CH₂)₁₁OCH₂CH₂ |
| octadecyl | EtOCH₂CH₂ |
| Et | MeSCH₂CH₂ |
| Pr | CH₃(CH₂)₁₁SCH₂CH₂ |
| *i*-Pr | Bzyl |
| Bu | Ph-(CH)Me |
| *i*-Bu | 1-naphthalenylmethyl |
| *sec*-Bu | 2-naphthalenylmethyl |
| ClCH₂CH₂ | 2-(2-naphthalenyl)ethyl |
| Cl₂CHCH₂ | PhOCH₂CH₂ |
| Cl₃CCH₂ | 4-Cl-Bzl |
| BrCH₂CH₂ | 2,4-diCl-Bzl |
| F(CH₂)₃ | 4-Br-Bzl |
| CF₃CH₂ | 4-F-Bzl |
| ICH₂CH₂ | 4-I-Bzl |
| MeOCH₂CH₂ | 4-NO₂-Bzl |
| 4-Me-Bzl | CH₂=CH |
| 4-Bu-Bzl | CH₃(CH₂)₆CH=CHCH₂ |
| 4-CF₃-Bzl | CH₂=CHCH₂ |
| 4-MeS-Bzl | ClCH₂CH=CHCH₂ |
| 4-MeO-Bzl | Cl₃CCH=CHCH₂ |
| 4-BuO-Bzl | BrCH₂CH=CHCH₂ |
| 1-Cl-2-naphthalenylmethyl | FCH₂CH=CHCH₂ |
| 1,4-diCl-naphthalenylmethyl | ICH₂CH=CHCH₂ |
| 1-Br-2-naphthalenylmethyl | MeOCH₂CH=CHCH₂ |
| 1-F-2-naphthalenylmethyl | BuOCH₂CH=CHCH₂ |
| 1-I-2-naphthalenylmethyl | HC≡CCH₂ |
| 1-NO₂-2-naphthalenylmethyl | CH₃(CH₂)₄C≡CCH₂ |
| 1-Me-2-naphthalenylmethyl | cyclopropyl |
| 1-Bu-2-naphthalenylmethyl | cyclopentyl |
| 1-CF₃-2-naphthalenylmethyl | cyclohexyl |
| 1-MeS-2-naphthalenylmethyl | cycloheptyl |
| 1-MeO-2-naphthalenylmethyl | cyclododecyl |
| 1-BuO-2-naphthalenylmethyl | 2-cyclopentenyl |
| (4-Cl-Ph)OCH₂CH₂ | 2-cyclohexenyl |
| (2,4-diCl-Ph)OCH₂CH₂ | 2-Me-cyclopentyl |
| (4-F-Ph)OCH₂CH₂ | 2-Me-cyclohexyl |
| (4-I-Ph)OCH₂CH₂ | cyclopentylmethyl |
| (4-NO₂-Ph)OCH₂CH₂ | cyclohexylmethyl |
| (4-Me-Ph)OCH₂CH₂ | 1-naphthalenyl |
| (4-Bu-Ph)OCH₂CH₂ | 2-naphthalenyl |
| (4-CF₃-Ph)OCH₂CH₂ | 2-thienyl |
| (4-MeS-Ph)OCH₂CH₂ | 3-thienyl |
| (4-MeO-Ph)OCH₂CH₂ | 4-Cl-Ph |
| (4-BuO-Ph)OCH₂CH₂ | 2,4-diCl-Ph |
| 4-Br-Ph | 1-Bu-2-naphthalenyl |
| 4-F-Ph | 1-CF₃-2-naphthalenyl |
| 4-I-Ph | 1-MeS-2-naphthalenyl |
| 4-NO₂-Ph | 1-MeO-2-naphthalenyl |
| 4-Me-Ph | 1-BuO-2-naphthalenyl |
| 4-Bu-Ph | 5-Cl-2-thienyl |
| 4-CF₃-Ph | 4,5-diCl-thienyl |
| 4-MeS-Ph | 5-Br-2-thienyl |
| 4-MeO-Ph | 5-F-2-thienyl |
| 4-BuO-Ph | 5-I-2-thienyl |
| 1-Cl-2-naphthalenyl | 5-NO₂-2-thienyl |
| 1,3-diCl-2-naphthalenyl | 5-Me-2-thienyl |
| 1-Br-2-naphthalenyl | 5-Bu-2-thienyl |
| 1-F-2-naphthalenyl | 5-CF₃-2-thienyl |
| 1-I-2-naphthalenyl | 5-MeS-2-thienyl |
| 1-NO₂-2-naphthalenyl | 5-MeO-2-thienyl |
| 1-Me-2-naphthalenyl | 5-BuO-2-thienyl |

**TABLE 10**

| Compounds of Formula I wherein | | | |
|---|---|---|---|
| R¹ = Me, R² = 4-PhO-Ph, R³ = Ph, R⁴ = H, A = N-C(=O)NR¹⁹R²⁰ and: | | | |
| R¹⁹ | R²⁰ | R¹⁹ | R²⁰ |
| H | H | Bu | H |
| Me | H | decyl | H |
| Et | H | cyclopentyl | H |
| Pr | H | cyclohexyl | H |
| i-Pr | H | Ph | H |
| 4-Cl-Ph | H | Me | Me |
| 3,5-diCl-Ph | H | Me | Et |
| 4-Br-Ph | H | Et | Et |
| 2-F-Ph | H | Pr | Pr |
| 4-I-Ph | H | Bu | Bu |
| 4-NO₂-Ph | H | -CH₂CH₂CH₂CH₂- | |
| 4-Me-Ph | H | -CH₂CH₂CH₂CH₂CH₂- | |
| 4-Bu-Ph | H | -CH₂CH₂OCH₂CH₂- | |
| 4-CF₃-Ph | H | -CH₂(CH₂)₄CH₂- | |
| 4-MeS-Ph | H | -CH₂CH(Me)CH₂CH(Me)CH₂- | |
| 4-MeO-Ph | H | -CH₂CH(Me)OCH(Me)CH₂- | |
| 4-BuO-Ph | H | | |

**TABLE 11**

| Compounds of Formula I wherein: | | | |
|---|---|---|---|
| R¹ = Me, R² = 4-PhO-Ph, R³ = Ph, R⁴ = H, A = N-P(=O)LM and: | | | |
| L | M | L | M |
| Me | Me | Pr | Pr |
| Me | Et | Bu | Bu |
| Et | Et | | |

**TABLE 12**

| Compounds of Formula I wherein | |
|---|---|
| R¹ = Me, R² = 4-PhO-Ph, R³ = Ph, R⁴ = H, A = N-OG and: | |
| G | G |
| H | CH₃(CH₂)₃C(=O) |
| H (HBr salt) | MeOC(=O) |
| Me | EtOC(=O) |
| Me (HBr salt) | PrOC(=O) |
| hexyl | BuOC(=O) |
| Bzl | *i*-BuOC(=O) |
| 4-Cl-Bzl | MeNHC(=O) |
| 2,4-diCl-Bzl | BuNHC(=O) |
| 4-Br-Bzl | CH₃(CH₂)₅NHC(=O) |
| 4-F-Bzl | Ph-NH(C=O) |
| 4-I-Bzl | (4-Cl-Ph)NH(C=O) |
| 4-NO₂-Bzl | (3,5-diCl-Ph)NH(C=O) |
| 4-Me-Bzl | (4-Br-Ph)NH(C=O) |
| 4-Bu-Bzl | (2-F-Ph)NHC(=O) |
| 4-CF₃-Bzl | (2-I-Ph)NHC(=O) |
| 4-MeS-Bzl | (4-NO₂-Ph)NHC(=O) |
| 4-MeO-Bzl | (4-Me-Ph)NHC(=O) |
| 4-BuO-Bzl | (4-Bu-Ph)NHC(=O) |
| MeC(=O) | (4-CF₃-Ph)NHC(=O) |
| MeCH₂C(=O) | (4-MeS-Ph)NHC(=O) |
| MeC(CH₂)₂C(=O) | (4-MeO-Ph)NHC(=O) |
| CH₃CH(CH₃)C(=O) | (4-BuO-Ph)NHC(=O) |

**TABLE 13**

| Compounds of Formula I wherein | |
|---|---|
| R¹ = Me, R² = 2,4-di-F-Ph, R³ = Ph, R⁴ = H, A = N-J and: | |
| J | J |
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | CH₂-CHCH₂OC(=O) |
| EtC(=O) | MeSC(=O) |
| Me₃CC(=O) | EtSC(=O) |
| BrCH₂C(=O) | H₂NC(=O) |
| CH₂=CH-C(=O) | MeNHC(=O) |
| CH₂=C(CH₃)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | CH₃C(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | Me₂P (=O) |

**TABLE 14**

| Compounds of Formula I wherein | |
|---|---|
| R¹ = Me, R² = octyl, R³ = Ph, R⁴ = H, A = N-J and: | |
| J | J |
| H | EtOC(=O)C(=O) |
| H (HBr salt) | MeOC(-O) |
| Me | EtC(=O) |
| Bzl | PrC(=O) |
| HC(=O) | BuC(=O) |
| MeC(=O) | CH₂=CHCH₂OC(=O) |
| EtC(=O) | MeSC(=O) |
| Me₃CC(=O) | EtSC(=O) |
| BrCH₂C(=O) | H₂NC(=O) |
| CH₂=CH-C(=O) | MeNHC(=O) |
| CH₂-C(CH₃)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | CH₃C(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | Me₂P(=O) |

**TABLE 15**

| Compounds of Formula I wherein | |
|---|---|
| | |

| R¹ = Me, R² = 2-F-6-PhO-3-pyridinyl, R³ = Ph, R⁴ = H, A = N-J and: | |
|---|---|
| J | J |
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | CH₂=CHCH₂OC(=O) |
| EtC(=O) | MeSC(=O) |
| Me₃CC(=O) | EtSC(=O) |
| BrCH₂C(=O) | H₂NC(=O) |
| CH₂=CH-C(=O) | MeNHC(=O) |
| CH₂=C(CH₃)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | CH₃C(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | Me₂P(=O) |

**TABLE 16**

| Compounds of Formula I wherein | |
|---|---|
| R¹ = Me, R² = 4-(benzoyloxy)Ph, R³ = Ph, R⁴ = H, A = N-J and: | |
| J | J |
| H | EtOC(=O)C(=O) |
| H (HBr salt) | MeOC(=O) |
| Me | EtC(=O) |
| Bzl | PrC(=O) |
| HC(=O) | BuC(=O) |
| MeC(=O) | CH₂=CHCH₂OC (=O) |
| EtC(=O) | MeSC(=O) |
| Me₃CC(=O) | EtSC(=O) |
| BrCH₂C(=O) | H₂NC(=O) |
| CH₂=CH-C(=O) | MeNHC(=O) |
| CH₂=C(CH₃)-C(=O) | PhNHC(=O) |
| PhC(=O) | (4-Cl-Ph)NHC(=O) |
| 4-Cl-PhC(=O) | (3,5-diCl-Ph)NHC(=O) |
| 2-naphthoyl | (4-Me-Ph)NHC(=O) |
| 2-furoyl | HO |
| 2-thienyl | MeO |
| PhC(=O)C(=O) | CH₃C(=O)O |
| 3-pyridinyl-C(=O) | MeOC(=O)O |
| 4-pyridinyl-C(=O) | MeNHC(=O)O |
| MeOC(=O)C(=O) | PhNHC(=O)O |
| | Me₂P(=O) |

**TABLE 17**

| Compounds of Formula I wherein | |
|---|---|
| R² = 2,4-di-F-Ph, R³ = Ph, R⁴ = H, A = N-H and: | |
| R¹ | R¹ |
| Me | Et |
| Bu | CF₃ |
| CF₃(CH₂)₃ | 4-Br-Bzl |
| allyl | 4-I-Bzl |
| cyclopropyl | 4-NO₂-Bzl |
| cyclohexyl | 4-Me-Bzl |
| vinyl | 4-Bu-Bzl |
| CH₃CH=CHCH₂ | 4-CF₃-Bzl |
| MeO(C=O) | 4-MeS-Bzl |
| PrO(C=O) | 4-MeO-Bzl |
| Bzl | 4-BuO-Bzl |
| 4-Cl-Bzl | Ph-(CH)-Me |
| 2,4-diCl-Bzl | Ph-(CH)-Bu |

**TABLE 18**

| Compounds of Formula I wherein | |
|---|---|
| R¹ = Me, R² = 2,4-di-F-Ph, R⁴ = H, A = N-H and: | |
| _{R}3 | _{R}3 |
| 2-pyridyl | 4-F-Ph |
| 3-pyridyl | 4-I-Ph |
| 4-pyridyl | 4-NO₂-Ph |
| 2-pyrimidinyl | 4-NC-Ph |
| 4-pyrimidinyl | 4-Me-Ph |
| 5-pyrimidinyl | 4-Bu-Ph |
| 4-Cl-Ph | 4-CF₃-Ph |
| 3,4-diCl-Ph | 4-MeO-Ph |
| 4-Br-Ph | 4-BuO-Ph |
| 4-MeS-Ph | 3-Cl-2-pyrimidinyl |
| 4-BuS-Ph | 3,5-diCl-2-pyrimidinyl |
| 4-CF₃O-Ph | 3-Br-2-pyrimidinyl |
| 5-Cl-2-pyridyl | 3-F-2-pyrimidinyl |
| 3,5-diCl-2-pyridyl | 3-I-2-pyrimidinyl |
| 5-Br-2-pyridyl | 3-NO₂-2-pyrimidinyl |
| 5-F-2-pyridyl | 3-NC-2-pyrimidinyl |
| 5-I-2-pyridyl | 3-Me-2-pyrimidinyl |
| 5-NO₂-2-pyridyl | 3,5-di-Me-2-pyrimidinyl |
| 5-CN-2-pyridyl | 3-Bu-2-pyrimidinyl |
| 6-Me-2-pyridyl | 3-CF₃-2-pyrimidinyl |
| 6-Bu-2-pyridyl | 3-MeO-2-pyrimidinyl |
| 5-CF₃-2-pyridyl | 3,5-di-MeO-2-pyrimidinyl |
| 5-MeO-2-pyridyl | 3-BuO-2-pyrimidinyl |
| 5-BuO-2-pyridyl | 3-MeS-2-pyrimidinyl |
| 5-MeS-2-pyridyl | 3-BuS-2-pyrimidinyl |
| 5-BuS-2-pyridyl | 3-CF₃O-2-pyrimidinyl |
| 5-CF₃O-2-pyridyl | Bzl |

**TABLE 19**

| Compounds of Formula I wherein | |
|---|---|
| R¹ = Me, R³ = Ph, R⁴ = Me, A = N-H and: | |
| R² | R² |
| Ph | octyl |
| 4-PhO-Ph | 2-F-6-PhO-3-pyridinyl |
| 2,4-di-F-Ph | 4-[Ph(C=O)O]Ph |

**TABLE 20**

| Compounds of Formula Ia wherein | |
|---|---|
| R¹ = Me, R³ = Ph, R⁴ = H and: | |
| R² | R² |
| Et | 2-(MeS(O))Et |
| heptyl | 2-(EtSO₂)Et |
| octyl | EtOC(=O)(CH₂)₄ |
| decyl | 3-hydroxypropyl |
| undecyl | HO₂C(CH₂)₇ |
| eicosanyl | (MeO)₂P(=O)OCH₂(CH₂)₃CH₂ |
| 3-Me-Bu | (BuO)₂P(=S)OCH₂(CH₂)₃CH₂ |
| 3-Me-hexyl | 8-(MeSO₃)octyl |
| 2-Me-pentyl | 5-(BuSO₃)pentyl |
| 9, 9, 9-triF-nonyl | (PhSO₃)CH₂CH₂ |
| 2,4-diCl-octyl | 3-(4-F-PhSO₃)propyl |
| 4-(*c*-propyl) Bu | (4-Et-PhSO₃)Bu(CH₂)₄ |
| 8-(*c*-hexyl)octyl | (Me₃N⁺)(CH₂)₄ I⁻ |
| 6-Ph-octyl | (H₃N⁺)(CH₂) ₄ Cl⁻ |
| 4-(3-CF₃-Ph)Bu | MeC(=O)NMe(CH₂)₄ |
| 8-(4-F₃CO-Ph)octyl | 3-(PhSO₂)propyl |
| 10-(2,4-diF-Ph)decyl | (2,6-diMe-PhSO₂)(CH₂)₄ |
| 4-(4-Cl-Ph)Bu | 6-(2-EtO-PbSO₂)hexyl |
| 5-(4-Et-Ph)pentyl | 8-(PhS(O))octyl |
| 8-(3-propyloxy-Ph)octyl | 7-(4-F-PhS(O))heptyl |
| 6-(4-(4-F-Pho)Ph)hexyl | (3-Me-PhS(O))CH₂CH₂ |
| 7-(3-(4-Et-PhO)Ph)heptyl | 5-(3-BuO-PhS(O))pentyl |
| 10-(4-(3-MeS-PhO)Ph)decyl | (4-pyridyl)CH₂ |
| 2-CN-propyl | Me₂N(CH₂)₆ |
| 3-NO₂-propyl | 8-(2,6-diMe-4-pyridyl)octyl |
| 2-MeS-hexyl | 10-(6-Cl-3-pyridyl)decyl |
| 6-(4-pyridyloxy)hexyl | 3-Ph-2-propenyl |
| 3-(2,6-diCl-4-pyridyloxy) propyl | 4-PhO-2-butenyl |
| (4-MeO-2-pyridyloxy)(CH₂)₄ | (Z)-(4-PhO-2-butenyl) |
| 3-(2-thienyl)propyl | 7-octynyl |
| 6-(3-Me-2-thienyl)hexyl | 2-hydroxy-4-butynyl |
| 8-(2-pyrimidinyl)octyl | 2-EtO-6-heptynyl |
| 7-(4-MeO-2-pyrimidinyl)heptyl | 2,4-diF-7-octynyl |
| 10-(2-furanyl)decyl | 3-*c*-hexenyl |
| (5-Et-2-furanyl)(CH₂)₄ | 3-*c*-pentenyl |
| 3-(1-naphthalenyl)propyl | (CH₂CH₂OCH₂CH₂)CH |
| 6-(6-F-2-naphthalenyl)hexyl | (CH₂CH₂SCH₂CH₂)CH |
| 5-(2-pyrimidinyloxy)pentyl | (CH₂CH₂SO₂CH₂CH₂)CH |
| 8-(1-naphthalenylozy)octyl | PhOCH₂CH₂CH₂ |
| 3-(2-tetrahydropyranyl)propyl | PhO(CH₂)₄ |
| 8-(2-tetrahydropyranyloxy) octyl | PhO(CH₂)₅ |
| 2-octenyl | PhO(CH₂)₈ |
| 2-decenyl | (3-CF₃-PhO)(CH₂)₃ |
| 4-octenyl | (3,5-diCl-PhO)(CH₂)₆ |
| 3-EtS-2-propenyl | (2-F-PhO)(CH₂)₃ |
| 4-*c*-hexylozy-2-butenyl | (2-Me-PhO)(CH₂)₃ |
| 5-*c*-pentyloxy-3-pentenyl | (3,5-diMeO-PhO)(CH₂)₈ |
| 6-hydroxy-4-hexenyl | (4-(4-F-PhO)PhO)(CH₂)₃ |
| 3-(MeC(=O)O)-2-propenyl | (3-(3,5-diMe-PhO)PhO)(CH₂)₆ |
| 3-(4-pyridyl)-2-propenyl | PhS(CH₂)₃ |
| 5-EtO-2-pentenyl | (3-CF₃-PhS)(CH₂)₇ |
| 3-CF₃-2-propenyl | (3-F₃CO-PhS)(CH₂)₆ |
| (2,4-diF-PhS)(CH₂)₅ | 3-Cl-Ph |
| (4-Et-PhS) (CH₂)₅ | 3-F-Ph |
| (2-EtO-PhS)(CH₂)₁₀ | 4-Br-Ph |
| (3-(4-Cl-PhO)PhS)(CH₂)₇ | 3,4-diCl-Ph |
| (3-(2,4-diMe-PhO)PhS)(CH₂)₃ | 2,3-diF-Ph |
| (4-(4-EtO-PhO)PhS)CH₂)₇ | Ph |
| (3-(2-MeS-PhO)PhS)(CH₂)₃ | 2,5-diF-Ph |
| FCH₂CH₂O(CH₂)₆ | 3,5-diF-Ph |
| (*c*-hexyloxy)(CH₂)₆ | 2,6-diF-Ph |
| (*c*-pentyloxy)(CH₂)₈ | 3,4-diF-Ph |
| (CH₂=CHCH₂O)(CH₂)₇ | 5-Cl-2-F-Ph |
| CH≡CCH₂OCH₂CH₂ | 2-F-5-Me-Ph |
| CH(=O)CH₂CH₂ | 2,5-diF-4-Me-Ph |
| MeC(=O)CH₂CH₂CH₂ | 2-F-Ph |
| BuC(=O)(CH₂)₇ | 4-Me-Ph |
| MeC(=NMe)CH₂ | 4-NO₂-Ph |
| MeC(=NOMe)CH₂CH₂CH₂ | 4-hexyl-Ph |
| MeC(=NNHPh)CH₂CH₂CH₂ | 4-(cyclohexyl)Ph |
| hexyl | 4-CF₃-Ph |
| cyclopentyl | 4-MeS-Ph |
| cyclohexyl | 4-(hexylthio)Ph |
| vinyl | 3-MeO-Ph |
| 3-hexenyl | 4-(pentyloxy)Ph |
| 2-cyclohexenyl | 4-CF₃O-Ph |
| 4-F-Ph | 4-(cyclohexyloxy)Ph |
| 4-Cl-Ph | 4-(BuOCH₂)Ph |
| 4-MeOCH₂-Ph | 4-(4-MeOC(=O)-PhO)Ph |
| 4-(2-propenyl)Ph | 4-(4-allyl-PhO)Ph |
| 4-(3-pentenyl)Ph | 4-(4-propargyl-PhO)Ph |
| 4-(Cl₂C=CHCH₂)Ph | 4-((3-PhO)PhO)Ph |
| 4-(2-propenyloxy)Ph | 4-(3-(2-Cl-PhO)PhO)Ph |
| 4-(2-hexenyloxy)Ph | 4-(3-(4-Me-PhO)PhO)Ph |
| 4-(propargyl)Ph | 4-(3-(3-MeO-PhO)PhO)Ph |
| 4-(2-butynyloxy)Ph | 4-PhCH=CH-Ph |
| 4-BuSO₂-Ph | 4-(3-Br-Ph)CH=CH-Ph |
| 4-PhO-Ph | 4-(2-F-Ph)CH=CH-Ph |
| 4-(4-Cl-PhO)Ph | 4-(3-Cl-Ph)CH=CH-Ph |
| 4-(3-Cl-PhO)Ph | 4-(2,4-diF-Ph)CH=CH-Ph |
| 4-(2,4-diF-PhO)Ph | 4-(4-NO₂-Ph)CH=CH-Ph |
| 4-(4-NO₂-PhO)Ph | 4-(3-Me-Ph)CH=CH-Ph |
| 4-(4-CN-PhO)Ph | 4-(3-Bu-Ph)CH=CH-Ph |
| 4-(4-Me-PhO)Ph | 4-(4-CF₃-Ph)CH=CH-Ph |
| 4-(3-hexyl-PhO)Ph | 4-(2-MeS-Ph)CH=CH-Ph |
| 4-(3-cyclohexyl-Pho)Ph | 4-(4-MeO-Ph)CH=CH-Ph |
| 4-(3-CF₃-Pho)Ph | 4-(4-BuO-Ph)CH=CH-Ph |
| 4-(4-MeS-PhO)Ph | 4-PhCH₂CH₂-Ph |
| 4-(4-MeSO₂-PhO)Ph | 4-(3-Br-Ph)CH₂CH₂-Ph |
| 4-(3-BuSO₂-PhO)Ph | 4-(2-F-Ph)CH₂CH₂-Ph |
| 4-(3-MeO-PhO)Ph | 4-(3-Cl-Ph)CH₂CH₂-Ph |
| 4-(3-hexyloxy-PhO)Ph | 4-(2,4-diF-Ph)CH₂CH₂-Ph |
| 4-(4-CF₃O-PhO)Ph | 4-(4-NO₂-Ph)CH₂CH₂-Ph |
| 4-(4-cyclohexyloxy-PhO)Ph | 4-(3-Me-Ph)CH₂CH₂-Ph |
| 4-(3-Bu-Ph)CH₂CH₂-Ph | 4-(2-MeS-Ph)CH₂-Ph |
| 4-(4-CF₃-Ph)CH₂CH₂-Ph | 4-(4-MeO-Ph)CH₂-Ph |
| 4-(2-MeS-Ph)CH₂CH₂-Ph | 4-(4-BuO-Ph)CH₂-Ph |
| 4-(4-MeO-Ph)CH₂CH₂-Ph | 4-PhS-Ph |
| 4-(4-BuO-Ph)CH₂CH₂-Ph | 4-(3-Br-Ph)S-Ph |
| 4-PhOCH₂-Ph | 4-(2-F-Ph)S-Ph |
| 4-(3-Br-Ph)OCH₂-Ph | 4-(3-Cl-Ph)S-Ph |
| 4-(2-F-Ph)OCH₂-Ph | 4-(2,4-diF-Ph)S-Ph |
| 4-(3-Cl-Ph)OCH₂-Ph | 4-(4-NO₂-Ph)S-Ph |
| 4-(2,4-diF-Ph)OCH₂-Ph | 4-(3-Me-Ph)S-Ph |
| 4-(4-NO₂-Ph)OCH₂-Ph | 4-(3-Bu-Ph)S-Ph |
| 4-(3-Me-Ph)OCH₂-Ph | 4-(4-CF₃-Ph)S-Ph |
| 4-(3-Bu-Ph)OCH₂-Ph | 4-(2-MeS-Ph)S-Ph |
| 4-(4-CF₃-Ph)OCH₂-Ph | 4-(4-MeO-Ph)S-Ph |
| 4-(2-MeS-Ph)OCH₂-Ph | 4-(4-BuO-Ph)S-Ph |
| 4-(4-MeO-Ph)OCH₂-Ph | 4-Ph-Ph |
| 4-(4-BuO-Ph)OCH₂-Ph | 4-(3-Br-Ph)Ph |
| 4-PhCH₂-Ph | 4-(2-F-Ph)Ph |
| 4-(3-Br-Ph)CH₂-Ph | 4-(3-Cl-Ph)Ph |
| 4-(2-F-Ph)CH₂-Ph | 4-(2,4-diF-Ph)Ph |
| 4-(3-Cl-Ph)CH₂-Ph | 4-(4-NO₂-Ph)Ph |
| 4-(2,4-diF-Ph)CH₂-Ph | 4-(3-Me-Ph)Ph |
| 4-(4-NO₂-Ph)CH₂-Ph | 4-(3-Bu-Ph)Ph |
| 4-(3-Me-Ph)CH₂-Ph | 4-(4-CF₃-Ph)Ph |
| 4-(3-Bu-Ph)CH₂-Ph | 4-(2-MeS-Ph)Ph |
| 4-(4-CF₃-Ph)CH₂-Ph | 4-(4-MeO-Ph)Ph |
| 4-(4-BuO-Ph)Ph | 4-[octyl-NHC(=O)O]Ph |
| 4-PhCH₂O-Ph | 4-[PhNHC(=O)O]Ph |
| 4-(3-Br-Ph)CH₂O-Ph | 4-[(3-Br-Ph)NHC(=O)O]Ph |
| 4-(2-F-Ph)CH₂O-Ph | 4-[(2-F-Ph)NHC(=O)O]Ph |
| 4-(3-Cl-Ph)CH₂O-Ph | 4-[(3-Cl-Ph)NHC(=O)O]Ph |
| 4-(2,4-diF-Ph)CH₂O-Ph | 4-[(2,4-diF-Ph)NHC(=O)O]Ph |
| 4-(4-NO₂-Ph)CH₂O-Ph | 4-[(4-NO₂-Ph)NHC(=O)O]Ph |
| 4-(3-Me-Ph)CH₂O-Ph | 4-[(3-Me-Ph)NHC(=O)O]Ph |
| 4-(3-Bu-Ph)CH₂O-Ph | 4-[(3-Bu-Ph)NHC(=O)O]Ph |
| 4-(4-CF₃-Ph)CH₂O-Ph | 4-[(4-CF₃-Ph)NHC(=O)O]Ph |
| 4-(2-MeS-Ph)CH₂O-Ph | 4-[(2-MeS-Ph)NHC(=O)O]Ph |
| 4-(4-MeO-Ph)CH₂O-Ph | 4-[(4-MeO-Ph)NHC(=O)O]Ph |
| 4-(4-BuO-Ph)CH₂O-Ph | 4-[(4-BuO-Ph)NHC(=O)O]Ph |
| 4-(4-CN-Ph)CH₂O-Ph | 4-[(4-CN-Ph)NHC(=O)O]Ph |
| 4-(3-PhO-Ph)CH₂O-Ph | 4-[(3-PhO-Ph)NHC(=O)O]Ph |
| 4-[3-(3-F-PhO)Ph]CH₂O-Ph | 4-[3-(3-F-PhO)Ph-NHC(=O)O]Ph |
| 4-[4-(2-NO₂-PhO)Ph]CH₂O-Ph | 4-[4-(2-NO₂-PhO)Ph-NHC(=O)O]Ph |
| 4-[3-(4-Me-PhO)Ph]CH₂O-Ph | 4-[2-(4-Me-PhO)Ph-NHC(=O)O]Ph |
| 4-[4-(4-Bu-PhO)Ph]CH₂O-Ph | 4-[4-(4-Bu-PhO)Ph-NHC(=O)O]Ph |
| 4-[3-(3-CF₃-PhO)Ph]CH₂O-Ph | 4-(4-(3-CF₃-PhO)Ph-NNC(=O)O]Ph |
| 4-[2-(3-MeS-PhO)Ph]CH₂O-Ph | 4-(4-(3-MeS-PhO)Ph-NHC(=O)O]Ph |
| 4-[4-(4-MeO-PhO)Ph]CH₂O-Ph | 4-[3-(4-MeO-PhO)Ph-NHC(=O)O]Ph |
| 4-[3-(3-BuO-PhO)Ph]CH₂O-Ph | 4-[3-(3-BuO-PhO)Ph-NHC(=O)O]Ph |
| 4-(2-F-4-MeO-Ph)CH₂O-Ph | 4-[(2-F-4-MeO-Ph)NHC(=O)O]Ph |
| 3-(2-CF₃-4-Et-Ph)CH₂O-Ph | 3-[(2-CF₃-4-Et-Ph)NHC(=O)O]Ph |
| 4-[MeNHC(=O)O]Ph | 4-[(3-pyridyl)NHC(=O)O]Ph |
| 4-[(3-Br-2-pyridyl)NHC(=O)O]Ph | 4-[3-Br-PhO-C(=O)]Ph |
| 4-[(2-F-3-pyridyl)NHC(=O)O]Ph | 4-[2-F-PhO-C(=O)]Ph |
| 4-[(3-Cl-4-pyridyl)NHC(=O)O]Ph | 4-[3-Cl-PhO-C(=O)]Ph |
| 4-[(2,4-diF-3-pyridyl)NHC(=O)O]Ph | 4-[2,4-diF-PhO-C(=O)]Ph |
| 4-[(4-NO₂-2-pyridyl)NHC(=O)O]Ph | 4-[4-NO₂-PhO-(=O)]Ph |
| 4-[(3-Me-2-pyridyl)NHC(=O)O]Ph | 4-[3-Me-PhO-C(=O)]Ph |
| 4-[(3-Bu-2-pyridyl)NHC(=O)O]Ph | 4-[3-Bu-PhO-C(=O)]Ph |
| 4-[(4-CF₃-3-pyridyl)NNC(=O)O]Ph | 4-[4-CF₃-PhO-C(=O)]Ph |
| 4-[(2-MeS-4-pyridyl)NHC(=O)O]Ph | 4-[2-MeS-PhO-C(=O)]Ph |
| 4-[(4-MeO-3-pyridyl)NHC(=O)O]Ph | 4-[4-MeO-PhO-C(=O)]Ph |
| 4-[(4-BuO-3-pyridyl)NHC(=O)O]Ph | 4-[4-BuO-PhO-C(=O)]Ph |
| 4-[(4-CN-2-pyridyl)NHC(=O)O]Ph | 4-[4-CN-PhO-C(=O)]Ph |
| 4-[(3-PhO-2-pyridyl)NHC(=O)O]Ph | 4-[3-PhO-PhO-C(=O)]Ph |
| 4-[3-(3-F-PhO)-2-pyridyl-NHC(=O)O]Ph | 4-[3-(3-F-PhO)PhO-C(=O)]Ph |
| 4-[4-(2-NO₂-PhO)-3-pyridyl-NHC(=O)O]Ph | 4-[4-(2-NO₂-PhO)PhO-C(=O)]Ph |
| 4-[2-(4-Me-PhO)-4-pyridyl-NBC(=O)O]Ph | 4-[2-(4-Me-PhO)PhO-C(=O)]Ph |
| 4-(4-(4-Bu-PhO)-3-pyridyl-NHC(=O)O]Ph | 4-[4-(4-Bu-PhO)PhO-C(=O)]Ph |
| 4-[4-(3-CF₃-PhO)-2-pyridyl-NHC(=O)O]Ph | 4-[4-(3-CF₃-PhO)PhO-C(=O)]Ph |
| 4-[4-(3-MeS-PhO)-3-pyridyl-NHC(=O)O]Ph | 4-[4-(3-MeS-PhO)PhO-C(=O)]Ph |
| 4-[3-(4-MeO-PhO)-2-pyridyl-NHC(=O)O]Ph | 4-[3-(4-MeO-PhO)PhO-C(=O)]Ph |
| 4-[3-(3-BuO-PhO)-2-pyridyl-NHC(=O)O]Ph | 4-[3-(3-BuO-PhO)PhO-C(=O)]Ph |
| 4-[(2-F-4-MeO-3-pyridyl)NHC(=O)O]Ph | 4-[(2-F-4-MeO-PhO-C(=O)]Ph |
| 3-[(2-CF₃-4-Et-3-pyridyl)NHC(=O)O]Ph | 3-[(2-CF₃-4-Et-PhO-C(=O)]Ph |
| 4-[MeO-C(=O)]Ph | 4-[(3-pyridyloxy)C(=O)]Ph |
| 4-[octyloxy-C(=O)]Ph | 4-[(3-Br-2-pyridyloxy)C(=O)]Ph |
| 4-[PhO-C(=O)]Ph | 4-[(2-F-3-pyridyloxy)C(=O)]Ph |
| 4-[(3-Cl-4-pyridyloxy)C(=O)]Ph | 4-[3-Cl-PhC(=O)O]Ph |
| 4-[(2,4-diF-3-pyridyloxy)C(=O)]Ph | 4-[2,4-diF-PhC(=O)O]Ph |
| 4-[(4-NO₂-2-pyridyloxy)C(=O)]Ph | 4-[4-NO₂-Ph(=O)O]Ph |
| 4-[(3-Me-2-pyridyloxy)O(=O)]Pb | 4-(3-Me-PhC(=O)O]Ph |
| 4-[(3-Bu-2-pyridyloxy)C(=O)]Ph | 4-[3-Bu-PhC(=O)O]Ph |
| 4-[(4-CF₃-3-pyridyloxy)C(=O)]Ph | 4-[4-CF₃-PhC(=O)O]Ph |
| 4-[(2-MeS-4-pyridylosy)C(=O)]Ph | 4-[2-MeS-PhC(=O)O]Ph |
| 4-[(4-MeO-3-pyridyloxy)C(=O)]Ph | 4-[4-MeO-PhC(=O)O]Ph |
| 4-[(4-BuO-3-pyridyloxy)C(=O)]Ph | 4-[4-BuO-PhC(=O)O]Ph |
| 4-[(4-CN-2-pyridyloxy)C(=O)]Ph | 4-[4-CN-PhC(=O)O]Ph |
| 4-[(3-PhO-2-pyridyloxy)C(=O)]Ph | 4-[3-PhO-PhC(=O)O]Ph |
| 4-[3-(3-F-PhO)-2-pyridyloxy-C(=O)]Ph | 4-[3-(3-F-PhO)PhC(=O)O]Ph |
| 4-[4-(2-NO₂-PhO)-3-pyridyloxy-C(=O)]Ph | 4-[4-(2-NO₂-PhO)PhC(=O)O]Ph |
| 4-[2-(4-Me-PhO)-4-pyridyloxy-C(=O)]Ph | 4-[2-(4-Me-PhO)PhC(=O)O]Ph |
| 4-[4-(4-Bu-PhO)-3-pyridyloxy-C(=O)]Ph | 4-[4-(4-Bu-PhO)PhC(=O)O]Ph |
| 4-[4-(3-CF₃-PhO)-2-pyridyloxy-C(=O)]Ph | 4-[4-(3-CF₃-PhO)PhC(=O)O]Ph |
| 4-[4-(3-MeS-PhO)-3-pyridyloxy-C(=O)]Ph | 4-[4-(3-MeS-PhO)PhC(=O)O]Ph |
| 4-[3-(4-MeO-PhO)-2-pyridyloxy-C(=O)]Ph | 4-[3-(4-MeO-PhO)PhC(=O)O]Ph |
| 4-[3-(3-BuO-PhO)-2-pyridyloxy-C(=O)]Ph | 4-[3-(3-BuO-PhO)PhC(=O)O]Ph |
| 4-[(2-F-4-MeO-3-pyridyloxy)C(=O)]Ph | 4-[(2-F-4-MeO-Ph)C(=O)O]Ph |
| 3-[(2-CF₃-4-Et-3-pyridyloxy)C(=O)]Ph | 3-[(2-CF₃-4-Et-Ph)C(=O)O]Ph |
| 4-[MeC(=O)O]Ph | 4-[(3-pyridyl)C(=O)O]Ph |
| 4-[octyl-C(=O)O]Ph | 4-[(3-Br-2-pyridyl)C(=O)O]Ph |
| 4-[PhC(=O)O]Ph | 4-[(2-F-3-pyridyl)C(=O)O]Ph |
| 4-[3-Br-PhC(=O)O]Ph | 4-[(3-Cl-4-pyridyl)C(=O)O]Ph |
| 4-[2-F-PhC(=O)O]Ph | 4-[(2,4-diF-3-pyridyl)C(=O)O]Ph |
| 4-[(4-NO₂-2-pyridyl)C(=O)O]Ph | 2-F-4-[octyl-NHC(=O)O]Ph |
| 4-[(3-Me-2-pyridyl)C(=O)O]Ph | 4-F-3-thienyl |
| 4-[(3-Bu-2-pyridyl)C(=O)O]Ph | 4-Cl-2-thienyl |
| 4-[(4-CF₃-3-pyridyl)C(=O)O]Ph | 5-Cl-2-thienyl |
| 4-[(2-MeS-4-pyridyl)C(=O)O]Ph | 3-F-2-thienyl |
| 4-[(4-MeO-3-pyridyl)C(=O)O]Ph | 4-Br-2-thienyl |
| 4-[(4-BuO-3-pyridyl)C(=O)O]Ph | 3,4-diCl-2-thienyl |
| 4-[(4-CN-2-pyridyl)C(=O)O]Ph | 2,4-diF-3-thienyl |
| 4-[(3-PhO-2-pyridyl)C(=O)O]Ph | 2-thienyl |
| 4-[3-(3-F-PhO)-2-pyridyl-C(=O)O]Ph | 3-thienyl |
| 4-[4-(2-NO₂-PhO)-3-pyridyl-C(=O)O]Ph | 2,5-diF-3-thienyl |
| 4-[2-(4-Me-PhO)-4-pyridyl-C(=O)O]Ph | 3,5-diF-2-thienyl |
| 4-[4-(4-Bu-PhO)-3-pyridyl-C(=O)O]Ph | 3,4-diF-2-thienyl |
| 4-[4-(3-CF₃-PhO)-2-pyridyl-C(=O)O]Ph | 5-Cl-2-F-3-thienyl |
| 4-[4-(3-MeS-PhO)-3-pyridyl-C(=O)O]Ph | 2-F-5-Me-3-thienyl |
| 4-[3-(4-MeO-PhO)-2-pyridyl-C(=O)O]Ph | 2,5-diF-4-Me-3-thienyl |
| 4-[3-(3-BuO-PhO)-2-pyridyl-C(=O)O]Ph | 2-F-3-thienyl |
| 4-[(2-F-4-MeO-3-pyridyl)C(=O)O]Ph | 4-Me-2-thienyl |
| 3-[(2-CF₃-4-Et-3-pyridyl)C(=O)O]Ph | 4-NO₂-2-thienyl |
| 2-F-4-PhCH₂CH₂-Ph | 4-hexyl-2-thienyl |
| 3-Cl-4-(2-F-Ph)OCH₂-Ph | 5-cyclohexyl-3-thienyl |
| 3-Me-4-(4-MeO-Ph)CH₂-Ph | 5-CF₃-3-thienyl |
| 2-F-4-PhS-Ph | 4-MeS-2-thienyl |
| 3-BuO-4-Ph-Ph | 4-hexylthio-2-thienyl |
| 3-MeS-4-PhO-Ph | 5-MeO-3-thienyl |
| 2-F-4-PhCH₂O-Ph | 4-pentyloxy-2-thienyl |
| 4-CF₃O-2-thienyl | 5-(3-hexyloxy-PhO)-3-thienyl |
| 5-cyclohexyloxy-3-thienyl | 5-(4-CF₃O-PhO)-3-thienyl |
| 4-BuOCH₂-2-thienyl | 5-(4-cyclohexyloxy-PhO)-3-thienyl |
| 4-MeOCH₂-2-thienyl | 4-(4-MeOC(=O)-PhO)-2-thienyl |
| 5-(2-propenyl)-3-thienyl | 4-(4-allyl-PhO)-2-thienyl |
| 5-(3-pentenyl)-3-thienyl | 4-(4-propargyl-PhO)-2-thienyl |
| 4-(Cl₂C=CHCH₂)-2-thienyl | 5-((3-PhO) PhO)-3-thienyl |
| 4-(2-propenyloxy)-2-thienyl | 5-(3-(2-Cl-PhO)PhO)-3-thienyl |
| 4-(2-hexenyloxy)-2-thienyl | 5-(3-(4-Me-PhO)PhO)-3-thienyl |
| 5-(propargyl)-3-thienyl | 5-(3-(3-MeO-PhO)PhO)-3-thienyl |
| 4-(2-butynyloxy)-2-thienyl | 4-PhCH=CH-2-thienyl |
| 5-BuSO₂-3-thienyl | 4-(3-Br-Ph)CH=CH-2-thienyl |
| 5-PhO-3-thienyl | 4-(2-F-Ph)CH=CH-2-thienyl |
| 4-(4-Cl-PhO)-2-thienyl | 4-(3-Cl-Ph)CH=CH-2-thienyl |
| 4-(3-Cl-PhO)-2-thienyl | 5-(2,4-diF-Ph)CH=CH-3-thienyl |
| 5-(2,4-diF-PhO)-3-thienyl | 5-(4-NO₂-Ph)CH=CH-3-thienyl |
| 5-(4-NO₂-PhO)-3-thienyl | 5-(3-Me-Ph)CH=CH-3-thienyl |
| 5-(4-CN-PhO)-3-thienyl | 5-(3-Bu-Ph)CH=CH-3-thienyl |
| 5-(4-Me-PhO)-3-thienyl | 4-(4-CF₃-Ph)CH=CH-2-thienyl |
| 4-(3-hexyl-PhO)-2-thienyl | 4-(2-MeS-Ph)CH=CH-2-thienyl |
| 4-(3-cyclohexyl-PhO)-2-thienyl | 4-(4-MeO-Ph)CH=CH-2-thienyl |
| 4-(3-CF₃-PhO)-2-thienyl | 4-(4-BuO-Ph)CH=CH-2-thienyl |
| 4-(4-MeS-PhO)-2-thienyl | 5-PhCH₂CH₂-3-thienyl |
| 5-(4-MeSO₂-PhO)-3-thienyl | 5-(3-Br-Ph)CH₂CH₂-3-thienyl |
| 5-(3-BuSO₂-PhO)-3-thienyl | 4-(2-F-Ph)CH₂CH₂-2-thienyl |
| 5-(3-MeO-PhO)-3-thienyl | 4-(3-Cl-Ph)CH₂CH₂-2-thienyl |
| 4-(2,4-diF-Ph)CH₂CH₂-2-thienyl | 5-(3-Me-Ph)CH₂-3-thienylPh |
| 5-(4-NO₂-Ph)CH₂CH₂-3-thienyl | 4-(3-Bu-Ph)CH₂-2-thienyl |
| 5-(3-Me-Ph)CH₂CH₂-3-thienyl | 4-(4-CF₃-Ph)CH₂-2-thienyl |
| 4-(3-Bu-Ph)CH₂CH₂-2-thienyl | 5-(2-MeS-Ph)CH₂-3-thienyl |
| 5-(4-CF₃-Ph)CH₂CH₂-3-thienyl | 4-(4-MeO-Ph)CH₂-2-thienyl |
| 4-(2-MeS-Ph)CH₂CH₂-2-thienyl | 4-(4-BuO-Ph)CH₂-2-thienyl |
| 5-(4-MeO-Ph)CH₂CH₂-3-thienyl | 5-PhS-3-thienyl |
| 4-(4-BuO-Ph)CH₂CH₂-2-thienyl | 4-(3-Br-Ph)S-2-thienyl |
| 4-PhOCH₂-2-thienyl | 5-(2-F-Ph)S-3-thienyl |
| 5-(3-Br-Ph)OCH₂-3-thienyl | 4-(3-Cl-Ph)S-2-thienyl |
| 5-(2-F-Ph)OCH₂-3-thienyl | 5-(2,4-diF-Ph)S-3-thienyl |
| 4-(3-Cl-Ph)OCH₂-2-thienyl | 4-(4-NO₂-Ph)S-2-thienyl |
| 5-(2,4-diF-Ph)OCH₂-3-thienyl | 5-(3-Me-Ph)S-3-thienyl |
| 5-(4-NO₂-Ph)OCH₂-3-thienyl | 4-(3-Bu-Ph)S-2-thienyl |
| 4-(3-Me-Ph)OCH₂-2-thienyl | 5-(4-CF₃-Ph)S-3-thienyl |
| 5-(3-Bu-Ph)OCH₂-3-thienyl | 4-(2-MeS-Ph)S-2-thienyl |
| 5-(4-CF₃-Ph)OCH₂-3-thienyl | 5-(4-MeO-Ph)S-3-thienyl |
| 4-(2-MeS-Ph)OCH₂-2-thienyl | 5-(4-BuO-Ph)S-3-thienyl |
| 5-(4-MeO-Ph)OCH₂-3-thienyl | 4-Ph-2-thienyl |
| 4-(4-BuO-Pb)OCH₂-2-thienyl | 4-(3-Br-Ph)-2-thienyl |
| 5-PhCH₂-3-thienyl | 4-(2-F-Ph)-2-thienyl |
| 5-(3-Br-Ph)CH₂-3-thienyl | 5-(3-Cl-Ph)-3-thienyl |
| 5-(2-F-Ph)CH₂-3-thienyl | 5-(2,4-dir-Ph)-3-thienyl |
| 5-(3-Cl-Ph)CH₂-3-thienyl | 5-(4-NO₂-Ph)-3-thienyl |
| 4-(2,4-diF-Ph)CH₂-2-thienyl | 4-(3-Me-Ph)-2-thienyl |
| 4-(4-NO₂-Ph)CH₂-2-thienyl | 5-(3-Bu-Ph)-3-thienyl |
| 4-(4-CF₃-Ph)-2-thienyl | |
| 5-(2-MeS-Ph)-3-thienyl | |
| 5-(4-MeO-Ph)-3-thienyl | |
| 4-(4-BuO-Ph)-2-thienyl | |
| 5-PhCH₂O-3-thienyl | |
| 4-(3-Br-Ph)CH₂O-2-thienyl | |
| 5-(2-F-Ph)CH₂O-3-thienyl | |
| 5-(3-Cl-Ph)CH₂O-3-thienyl | |
| 5-(2,4-diF-Ph)CH₂O-3-thienyl | |
| 4-(4-NO₂-Ph)CH₂O-2-thienyl | |
| 5-(3-Me-Ph)CH₂O-3-thienyl | |
| 4-(3-Bu-Ph)CH₂O-2-thienyl | |
| 4-(4-CF₃-Ph)CH₂O-2-thienyl | |
| 5-(2-MeS-Ph)CH₂O-3-thienyl | |
| 4-(4-MeO-Ph)CH₂O-2-thienyl | |
| 5-(4-BuO-Ph)CH₂O-3-thienyl | |
| 4-(4-CN-Ph)CH₂O-2-thienyl | |
| 5-(3-PhO-Ph)CH₂O-3-thienyl | |
| 5-[3-(3-F-PhO)Ph]CH₂O-3-thienyl | |
| 4-[4-(2-NO₂-PhO)Ph]CH₂O-2-thienyl | |
| 4-[3-(4-Me-PhO)Ph]CH₂O-2-thienyl | |
| 4-[4-(4-Bu-PhO)Ph]CH₂O-2-thienyl | |
| 5-[3-(3-CF₃-PhO)Ph]CH₂O-3-thienyl | |
| 5-[2-(3-MeS-PhO)Ph]CH₂O-3-thienyl | |
| 4-[4-(4-MeO-PhO)Ph]CH₂O-2-thienyl | |
| 5-[3-(3-BuO-PhO)Ph]CH₂O-3-thienyl | |

| R² | |
|---|---|
| 4-(2-F-4-MeO-Ph)CH₂O-2-thienyl | |
| 5-(2-CF₃-4-Et-Ph)CH₂O-3-thienyl | |
| 4-[MeNHC(=O)O]-2-thienyl | |
| 4-[octyl-NHC(=O)O]-2-thienyl | |
| 5-[PhNHC(=O)O]-3-thienyl | |
| 5-[(3-Br-Ph)NHC(=O)O]-3-thienyl | |
| 4-((2-F-Ph)NHC(=O)O]-2-thienyl | |
| 5-[(3-Cl-Ph)NHC(=O)O]-3-thienyl | |
| 5-[(2,4-diF-Ph)NHC(=O)O]-3-thienyl | |
| 5-[(4-NO₂-Ph)NHC(=O)O]-3-thienyl | |
| 4-[(3-Me-Ph)NHC(=O)O]-2-thienyl | |
| 5-[(3-Bu-Ph)NHC(=O)O]-3-thienyl | |
| 5-[(4-CF₃-Ph)NHC(=O)O]-3-thienyl | |
| 4-[(2-MeS-Ph)NHC(=O)O]-2-thienyl | |
| 4-[(4-MeO-Ph)NHC(=O)O]-2-thienyl | |
| 5-[(4-BuO-Ph)NHC(=O)O]-3-thienyl | |
| 5-(4-CN-Ph)NHC(=O)O]-3-thienyl | |
| 4-[(3-PhO-Ph)NHC(=O)O]-2-thienyl | |
| 4-[3-(3-r-PhO)Ph-NHC(=O)O]-2-thienyl | |
| 4-[4-(2-NO₂-PhO)Ph-NHC(=O)O]-2-thienyl | |
| 4-[2-(4-Me-PhO)Ph-NHC(=O)O]-2-thienyl | |
| 5-(4-(4-Bu-PhO)Ph-NHC(=O)O]-3-thienyl | |
| 5-(4-(3-CF₃-PhO)Ph-NHC(=O)O]-3-thienyl | |
| 5-(4-(3-MeS-PhO)Ph-NHC(=O)O]-3-thienyl | |
| 4-[3-(4-MeO-PhO)Ph-NHC(=O)O]-2-thienyl | |
| 5-[3-(3-BuO-PhO)Ph-NHC(=O)O]-3-thienyl | |
| 4-[(2-F-4-MeO-Ph)NHC(=O)O]-2-thienyl | |
| 5-[(2-CF₃-4-Et-Ph)NHC(=O)O]-3-thienyl | |
| 5-[(3-pyridyl)NHC(=O)O]-3-thienyl | |
| 4-[(3-Br-2-pyridyl)NHC(=O)O]-2-thienyl | |
| 5-[(2-F-3-pyridyl)NHC(=O)O]-3-thienyl | |
| 5-[(3-Cl-4-pyridyl)NHC(=O)O]-3-thienyl | |
| 5-[(2,4-diF-3-pyridyl)NHC(=O)O]-3-thienyl | |
| 4-[(4-NO₂-2-pyridyl)NHC(=O)O]-2-thienyl | |
| 4-[(3-Me-2-pyridyl)NHO(=O)O]-2-thienyl | |
| 5-[(3-Bu-2-pyridyl)NHC(=O)O]-3-thienyl | |
| 5-[(4-CF₃-3-pyridyl)NHC(=O)O]-3-thienyl | |
| 4-[(2-MeS-4-pyridyl)NHC(=O)O]-2-thienyl | |
| 5-[(4-MeO-3-pyridyl)NHC(=O)O]-3-thienyl | |
| 4-[(4-BuO-3-pyridyl)NHC(=O)O]-2-thienyl | |
| 5-[(4-CN-2-pyridyl)NHC(=O)O]-3-thienyl | |
| 4-[(3-PhO-2-pyridyl)NHC(=O)O]-2-thienyl | |
| 4-[3-(3-F-PhO)-2-pyridyl-NHC(=O)O]-2-thienyl | |
| 5-[4-(2-NO₂-PhO)-3-pyridyl-NHC(=O)O]-3-thienyl | |
| 4-[2-(4-Me-PhO)-4-pyridyl-NHC(=O)O]-2-thienyl | |
| 5-[4-(4-Bu-PhO)-3-pyridyl-NHC(=O)O]-3-thienyl | |
| 4-[4-(3-CF₃-PhO)-2-pyridyl-NHC(=O)O]-2-thienyl | |
| 5-[4-(3-MeS-PhO)-3-pyridyl-NHC(=O)O]-3-thienyl | |
| 4-[3-(4-MeO-PhO)-2-pyridyl-NHC(=O)O]-2-thienyl | |
| 5-[3-(3-BuO-PhO)-2-pyridyl-NHC(=O)O]-3-thienyl | |
| 5-[(2-F-4-MeO-3-pyridyl)NHC(=O)O]-3-thienyl | |
| 4-[(2-CF₃-4-Et-3-pyridyl)NHC(=O)O]-2-thienyl | |
| 4-[MeO-C(=O)]-2-thienyl | |
| 4-[octyloxy-C(=O)]-2-thienyl | |
| 5-[PhO-C(=O)]-3-thienyl | |
| 5-[3-Br-PhO-C(=O)]-3-thienyl | |
| 4-[2-F-PhO-C(=O)]-2-thienyl | |
| 4-[3-Cl-PhO-C(=O)]-2-thienyl | |
| 5-[2,4-diF-PhO-C(=O)]-3-thienyl | |
| 5-[4-NO₂-PhO-(=O)]-3-thienyl | |
| 4-[3-Me-PhO-C(=O)]-2-thienyl | |
| 4-[3-Bu-PhO-C(=O)]-2-thienyl | |
| 5-(4-CF₃-PhO-C(=O)]-3-thienyl | |
| 5-[2-MeS-PhO-C(=O)]-3-thienyl | |
| 4-[4-MeO-PhO-C(=O)]-2-thienyl | |
| 4-[4-BuO-PhO-C(=O)]-2-thienyl | |
| 5-[4-CN-PhO-C(=O)]-3-thienyl | |
| 5-[3-PhO-PhO-C(=O)]-3-thienyl | |
| 4-[3-(3-F-PhO)PhO-C(=O)]-2-thienyl | |
| 5-[4-(2-NO₂-PhO)PhO-C(=O)]-3-thienyl | |
| 4-[2-(4-Me-PhO)PhO-C(=O)]-2-thienyl | |
| 5-[4-(4-Bu-PhO)PhO-C(=O)]-3-thienyl | |
| 4-(4-(3-CF₃-PhO)PhO-C(=O)]-2-thienyl | |
| 5-(4-(3-MeS-PhO)PhO-C(=O)]-3-thienyl | |
| 4-[3-(4-MeO-PhO)PhO-C(=O)]-2-thienyl | |
| 5-[3-(3-BuO-PhO)PhO-C(=O)]-3-thienyl | |
| 4-[(2-F-4-MeO-PhO-C(=O)]-2-thienyl | |
| 5-[(2-CF₃-4-Et-PhO-C(=O)]-3-thienyl | |
| 5-[(3-pyridyloxy)C(=O)]-3-thienyl | |
| 4-[(3-Br-2-pyridyloxy)C(=O)]-2-thienyl | |
| 5-[(2-F-3-pyridyloxy)C(=O)]-3-thienyl | |
| 5-[(3-Cl-4-pyridyloxy)C(=O)]-3-thienyl | |
| 4-[(2,4-diF-3-pyridyloxy)C(=O)]-2-thienyl | |
| 4-[(4-NO₂-2-pyridyloxy)C(=O)]-2-thienyl | |
| 5-[(3-Me-2-pyridyloxy)C(=O)]-3-thienyl | |
| 5-[(3-Bu-2-pyridyloxy)C(=O)]-3-thienyl | |
| 5-[(4-CF₃-3-pyridyloxy)C(=O)]-3-thienyl | |
| 5-[(2-MeS-4-pyridyloxy)C(=O)]-3-thienyl | |
| 5-[(4-MeO-3-pyridyloxy)C(=O)]-3-thienyl | |
| 4-[(4-BuO-3-pyridyloxy)C(=O)]-2-thienyl | |
| 4-[(4-CN-2-pyridyloxy)C(=O)]-2-thienyl | |
| 4-[(3-PhO-2-pyridyloxy)C(=O)]-2-thienyl | |
| 5-[3-(3-F-PhO)-2-pyridyloxy-C(=O)]-3-thienyl | |
| 5-[4-(2-NO₂-PhO)-3-pyridyloxy-C(=O)]-3-thienyl | |
| 5-[2-(4-Me-PhO)-4-pyridyloxy-C(=O)]-3-thienyl | |
| 5-[4-(4-Bu-PhO)-3-pyridyloxy-C(=O)]-3-thienyl | |
| 4-[4-(3-CF₃-PhO)-2-pyridyloxy-C(=O)]-2-thienyl | |
| 4-[4-(3-MeS-PhO)-3-pyridyloxy-C(=O)]-2-thienyl | |
| 4-[3-(4-MeO-PhO)-2-pyridyloxy-C(=O)]-2-thienylPh | |
| 5-[3-(3-BuO-PhO)-2-pyridyloxy-C(=O)]-3-thienyl | |
| 4-[(2-F-4-MeO-3-pyridyloxy)C(=O)]-2-thienyl | |
| 4-[(2-CF₃-4-Et-3-pyridyloxy)C(=O)]-2-thienyl | |
| 4-[MeC(=O)O]-2-thienyl | |
| 4-[octyl-C(=O)O]-2-thienyl | |
| 5-[PhC(=O)O]-3-thienyl | |
| 4-[3-Br-PhC(=O)O]-2-thienyl | |
| 5-[2-F-PhC(=O)O]-3-thienyl | |
| 5-[3-Cl-PhC(=O)O]-3-thienyl | |
| 4-[2,4-diF-PhC(=O)O]-2-thienyl | |
| 5-[4-NO₂-Ph(=O)O]-3-thienyl | |
| 4-[3-Me-PhC(=O)O]-2-thienyl | |
| 5-[3-Bu-PhC(=O)O]-3-thienyl | |
| 4-[4-CF₃-PhC(=O)O]-2-thienyl | |
| 4-[2-MeS-PhC(=O)O]-2-thienyl | |
| 5-[4-MeC-PhC(=O)O]-3-thienyl | |
| 5-[4-BuO-PhC(=O)O]-3-thienyl | |
| 4-[4-CN-PhC(=O)O]-2-thienyl | |
| 4-[3-PhO-PhC(=O)O]-2-thienyl | |
| 5-[3-(3-F-PhO)PhC(=O)O]-3-thienyl | |
| 4-[4-(2-NO₂-PhO)PhC(=O)O] -2-thienyl | |
| 5-[2-(4-Me-PhO)PhC(=O)O]-3-thienyl | |
| 4-[4-(4-Bu-PhO)PhC(=O)O]-2-thienyl | |
| 5-[4-(3-CF₃-PhO)PhC(=O)O]-3-thienyl | |
| 4-[4-(3-MeS-PhO)PhC(=O)O]-2-thienyl | |
| 5-[3-(4-MeO-PhO)PhC(=O)O]-3-thienyl | |
| 5-[3-(3-BuO-PhO)PhC(=O)O]-3-thienyl | |
| 4-[(2-F-4-MeO-Ph)C(=O)O]-2-thienyl | |
| 5-[(2-CF₃-4-Et-Ph)C(=O)O]-3-thienyl | |
| 5-[(3-pyridyl)C(=O)O]-3-thienyl | |
| 4-[(3-Br-2-pyridyl)C(=O)O]-2-thienyl | |
| 4-[(2-F-3-pyridyl)C(=O)O]-2-thienyl | |
| 5-[(3-Cl-4-pyridyl)C(=O)O]-3-thienyl | |
| 5-[(2,4-diF-3-pyridyl)C(=O)O]-3-thienyl | |
| 5-[(4-NO₂-2-pyridyl)C(=O)O]-3-thienyl | |
| 5-[(3-Me-2-pyridyl)C(=O)O]-3-thienyl | |
| 5-[(3-Bu-2-pyridyl)C(=O)O]-3-thienyl | |
| 4-[(4-CF₃-3-pyridyl)C(=O)O]-2-thienyl | |
| 5-[(2-MeS-4-pyridyl)C(=O)O]-3-thienyl | |
| 5-[(4-MeO-3-pyridyl)C(=O)O]-3-thienyl | |
| 4-[(4-BuO-3-pyridyl)C(=O)O]-2-thienyl | |
| 4-[(4-CN-2-pyridyl)C(=O)O]-2-thienyl | |
| 5-[(3-PhO-2-pyridyl)C(=O)O]-3-thienyl | |
| 5-[3-(3-F-PhO)-2-pyridyl-C(=O)O]-3-thienyl | |
| 5-[4-(2-NO₂-PhO)-3-pyridyl-C(=O)O]-3-thienyl | |
| 4-[2-(4-Me-PhO)-4-pyridyl-C(=O)O]-2-thienyl | |
| 5-[4-(4-Bu-PhO)-3-pyridyl-C(=O)O]-3-thienyl | |
| 5-[4-(3-CF₃-PhO)-2-pyridyl-C(=O)O]-3-thienyl | |
| 5-[4-(3-MeS-PhO)-3-pyridyl-C(=O)O]-3-thienyl | |
| 4-[3-(4-MeO-PhO)-2-pyridyl-C(=O)O]-2-thienyl | |
| 4-[3-(3-BuO-PhO)-2-pyridyl-C(=O)O]-2-thienyl | |
| 5-[(2-F-4-MeO-3-pyridyl)C(=O)O]-3-thienyl | |
| 4-[(2-CF₃-4-Et-3-pyridyl)C(=O)O]-2-thienyl | |
| 3-F-4-PhCH₂CH₂-2-thienyl | |
| 3-F-4-(2-F-Ph)OCH₂-2-thienyl | |
| 4-F-5-(4-MeO-Ph)CH₂-3-thienyl | |
| 2-F-5-PhS-3-thienyl | |

| R² | R² |
|---|---|
| 5-MeS-4-PhO-2-thienyl | 6-pentyloxy-3-pyridyl |
| 2-F-5-PhCH₂O-3-thienyl | 6-CF₃O-3-pyridyl |
| 2-F-5-[octyl-NHC(=O)O]-3-thienyl | 6-cyclohexyloxy-3-pyridyl |
| 4-F-3-pyridyl | 6-(BuOCH₂)-3-pyridyl |
| 4-Cl-3-pyridyl | 5-MeOCH₂-2-pyridyl |
| 3-F-2-pyridyl | 6-(2-propenyl)-3-pyridyl |
| 4-Br-3-pyridyl | 5-(3-pentenyl)-2-pyridyl |
| 2,3-diF-4-pyridyl | 4-(Cl₂C=CHCH₂)-2-pyridyl |
| 2-pyridyl | 4-(2-propenyloxy)-2-pyridyl |
| 3-pyridyl | 6-(2-hexenyloxy)-3-pyridyl |
| 4-pyridyl | 6-(propargyl)-3-pyridyl |
| 2,5-diF-3-pyridyl | 2-(2-butynyloxy)-4-pyridyl |
| 3,5-diF-4-pyridyl | 4-BuSO₂-2-pyridyl |
| 2,6-diF-3-pyridyl | 6-PhO-3-pyridyl |
| 3,4-diF-2-pyridyl | 6-(4-Cl-PhO)-3-pyridyl |
| 5-Cl-2-F-3-pyridyl | 6-(3-Cl-PhO)-3-pyridyl |
| 2-F-5-Me-3-pyridyl | 5-(2,4-diF-PhO)-2-pyridyl |
| 2-F-4-pyridyl | 5-(4-NO₂-PhO)-3-pyridyl |
| 4-Me-2-pyridyl | 2-(4-CN-PhO)-4-pyridyl |
| 4-NO₂-2-pyridyl | 5-(4-Me-PhO)-2-pyridyl |
| 5-hexyl-3-pyridyl | 6-(3-hexyl-PhO)-3-pyridyl |
| 5-(cyclohexyl)-3-pyridyl | 6-(3-cyclohexyl-PhO)-3-pyridyl |
| 5-CF₃-3-pyridyl | 4-(3-CF₃-PhO)-2-pyridyl |
| 2-MeS-4-pyridyl | 4-(4-MeS-PhO)-2-pyridyl |
| 4-hexylthio-2-pyridyl | 5-(4-MeSO₂-PhO)-2-pyridyl |
| 5-MeO-4-pyridyl | 6-(3-BuSO₂-PhO)-3-pyridyl |
| 6-(3-MeO-PhO)-3-pyridyl | 2-(3-Cl-Ph)CH₂CH₂-4-pyridyl |
| 6-(3-hexyloxy-PhO)-3-pyridyl | 5-(2,4-diF-Ph)CH₂CH₂-3-pyridyl |
| 6-(4-CF₃O-PhO)-3-pyridyl | 6-(4-NO₂-Ph)CH₂CH₂-3-pyridyl |
| 5-(4-cyclohexyloxy-PhO)-2- | 4-(3-Me-Ph)CH₂CH₂-2-pyridyl |
| pyridyl | 5-(3-Bu-Ph)CH₂CH₂-2-pyridyl |
| 5-(4-MeOC(=O)-PhO)-2-pyridyl | 6-(4-CF₃-Ph)CH₂CH₂-3-pyridyl |
| 5-(4-allyl-PhO)-3-pyridyl | 6-(2-MeS-Ph)CH₂CH₂-3-pyridyl |
| 6-(4-propargyl-PhO)-3-pyridyl | 5-(4-MeO-Ph)CH₂CH₂-2-pyridyl |
| 6-((3-PhO)PhO)-3-pyridyl | 6-(4-BuO-Ph)CH₂CH₂-3-pyridyl |
| 2-(3-(2-Cl-PhO)PhO)-4-pyridyl | 6-PhOCH₂-3-pyridyl |
| 4-(3-(4-Me-PhO)PhO)-2-pyridyl | 6-(3-Br-Ph)OCH₂-3-pyridyl |
| 5-(3-(3-MeO-PhO)PhO)-2-pyridyl | 5-(2-F-Ph)OCH₂-2-pyridyl |
| 6-PhCH=CH-3-pyridyl | 2-(3-Cl-Ph)OCH₂-4-pyridyl |
| 6-(3-Br-Ph)CH=CH-3-pyridyl | 5-(2,4-diF-Ph)OCH₂-3-pyridyl |
| 5-(2-F-Ph)CH=CH-2-pyridyl | 6-(4-NO₂-Ph)OCH₂-3-pyridyl |
| 2-(3-Cl-Ph)CH=CH-4-pyridyl | 4-(3-Me-Ph)OCH₂-2-pyridyl |
| 5-(2,4-diF-Ph)CH=CH-3-pyridyl | 5-(3-Bu-Ph)OCH₂-2-pyridyl |
| 6-(4-NO₂-Ph)CH=CH-3-pyridyl | 6-(4-CF₃-Ph)OCH₂-3-pyridyl |
| 4-(3-Me-Ph)CH=CH-2-pyridyl | 6-(2-MeS-Ph)OCH₂-3-pyridyl |
| 5-(3-Bu-Ph)CH-CH-2-pyridyl | 5-(4-MeO-Ph)OCH₂-2-pyridyl |
| 6-(4-CF₃-Ph)CH=CH-3-pyridyl | 6-(4-BuO-Ph)OCH₂-3-pyridyl |
| 6-(2-MeS-Ph)CH=CH-3-pyridyl | 6-PhCH₂-3-pyridyl |
| 5-(4-MeO-Ph)CH=CH-2-pyridyl | 6-(3-Br-Ph)CH₂-3-pyridyl |
| 6-(4-BuO-Ph)CH=CH-3-pyridyl | 5-(2-F-Ph)CH₂-2-pyridyl |
| 6-PhCH₂CH₂-3-pyridyl | 2-(3-Cl-Ph)CH₂-4-pyridyl |
| 6-(3-Br-Ph)CH₂CH₂-3-pyridyl | 5-(2,4-diF-Ph)CH₂-3-pyridyl |
| 5-(2-F-Ph)CH₂CH₂-2-pyridyl | 6-(4-NO₂-Ph)CH₂-3-pyridyl |

| R² | |
|---|---|
| 4-(3-Me-Ph)CH₂-2-pyridyl | |
| 5-(3-Bu-Ph)CH₂-2-pyridyl | |
| 6-(4-CF₃-Ph)CH₂-3-pyridyl | |
| 6-(2-MeS-Ph)CH₂-3-pyridyl | |
| 5-(4-MeO-Ph)CH₂-2-pyridyl | |
| 6-(4-BuO-Ph)CH₂-3-pyridyl | |
| 6-PhS-3-pyridyl | |
| 6-(3-Br-Ph)S-3-pyridyl | |
| 5-(2-F-Ph)S-2-pyridyl | |
| 2-(3-Cl-Ph)S-4-pyridyl | |
| 5-(2,4-diF-Ph)S-3-pyridyl | |
| 6-(4-NO₂-Ph)S-3-pyridyl | |
| 4-(3-Me-Ph)S-2-pyridyl | |
| 5-(3-Bu-Ph)S-2-pyridyl | |
| 6-(4-CF₃-Ph)S-3-pyridyl | |
| 6-(2-MeS-Ph)S-3-pyridyl | |
| 5-(4-MeO-Ph)S-2-pyridyl | |
| 6-(4-BuO-Ph)S-3-pyridyl | |
| 6-Ph-3-pyridyl | |
| 6-(3-Br-Ph)-3-pyridyl | |
| 5-(2-F-Ph)-2-pyridyl | |
| 2-(3-Cl-Ph)-4-pyridyl | |
| 5-(2,4-diF-Ph)-3-pyridyl | |
| 6-(4-NO₂-Ph)-3-pyridyl | |
| 4-(3-Me-Ph)-2-pyridyl | |
| 5-(3-Bu-Ph)-2-pyridyl | |
| 6-(4-CF₃-Ph)-3-pyridyl | |
| 6-(2-MeS-Ph)-3-pyridyl | |
| 5-(4-MeO-Ph)-2-pyridyl | |
| 6-(4-BuO-Ph)-3-pyridyl | |
| 6-PhCH₂O-3-pyridyl | |
| 5-(3-Br-Ph)CH₂O-2-pyridyl | |
| 6-(2-F-Ph)CH₂O-3-pyridyl | |
| 4-(3-Cl-Ph)CH₂O-2-pyridyl | |
| 2-(2,4-diF-Ph)CH₂O-4-pyridyl | |
| 6-(4-NO₂-Ph)CH₂O-3-pyridyl | |
| 6-(3-Me-Ph)CH₂O-3-pyridyl | |
| 5-(3-Bu-Ph)CH₂O-2-pyridyl | |
| 2-(4-CF₃-Ph)CH₂O-4-pyridyl | |
| 5-(2-MeS-Ph)CH₂O-3-pyridyl | |
| 5-(4-MeO-Ph)CH₂O-2-pyridyl | |
| 6-(4-BuO-Ph)CH₂O-3-pyridyl | |
| 5-(4-CN-Ph)CH₂O-2-pyridyl | |
| 6-(3-PhO-Ph)CH₂O-3-pyridyl | |
| 6-[3-(3-F-PhO)Ph]CH₂O-3-pyridyl | |
| 6-[4-(2-NO₂-PhO)Ph]CH₂O-3-pyridyl | |
| 5-[3-(4-Me-PhO)Ph]CH₂O-2-pyridyl | |
| 5-[4-(4-Bu-PhO)Ph]CH₂O-2-pyridyl | |
| 6-[3-(3-CF₃-PhO)Ph]CH₂O-3-pyridyl | |
| 6-[2-(3-MeS-PhO)Ph]CH₂O-3-pyridyl | |
| 6-[4-(4-MeO-PhO)Ph]CH₂O-3-pyridyl | |
| 5-[3-(3-BuO-PhO)Ph]CH₂O-2-pyridyl | |
| 6-(2-F-4-MeO-Ph)CH₂O-3-pyridyl | |
| 6-(2-CF₃-4-Et-Ph)CH₂O-3-pyridyl | |
| 6-[(3-pyridyl)NHC(=O)O]-3-pyridyl | |
| 5-[(3-Br-2-pyridyl)NHC(=O)O]-2-pyridyl | |
| 6-[(2-F-3-pyridyl)NHC(=O)O]-3-pyridyl | |
| 4-[(3-Cl-4-pyridyl)NHC(=O)O]-2-pyridyl | |
| 2-[(2,4-diF-3-pyridyl)NHC(=O)O]-4-pyridyl | |
| 6-[(4-NO₂-2-pyridyl)NHC(=O)O]-3-pyridyl | |
| 6-[(3-Me-2-pyridyl)NHC(=O)O]-3-pyridyl | |
| 5-[(3-Bu-2-pyridyl)NHC(=O)O]-2-pyridyl | |
| 2-[(4-CF₃-3-pyridyl)NHC(=O)O]-4-pyridyl | |
| 5-[(2-MeS-4-pyridyl)NHC(=O)O]-3-pyridyl | |
| 5-[(4-MeO-3-pyridyl)NHC(=O)O]-2-pyridyl | |
| 2-[(4-BuO-3-pyridyl)NHC(=O)O]-4-pyridyl | |
| 5-[(4-CN-2-pyridyl)NHC(=O)O]-3-pyridyl | |
| 5-[(3-PhO-2-pyridyl)NHC(=O)O]-2-pyridyl | |
| 6-[3-(3-F-PhO)-2-pyridyl-NHC(=O)O]-3-pyridyl | |
| 5-[4-(2-NO₂-PhO)-3-pyridyl-NHC(=O)O]-2-pyridyl | |
| 6-[2-(4-Me-PhO)-4-pyridyl-NHC(=O)O]-3-pyridyl | |
| 6-[4-(4-Bu-PhO)-3-pyridyl-NHC(=O)O]-3-pyridyl | |
| 6-[4-(3-CF₃-PhO)-2-pyridyl-NHC(=O)O]-3-pyridyl | |
| 5-[4-(3-MeS-PhO)-3-pyridyl-NHC(=O)O]-2-pyridyl | |
| 5-[3-(4-MeO-PhO)-2-pyridyl-NHC(=O)O]-2-pyridyl | |
| 6-[3-(3-BuO-PhO)-2-pyridyl-NHC(=O)O]-3-pyridyl | |
| 6-[(2-F-4-MeO-3-pyridyl)NHC(=O)O]-3-pyridyl | |
| 6-[(2-CF₃-4-Et-3-pyridyl)NHC(=O)O]-3-pyridyl | |
| 6-[(3-pyridyloxy)C(=O)]-3-pyridyl | |
| 5-[(3-Br-2-pyridyloxy)C(=O)]-2-pyridyl | |
| 6-[(2-F-3-pyridyloxy)C(=O)]-3-pyridyl | |
| 4-[(3-Cl-4-pyridyloxy)C(=O)]-2-pyridyl | |
| 2-[(2,4-diF-3-pyridyloxy)C(=O)]-4-pyridyl | |
| 6-[(4-NO₂-2-pyridyloxy)C(=O)]-3-pyridyl | |
| 6-[(3-Me-2-pyridyloxy)C(=O)]-3-pyridyl | |
| 5-[(3-Bu-2-pyridyloxy)C(=O)]-2-pyridyl | |
| 2-[(4-CF₃-3-pyridyloxy)C(=O)]-4-pyridyl | |
| 5-[(2-MeS-4-pyridyloxy)C(=O))-3-pyridyl | |
| 5-[(4-MeO-3-pyridyloxy)C(=O)]-2-pyridyl | |
| 4-[(4-BuO-3-pyridyloxy)C(=O)]-3-pyridyl | |
| 2-[(4-CN-2-pyridyloxy)C(=O)]-4-pyridyl | |
| 5-[(3-PhO-2-pyridyloxy)C(=O)]-2-pyridyl | |
| 5-[3-(3-F-PhO)-2-pyridyloxy-C(=O)]-3-pyridyl | |
| 6-[4-(2-NO₂-PhO)-3-pyridyloxy-C(=O)]-3-pyridyl | |
| 5-[2-(4-Me-PhO)-4-pyridyloxy-C(=O)]-2-pyridyl | |
| 6-[4-(4-Bu-PhO)-3-pyridyloxy-C(=O)]-3-pyridyl | |
| 6-[4-(3-CF₃-PhO)-2-pyridyloxy-C(=O)]-3-pyridyl | |
| 6-[4-(3-MeS-PhO)-3-pyridyloxy-C(=O)]-3-pyridyl | |
| 5-[3-(4-MeO-Pho)-2-pyridyloxy-C(=O)]-2-pyridyl | |
| 5-[3-(3-BuO-PbO)-2-pyridyloxy-C(=O)]-2-pyridyl | |
| 6-[(2-F-4-MeO-3-pyridyloxy)C(=O)]-3-pyridyl | |
| 6-[(2-CF₃-4-Et-3-pyridyloxy)C(=O))-3-pyridyl | |
| 6-[(3-pyridyl)C(=O)O]-3-pyridyl | |
| 5-[(3-Br-2-pyridyl)C(=O)O]-2-pyridyl | |
| 6-[(2-F-3-pyridyl)C(=O)O]-3-pyridyl | |
| 4-[(3-Cl-4-pyridyl)C(=O)O]-2-pyridyl | |
| 2-[(2,4-diF-3-pyridyl)C(=O)O]-4-pyridyl | |
| 6-[(4-NO₂-2-pyridyl)C(=O)O]-3-pyridyl | |
| 6-[(3-Me-2-pyridyl)C(=O)O]-3-pyridyl | |
| 5-[(3-Bu-2-pyridyl)C(=O)O]-2-pyridyl | |
| 2-[(4-CF₃-3-pyridyl)C(=O)O]-4-pyridyl | |
| 5-[(2-MeS-4-pyridyl)C(=O)O]-3-pyridyl | |
| 5-[(4-MeO-3-pyridyl)C(=O)O]-2-pyridyl | |
| 2-[(4-BuO-3-pyridyl)C(=O)O]-4-pyridyl | |
| 5-[(4-CN-2-pyridyl)C(=O)O]-3-pyridyl | |
| 5-[(3-PhO-2-pyridyl)C(=O)O]-2-pyridyl | |
| 6-[3-(3-F-PhO)-2-pyridyl-C(=O)O]-3-pyridyl | |
| 5-[4-(2-NO₂-PhO)-3-pyridyl-C(=O)O]-2-pyridyl | |
| 6-[2-(4-Me-PhO)-4-pyridyl-C(=O)O]-3-pyridyl | |
| 6-[4-(4-Bu-PhO)-3-pyridyl-C(=O)O]-3-pyridyl | |
| 6-[4-(3-CF₃-PhO)-2-pyridyl-C(=O)O]-3-pyridyl | |
| 5-[4-(3-MeS-PhO)-3-pyridyl-C(=O)O]-2-pyridyl | |
| 5-[3-(4-MeO-PhO)-2-pyridyl-C(=O)O]-2-pyridyl | |
| 6-[3-(3-BuO-PhO)-2-pyridyl-C(=O)O]-3-pyridyl | |
| 6-[(2-F-4-MeO-3-pyridyl)C(=O)O]-3-pyridyl | |
| 6-[(2-CF₃-4-Et-3-pyridyl)C(=O)O]-3-pyridyl | |
| 5-[MeNHC(=O)O]-2-pyridyl | |
| 2-F-6-[octyl-NHC(=O)O]-3-pyridyl | |
| 5-[PhNHC(=O)O]-3-pyridyl | |
| 2-[(3-Br-Ph)NHC(=O)O]-4-pyridyl | |
| 5-[(2-F-Ph)NHC(=O)O]-3-pyridyl | |
| 2-F-6-[(3-Cl-Ph)NHC(=O)O]-3-pyridyl | |
| 6-[(2,4-diF-Ph)NHC(=O)O]-3-pyridyl | |
| 3-F-2-[(4-NO₂-Ph)NHC(=O)O]-4-pyridyl | |
| 6-[(3-Me-ph)NHC(=O)O)-3-pyridyl | |
| 5-[(3-Bu-Ph)NHC(=O)O]-2-pyridyl | |
| 6-[(4-CF₃-Ph)NHC(=O)O]-3-pyridyl | |
| 2-F-6-[(2-MeS-Ph)NHC(=O)O]-3-pyridyl | |
| 5-[(4-MeO-Ph)NHC(=O)O]-2-pyridyl | |
| 4-[(4-BuO-Ph)NHC(=O)O]-2-pyridyl | |
| 6-[(4-CN-Ph)NHC(=O)O]-3-pyridyl | |
| 2-F-6-[(3-PhO-Ph)NHC(=O)O]-3-pyridyl | |
| 2-[3-(3-F-PhO)Ph-NHC(=O)O]-4-pyridyl | |
| 5-[4-(2-NO₂-PhO)Ph-NHC(=O)O]-2-pyridyl | |
| 5-[2-(4-Me-PhO)Ph-NHC(=O)O]-3-pyridyl | |
| 5-[4-(4-Bu-PhO)Ph-NHC(=O)O]-2-pyridyl | |
| 6-[4-(3-CF₃-PhO)Ph-NHC(=O)O]-3-pyridyl | |
| 6-[4-(3-MeS-PhO)Ph-NHC(=O)O]-3-pyridyl | |
| 6-[3-(4-MeO-PhO)Ph-NHC(=O)O]-3-pyridyl | |
| 2-F-6-[3-(3-BuO-PhO)Ph-NHC(=O)O]-3-pyridyl | |
| 5-[(2-F-4-MeO-Ph)NHC(=O)O]-2-pyridyl | |
| 6-[(2-CF₃-4-Et-Ph)NHC(=O)O]-2-pyridyl | |
| 5-[MeOC(=O)]-2-pyridyl | |
| 2-F-6-[octyloxy-C(=O)]-3-pyridyl | |
| 5-[PhOC(=O)]-3-pyridyl | |
| 2-[(3-Br-PhO)C(=O)]-4-pyridyl | |
| 5-[(2-F-PhO)C(=O)]-3-pyridyl | |
| 2-F-6-[(3-Cl-PhO)C(=O)]-3-pyridyl | |
| 6-[(2,4-diF-PhO)C(-O)]-3-pyridyl | |
| 3-F-2-[(4-NO₂-PhO)C(=O)]-4-pyridyl | |
| 6-[(3-Me-PhO)C(=O)]-3-pyridyl | |
| 5-[(3-Bu-PhO)C(=O)]-2-pyridyl | |
| 6-[(4-CF₃-PhO)C(=O)]-3-pyridyl | |
| 2-F-6-[(2-MeS-PhO)C(=O)]-3-pyridyl | |
| 5-[(4-Meo-PhO)C(=O)]-2-pyridyl | |
| 4-[(4-BuO-PhO)C(=O)]-2-pyridyl | |
| 6-[(4-CN-PhO)C(=O)]-3-pyridyl | |
| 2-F-6-[(3-PhO-PhO)C(=O)]-3-pyridyl | |
| 2-[3-(3-F-PhO)PhO-C(=O)]-4-pyridyl | |
| 5-[4-(2-NO₂-PhO)PhO-C(=O)]-2-pyridyl | |
| 5-[2-(4-Me-PhO)PhO-C(=O)]-3-pyridyl | |
| 5-[4-(4-Bu-PhO)PhO-C(=O)]-2-pyridyl | |
| 6-[4-(3-CF₃-PhO)PhO-C(=O]-3-pyridyl | |
| 6-[4-(3-MeS-PhO)PhO-C(=O)]-3-pyridyl | |
| 6-[3-(4-MeO-PhO)PhO-C(=O)1-3-pyridyl | |
| 2-F-6-[3-(3-BuO-PhO)PhO-C(=O)]-3-pyridyl | |
| 5-[(2-F-4-MeO-PhO)C(=O)]-2-pyridyl | |
| 6-[(2-CF₃-4-Et-PhO)C(=O)]-2-pyridyl | |
| S-[MeC(=O)O]-2-pyridyl | |
| 2-F-6-[octyl-C(=O)O]-3-pyridyl | |
| S-[PhC(=O)O]-3-pyridyl | |
| 2-[(3-Br-Ph)C(=O)O]-4-pyridyl | |

| R² | R² |
|---|---|
| 5-[(2-F-Ph)C(=O)O]-3-pyridyl | 5-NO₂-3-furanyl |
| 2-F-6-[(3-Cl-Ph)C(=O)O]-3-pyridyl | 3,4-diF-2-furanyl |
| 6-[(2,4-diF-Ph)C(=O)O]-3-pyridyl | 4-Me-2-furanyl |
| 3-F-2-[(4-NO₂-Ph)C(=O)O]-4-pyridyl | 5-Bu-3-furanyl |
| 6-[(3-Me-Ph)C(=O)O]-3-pyridyl | 4-CF₃-2-furanyl |
| 5-[(3-Bu-Ph)C(=O)O]-2-pyridyl | 5-MeS-2-furanyl |
| 6-[(4-CF₃-Ph)C(=O)O]-3-pyridyl | 5-MeO-3-furanyl |
| 2-F-6-[(2-MeS-Ph)C(=O)O]-3-pyridyl | 4-BuO-2-furanyl |
| 5-[(4-MeO-Ph)C(=O)O]-2-pyridyl | |
| 4-[(4-BuO-Ph)C(=O)O]-2-pyridyl | |
| 6-[(4-CN-Ph)C(=O)O]-3-pyridyl | |
| 2-F-6-[(3-PhO-Ph)C(=O)O]-3-pyridyl | |
| 2-[3-(3-F-PhO)Ph-C(=O)O]-4-pyridyl | |
| 5-[4-(2-NO₂-PhO)Ph-C(=O)O]-2-pyridyl | |
| 5-[2-(4-Me-PhO)Ph-C(=O)O]-3-pyridyl | |
| 5-[4-(4-Bu-PhO)Ph-C(=O)O]-2-pyridyl | |
| 6-[4-(3-CF₃-PhO)Ph-C(=O)O]-3-pyridyl | |
| 6-[4-(3-MeS-PhO)Ph-C(=O)O]-3-pyridyl | |
| 6-[3-(4-MeO-PhO)Ph-C(=O)O]-3-pyridyl | |
| 2-F-6-[3-(3-BuO-Ph)PhO-C(=O)O]-3-pyridyl | |
| 5-[(2-F-4-MeO-Ph)C(=O)O]-2-pyridyl | |
| 6-[(2-CF₃-4-Et-Ph)C(=O)O]-2-pyridyl | |
| 2-furanyl | |
| 3-furanyl | |
| 2-F-3-furanyl | |
| 5-Cl-3-furanyl | |

**TABLE 21**

| Compounds of Formula If wherein A = NH and: | | | |
|---|---|---|---|
| n | R⁴³ | n | R⁴³ |
| 5 | Me | 6 | 4-F-PhO |
| 6 | Ph | 8 | 4-Et-PhO |
| 8 | 4-CF₃-Ph | 2 | 4-EtO-PhO |
| 9 | 3-F₃CO-Ph | 1 | 4-(4-F-PhO)PhO |
| 5 | 2,4-diCl-Ph | 2 | 4-(3-Et-PhO)PhO |
| 1 | 4-F-Ph | 3 | 4-(3-Bu-PhO)PhO |
| 2 | 4-Et-Ph | 3 | MeNH |
| 3 | 3-EtO-Ph | 4 | Me₂N |
| 4 | 4-(4-F-PhO)Ph | 1 | PhNH |
| 3 | 4-(3-Et-PhO)Ph | 5 | PhN(Me) |
| 1 | 4-(3-BuO-PhO)Ph | 6 | 4-CF₃-PhNH |
| 5 | 3-(4-CF₃-PhO)Ph | 2 | 3-F₃-CO-PhNH |
| 8 | MeO | 3 | 2,4-diCl-PhNH |
| 6 | PhO | 8 | 4-Et-PhNH |
| 2 | 4-F₃CO-PhO | 4 | 4-(4-F-PhO)PhNH |
| 4 | 3,5-diCl-PhO | 3 | 4-(4-CF₃-PhO)PhNH |

**TABLE 22**

| Compounds of Formula Ig wherein A = NH and: | | | | | |
|---|---|---|---|---|---|
| n | T | W | n | T | W |
| 1 | O | H | 6 | O | 4-F-Ph |
| 4 | O | Et | 7 | O | 3,5-diMe-Ph |
| 2 | O | Bzl | 8 | O | 4-pyridyl |
| 3 | O | 4-Cl-Bzl | 1 | O | 2,6-diEt-4-pyridyl |
| 5 | O | Ph | 4 | O | MeC(=O) |
| 1 | O | 2,6-diF-Ph | 2 | O | PhC(=O) |
| 1 | O | 3,5-diF-PhC(=O) | 3 | NH | 3,5-diF-Ph |
| 3 | O | 3,5-MeO-PhC(=O) | 2 | NH | 4-pyridyl |
| 6 | O | 4-pyridyl-C(=O) | 1 | NH | EtC(=O) |
| 7 | O | 3,5-diCl-4-pyridyl-C(=O) | 4 | NH | PhC(=O) |
| 4 | O | BuOC(=O) | 5 | NH | (4-pyridyl)C(=O) |
| 2 | O | PhOC(=O) | 6 | NH | MeOC(=O) |
| 5 | O | (3,5-diF-Ph)OC(=O) | 3 | NH | PhOC(=O) |
| 3 | O | (4-EtO-Ph)OC(=O) | 1 | NH | (3-pyridyl)OC(=O) |
| 6 | O | (4-pyridyl)OC(=O) | 1 | NH | MeNHC(=O) |
| 2 | O | NHMeC(=O) | 2 | N-Me | Me |
| 5 | O | NHPhC(=O) | 3 | N-Me | Ph |
| 1 | O | NHpyridylC(=O) | 4 | N-Ph | Ph |
| 4 | NH | Me | 5 | --- | H |
| 1 | NH | Bzl | 1 | --- | Me |
| 8 | NH | Ph | 2 | --- | Ph |

**TABLE 23**

| Compounds of Formula Ih wherein A = NH and: | | | | | |
|---|---|---|---|---|---|
| n | m | R³⁵ | n | m | R³⁵ |
| 2 | 1 | MeS | 3 | 1 | MeC(=O)O |
| 3 | 1 | FCH₂CH₂O | 2 | 2 | PhOC(=O)O |
| 1 | 1 | c-hexyloxy | 1 | 3 | MeNHC(=O)O |
| 1 | 1 | EtOC(=O) | 2 | 1 | PhNH |
| 1 | 2 | OH | 2 | 3 | (MeO)₂P(=O)O |
| 1 | 2 | MeSO₃ | 2 | 2 | PhO |
| 3 | 2 | 4-pyridyl | 2 | 6 | H |
| 1 | 3 | 4-pyridyloxy | 2 | 8 | H |
| 2 | 2 | 2-pyrimidyl | 1 | 7 | EtS |
| 2 | 1 | c-hexyl | 3 | 5 | CF₃O |
| 1 | 2 | c-pentyl | 3 | 5 | c-hexyl |
| 1 | 2 | EtO | 1 | 8 | Ph |
| 1 | 1 | Ph | | | |

### Formulations

Useful formulations of the compounds of Formula I can be prepared in conventional forms such as dusts, granules, pellets, solutions, suspensions, emulsions, wettable powders, emulsifiable concentrates and the like. Many of these can be applied directly. Sprayable formulations can be extended in suitable media and used at spray volumes of from a few liters to several hundred liters per hectare. High-strength compositions are primarily used as intermediates for further formulation. The formulations, can contain from 0.1% to 99% by weight of active compound(s) and at least one of (a) 0.1% to 20% surfactant(s) and (b) 1% to 99.9% solid or liquid diluent(s). More specifically, they will contain these ingredients in the following approximate proportions:

| | Weight Percent* | | |
|---|---|---|---|
| Formulation | Compound | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High-Strength Compositions | 90-99 | 0-10 | 0-2 |

| | | | |
|---|---|---|---|
| *Active compound plus at least one of a surfactant or a diluent equals 100 weight percent. | | | |

Lower or higher levels of active compound can, of course, be present depending on the intended use and the physical properties of the compound. Higher ratios of surfactant to active compound are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, can be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, U.S. Patent 3,060,084). Granules and pellets can be made by spraying the active material upon preformed granular carriers or by agglomeration techniques. See J. E. Browning, "Agglomeration", *Chemical Engineering,* December 4, 1967, pp 147ff. and "Perry's Chemical Engineer's Handbook", 5th Ed., McGraw-Hill, New York, 1973, pp. 8-57ff.

For further information regarding the art of formulation, see for example:
U.S. Patent 3,235,361;
U.S. Patent 3,309,192;
U.S. Patent 2,891,855;
G. C. Klingman, "Weed Control as a Science", John Wiley and Sons, Inc., New York, 1961, pp 81-96; and
J. D. Fryer et al., "Weed Control Handbook", 5th Ed., Blackwell Scientific Publications, Oxford, 1968, pp 101-103.

In the following examples of formulations of the compounds of Formula I, all parts are by weight unless otherwise indicated.

### Example A

| Wettable Powder | |
|---|---|
| 5-(2,4-diF-Ph)-5-Me-3-(phenylamino)-4-thioxooxazolidin-2-one | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

### Example B

| Wettable Powder | |
|---|---|
| 5-(2,4-diF-Ph)-5-Me-3-(phenylamino)-4-thioxooxazolidin-2-one | 50% |
| sodium alkylnaphthalenesulfonate | 2% |
| low viscosity Me cellulose | 2% |
| diatomaceous earth | 46% |

The ingredients are blended, coarsely hammer-milled and then air-milled to produce particles essentially all below 10 microns in diameter. The product is reblended before packaging.

### Example C

| Granule | |
|---|---|
| Wettable Powder of Example A | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules aredried and packaged.

### Example D

| Extruded Pellet | |
|---|---|
| 5-(2,4-diF-Ph)-5-Me-3-(phenylamino)-4-thioxooxazolidin-2-one | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These can be used directly after drying, or the dried pellets can be crushed to pass a U.S.S. No. 20 sieve (0.84 mm openings). The granules held on a U.S.S. No. 40 sieve (0.42 mm openings) can be packaged for use and the fines recycled.

### Example E

| Oil Suspension | |
|---|---|
| 5-(2,4-diF-Ph)-5-Me-3-(phenylamino)-4-thioxooxazolidin-2-one | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension can be applied directly, but preferably after being extended with oils or emulsified in water.

### Example F

| Wettable Powder | |
|---|---|
| 5-(2,4-diF-Ph)-5-Me-3-(phenylamino)-4-thioxooxazolidin-2-one | 20% |
| sodium alkylnaphthalenesulfonate | 4% |
| sodium ligninsulfonate | 4% |
| low viscosity Me cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3mm opening) and packaged.

### Example G

| Low Strength Granule | |
|---|---|
| 5-(2,4-diF-Ph)-5-Me-3-(phenylamino)-4-thioxooxazolidin-2-one | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

### Example H

| Aqueous Suspension | |
|---|---|
| 5-(2,4-diF-Ph)-5-Me-3-(phenylamino)-4-thioxooxazolidin-2-one | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

### Example I

| Low Strength Granule | |
|---|---|
| 5-(2,4-diF-Ph)-5-Me-3-(phenylamino)-4-thioxooxazolidin-2-one | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

### Example J

| Granule | |
|---|---|
| 5-(2,4-diF-Ph)-5-Me-3-(phenylamino)-4-thioxooxazolidin-2-one | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packagedfor use.

### Example K

| High Strength Concentrate | |
|---|---|
| 5-(2,4-diF-Ph)-5-Me-3-(phenylamino)-4-thioxooxazolidin-2-one | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate can be formulated further if necessary.

### Example L

| Wettable Powder | |
|---|---|
| 5-(2,4-diF-Ph)-5-Me-3-(phenylamino)-4-thioxooxazolidin-2-one | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

### Example M

| Wettable Powder | |
|---|---|
| 5-(2,4-diF-Ph)-5-Me-3-(phenylamino)-4-thioxooxazolidin-2-one | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

### Example N

| Oil Suspension | |
|---|---|
| 5-(2,4-diF-Ph)-5-Me-3-(phenylamino)-4-thioxooxazolidin-2-one | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

### Example O

| Emulsifiable Concentrate | |
|---|---|
| 5-(2,4-diF-Ph)-5-Me-3-(phenylamino)-4-thioxooxazolidin-2-one | 20% |
| chlorobenzene | 74% |
| sorbitan monostearate and polyoxyethylene condensates thereof | 6% |

The ingredients are combined and stirred to produce a solution which can be emulsified in water for application.

### Example P

| Wettable Powder | |
|---|---|
| 4-imino-5-methyl-5-(4-phenoxyphenyl)-3-(phenylamino)-2-oxazolidinone hydrobromide | 50% |
| sodium alkylnaphthalenesulfonate | 2% |
| low viscosity Me cellulose | 2% |
| diatomaceous earth | 46% |

The ingredients are blended, coarsely hammer-milled and then air-milled to produce particles essentially all below 10 microns in diameter. The product is reblended before packaging.

### Example Q

| Emulsifiable Concentrate | |
|---|---|
| 4-imino-5-methyl-5-(4-phenoxyphenyl)-3-(phenylamino)-2-oxazolidinone hydrododecylbenzene sulfonate | 20% |
| chlorobenzene | 74% |
| sorbitan monostearate and polyoxyethylene condenates thereof | 6% |

The ingredients are combined and stirred to produce a solution which can be emulsified in water for application.

### UTILITY

The compounds of this invention are useful as plant disease control agents. They provide control of diseases caused by a broad spectrum of fungal plant pathogens in the *Basidiomycete, Ascomycete* and *Oomycete* classes. They are effective in controlling a broad spectrum of plant diseases, particularly foliar pathogens of ornamental, vegetable, field, cereal, and fruit crops. These pathogens include, *Cercosporidium personatum, Cercospora* *arachidicola, Cercospora beticola, Pseudocercosporella herpotrichoides, Puccinia recondita, Puccinia gramminis, Hemileia vastatrix, Puccinia striiformis, Puccinia arachidis, Phytophthora infestans, Plasmopara viticola, Peronospora tabacina, Pseudoperonospora cubensis, Pythium aphanidermatum, Alternaria brassicae, Septoria nodorum,* and other species closely related to these pathogens. They also control seed pathogens.

The compounds of this invention can be mixed with various agents such as fungicides, bactericides, acaricides, nematicides, insecticides or other biologically active compounds in order to achieve desired results with a minimum of expenditure of time, effort and material. Suitable agents of this type are well-known to those skilled in the art. Some of these agents are listed below:

### Fungicides

methyl 2-benzimidazolecarbamate (carbendazim)
tetramethylthiuram disulfide (thiuram)
*n*-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
2-cyano-*N*-ethylcarbamoyl-2-methoxyiminoacetamide (cymoxanil)
*N*-trichloromethylthiotetrahydrophthalamide (captan)
*N*-trichloromethylthiophthalimide (folpet)
dimethyl 4,4'-(*o*-phenylene) bis (3-thioallophanate) (thiophanate-methyl)
2-(thiazol-4-yl)benzimidazole (thiabendazole)
aluminum tris(*O*-ethylphosphonate)(phosethyl aluminum)
tetrachloroisophthalonitrile (chlorothalonil)
2,6-dichloro-4-nitroaniline (dichloran)
*N-*(2,6-dimethylphenyl)*-N-*(methoxyacetyl)alanine methyl ester (metalaxyl)
2-methoxy-*N*-(2-oxo-1,3-oxazolidin-3-yl)acet2',6'-xylidide (oxadixyl)
*cis*-*N*-[1,1,2,2-tetrachloroethyl)thio]cyclohex-4-ene-1,2-dicarbioximide (captafol)
3-(3,5-dichlorophenyl)-N-(1-methylethyl)-2,4-dioxo-1-imidazolidine carboxamide (iprodione)
3-(3,5-dichlorophenyl)-5-ethenyl-5-methyl-2,4-oxazolidinedione (vinclozolin)
kasugamycin
*O*-ethyl-*S*,*S*-diphenylphosphorodithioate (edifenphos) 4-(3-(4-(1,1-dimethylethyl)phenyl)-2-methyl)propyl-2,6-dimethylmorpholine (fenpropimorph)
4-(3-4(1,1-dimethylethyl)phenyl)-2-methyl)propylpiperidine (fenpropidine)
1-(4-chlorophenoxy)-3,3-dimethyl-1-1H-1,2,4-triazol-1-yl)butanone (triadimefon)
2-(4-chlorophenyl)-2-(1H-1,2,4-triazol-1-ylmethyl)hexanenitrile (myclobutanil)
1-[2-(4-chlorophenyl)ethyl]-1-(1,1-dimethylethyl)-1-(1H-1,2,4-triazole-1-yl)ethanol (tebuconazol)
3-chloro-4-[4-methyl-2-(1H-1,2,4-triazol)-1-ylmethyl)-1,3-dioxolan-2-yl]phenyl-4-chlorophenyl ether (difenoconazole)
1-[2-(2,4-dichlorophenyl)pentyl]1H-1,2,4-triazole (penconazole)
2,4'-difluoro-1-(1H-1,2,4-triazole-1-ylmethyl)benzhydryl alcohol (flutriafol)
1-[[[bis(4-fluorophenyl)]methylsilyl]methyl]-1H-1,2,4-triazole (flusilazole)
*N*-propyl-*N*-[2-(2,4,6-trichlorophenoxy)ethyl]imidazole-1-carboxamide (prochloraz)
1-[2-(2,4-dichlorophenyl)-4-propyl-1,3-dioxolan-2-ylmethyl]-1H-1,2,4-triazole (propiconazole)
1-(2-chlorophenyl)-1-(4-chlorophenyl)-1-(5-pyrimidin)methanol (fenarimol)
1-(4-chlorophenoxy)-3,3-dimethyl-1-(1H-1,2,4-triazole-1-yl)butan-2-ol (triadimenol)
1-(2,4-dichlorophenyl)-4,4-dimethyl-2-(1H-1,2,4-triazol-1-yl)pentan-3-ol (diclobutrazol)
copper oxychloride
methyl *N-*(2,6-dimethylphenyl)*-N-*(2-furanylcarbonyl)-DL-alaninate (furalaxyl)

### Bactericides

tribasic copper sulfate
streptomycin sulfate
oxytetracycline

### Acaricides

senecioic acid, ester with 2-sec-butyl-4,6-dinitro-phenol (binapacryl)
6-methyl-1,3-dithiolo[2,3-B]quinonolin-2-one (oxythio-quinox)
2,2,2-trichloro-1,1-bis (4-chlorophenyl)ethanol (dicofol)
bis(pentachloro-2,4-oyolopentadien-1-yl)(dienochlor)
tricyclohexyltin hydroxide (cyhexatin)
hexakis (2-methyl-2-phenylpropyl)distannoxane (fenbutin oxide)

### Nematicides

2-[diethoxyphosphinylimino]-1,3-diethietane (fosthietan)
*S*-methyl-1-(dimethylcarbamoyl)-*N*-(methylcarbamoyloxy)thioformimidate (oxamyl)
*S*-methyl-1-carbamoyl-*N*-(methylcarbamoyloxy)thioformimidate
*N*-isopropylphosphoramidic acid, *O*-ethyl-*O*'-[4-(methylthio)-*m*-tolyl]diester (fenamiphos)

### Insecticides

3-hydroxy-*N*-methylcrotonamide(dimethylphosphate)ester (monocrotophos)
methylcarbamic acid, ester with 2,3-dihydro-2,2-dimethyl-7-benzofuranol (carbofuran)
*O*-[2,4,5-trichloro-a-(chloromethyl)benzyl]phosphoric acid, *O'*,*O'*-dimethyl ester (tetrachlorvinphos)
2-mercaptosuccinic acid, diethyl ester, *S*-ester with thionophosphoric acid, dimethyl ester (malathion)
phosphorothioic acid, *O*,*O*-dimethyl, *O-p-*nitrophenyl ester (methyl parathion)
methylcarbamic acid, ester with α-naphthol (carbaryl)
methyl *N*-[[(methylamino)carbonyl]oxy]ethanimidothioate (methomyl)
*N'*-(4-chloro-o-tolyl)-*N*,*N*-dimethylformamidine (chlordimeform)
*O*,*O*-diethyl-*O*-(2-isopropyl-4-methyl-6-pyrimidyl)phosphorothioate (diazinon)
octachlorocamphene (toxaphene)
*O*-ethyl *O*-*p*-nitrophenyl phenylphosphonothioate (EPN)
cyano(3-phenoxyphenyl)-methyl 4-chloro-α-(1-methylethyl)benzeneacetate (fenvalerate)
(3-phenoxyphenyl)methyl 3-(2,2-dichloro-ethenyl)-2,2-dimethylcyclopropanecarboxylate (permethrin)
dimethyl *N*,*N*'-[thiobis(*N*-methylimmo)carbonyloxy]]-bis[ethanimidothioate] (thiodicarb)
phosphorothiolothionic acid, *O*-ethyl-*O*-[4-(methylthio)phenyl]-*S*-*n*-propyl ester (sulprofos)
alpha-cyano-3-phenoxybenzyl 3-(2,2-dichlorovinyl)-2,2-dimethylcyclopropanecarboxylate (cypermethrin)
cyano(3-phenoxyphenyl)methyl 4-(difluoromethoxy)-alpha-(methylethyl)benzeneacetate (flucythrinate)
*O*,*O*-diethyl-*O*-(3,5,6-trichloro-2-pyridyl)phosphorothioate (chlorpyrifos)
*O*,*O*-dimethyl-*S*-[(4-oxo-1,2,3-benzotriazin-3-(4H)-yl)methyl]phosphorodithioate (azinphos-methyl)
5,6-dimethyl-2-dimethylamino-4-pyrimidinyl dimethyl carbamate (pirimicarb)
*S*-(*N*-formyl-*N*-methylcarbamoylmethyl)-*O*,*O*-dimethyl phosphorodithioate (formothion)
*S*-2-(ethylthioethyl)-*O*,*O*-dimethyl phosphiorothioate (demeton-S-methyl)
(5)-α-cyano-3-phenoxybenzyl (1R,3R)-3-(2,2-dibromovinyl)-2,2-dimethylcyclopropanecarboxylate (deltamethrin)
cyano(3-phenoxyphenyl)methyl ester of *N*-(2-chloro-4-trifluoromethylphenyl)alanine(fluvalinate)

### APPLICATION METHOD

Disease control is ordinarily accomplished by applying an effective amount of the compounds of the invention either pre-infection or post-infection to the portion of the plant to be protected such as the roots, stems, foliage, fruit, seeds, tubers or bulbs, or to the media (soil or sand) in which the plants to be protected are growing. The compound also may be applied to the seed, to protect the seed and seedling.

Rates of application for these compounds can be influenced by many factors of the environment and should be determined under actual use conditions. Foliage can normally be protected when treated at a rate of from less than 1 g/ha to 5000 g/ha of active ingredient. Plants growing in soil treated at a concentration from 0.1 to about 20 kg/ha can be protected from disease. Seed and seedlings can normally be protected when seed is treated at a rate of from 0.1 to 10 g per kilogram of seed. The efficacy of the compounds for disease control is evaluated according to Tests A-G below.

### Test A

The test compounds are dissolved in acetone in an amount equal to 3% of the final volume and then suspendec at a concentration of 200 ppm in purified water containing 250 ppm of the surfactant Trem 014 (polyhydric alcohol esters). This suspension is sprayed to the point of run-off on wheat seedlings. The following day the seedlings are inoculated with a spore dust of *Erysiphe graminis* f. sp. tritici, (the causal agent of wheat powdery mildew) and incubated in a growth chamber at 20°C for 7 days, after which disease ratings are made.

### Test B

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on wheat seedlings. The following day the seedlings are inoculated with a spore suspension of *Puccinia recondita* (the causal agent of wheat leaf rust) and incubated in a saturated atmosphere at 20°C for 24 h, and then moved to a growth chamber at 20°C for 6 days, after which disease ratings are made.

### Test C

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on rice seedlings. The following day the seedlings are inoculated with a spore suspension of *Pyricularia oryzae* (the causal agent of rice blast) and incubated in a saturated atmosphere at 27°C for 24 h, and then moved to a growth chamber at 30°C for 5 days, after which disease ratings are made.

### Test D

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on tomato seedlings. The following day the seedlings are inoculated with a spore suspension of *Phytophthora infestans* (the causal agent of potato and tomato late blight) and incubated in a saturated atmosphere at 20°C for 24 h, and then moved to a growth chamber at 20°C for 5 days, after which disease ratings are made.

### Test E

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on potato seedlings. The following day the seedlings are inoculated with a spore suspension of *Phytophthora infestans* (the causal agent of potato late blight) and incubated in a high humidity atmosphere at 20°C for 24 h, and then moved to a greenhouse at 20 to 25°C for 7 days, after which disease ratings are made.

### Test F

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on grape seedlings. The following day the seedlings are inoculated with a pore suspension of *Plasmopara viticola* (the causal agent of grape downy mildew) and incubated in a saturated atmosphere at 20°C for 24 h, moved to a growth chamber at 20°C for 6 days, and then incubated in a saturated atmosphere at 20°C for 24 h, after which disease ratings are made.

### Test G

The test compounds are dissolved in acetone in an amount equal to 3% of the final volume and then suspended at a concentration of 200 ppm in purified water containing 250 ppm of the surfactant Trem 014 (polyhydric alcohol esters). This suspension is sprayed to the point of run-off on cucumber seedlings. The following day the seedlings are inoculated with a spore suspension of *Botrytis cinerea* (the causal agent of gray mold on many crops) and incubated in a saturated atmosphere at 20°C for 48 h, and moved to a growth chamber at 20°C for 5 days, after which disease ratings are made.

Results for Test A to G are given in Table 1. In the table, a rating of 100 indicates 100% disease control and a rating of 0 indicated no disease control (relative to the carrier sprayed controls). NT indicates that no test was performed.

The Index Table below identifies the compounds evaluated in Tests A-G.

**INDEX TABLE**

| Compounds of Formula I wherein | | | |
|---|---|---|---|
| R¹ = Me, R³ = Ph, R⁴ = H | | | |
| Cmpd. No. | R² | A | m.p.(°C) |
| 1 | Me | H₂N⁺Br⁻ | 223 dec |
| 2 | n-octyl | H₂N⁺Br⁻ | 183-186 dec |
| 3 | 4-PhO-Ph | H₂N⁺Br⁻ | 161 dec |
| 4 | Me | PhNHC(=O)N | 157-158 dec |
| 5 | 4-PhO-Ph | BrCH₂C(=O)N | 156-158 dec |
| 6 | 4-PhO-Ph | Me₃C(=O)N | 152-155 dec |
| 7 | 2,4-diF-Ph | HN | 186-189 |
| 8 | 4-PhO-Ph | HN | 137-139 |
| 9 | 4-PhO-Ph | MeC(=O) | 160-162 dec |
| 10 | 4-PhO-Ph | MeOC(=O)N | 147-149 dec |
| 11 | 4-PhO-Ph | PhNHC(=O)N | 104-106 dec |
| 12 | 4-PhO-Ph | PhC(=O) | 100-104 dec |
| 13 | 4-PhO-Ph | Me₂P(=O) | 172-180 |
| 14 | 2,4-diF-Ph | S | 124 |
| 15 | 4-PhO-Ph | S | 110-112 |
| 16 | n-octyl | S | oil¹ |
| 17 | 2,4-diF-Ph | BrCH₂C(=O)N | 128-130 dec |
| 18 | 4-PhO-Ph | H₂N⁺(dodecylbenzenesulfonate)⁻ | 67-75 |

| | | | |
|---|---|---|---|
| ¹Anal. Calcd. for C₁₈H₂₆N₂O₂S: C 64.63; H 7.84; N 8.38; S 9.59; Found (average of 2): C 62.98; H 7.68; N 8.00; S 12.34; IR: 1807 cm⁻¹ | | | |

**TABLE I**

| CMPD. NO. | TEST A | TEST B | TEST C | TEST D | TEST E | TEST F | TEST G |
|---|---|---|---|---|---|---|---|
| 1 | 0 | 84 | 14 | 0 | NT | 14 | 35 |
| 2 | 94 | 5 | 12 | 96 | 63* | 100 | 3 |
| 3 | 59 | 100 | 81 | 99 | 93 | 100 | 56 |
| 4 | 0 | 0 | 7 | 75 | 32 | 99 | 75 |
| 5 | 0 | 84 | 27 | 74 | 33* | 99 | 60 |
| 6 | 53 | 93 | 0 | 84 | 29* | 100 | 38 |
| 7 | 53 | 83 | 0 | 92 | 68* | 100 | 38 |
| 8 | 0 | 96 | 27 | 69 | NT | 100 | 8 |
| 9 | 0 | 80* | 0 | 30 | NT | 100 | 0 |
| 10 | 57 | 55 | 0 | 69 | NT | 100 | 0 |
| 11 | 0 | 0 | 0 | 30 | NT | 100 | 8 |
| 12 | 0 | 80 | 0 | 52 | NT | 100 | 0 |
| 13 | 57 | 91 | 27 | 82 | NT | 100 | 0 |
| 14 | 0 | 89 | 0 | 91 | NT | 100 | NT |
| 15 | 0 | 93 | 0 | 73 | 9* | 100 | 0 |
| 16 | 83 | 0 | 28 | 73 | 66* | 85 | 38 |
| 17 | 83 | 17 | 0 | 73 | NT | 100 | 0 |

| | | | | | | | |
|---|---|---|---|---|---|---|---|
| * = Plants were sprayed at a concentration of 40 ppm. | | | | | | | |

From the foregoing description, one skilled in the art can easily ascertain the essential characteristics of this invention, and without departing from the spirit and scope thereof, can make various changes and modifications of the invention to adapt it to various usages and conditions.

## Claims

1. A fungicidally active compound of Formula I, and agriculturally suitable salts thereof, comprising 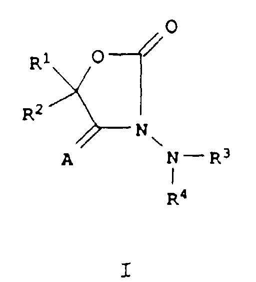 wherein:
A is S or N-J;
J r is R¹⁵; C(=O)R¹⁶; C(=O)OR¹⁷; C(=O)SR¹⁸; C(=O)NR¹⁹R²⁰; P(=O)(C₁-C₄ alkyl)₂; or OG;
G is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
R¹ is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
R² is C₁-C₂₀ alkyl optionally substituted with R²²; C₂-C₂₀ alkoxyalkyl optionally substituted with R³⁵; C₂-C₂₀ alkenyl optionally substituted with R⁴²; C₂-C₂₀ alkynyl optionally substituted with R⁴¹; (CH₂CH₂OCH₂CH₂)CH-; (CH₂CH₂SCH₂CH₂)CH-; (CH₂CH₂SO₂CH₂CH₂)CH-; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷; or
R¹ and R² can be taken together to form structures selected from the group consisting of -CH₂(CH₂)₂CH₂-,-CH₂(CH₂)₃CH₂-, -CH₂(CH₂)₄CH₂-, -CH₂CH₂OCH₂CH₂-, -CH₂CH₂SCH₂CH₂-, 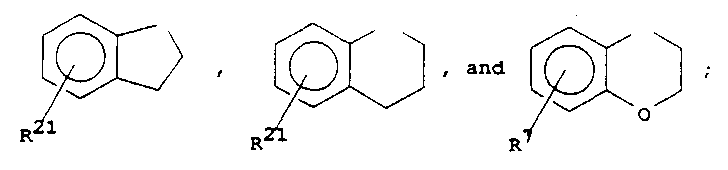
R³ is phenyl, pyridyl, or pyrimidinyl each optionally substituted with R¹⁰ or phenylmethyl;
R⁴ is H or methyl;
_{R}5 is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁶; phenylmethyl, phenoxymethyl, phenethyl, or styryl each optionally substituted with R²⁶ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
R⁶, R⁷, R¹², R¹³, R²⁴, R²⁶ and R³⁴ are independently 1-2 halogen; nitro; C₁-C₄ alkyl; trifluoromethyl; methylthio; or C₁-C₄ alkoxy;
R⁸, R¹⁴, R²⁰, R⁴⁰ and R³⁸ are independently H or C₁-C₄ alkyl;
R⁹ is C₁-C₁₈ alkyl; or phenyl optionally substituted with R⁷;
R¹⁰, R²⁵ and R³³ are independently 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
R¹¹ and R³⁶ are independently C₁-C₆ alkyl; or phenyl optionally substituted with R¹²;
_{R}15 is H; C₁-C₈ alkyl optionally substituted with C₁-C₂ alkoxy; C₃-C₆ cycloalkyl; C₃-C₈ alkenyl; C₃-C₈ alkynyl; phenyl optionally substituted with R¹³; benzyl optionally substituted with R¹³ on the phenyl ring and with R²⁰ on the benzylic carbon; or pyridyl optionally substituted with R¹³;
R¹⁶ is H; C₁-C₁₇ alkyl optionally substituted with R³¹; C₂-C₁₇ alkenyl optionally substituted with R³²; C₂-C₇ alkynyl; C₃-C₈ cycloalkyl; C₅-C₆ cycloalkenyl; C₆-C₇ alkylcycloalkyl; C₄-C₈ cycloalkylalkyl; phenyl optionally substituted with R³³; naphthalenyl, furanyl, thienyl, benzoyl, or pyridyl each optionally substituted with R³⁴; or C₂-C₅ alkoxycarbonyl;
R¹⁷ and R¹⁸ are independently C₁-C₁₈ alkyl optionally substituted with R²³; C₂-C₁₀ alkenyl optionally substituted with R³²; C₃-C₈ alkynyl;
C₃-C₁₂ cycloalkyl; C₅-C₆ cycloalkenyl;
C₆-C₇ alkylcycloalkyl; C₆-C₇ cycloalkylalkyl; or phenyl, naphthalenyl, or thienyl each optionally substituted with R³⁴;
R¹⁹ is H; C₁-C₁₀ alkyl; C₅-C₆ cycloalkyl; or phenyl optionally substituted with R³⁴; or
R¹⁹ and R²⁰ can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH (Me)CH₂-, and -CH₂CH(Me)OCH (Me)CH₂-;
R²¹ L is 1-2 halogen; nitro; C₁-C₄ alkyl; trifluoromethyl; methylthio; or C₁-C₄ alkoxy; or phenoxy optionally substituted with R²⁶;
R²² is cyano; nitro; C₁-C₁₉ alkylthio; C₁-C₁₉ alkylsulfinyl; C₁-C₁₉ haloalkoxy; C₅-C₆ cycloalkyloxy; C₃-C₁₉ alkenyloxy; C₃-C₁₉ alkynyloxy; C₁-C₁₉ alkylsulfonyl; C₂-C₁₉ alkoxycarbonyl; hydroxyl; hydroxycarbonyl; R²⁸C(=O)O; R²⁸OC(=O)O; R²⁸R⁴⁰NC(=O)O; R³⁹R⁴⁰N; (C₁-C₄ alkoxy)₂P(=E)O; R¹¹SO₃; R⁴⁰R¹⁴R³⁸N⁺; phenyl, phenylthio, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; C₃-C₆ cycloalkyl; 2-tetrahydropyranyloxy; or C(=Q)R⁴⁰;
E is O or S;
Q is is O or N-T-W;
T is O; NR³⁷; or a direct bond;
W is H; C₁-C₈ alkyl, C₃-C₈ alkenyl; phenylmethyl optionally substituted with R⁷ on the phenyl ring and R¹⁴ on the benzylic carbon; phenyl or pyridyl each optionally substituted with R⁷; C(=O)R²⁸; C(=O)OR²⁸; or C(=O)NR²⁸R¹⁴;
R²³ is 1-3 halogen; C₁-C₁₂ alkoxy; C₁-C₁₂ alkylthio; phenyl or naphthalenyl each optionally substituted with R³⁴; or phenoxymethyl optionally substituted with R³⁴ on the phenyl ring;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl;
C₅-C₆ cycloalkyloxy; C₂-C₆ alkenyl;
C₂-C₆ haloalkenyl; C₂-C₆ alkynyl;
hydroxycarbonyl; C₂-C₄ alkoxycarbonyl; or phenoxy optionally substituted with R²⁴;
R²⁸ is C₁-C₈ alkyl; or phenyl or pyridyl each optionally substituted with R³⁰;
R³⁰ is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
R³¹ is 1-3 halogen; C₁-C₁₈ alkoxy; allyloxy; C₁-C₁₈ alkylthio; phenyl, phenoxy, benzyloxy, or phenylthio each optionally substituted with R³⁴ on the phenyl ring; acetyl; or C₂-C₅ alkoxycarbonyl;
R³² is 1-3 halogen; or C₁-C₄ alkoxy;
R³⁵ is cyano; nitro; C₁-C₁₇ alkylthio; C₁-C₁₇ alkylsulfinyl; C₁-C₁₇ haloalkoxy; C₅-C₆ cycloalkyloxy; C₂-C₁₇ haloalkenyl; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; C₂-C₁₇ alkoxyalkoxy; C₃-C₁₇ alkynyl; C₃-C₆ cycloalkyl; or C₂-C₁₇ haloalkoxyalkoxy;
R³⁷ is H; C₁-C₆ alkyl; or phenyl optionally substituted with R⁷;
R³⁹ is C₁-C₁₉ alkyl; C₂-C₁₉ alkylcarbonyl; C₂-C₁₉ alkoxycarbonyl; (R⁹R⁴⁰N)C=O; phenyl optionally substituted with R²⁵; or phenoxycarbonyl optionally substituted with R⁷;
R⁴¹ is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; or C₃-C₆ cycloalkyl;
R⁴² is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; C₃-C₁₇ alkynyl; or C₃-C₆ cycloalkyl;
provided that
i) when R² is a substituted phenyl or heterocyclic ring, then only H or F can be substituted on the carbon atom(s) of the phenyl or heterocyclic ring adjacent to the carbon bearing the oxazolidinone ring;
ii) when R³ is a phenyl or heterocyclic ring disubstituted with two alkyl or alkoxy groups, or one alkyl and one alkoxy group, then at least one of the alkyl and alkoxy groups is methyl or methoxy; and
iii) the total number of carbons in R², R¹⁶, R¹⁷, and R¹⁸ is each less than or equal to 20.

2. A fungicidally active compound of Formula I comprising 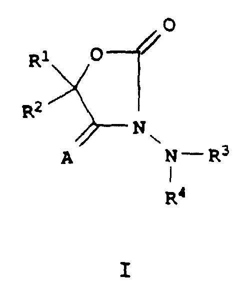 wherein:
A is S or N-OG;
G is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
R¹ is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
R² is C₁-C₆ alkyl; C₅-C₇ cycloalkyl; C₂-C₆ alkenyl; C₅-C₇ cycloalkenyl; phenyl optionally substituted with R⁵ and R⁷;
2-naphthalenyl; thienyl optionally substituted with R⁵ and R⁷; furyl optionally substituted with R⁷; or pyridyl optionally substituted with R⁷; or phenoxy or phenylthio each optionally substituted with R⁷; or
R¹ and R² can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 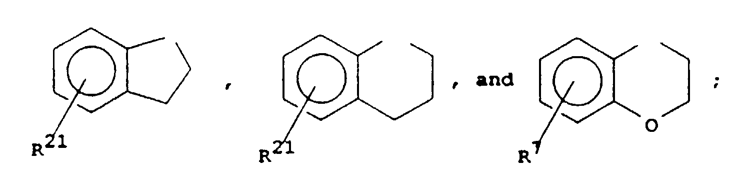
R³ is phenyl, pyridyl, or pyrimidinyl each optionally substituted with R¹⁰; or phenylmethyl;
R⁴ is H or methyl;
R⁵ is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; and C₂-C₆ alkoxycarbonyl;
R⁶, R⁷, R²⁴, R²⁶ and R³⁴ are independently 1-2 halogen; nitro; C₁-C₄ alkyl; trifluoromethyl; methylthio; or C₁-C₄ alkoxy;
R⁸ is H or C₁-C₄ alkyl;
R¹⁰ is 1-2 substituents selected from the group consisting of halogen, nitro, cyano, C₁-C₄ alkyl, trifluoromethyl, C₁-C₄ alkylthio, C₁-C₄ alkoxy and trifluoromethoxv:
R²¹ is halogen; nitro; C₁-C₄ alkyl; trifluoromethyl; methylthio; or C₁-C₄ alkoxy; or phenoxy optionally substituted with R²⁶;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; C₂-C₄ alkoxycarbonyl; or phenoxy; and
R³⁶ is C₁-C₆ alkyl; or phenyl optionally substituted with R¹².

3. A fungicidally active compound according to Claim 1 wherein:
J is H; OH; C(=O)R¹⁶; C(=O)OR¹⁷; C(=O)NHR¹⁹;
R¹ is C₁-C₄ alkyl; C₁-C₄ haloalkvl: or vinyl;
R² is C₁-C₂₀ alkyl; C₂-C₂₀ alkoxyalkyl; C₂-C₂₀ haloalkyl; C₃-C₈ alkyl substituted with phenoxy or phenylthio each optionally substituted with R³⁰; C₅-C₇ cycloalkyl; C₂-C₂₀ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
R³ is phenyl optionally substituted with R¹⁰;
R¹⁶ is H; C₁-C₆ alkyl; or phenyl optionally substituted with R³⁴;
R¹⁷ is C₁-C₆ alkyl;
R¹⁹ is C₁-C₆ alkyl; or phenyl optionally substituted with R³⁴;
provided that when R² is phenyl and R⁵ is not F, then
R⁵ is attached to the para-position relative to the oxazolidinone ring.

4. A fungicidally active compound according to Claim 3 wherein:
J is H; OH or C(=O)R¹⁶;
R¹ is C₁-C₂ alkyl or vinyl;
R² is C₁-C₁₂ alkyl; C₅-C₇ cycloalkyl; phenyl optionally substituted with R⁵ and R⁷; thienyl optionally substituted with R⁷; or pyridyl optionally substituted with R⁵ and R⁷;
R³ is phenyl optionally substituted with F; Cl; or methyl;
R⁴ is H;
R⁵ is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring or -OC(=O)NHR²⁸;
R⁷ and R²⁴ are independently F; C₁-C₂ alkyl; methylthio or C₁-C₂ alkoxy;
R¹⁶ is C₁-C₆ alkyl;
R¹⁹ is phenyl optionally substituted with R³⁴;
R²⁷ is 1-2 halogen; cyano; C₁-C₄ alkyl; trifluoromethyl; C₁-C₄ alkoxy; C₁-C₄ haloalkoxy; C₁-C₄ alkylthio; C₅-C₆ cycloalkyloxy; or allyl;
R³⁰ is 1-2 halogen, cyano, C₁-C₄ alkyl, trifluoromethyl, C₁-C₄ alkoxy or trifluoromethoxy; and
R³⁴ is 1-2 halogen; nitro; C₁-C₂ alkyl; or C₁-C₂ alkoxy.

5. A fungicidally active compound according to Claim 4 wherein:
J is H;
R¹ is methyl;
R² is C₁-C₁₂ alkyl; phenyl optionally substituted with R⁵ and R⁷; pyridyl optionally substituted with R⁵ and R⁷; and
R⁵ is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸.

6. A fungicidally active compound according to Claim 5 selected from the group consisting of
5-(2,4-Difluorophenyl)-5-methyl-3-(phenylamino)-4-thioxo-2-oxazolidinone;
4-Imino-5-methyl-5-(4-phenoxyphenyl)-3-(phenylamino)-2-oxazolidinone;
4-Imino-5-methyl-5-(4-phenoxyphenyl)-3-(phenylamino)-2-oxazolidinone hydrobromide; and
5-(2,4-Difluorophenyl)-4-imino-5-methyl-3-(phenylamino)-2-oxazolidinone.

7. A fungicidally active composition comprising the compounds of any one of Claims 1-6.

8. A method of controlling disease in plants comprising applying to said plants a fungicidally active compound of Formula I 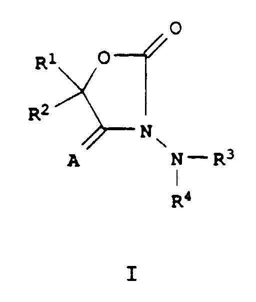 wherein:
A is S or N-J;
J is R¹⁵; C(=O)R¹⁶; C(=O)OR¹⁷; C(=O)SR¹⁸; C(=O)NR¹⁹R²⁰; P(=O) (C₁-C₄ alkyl)₂; or OG;
G is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
R¹ is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
R² is C₁-C₂₀ alkyl optionally substituted with R²²; C₂-C₂₀ alkoxyalkyl optionally substituted with R³⁵; C₂-C₂₀ alkenyl optionally substituted with R⁴²; C₂-C₂₀ alkynyl optionally substituted with R⁴¹; (CH₂CH₂OCH₂CH₂)CH-; (CH₂CH₂SCH₂CH₂)CH-; (CH₂CH₂SO₂CH₂CH₂)CH-; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷; or
R¹ and R² can be taken together to form structures selected from the group consisting of -CH₂(CH₂)₂CH₂-,-CH₂(CH₂)₃CH₂-,-CH₂(CH₂)₄CH₂-, -CH₂CH₂OCH₂CH₂-, -CH₂CH₂SCH₂CH₂-, 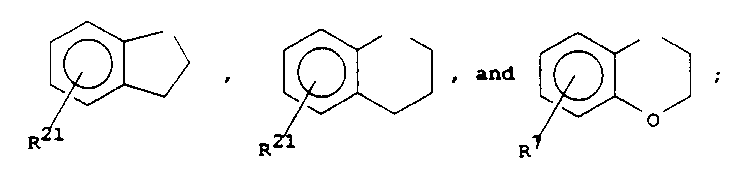
R³ is phenyl, pyridyl, or pyrimidinyl each optionally substituted with R¹⁰ or phenylmethyl;
R⁴ is H or methyl;
R⁵ is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁶; phenylmethyl, phenoxymethyl, phenethyl, or styryl each optionally substituted with R²⁶ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
R⁶, R⁷, R¹², R¹³, R²⁴, R²⁶ and R³⁴ are independently 1-2 halogen; nitro; C₁-C₄ alkyl; trifluoromethyl; methylthio; or C₁-C₄ alkoxy;
R⁸, R¹⁴, R²⁰, R⁴⁰ and R³⁸ are independently H or C₁-C₄ alkyl;
R⁹ is C₁-C₁₈ alkyl; or phenyl optionally substituted with R⁷;
R¹⁰, R²⁵ and R³³ are independently 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
R¹¹ and R³⁶ are independently C₁-C₆ alkyl; or phenyl optionally substituted with R¹²;
R¹⁵ is H; C₁-C₈ alkyl optionally substituted with C₁-C₂ alkoxy; C₃-C₆ cycloalkyl; C₃-C₈ alkenyl; C₃-C₈ alkynyl; phenyl optionally substituted with R¹³; benzyl optionally substituted with R¹³ on the phenyl ring and with R²⁰ on the benzylic carbon; or pyridyl optionally substituted with R¹³;
R¹⁶ is H; C₁-C₁₇ alkyl optionally substituted with R³¹; C₂-C₁₇ alkenyl optionally substituted with R³²; C₂-C₇ alkynyl; C₃-C₈ cycloalkyl; C₅-C₆ cycloalkenyl; C₆-C₇ alkylcycloalkyl; C₄-C₈ cycloalkylalkyl; phenyl optionally substituted with R³³; naphthalenyl, furanyl, thienyl, benzoyl, or pyridyl each optionally substituted with R³⁴; or C₂-C₅ alkoxycarbonyl;
R¹⁷ and R¹⁸ are independently C₁-C₁₈ alkyl optionally substituted with R²³; C₂-C₁₀ alkenyl optionally substituted with R³²; C₃-C₈ alkynyl; C₃-C₁₂ cycloalkyl; C₅-C₆ cycloalkenyl; C₆-C₇ alkylcycloalkyl; C₆-C₇ cycloalkylalkyl; or phenyl, naphthalenyl, or thienyl each optionally substituted with R³⁴;
R¹⁹ is H; C₁-C₁₀ alkyl; C₅-C₆ cycloalkyl; or phenyl optionally substituted with R³⁴; or
R¹⁹ and R²⁰ can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, and -CH₂CH(Me)OCH(Me)CH₂-;
R²¹ is 1-2 halogen; nitro; C₁-C₄ alkyl; trifluoromethyl; methylthio; or C₁-C₄ alkoxy; or phenoxy optionally substituted with R²⁶;
R²² is cyano; nitro; C₁-C₁₉ alkylthio; C₁-C₁₉ alkylsulfinyl; C₁-C₁₉ haloalkoxy; C₅-C₆ cycloalkyloxy; C₃-C₁₉ alkenyloxy; C₃-C₁₉ alkynyloxy; C₁-C₁₉ alkylsulfonyl; C₂-C₁₉ alkoxycarbonyl; hydroxyl; hydroxycarbonyl; R²⁸C(=O)O; R²⁸OC(=O)O; R²⁸R⁴⁰NC(=O)O; R³⁹R⁴⁰N; (C₁-C₄ alkoxy)₂P(=E)O; R¹¹SO₃; R⁴⁰R¹⁴R³⁸N⁺; phenyl, phenylthio, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; C₃-C₆ cycloalkyl; 2-tetrahydropyranyloxy; or C(=Q)R⁴⁰;
E is O or S;
Q is O or N-T-W;
T is O; NR³⁷; or a direct bond;
W is H; C₁-C₈ alkyl, C₃-C₈ alkenyl; phenylmethyl optionally substituted with R⁷ on the phenyl ring and R¹⁴ on the benzylic carbon; phenyl or pyridyl each optionally substituted with R⁷; C(=O)R²⁸; C(=O)OR²⁸; or C(=O)NR²⁸R¹⁴;
R²³ is 1-3 halogen; C₁-C₁₂ alkoxy; C₁-C₁₂ alkylthio; phenyl or naphthalenyl each optionally substituted with R³⁴; or phenoxymethyl optionally substituted with R³⁴ on the phenyl ring;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
R²⁸ is C₁-C₈ alkyl; or phenyl or pyridyl each optionally substituted with R³⁰;
R³⁰ is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
R³¹ is 1-3 halogen; C₁-C₁₈ alkoxy; allyloxy; C₁-C₁₈ alkylthio; phenyl, phenoxy, benzyloxy, or phenylthio each optionally substituted with R³⁴ on the phenyl ring; acetyl; or C₂-C₅ alkoxycarbonyl;
R³² is 1-3 halogen; or C₁-C₄ alkoxy;
R³⁵ is cyano; nitro; C₁-C₁₇ alkylthio; C₁-C₁₇ alkylsulfinyl; C₁-C₁₇ haloalkoxy; C₅-C₆ cycloalkyloxy; C₂-C₁₇ haloalkenyl; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; C₂-C₁₇ alkoxyalkoxy; C₃-C₁₇ alkynyl; C₃-C₆ cycloalkyl; or C₂-C₁₇ haloalkoxyalkoxy;
R³⁷ is H; C₁-C₆ alkyl; or phenyl optionally substituted with R⁷;
R³⁹ is C₁-C₁₉ alkyl; C₂-C₁₉ alkylcarbonyl; C₂-C₁₉ alkoxycarbonyl; (R⁹R⁴⁰N)C=O; phenyl optionally substituted with R²⁵; or phenoxycarbonyl optionally substituted with R⁷;
R⁴¹ is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; or C₃-C₆ cycloalkyl;
R⁴² is cyano; nitro; C₁-C₁₇ alkylthio; C₁-C₁₇ alkylsulfinyl; C₁-C₁₇ haloalkoxy; C₅-C₆ cycloalkyloxy; C₃-C₁₇ alkenyloxy; C₃-C₁₇ haloalkynyl; C₃-C₁₇ alkynyloxy; C₁-C₁₇ alkylsulfonyl; C₂-C₁₇ alkoxycarbonyl; hydroxyl; hydroxycarbonyl; R²⁸C(=O)O; R²⁸OC(=O)O; R²⁸R⁴⁰NC(=O)O; R⁴⁰R³⁹N; (C₁-C₄ alkoxy)₂P(=E)O; R¹¹SO₃; R⁴⁰R¹⁴R³⁸N⁺; phenyl, phenoxy, phenylthio, phenylsulfonyl, phenylsulfinyl, pyridyl or pyridyloxy each optionally substituted with R³⁰; thienyl, pyrimidinyl, furanyl, naphthalenyl, pyrimidinyloxy, naphthalenyloxy each optionally substituted with R⁷; tetrahydropyranyl; 2-tetrahydropyranyloxy; C₁-C₁₇ alkoxy; 1-3 halogen; C₂-C₁₇ alkoxyalkoxy; C₃-C₁₇ alkynyl; or C₃-C₆ cycloalkyl;
provided that
i) when R² is a substituted phenyl or heterocyclic ring, then only H or F can be substituted on the carbon atom(s) of the phenyl or heterocyclic ring adjacent to the carbon bearing the oxazolidinone ring;
ii) when R³ is a phenyl or heterocyclic ring disubstituted with two alkyl or alkoxy groups, or one alkyl and one alkoxy group, then at least one of the alkyl and alkoxy groups is methyl or methoxy; and
iii) the total number of carbons in R², R¹⁶, R¹⁷, and R¹⁸ is each less than or equal to 20.

9. A method of controlling disease in plants comprising applying to said plants a fungicidally active compound of Formula I 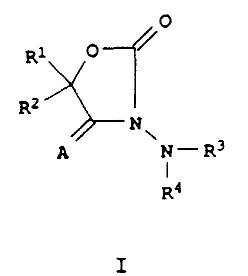 wherein:
A is S or N-OG;
G is H; C₁-C₆ alkyl; benzyl optionally substituted with R³⁴ on the phenyl ring; C(=O)(C₁-C₄ alkyl); C(=O)(C₁-C₄ alkoxy); or C(=O)NHR³⁶;
R¹ is C₁-C₄ alkyl; C₁-C₄ haloalkyl; C₃-C₆ cycloalkyl; C₂-C₄ alkenyl; C₂-C₄ alkoxycarbonyl; or phenylmethyl optionally substituted with R⁶ on the phenyl ring and with R⁸ on the benzylic carbon;
R² is C₁-C₆ alkyl; C₅-C₇ cycloalkyl; C₂-C₆ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁷; or phenoxy or phenylthio each optionally substituted with R⁷; or
R¹ and R² can be taken together to form structures selected from the group consisting of -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 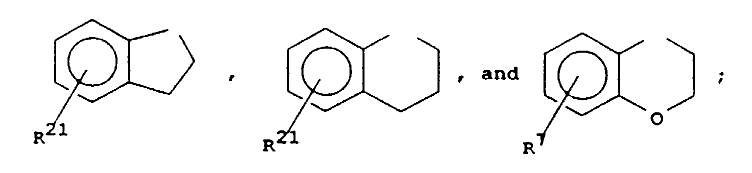
R³ is phenyl, pyridyl, or pyrimidinyl each optionally substituted with R¹⁰; or phenylmethyl;
R⁴ is H or methyl;
R⁵ is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; and C₂-C₆ alkoxycarbonyl;
R⁶, R⁷, R²⁴, R²⁶ and R³⁴ are independently 1-2 halogen; nitro; C₁-C₄ alkyl; trifluoromethyl; methylthio; or C₁-C₄ alkoxy;
R⁸ is H or C₁-C₄ alkyl;
R¹⁰ is 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
R²¹ is halogen; nitro; C₁-C₄ alkyl; trifluoromethyl; methylthio; or C₁-C₄ alkoxy; or phenoxy optionally substituted with R²⁶;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; C₂-C₄ alkoxycarbonyl; or phenoxy; and
R³⁶ is C₁-C₆ alkyl; or phenyl optionally substituted with R¹².

10. A method of Claim 8 wherein:
J is H; OH; C(=O)R¹⁶; C(=O)OR¹⁷ or C(=O)NHR¹⁹;
R¹ is C₁-C₄ alkyl; C₁-C₄ haloalkyl; or vinyl;
R² is C₁-C₂₀ alkyl; C₂-C₂₀ alkoxyalkyl; C₂-C₂₀ haloalkyl; C₃-C₈ alkyl substituted with phenoxy or phenylthio each optionally substituted with R³⁰; C₅-C₇ cycloalkyl; C₂-C₂₀ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
R³ is phenyl optionally substituted with R¹⁰;
R¹⁶ is H; C₁-C₆ alkyl; or phenyl optionally substituted with R³⁴;
R¹⁷ is C₁-C₆ alkyl;
R¹⁹ is C₁-C₆ alkyl; or phenyl optionally substituted with R³⁴;
provided that when R² is phenyl and R⁵ is not F, then
R⁵ is attached to the para-position relative to the oxazolidinone ring.

11. The method of Claim 8 wherein:
J is H; OH; or C(=O)R¹⁶;
R¹ is C₁-C₂ alkyl or vinyl;
R² is C₁-C₁₂ alkyl; C₅-C₇ cycloalkyl; phenyl optionally substituted with R⁵ and R⁷; thienyl optionally substituted with R⁷; or pyridyl optionally substituted with R⁵ and R⁷;
R³ is phenyl optionally substituted with F; Cl; or methyl;
R⁴ is H;
R⁵ is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring; -OC(=O)NHR²⁸;
R⁷ and R²⁴ are independently F; C₁-C₂ alkyl; methylthio; or C₁-C₂ alkoxy;
R¹⁶ is C₁-C₆ alkyl;
R¹⁹ is phenyl optionally substituted with R³⁴;
R²⁷ is 1-2 halogen; cyano; C₁-C₄ alkyl; trifluoromethyl; C₁-C₄ alkoxy; C₁-C₄ haloalkoxy; C₁-C₄ alkylthio; C₅-C₆ cycloalkyloxy; or allyl;
R³⁰ is 1-2 halogen, cyano, C₁-C₄ alkyl, trifluoromethyl, C₁-C₄ alkoxy or trifluoromethoxy; and
R³⁴ is 1-2 halogen; nitro; C₁-C₂ alkyl; or C₁-C₂ alkoxy.

12. A method of Claim 11 wherein:
J is H;
R¹ is methyl;
R² is C₁-C₁₂ alkyl; phenyl optionally substituted with R⁵ and R⁷; pyridyl optionally substituted with R⁵ and R⁷; and
R⁵ is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸.

13. A method of Claim 8 wherein said compound is selected from the group consisting of
5-(2,4-Difluorophenyl)-5-methyl-3-(phenylamino)-4-thioxo-2-oxazolidinone;
4-Imino-5-methyl-5-(4-phenoxyphenyl)-3-(phenylamino)-2-oxazolidinone;
4-Imino-5-methyl-5-(4-phenoxyphenyl)-3-(phenylamino) -2-oxazolidinone hydrobromide; and
5-(2,4-Difluorophenyl)-4-imino-5-methyl-3-(phenylamino)-2-oxazolidinone.

14. A method for controlling disease in plants comprising applying to said plants a composition comprising a fungicidally active compound of any one of Claims 1-6.

## Patentansprüche

1. Fungizide aktive Verbindung der Formel I,
und deren landwirtshaftlich geeignete Salze, umfassend: 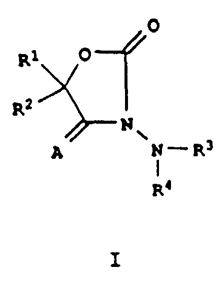 worin sind:
A S oder N-J;
J R¹⁵; C(=O)R¹⁶; C(=O)OR¹⁷; C(=O)SR¹⁸; C(=O)NR¹⁹R²⁰; P(=O)(C₁-C₄-Alkyl)₂; oder OG;
G H; C₁-C₆-Alkyl; Benzyl, wahlweise am Phenyl-Ring substituiert mit R³⁴; C(=O)(C₁-C₄-Alkyl); C(=O)(C₁-C₄-Alkoxy); oder C(=O)NHR³⁶;
R¹ C₁-C₄-Alkyl; C₁-C₄-Halogenalkyl; C₃-C₆-Cycloalkyl; C₂-C₄-Alkenyl; C₂-C₄-Alkoxycarbonyl; oder Phenylmethyl, am Phenyl-Ring wahlweise substituiert mit R⁶ und am Benzyl-Kohlenstoff mit R⁸;
R² C₁-C₂₀-Alkyl, wahlweise substituiert mit R²²; C₂-C₂₀-Alkoxyalkyl, wahlweise substituiert mit R³⁵; C₂-C₂₀-Alkenyl, wahlweise substituiert mit R⁴²; C₂-C₂₀-Alkinyl, wahlweise substituiert mit R⁴¹; (CH₂CH₂OCH₂CH₂)CH-; (CH₂CH₂SCH₂CH₂)CH-; (CH₂CH₂SO₂CH₂CH₂)CH-; C₅-C₇-Cycloalkyl; C₅-C₇-Cycloalkenyl; Phenyl, wahlweise substituiert mit R⁵ und R⁷; 2-Naphthalenyl; Thienyl, wahlweise substituiert mit R⁵ und R⁷; Furyl, wahlweise substituiert mit R⁷; oder Pyridyl, wahlweise substituiert mit R⁵ und R⁷; oder
R¹ und R² können gemeinsam Strukturen bilden, ausgewählt aus der Gruppe, bestehend aus -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 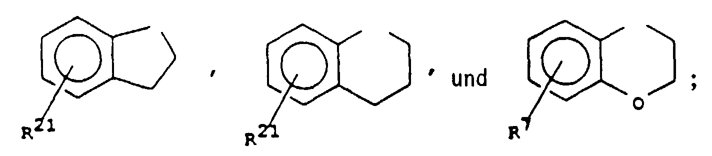
R³ Phenyl, Pyridyl oder Pyrimidinyl, jeweils wahlweise substituiert mit R¹⁰ oder Phenylmethyl;
R⁴ H oder Methyl;
R⁵ Halogen; Nitro; Cyano; C₁-C₆-Alkyl; C₅-C₆-Cycloalkyl; C₁-C₆-Halogenalkyl; C₁-C₆-Alkylthio; C₁-C₆-Halogenalkylthio; C₁-C₆-Alkoxy; C₁-C₆-Halogenalkoxy; C₅-C₆-Cycloalkyloxy; C₂-C₆-Alkoxyalkyl; C₂-C₆-Alkoxyalkoxy; C₃-C₆-Alkenyl ; C₃-C₆-Halogenalkenyl; C₃-C₆-Alkenyloxy; C₃-C₆-Alkinyl ; C₃-C₆-Halogenalkinyl; C₃-C₆-Alkinyloxy; C₁-C₆-Alkylsulfonyl; C₁-C₆-Halogenalkylsulfonyl; Phenyl oder Phenylthio, jeweils wahlweise substituiert mit R²⁶; Phenylmethyl, Phenoxymethyl, Phenethyl oder Styryl, jeweils wahlweise am Phenyl-Ring substituiert mit R²⁶; Phenoxy, wahlweise substituiert mit R²⁷; Benzyloxy, wahlweise am Phenyl-Ring substituiert mit R³⁰; -OC(=O)NHR²⁸; -C(=O)OR²⁸; oder -OC(=O)R²⁸;
R⁶, R⁷, R¹², R¹³, R²⁴, R²⁶ und R³⁴ unabhängig 1-2 Halogen; Nitro; C₁-C₄-Alkyl; Trifluormethyl; Methylthio; oder C₁-C₄-Alkoxy;
R⁸, R¹⁴, R²⁰, R⁴⁰ und R³⁸ unabhängig H oder C₁-C₄-Alkyl;
R⁹ C₁-C₁₈-Alkyl; oder Phenyl, wahlweise substituiert mit R⁷;
R¹⁰, R²⁵ und R³³ unabhängig 1-2 Substituenten, ausgewählt aus der Gruppe, bestehend aus: Halogen, Nitro, Cyano, C₁-C₄-Alkyl, Trifluormethyl, C₁-C₄-Alkylthio, C₁-C₄-Alkoxy und Trifluormethoxy;
R¹¹ und R³⁶ unabhängig C₁-C₆-Alkyl; oder Phenyl, wahlweise substituiert mit R¹²;
R¹⁵ H; C₁-C₈-Alkyl, wahlweise substituiert mit C₁-C₂-Alkoxy; C₃-C₆-Cycloalkyl ; C₃-C₈-Alkenyl; C₃-C₈-Alkinyl; Phenyl, wahlweise substituiert mit R¹³; Benzyl, wahlweise am Phenyl-Ring substituiert mit R¹³ und am Benzyl-Kohlenstoff mit R²⁰; oder Pyridyl, wahlweise substituiert mit R¹³;
R¹⁶ H; C₁-C₁₇-Alkyl, wahlweise substituiert mit R³¹; C₂-C₁₇-Alkenyl, wahlweise substituiert mit R³²; C₂-C₇-Alkinyl; C₃-C₈-Cycloalkyl; C₅-C₆-Cycloalkenyl; C₆-C₇-Alkylcycloalkyl; C₄-C₈-Cycloalkylalkyl ; Phenyl, wahlweise substituiert mit R³³; Naphthalenyl, Furanyl, Thienyl, Benzoyl oder Pyridyl, jeweils wahlweise substituiert mit R³⁴; oder C₂-C₅-Al koxycarbonyl;
R¹⁷ und R¹⁸ unabhängig C₁-C₁₈-Alkyl, wahlweise substituiert mit R²³; C₂-C₁₀-Alkenyl , wahlweise substituiert mit R³²; C₃-C₈-Alkinyl ; C₃-C₁₂-Cycloalkyl; C₅-C₆-Cycloalkenyl; C₆-C₇-Alkylcycloalkyl; C₆-C₇-Cycloalkylalkyl; oder Phenyl, Naphthalenyl; oder Thienyl, jeweils wahlweise substituiert mit R³⁴;
R¹⁹ H; C₁-C₁₀-Alkyl; C₅-C₆-Cycloalkyl; oder Phenyl, wahlweise substituiert mit R³⁴; oder
R¹⁹ und R²⁰ bilden gemeinsam Strukturen, ausgewählt aus der Gruppe, bestehend aus: -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, und -CH₂CH(Me)OCH(Me)CH₂-;
R²¹ 1-2 Halogen; Nitro; C₁-C₄-Alkyl; Trifluormethyl; Methylthio; oder C₁-C₄-Alkoxy; oder Phenoxy, wahlweise substituiert mit R²⁶;
R²² Cyano; Nitro; C₁-C₁₉-Alkylthio; C₁-C₁₉-Alkylsulfinyl; C₁-C₁₉-Halogenal koxy; C₅-C₆-Cycloalkyloxy; C₃-C₁₉-Alkenyloxy; C₃-C₁₉-Alkinyloxy; C₁-C₁₉-Alkylsulfonyl; C₂-C₁₉-Alkoxycarbonyl; Hydroxyl; Hydroxycarbonyl; R²⁸C(=O)O; R²⁸OC(=O)O; R²⁸R⁴⁰NC(=O)O; R³⁹R⁴⁰N; (C₁-C₄-Alkoxy)₂P(=E)O; R¹¹SO₃; R⁴⁰R¹⁴R³⁸N⁺; Phenyl, Phenylthio, Phenoxy, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; C₃-C₆-Cycloalkyl; 2-Tetrahydropyranyloxy; oder C(=Q)R₄O;
E O oder S;
Q O oder N-T-W;
T O; NR³⁷; oder eine direkte Bindung;
W H; C₁-C₈-Alkyl , C₃-C₈-Alkenyl; Phenylmethyl, wahlweise am Phenyl-Ring substituiert mit R⁷ und am Benzyl-Kohlenstoff mit R¹⁴; Phenyl oder Pyridyl, jeweils wahlweise substituiert mit R⁷; C(=O)R²⁸; C(=O)OR²⁸; oder C(=O)NR²⁸R¹⁴;
R²³ 1-3 Halogen; C₁-C₁₂-Alkoxy; C₁-C₁₂-Alkylthio; Phenyl oder Naphthalenyl, jeweils wahlweise substituiert mit R³⁴; oder Phenoxymethyl, wahlweise am Phenyl-Ring substituiert mit R³⁴;
R²⁷ 1-2 Halogen; Nitro; Cyano; C₁-C₆-Alkyl; C₁-C₆-Halogenalkyl; C₁-C₆-Alkoxy; C₁-C₆-Halogenalkoxy; C₁-C₄-Alkylsulfonyl; C₂-C₆-Alkoxyalkyl; C₁-C₄-Alkylthio; C₅-C₆-Cycloalkyl; C₅-C₆-Cycloalkyloxy; C₂-C₆-Alkenyl; C₂-C₆-Halogenalkenyl; C₂-C₆-Alkinyl; Hydroxycarbonyl; C₂-C₄-Alkoxycarbonyl; oder Phenoxy, wahlweise substituiert mit R²⁴;
R²⁸ C₁-C₈-Alkyl; oder Phenyl oder Pyridyl, jeweils wahlweise substituiert mit R³⁰;
R³⁰ 1-2 Substituenten, ausgewählt aus der Gruppe, bestehend aus: Halogen, Nitro, Cyano, C₁-C₄-Alkyl, Trifluormethyl, C₁-C₄-Alkoxy und Trifluormethoxy; oder Phenoxy, wahlweise substituiert mit R²⁶;
R³¹ 1-3 Halogen; C₁-C₁₈-Alkoxy; Allyloxy; C₁-C₁₈-Alkylthio; Phenyl, Phenoxy, Benzyloxy, oder Phenylthio, jeweils wahlweise am Phenyl-Ring substituiert mit R³⁴; Acetyl; oder C₂-C₅-Alkoxycarbonyl;
R³² 1-3 Halogen; oder C₁-C₄-Alkoxy;
R³⁵ Cyano; Nitro; C₁-C₁₇-Alkylthio; C₁-C₁₇-Alkylsulfinyl; C₁-C₁₇-Halogenalkoxy; C₅-C₆-Cycloalkyloxy; C₂-C₁₇-Halogenalkenyl; C₃-C₁₇-Alkenyloxy; C₃-C₁₇-Halogenalkinyl; C₃-C₁₇-Alkinyloxy; C₁-C₁₇-Alkylsulfonyl; C₂-C₁₇-Alkoxycarbonyl; Hydroxyl; Hydroxycarbonyl; R²⁸C(=O)O; R²⁸OC(=O)O; R²⁸R⁴⁰NC(=O)O; R⁴⁰R³⁹N; (C₁-C₄-Alkoxy)₂P(=E)O; R¹¹SO₃; R⁴⁰R¹⁴R³⁸N⁺; Phenyl, Phenoxy, Phenylthio, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; 2-Tetrahydropyranyloxy; C₁-C₁₇-Alkoxy; C₂-C₁₇-Alkoxyalkoxy; C₃-C₁₇-Alkinyl; C₃-C₆-Cycloalkyl; oder C₂-C₁₇-Halogenalkoxyalkoxy;
R³⁷ H; C₁-C₆-Alkyl; oder Phenyl, wahlweise substituiert mit R⁷;
R³⁹ C₁-C₁₉-Alkyl; C₂-C₁₉-Alkylcarbonyl; C₂-C₁₉-Alkoxycarbonyl; (R⁹R⁴⁰N)C=O; Phenyl, wahlweise substituiert mit R²⁵; oder Phenoxycarbonyl, wahlweise substituiert mit R⁷;
R⁴¹ Cyano; Nitro; C₁-C₁₇-Alkylthio; C₁-C₁₇-Alkylsulfinyl; C₁-C₁₇-Halogenalkoxy; C₅-C₆-Cycloalkyloxy; C₃-C₁₇-Alkenyloxy; C₃-C₁₇-Alkinyloxy; C₁-C₁₇-Alkylsulfonyl; C₂-C₁₇-Alkoxycarbonyl; Hydroxyl; Hydroxycarbonyl; R²⁸C(=O)O; R²⁸OC(=O)O; R²⁸R⁴⁰NC(=O)O; R⁴⁰R³⁹N; (C₁-C₄-Alkoxy)₂P(=E)O; R¹¹SO₃; R⁴⁰R¹⁴R³⁸N⁺; Phenyl, Phenoxy, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; 2-Tetrahydropyranyloxy; C₁-C₁₇-Alkoxy; 1-3-Halogen; C₂-C₁₇-Alkoxyalkoxy; oder C₃-C₆-Cycloalkyl;
R⁴² Cyano; Nitro; C₁-C₁₇-Alkylthio; C₁-C₁₇-Alkylsulfinyl; C₁-C₁₇-Halogenalkoxy; C₅-C₆-Cycloalkyloxy; C₃-C₁₇-Alkenyloxy; C₃-C₁₇-Halogenalkinyl; C₃-C₁₇-Alkinyloxy; C₁-C₁₇-Alkylsulfonyl; C₂-C₁₇-Alkoxycarbonyl; Hydroxyl; Hydroxycarbonyl; R²⁸C(=O)O; R²⁸OC(=O)O; R²⁸R⁴⁰NC(=O)O; R⁴⁰R³⁹N; (C₁-C₄-Alkoxy)₂P(=E)O; R¹¹SO₃; R⁴⁰R¹⁴R³⁸N⁺; Phenyl, Phenoxy, Phenylthio, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; 2-Tetrahydropyranyloxy; C₁-C₁₇-Alkoxy; 1-3 Halogen; C₂-C₁₇-Alkoxyalkoxy; C₃-C₁₇-Alkinyl; oder C₃-C₆-Cycloalkyl;
unter der Voraussetzung, daß:
i) wenn R² ein substituierter Phenyl- oder heterocyclischer Ring ist, dann lediglich H oder F an dem/den Kohlenstoffatom(en) des Phenyl- oder heterocyclischen Ringes angrenzend an dem Kohlenstoff, der den Oxazolidinon-Ring trägt, substituiert sein können;
ii) wenn R³ ein Phenyl- oder heterocyclischer Ring ist, disubstituiert mit zwei Alkyl- oder Alkoxy-Gruppen, oder einer Alkyl- und einer Alkoxy-Gruppe, dann mindestens eine der Alkyl- und Alkoxy-Gruppen Methyl oder Methoxy ist; sowie
iii) die Gesamtzahl der Kohlenstoffaktome in R², R¹⁶, R¹⁷ und R¹⁸ jeweils kleiner oder gleich 20 ist.

2. Fungizid aktive Verbindung nach Formel I, umfassend: 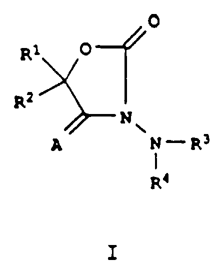 worin sind:
A S oder N-OG;
G H; C₁-C₆-Alkyl; Benzyl, wahlweise am Phenyl-Ring substituiert mit R³⁴; C(=O) (C₁-C₄-Alkyl); C(=O)(C₁-C₄-Alkoxy); oder C(=O)NHR³⁶;
R¹ C₁-C₄-Alkyl; C₁-C₄-Halogenalkyl; C₃-C₆-Cycloalkyl; C₂-C₄-Alkenyl; C₂-C₄-Alkoxycarbonyl ; oder Phenylmethyl, wahlweise am Phenyl-Ring substituiert mit R⁶ und am Benzyl-Kohlenstoff mit R⁸;
R² C₁-C₆-Alkyl; C₅-C₇-Cycloalkyl; C₂-C₆-Alkenyl; C₅-C₇-Cycloalkenyl; Phenyl, wahlweise substituiert mit R⁵ und R⁷; 2-Naphthalenyl; Thienyl, wahlweise substituiert mit R⁵ und R⁷; Furyl, wahlweise substituiert mit R⁷; oder Pyridyl, wahlweise substituiert mit R⁷; oder Phenoxy oder Phenylthio, jeweils wahlweise substituiert mit R⁷; oder
R¹ und R² bilden gemeinsam Strukturen, ausgewählt aus der Gruppe, bestehend aus: -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 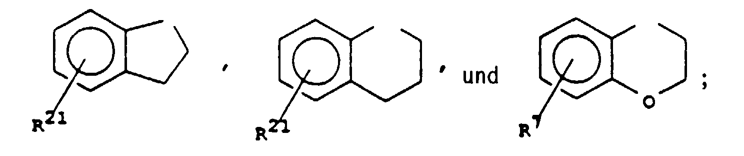
R³ Phenyl, Pyridyl oder Pyrimidinyl, jeweils wahlweise substituiert mit R¹⁰; oder Phenylmethyl;
R⁴ H oder Methyl;
R⁵ Halogen; Nitro; Cyano; C₁-C₆-Alkyl; C₅-C₆-Cycloalkyl; C₁-C₆-Halogenalkyl; C₁-C₆-Alkylthio; C₁-C₆-Halogenalkylthio; C₁-C₆-Alkoxy; C₁-C₆-Halogenalkoxy; C₅-C₆-Cycloalkyl oxy; C₂-C₆-Alkoxyalkyl; C₂-C₆-Alkoxyalkoxy; C₃-C₆-Alkenyl ; C₃-C₆-Halogenalkenyl; C₃-C₆-Alkenyloxy; C₃-C₆-Alkinyl; C₃-C₆-Halogenalkinyl; C₃-C₆-Alkinyloxy; C₁-C₆-Alkylsulfonyl; C₁-C₆-Halogenalkylsulfonyl; Phenyl oder Phenylthio, jeweils wahlweise substituiert mit R²⁴; Phenylmethyl, Phenoxymethyl, Phenethyl, oder Styryl, jeweils wahlweise substituiert am Phenyl-Ring mit R²⁴; Phenoxy, wahlweise substituiert mit R²⁷; und C₂-C₆-Alkoxycarbonyl;
R⁶, R⁷, R²⁴, R²⁶ und R³⁴ unabhängig 1-2 Halogen; Nitro; C₁-C₄-Alkyl; Trifluormethyl; Methylthio; oder C₁-C₄-Alkoxy;
R⁸ H oder C₁-C₄-Alkyl;
R¹⁰ 1-2 Substituenten, ausgewählt aus der Gruppe, bestend aus: Halogen, Nitro, Cyano, C₁-C₄-Alkyl, Trifluormethyl, C₁-C₄-Alkylthio, C₁-C₄-Alkoxy und Trifluormethoxy;
R²¹ Halogen; Nitro; C₁-C₄-Alkyl; Trifluormethyl; Methylthio; oder C₁-C₄-Alkoxy; oder Phenoxy, wahlweise substituert mit R²⁶;
R²⁷ 1-2 Halogen; Nitro; Cyano; C₁-C₆-Alkyl; C₁-C₆-Halogenalkyl; C₁-C₆-Alkoxy; C₁-C₆-Halogenalkoxy; C₁-C₄-Alkylsulfonyl; C₂-C₆-Alkoxyalkyl; C₁-C₄-Alkylthio; C₅-C₆-Cycloalkyl; C₅-C₆-Cycloalkyloxy; C₂-C₆-Alkenyl; C₂-C₆-Halogenalkenyl; C₂-C₆-Alkinyl; C₂-C₄-Alkoxycarbonyl; oder Phenoxy; und
R³⁶ C₁-C₆-Alkyl; oder Phenyl, wahlweise substituiert mit R¹².

3. Fungizid aktive Verbindung nach Anspruch 1, worin sind:
J H; OH; C(=O)R¹⁶; C(=O)OR¹⁷; C(=O)NHR¹⁹;
R¹ C₁-C₄-Alkyl; C₁-C₄-Halogenalkyl; oder Vinyl;
R² C₁-C₂₀-Alkyl; C₂-C₂₀-Alkoxyalkyl; C₂-C₂₀-Halogenalkyl; C₃-C₈-Alkyl, substituiert mit Phenoxy oder Phenylthio, jeweils wahlweise substituiert mit R³⁰; C₅-C₇-Cycloalkyl; C₂-C₂₀-Alkenyl; C₅-C₇-Cycloalkenyl; Phenyl, wahlweise substituiert mit R⁵ und R⁷; 2-Naphthalenyl; Thienyl, wahlweise substituiert mit R⁵ und R⁷; Furyl, wahlweise substituiert mit R⁷; oder Pyridyl, wahlweise substituiert mit R⁵ und R⁷;
R³ Phenyl, wahlweise substituiert mit R¹⁰;
R¹⁶ H; C₁-C₆-Alkyl; oder Phenyl, wahlweise substituiert mit R³⁴;
R¹⁷ C₁-C₆-Alkyl;
R¹⁹ C₁-C₆-Alkyl; oder Phenyl, wahlweise substituiert mit R³⁴; unter der Voraussetzung, daß, wenn R² Phenyl und R⁵ nicht F ist, dann
R⁵ sich in para-Stellung zu dem Oxazolidinon-Ring befindet.

4. Fungizid aktive Verbindung nach Anspruch 3, worin sind:
J H; OH oder C(=O)R¹⁶;
R¹ C₁-C₂-Alkyl oder Vinyl;
R² C₁-C₁₂-Alkyl; C₅-C₇-Cycloalkyl; Phenyl, wahlweise substituiert mit R⁵ und R⁷; Thienyl, wahlweise substituiert mit R⁷; oder Pyridyl, wahlweise substituiert mit R⁵ und R⁷;
R³ Phenyl, wahlweise substituiert mit F; Cl; oder Methyl;
R⁴ H;
R⁵ Halogen; Nitro; C₁-C₆-Alkyl; C₁-C₃-Halogenalkyl; Methylthio; C₁-C₆-Alkoxy; C₁-C₂-Halogenalkoxy; C₅-C₆-Cycloalkyloxy; Phenoxy, wahlweise substituiert mit R²⁷; Phenylthio, wahlweise substituiert mit R²⁴; Phenoxymethyl, wahlweise am Phenyl-Ring substituiert mit R²⁴; Benzyloxy, wahlweise am Phenyl-Ring substituiert mit R³⁰ oder -OC(=O)NHR²⁸;
R⁷ und R²⁴ unabhängig F; C₁-C₂-Alkyl; Methylthio oder C₁-C₂-Alkoxy;
R¹⁶ C₁-C₆-Alkyl;
R¹⁹ Phenyl, wahlweise substituiert mit R³⁴;
R²⁷ 1-2 Halogen; Cyano; C₁-C₄-Alkyl; Trifluormethyl; C₁-C₄-Alkoxy; C₁-C₄-Halogenalkoxy; C₁-C₄-Alkylthio; C₅-C₆-Cycloalkyloxy; oder Allyl;
R³⁰ 1-2 Halogen, Cyano, C₁-C₄-Alkyl, Trifluormethyl, C₁-C₄-Alkoxy oder Trifluormethoxy; und
R³⁴ 1-2 Halogen; Nitro; C₁-C₂-Alkyl; oder C₁-C₂-Alkoxy.

5. Fungizid aktive Verbindung nach Anspruch 4, worin sind:
J H;
R¹ Methyl;
R² C₁-C₁₂-Alkyl; Phenyl, wahlweise substituiert mit R⁵ und R⁷; Pyridyl, wahlweise substituiert mit R⁵ und R⁷; und
R⁵ F; Cl; Br; C₁-C₆-Alkyl; Trifluormethyl; C₁-C₆-Alkoxy; Trifluormethoxy; 2,2,2-Trifluorethoxy; C₅-C₆-Cycloalkyloxy; Methylthio; Phenoxy, wahlweise substituiert mit R²⁷; Phenylthio, wahlweise substituiert mit R²⁴; Benzyloxy, wahlweise am Phenyl-Ring substituiert mit R³⁰; oder -OC(=O)R²⁸.

6. Fungizid aktive Verbindung nach Anspruch 5, ausgewählt aus der Gruppe, bestehend aus:
5-(2,4-Difluorphenyl)-5-methyl-3-(phenylamino)-4-thioxo-2-oxazolidinon;
4-Imino-5-methyl-5-(4-phenoxyphenyl)-3-(phenylamino)-2-oxazolidinon;
4-Imino-5-methyl-5-(4-phenoxyphenyl)-3-(phenylamino)-2-oxazolidinonhydrobromid; und
5-(2,4-Difluorphenyl)-4-imino-5-methyl-3-(phenylamino)-2-oxazolidinon.

7. Fungizid aktive Zusammensetzung, umfassend die Verbindungen nach einem der Ansprüche 1 bis 6.

8. Verfahren zur Krankheitsbekämpfung in Pflanzen, umfassend das Aufbringen einer Fungizid aktiven Verbindung auf die Pflanzen, welche Verbindung die Formel I hat 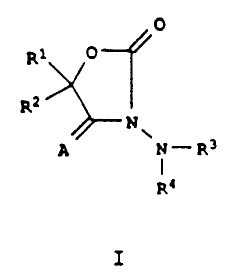 worin sind:
A S oder N-J;
J R¹⁵;C(=O)R¹⁶;C(=O)OR¹⁷; C(=O)SR¹⁸; C(=O)NR¹⁹R²⁰;P(=O)(C₁-C₄-Alkyl)₂; oder OG;
G H; C₁-C₆-Alkyl; Benzyl, wahlweise am Phenyl-Ring substituiert mit R³⁴; C(=O)(C₁-C₄-Alkyl); C(=O)(C₁-C₄-Alkoxy); oder C(=O)NHR³⁶;
R¹ C₁-C₄-Alkyl; C₁-C₄-Halogenalkyl; C₃-C₆-Cycloalkyl; C₂-C₄-Alkenyl; C₂-C₄-Alkoxycarbonyl; oder Phenylmethyl, wahlweise am Phenyl-Ring substituiert mit R⁶ und am Benzyl-Kohlenstoff mit R⁸;
R² C₁-C₂₀-Alkyl, wahlweise substituiert mit R²²; C₂-C₂₀-Alkoxyalkyl, wahlweise substituiert mit R³⁵; C₂-C₂₀-Alkenyl, wahlweise substituiert mit R⁴²; C₂-C₂₀-Alkinyl, wahlweise substituiert mit R⁴¹; (CH₂CH₂OCH₂CH₂)CH-; (CH₂CH₂SCH₂CH₂)CH-; (CH₂CH₂SO₂CH₂CH₂)CH-; C₅-C₇-Cycloalkyl; C₅-C₇-Cycloalkenyl; Phenyl, wahlweise substituiert mit R⁵ und R⁷; 2-Naphthalenyl; Thienyl , wahlweise substituiert mit R⁵ und R⁷; Furyl, wahlweise substituiert mit R⁷; oder Pyridyl, wahlweise substituiert mit R⁵ und R⁷; oder
R¹ und R² können gemeinsam Strukturen bilden, ausgewählt aus der Gruppe, bestehend aus -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 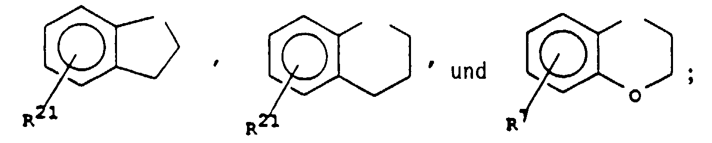
R³ Phenyl, Pyridyl, oder Pyrimidinyl, jeweils wahlweise substituiert mit R¹⁰ oder Phenylmethyl;
R⁴ H oder Methyl;
R⁵ Halogen; Nitro; Cyano; C₁-C₆-Alkyl; C₅-C₆-Cycloalkyl; C₁-C₆-Halogenalkyl; C₁-C₆-Alkylthio; C₁-C₆-Halogenalkylthio; C₁-C₆-Alkoxy; C₁-C₆-Halogenalkoxy; C₅-C₆-Cycloalkyloxy; C₂-C₆-Alkoxyalkyl ; C₂-C₆-Alkoxyalkoxy; C₃-C₆-Alkenyl; C₃-C₆-Halogenalkenyl; C₃-C₆-Alkenyloxy; C₃-C₆-Alkinyl; C₃-C₆-Halogenalkinyl; C₃-C₆-Alkinyloxy; C₁-C₆-Alkylsulfonyl; C₁-C₆-Halogenalkylsulfonyl; Phenyl oder Phenylthio, jeweils wahlweise substituiert mit R²⁶; Phenylmethyl, Phenoxymethyl, Phenethyl, oder Styryl, jeweils wahlweise am Phenyl-Ring substituiert mit R²⁶; Phenoxy, wahlweise substituiert mit R²⁷; Benzyloxy, wahlweise am Phenyl-Ring substituiert mit R³⁰; -OC(=O)NHR²⁸; -C(=O)OR²⁸; oder -OC(=O)R²⁸;
R⁶, R⁷, R¹², R¹³, R²⁴, R²⁶ und R³⁴ unabhängig 1-2 Halogen; Nitro; C₁-C₄-Alkyl; Trifluormethyl; Methylthio; oder C₁-C₄-Alkoxy;
R⁸, R¹⁴, R²⁰, R⁴⁰ und R³⁸ unabhängig H oder C₁-C₄-Alkyl;
R⁹ C₁-C₁₈-Alkyl; oder Phenyl, wahlweise substituiert mit R⁷;
R¹⁰, R²⁵ und R³³ unabhängig 1-2 Substituenten, ausgewählt aus der Gruppe, bestehend aus Halogen, Nitro, Cyano, C₁-C₄-Alkyl, Trifluormethyl, C₁-C₄-Alkylthio, C₁-C₄-Alkoxy und Trifluormethoxy;
R¹¹ und R³⁶ unabhängig C₁-C₆-Alkyl; oder Phenyl, wahlweise substituiert mit R¹²;
R¹⁵ H; C₁-C₈-Alkyl, wahlweise substituiert mit C₁-C₂-Alkoxy; C₃-C₆-Cycloalkyl; C₃-C₈-Alkenyl ; C₃-C₈-Alkinyl; Phenyl, wahlweise substituiert mit R¹³; Benzyl, wahlweise am Phenyl-Ring substituiert mit R¹³ und am Benzyl-Kohlenstoff mit R²⁰; oder Pyridyl, wahlweise substituiert mit R¹³;
R¹⁶ H; C₁-C₁₇-Alkyl, wahlweise substituiert mit R³¹; C₂-C₁₇-Alkenyl, wahlweise substituiert mit R³²; C₂-C₇-Alkinyl; C₃-C₈-Cycloalkyl; C₅-C₆-Cycloalkenyl; C₆-C₇-Alkylcycloalkyl; C₄-C₈-Cycloalkylalkyl; Phenyl, wahlweise substituiert mit R³³; Naphthalenyl, Furanyl, Thienyl, Benzoyl oder Pyridyl, jeweils s wahlweise substituiert mit R³⁴; oder C₂-C₅-Alkoxycarbonyl;
R¹⁷ und R¹⁸ unabhängig C₁-C₁₈-Alkyl, wahlweise substituiert mit R²³; C₂-C₁₀-Alkenyl, wahlweise substituiert mit R³²; C₃-C₈-Alkinyl; C₃-C₁₂-Cycloalkyl; C₅-C₆-Cycloalkenyl; C₆-C₇-Alkylcycloalkyl; C₆-C₇-Cycloalkylalkyl; oder Phenyl, Naphthalenyl, oder Thienyl, jeweils wahlweise substituiert mit R³⁴;
R¹⁹ H; C₁-C₁₀-Alkyl; C₅-C₆-Cycloalkyl; oder Phenyl, wahlweise substituiert mit R³⁴; oder
R¹⁹ und R²⁰ können gemeinsam Strukturen bilden, ausgewählt aus der Gruppe, bestehend aus:
-CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, und -CH₂CH(Me)OCH(Me)CH₂-;
R²¹ 1-2 Halogen; Nitro; C₁-C₄-Alkyl; Trifluormethyl; Methylthio; oder C₁-C₄-Alkoxy; oder Phenoxy, wahlweise substituiert mit R²⁶;
R²² Cyano; Nitro; C₁-C₁₉-Alkylthio; C₁-C₁₉-Alkylsulfinyl; C₁-C₁₉-Halogenalkoxy; C₅-C₆-Cycloalkyloxy; C₃-C₁₉-Alkenyloxy; C₃-C₁₉-Alkinyloxy; C₁-C₁₉-Alkylsulfonyl; C₂-C₁₉-Alkoxycarbonyl; Hydroxyl; Hydroxycarbonyl; R²⁸C(=O)O; R²⁸OC(=O)O; R²⁸R⁴⁰NC(=O)O; R³⁹R⁴⁰N; (C₁-C₄-Alkoxy)₂P(=E)O; R¹¹SO₃; R⁴⁰R¹⁴R³⁸N⁺; Phenyl, Phenylthio, Phenoxy, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; C₃-C₆-Cycloalkyl; 2-Tetrahydropyranyloxy; oder C(=Q)R⁴⁰;
E O oder S;
Q O oder N-T-W;
T O; NR³⁷; oder eine direkte Bindung;
W H; C₁-C₈-Alkyl, C₃-C₈-Alkenyl; Phenylmethyl, wahlweise am Phenyl-Ring substituiert mit R⁷ und am Benzyl-Kohlenstoff mit R¹⁴; Phenyl oder Pyridyl, jeweils wahlweise substituiert mit R⁷; C(=O)R²⁸; C(=O)OR²⁸; oder C(=O)NR²⁸R¹⁴;
R²³ 1-3 Halogen; C₁-C₁₂-Alkoxy; C₁-C₁₂-Alkylthio; Phenyl oder Naphthalenyl, jeweils wahlweise substituiert mit R³⁴; oder Phenoxymethyl, wahlweise am Phenyl-Ring substituiert mit R³⁴;
R²⁷ 1-2 Halogen; Nitro; Cyano; C₁-C₆-Alkyl; C₁-C₆-Halogenalkyl; C₁-C₆-Alkoxy; C₁-C₆-Halogenalkoxy; C₁-C₄-Alkylsulfonyl; C₂-C₆-Alkoxyalkyl; C₁-C₄-Alkylthio; C₅-C₆-Cycloalkyl; C₅-C₆-Cycloalkyloxy; C₂-C₆-Alkenyl; C₂-C₆-Halogenalkenyl; C₂-C₆-Alkinyl; Hydroxycarbonyl; C₂-C₄-Alkoxycarbonyl; oder Phenoxy, wahlweise substituiert mit R²⁴;
R²⁸ C₁-C₈-Alkyl; oder Phenyl oder Pyridyl, jeweils wahlweise substituiert mit R³⁰;
R³⁰ 1-2 Substituenten, ausgewählt aus der Gruppe, bestehend aus: Halogen, Nitro, Cyano, C₁-C₄-Alkyl, Trifluormethyl, C₁-C₄-Alkoxy und Trifluormethoxy; oder Phenoxy, wahlweise substituiert mit R²⁶;
R³¹ 1-3 Halogen; C₁-C₁₈-Alkoxy; Allyloxy; C₁-C₁₈-Alkylthio; Phenyl, Phenoxy, Benzyloxy oder Phenylthio, jeweils wahlweise am Phenyl-Ring substituiert mit R³⁴; Acetyl; oder C₂-C₅-Alkoxycarbonyl;
R³² 1-3 Halogen; oder C₁-C₄-Alkoxy;
R³⁵ Cyano; Nitro; C₁-C₁₇-Alkylthio; C₁-C₁₇-Alkylsulfinyl; C₁-C₁₇-Halogenalkoxy; C₅-C₆-Cycloalkyloxy; C₂-C₁₇-Halogenalkenyl; C₃-C₁₇-Alkenyloxy; C₃-C₁₇-Halogenalkinyl; C₃-C₁₇-Alkinyloxy; C₁-C₁₇-Alkylsulfonyl; C₂-C₁₇-Alkoxycarbonyl; Hydroxyl; Hydroxycarbonyl; R²⁸C(=O)O; R²⁸OC(=O)O; R²⁸R⁴⁰NC(=O)O; R⁴⁰R³⁹N; (C₁-C₄-Alkoxy)₂P(=E)O; R¹¹SO₃; R⁴⁰R¹⁴R³⁸N⁺; Phenyl, Phenoxy, Phenylthio, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; 2-Tetrahydropyranyloxy; C₁-C₁₇-Alkoxy; C₂-C₁₇-Alkoxyalkoxy; C₃-C₁₇-Alkinyl; C₃-C₆-Cycloalkyl; oder C₂-C₁₇-Halogenalkoxyalkoxy;
R³⁷ H; C₁-C₆-Alkyl; oder Phenyl, wahlweise substituiert mit R⁷;
R³⁹ C₁-C₁₉-Alkyl; C₂-C₁₉-Alkylcarbonyl; C₂-C₁₉-Alkoxycarbonyl; (R⁹R⁴⁰N)C=O; Phenyl, wahlweise substituiert mit R²⁵; oder Phenoxycarbonyl, wahlweise substituiert mit R⁷;
R⁴¹ Cyano; Nitro; C₁-C₁₇-Alkylthio; C₁-C₁₇-Alkylsulfinyl; C₁-C₁₇-Halogenalkoxy; C₅-C₆-Cycloal kyloxy; C₃-C₁₇-Alkenyloxy; C₃-C₁₇-Alkinyloxy; C₁-C₁₇-Alkylsulfonyl; C₂-C₁₇-Alkoxycarbonyl; Hydroxyl; Hydroxycarbonyl; R²⁸C(=O)O; R²⁸OC(=O)O; R²⁸R⁴⁰NC(=O)O; R⁴⁰R³⁹N; (C₁-C₄-Alkoxy)₂P(=E)O; R¹¹SO₃; R⁴⁰R¹⁴R³⁸N⁺; Phenyl, Phenoxy, Phenylsulfonyl, Phenylsulfinyl, Pyridyl oder Pyridyloxy, wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; 2-Tetrahydropyranyloxy; C₁-C₁₇-Alkoxy; 1-3 Halogen; C₂-C₁₇-Alkoxyalkoxy; oder C₃-C₆-Cycloalkyl;
R⁴² Cyano; Nitro; C₁-C₁₇-Alkylthio; C₁-C₁₇-Alkylsulfinyl; C₁-C₁₇-Halogenal koxy; C₅-C₆-Cycloalkyloxy; C₃-C₁₇-Alkenyloxy; C₃-C₁₇-Halogenalkinyl; C₃-C₁₇-Alkinyl oxy; C₁-C₁₇-Alkylsulfonyl; C₂-C₁₇-Alkoxycarbonyl; Hydroxyl; Hydroxycarbonyl; R²⁸C(=O)O; R²⁸OC(=O)O; R²⁸R⁴⁰NC(=O)O; R⁴⁰R³⁹N; (C₁-C₄-Alkoxy)₂P(=E)O; R¹¹SO₃; R⁴⁰R¹⁴R³⁸N⁺; Phenyl, Phenoxy, Phenylthio, Phenylsulfonyl, Phenylsulfinyl, Pyridyl oder Pyridyloxy, jeweils wahlweise substituiert mit R³⁰; Thienyl, Pyrimidinyl, Furanyl, Naphthalenyl, Pyrimidinyloxy, Naphthalenyloxy, jeweils wahlweise substituiert mit R⁷; Tetrahydropyranyl; 2-Tetrahydropyranyloxy; C₁-C₁₇-Alkoxy; 1-3 Halogen; C₂-C₁₇-Alkoxyalkoxy; C₃-C₁₇-Alkinyl; oder C₃-C₆-Cycloalkyl;
unter der Voraussetzung, daß,
i) wenn R² ein substituierter Phenyl- oder heterocyclischer Ring ist, dann lediglich H oder F an dem/den Kohlenstoffatom(en) des Phenyl- oder heterocyclischen Ringes angrenzend an dem Kohlenstoff, der den Oxazolidinon-Ring trägt, substituiert sein können;
ii) wenn R³ ein Phenyl- oder heterocyclischer Ring ist, disubstituiert mit zwei Alkyl- oder Alkoxy-Gruppen, oder eine Alkyl- und eine Alkoxy-Gruppe, dann mindestens eine der Alkyl- und Alkoxy-Gruppen Methyl oder Methoxy ist; und
iii) die Gesamtzahl der Kohlenstoffatome in R², R¹⁶, R¹⁷ und R¹⁸ jeweils kleiner oder gleich 20 ist.

9. Verfahren zur Krankheitsbekämpfung in Pflanzen, umfassend das Aufbringen einer Fungizid aktiven Verbindung der Formel I auf die Pflanzen; 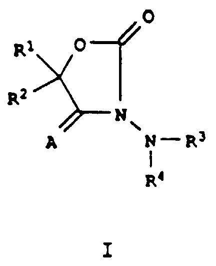 worin sind:
A S oder N-OG;
G H; C₁-C₆-Alkyl; Benzyl, wahlweise am Phenyl-Ring substituiert mit R³⁴; C(=O)(C₁-C₄-Alkyl); C(=O)(C₁-C₄-Alkoxy); oder C(=O)NHR³⁶;
R¹ C₁-C₄-Alkyl; C₁-C₄-Halogenalkyl; C₃-C₆-Cycloalkyl; C₂-C₄-Alkenyl; C₂-C₄-Alkoxycarbonyl; oder Phenylmethyl, wahlweise am Phenyl-Ring substituiert mit R⁶ und am Benzyl-Kohlenstoff mit R⁸;
R² C₁-C₆-Alkyl; C₅-C₇-Cycloalkyl; C₂-C₆-Alkenyl; C₅-C₇-Cycloalkenyl; Phenyl, wahlweise substituiert mit R⁵ und R⁷; 2-Naphthalenyl; Thienyl, wahlweise substituiert mit R⁵ und R⁷; Furyl, wahlweise substituiert mit R⁷; oder Pyridyl, wahlweise substituiert mit R⁷; oder Phenoxy oder Phenylthio, jeweils wahlweise substituiert mit R⁷; oder
R¹ und R² können gemeinsam Strukturen bilden, ausgewählt aus der Gruppe, bestehend aus -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 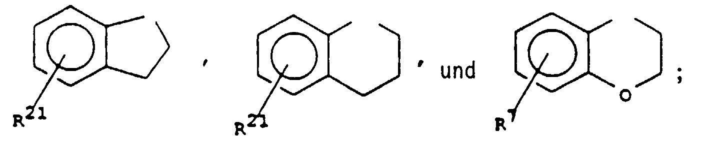
R³ Phenyl, Pyridyl oder Pyrimidinyl, jeweils wahlweise substituiert mit R¹⁰; oder Phenylmethyl;
R⁴ H oder Methyl;
R⁵ Halogen; Nitro; Cyano; C₁-C₆-Alkyl; C₅-C₆-Cycloalkyl; C₁-C₆-Halogenalkyl; C₁-C₆-Alkylthio; C₁-C₆-Halogenalkylthio; C₁-C₆-Alkoxy; C₁-C₆-Halogenalkoxy; C₅-C₆-Cycloalkyloxy; C₂-C₆-Alkoxyalkyl; C₂-C₆-Alkoxyalkoxy; C₃-C₆-Alkenyl; C₃-C₆-Halogenalkenyl; C₃-C₆-Alkenyloxy; C₃-C₆-Alkinyl ; C₃-C₆-Halogenalkinyl ; C₃-C₆-Alkinyloxy; C₁-C₆-Alkylsulfonyl; C₁-C₆-Halogenalkylsulfonyl; Phenyl oder Phenylthio, jeweils wahlweise substituiert mit R²⁴; Phenylmethyl, Phenoxymethyl, Phenethyl oder Styryl, jeweils wahlweise am Phenyl-Ring substituiert mit R²⁴; Phenoxy, wahlweise substituiert mit R²⁷; und C₂-C₆-Alkoxycarbonyl;
R⁶, R⁷, R²⁴, R²⁶ und R³⁴ unabhängig 1-2 Halogen; Nitro; C₁-C₄-Alkyl; Trifluormethyl; Methylthio; oder C₁-C₄-Alkoxy;
R⁸ H oder C₁-C₄-Alkyl;
R¹⁰ 1-2 Substituenten, ausgewählt aus der Gruppe, bestehend aus: Halogen, Nitro, Cyano, C₁-C₄-Alkyl, Trifluormethyl, C₁-C₄-Alkylthio, C₁-C₄-Alkoxy und Trifluormethoxy;
R²¹ Halogen; Nitro; C₁-C₄-Alkyl; Trifluormethyl; Methylthio; oder C₁-C₄-Alkoxy; oder Phenoxy, wahlweise substituiert mit R²⁶;
R²⁷ 1-2 Halogen; Nitro; Cyano; C₁-C₆-Alkyl; C₁-C₆-Halogenalkyl; C₁-C₆-Alkoxy; C₁-C₆-Halogenalkoxy; C₁-C₄-Alkylsulfonyl ; C₂-C₆-Alkoxyalkyl; C₁-C₄-Alkylthio; C₅-C₆-Cycloalkyl; C₅-C₆-Cycloalkyloxy; C₂-C₆-Alkenyl; C₂-C₆-Halogenalkenyl; C₂-C₆-Alkinyl; C₂-C₄-Alkoxycarbonyl; oder Phenoxy; und
R³⁶ C₁-C₆-Alkyl; oder Phenyl, wahlweise substituiert mit R¹².

10. Verfahren nach Anspruch 8, worin sind:
J H; OH; C(=O)R¹⁶; C(=O)OR¹⁷ oder C(=O)NHR¹⁹;
R¹ C₁-C₄-Alkyl ; C₁-C₄-Halogenalkyl; oder Vinyl;
R² C₁-C₂₀-Alkyl; C₂-C₂₀-Alkoxyal kyl; C₂-C₂₀-Halogenal kyl; C₃-C₈-Alkyl, substituiert mit Phenoxy, oder Phenylthio, jeweils wahlweise substituiert mit R³⁰; C₅-C₇-Cycloalkyl ; C₂-C₂₀-Alkenyl; C₅-C₇-Cycloalkenyl; Phenyl, wahlweise substituiert mit R⁵ und R⁷; 2-Naphthalenyl; Thienyl, wahlweise substituiert mit R⁵ und R⁷; Furyl, wahlweise substituiert mit R⁷; oder Pyridyl, wahlweise substituiert mit R⁵ und R⁷;
R³ Phenyl, wahlweise substituiert mit R¹⁰;
R¹⁶ H; C₁-C₆-Alkyl; oder Phenyl, wahlweise substituiert mit R³⁴;
R¹⁷ C₁-C₆-Alkyl;
R¹⁹ C₁-C₆-Alkyl; oder Phenyl, wahlweise substituiert mit R³⁴; unter der Voraussetzung, daß, wenn R² Phenyl und R⁵ nicht F ist, dann R⁵ sich in para-Stellung zum Oxazolidinon-Ring befindet.

11. Verfahren nach Anspruch 8, worin sind:
J H; OH; oder C(=O)R¹⁶;
R¹ C₁-C₂-Alkyl oder Vinyl;
R² C₁-C₁₂-Alkyl; C₅-C₇-Cycloalkyl; Phenyl, wahlweise substituiert mit R⁵ und R⁷; Thienyl, wahlweise substituiert mit R⁷; oder Pyridyl, wahlweise substituiert mit R⁵ und R⁷;
R³ Phenyl, wahlweise substituiert mit F; Cl; oder Methyl;
R⁴ H;
R⁵ Halogen; Nitro; C₁-C₆-Alkyl; C₁-C₃-Halogenalkyl; Methylthio; C₁-C₆-Alkoxy; C₁-C₂-Halogenalkoxy; C₅-C₆-Cycloalkyloxy; Phenoxy, wahlweise substituiert mit R²⁷; Phenylthio, wahlweise substituiert mit R²⁴; Phenoxymethyl, wahlweise am Phenyl-Ring substituiert mit R²⁴; Benzyloxy, wahlweise am Phenyl-Ring substituiert mit R³⁰; -OC(=O)NHR²⁸;
R⁷ und R²⁴ unabhängig F; C₁-C₂-Alkyl; Methylthio; oder C₁-C₂-Alkoxy;
R¹⁶ C₁-C₆-Alkyl;
R¹⁹ Phenyl, wahlweise substituiert mit R³⁴;
R²⁷ 1-2 Halogen; Cyano; C₁-C₄-Alkyl; Trifluormethyl; C₁-C₄-Alkoxy; C₁-C₄-Halogenalkoxy; C₁-C₄-Alkylthio; C₅-C₆-Cycloalkyloxy; oder Allyl;
R³⁰ 1-2 Halogen, Cyano, C₁-C₄-Alkyl, Trifluormethyl, C₁-C₄-Alkoxy oder Trifluormethoxy; und
R³⁴ 1-2 Halogen; Nitro; C₁-C₂-Alkyl; oder C₁-C₂-Alkoxy.

12. Verfahren nach Anspruch 11, worin sind:
J H;
R¹ Methyl;
R² C₁-C₁₂-Alkyl; Phenyl, wahlweise substituiert mit R⁵ und R⁷; Pyridyl, wahlweise substituiert mit R⁵ und R⁷; und
R⁵ F; Cl; Br; C₁-C₆-Alkyl; Trifluormethyl; C₁-C₆-Alkoxy; Trifluormethoxy; 2,2,2-Trifluorethoxy; C₅-C₆-Cycloalkyloxy; Methylthio; Phenoxy, wahlweise substituiert mit R²⁷; Phenylthio, wahlweise substituiert mit R²⁴; Benzyloxy, wahlweise am Phenyl-Ring substituiert mit R³⁰; oder -OC(=O)R²⁸.

13. Verfahren nach Anspruch 8, bei welchem die Verbindung ausgewählt wird aus der Gruppe, bestehend aus:
5-(2,4-Difluorphenyl)-5-methyl-3-(phenylamino)-4-thioxo-2-oxazolidinon;
4-Imino-5-methyl-5-(4-phenoxyphenyl)-3-(phenylamino)-2-oxazolidinon;
4-Imino-5-methyl-5-(4-phenoxyphenyl)-3-(phenylamino)-2-oxazolidinonhydrobromid; und
5-(2,4-Difluorphenyl)-4-imino-5-methyl-3-(phenylamino)-2-oxazolidinon.

14. Verfahren zur Krankheitsbekämpfung in Pflanzen, umfassend das Aufbringen einer Zusammensetzung auf die Pflanzen, welche Zusammensetzung eine Fungizid aktive Verbindung nach einem der Ansprüche 1 bis 6 umfaßt.

## Revendications

1. Un composé à activité fongicide de formule I, et ses sels qui sont appropriés à l'usage agricultural, 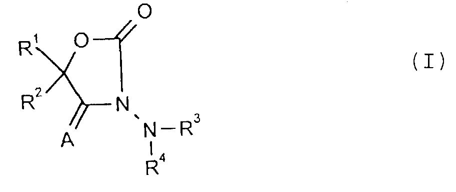 dans laquelle :
A est S ou N-J;
J est R¹⁵; C(=O)R¹⁶; C(=O)OR¹⁷; C(=O)SR¹⁸; C(=O)NR¹⁹R²⁰; P(=O) (alcoyl en C₁ à C₄)₂; ou OG;
G est H; un groupe alcoyl en C₁ à C₆; benzyle éventuellement substitué par R³⁴ sur le cycle phényle; C(=O)(alcoyl en C₁ à C₄); C(=O)(alcoxy en C₁ à C₄); ou C(=O)NHR³⁶;
R¹ est un groupe alcoyl en C₁ à C₄; halogénoalcoyl en C₁ à C₄; cycloalcoyl en C₃ à C₆; alcényle en C₂ à C₄; alcoxycarbonyle en C₂ à C₄; ou phénylméthyle éventuellement substitué par R⁶ sur le cycle phényle et par R⁸ sur le carbone benzylique;
R² est un groupe alcoyl en C₁ à C₂₀ éventuellement substitué par R²²; alcoxyalcoyl en C₂ à C₂₀ éventuellement substitué par R³⁵; alcényle en C₂ à C₂₀ éventuellement substitué par R⁴²; alcynyle en C₂ à C₂₀ éventuellement substitué par R⁴¹; (CH₂CH₂OCH₂CH₂)CH-; (CH₂CH₂SCH₂CH₂)CH-; (CH₂CH₂SO₂CH₂CH₂)CH-; cycloalcoyl en C₅ à C₇; cycloalcényle en C₅ à C₇; phényle éventuellement substitué par R⁵ et R⁷; 2-naphtalényle; thiényle éventuellement substitué par R⁵ et R⁷; furyle éventuellement substitué par R⁷; ou pyridyle éventuellement substitué par R⁵ et R⁷; ou bien
R¹ et R² peuvent, pris ensemble, former des structures choisies dans l'ensemble constitué par -CH₂(CH₂)₂CH₂-, -CH₂(CH₂)₃CH₂-, -CH₂(CH₂)₄CH₂-, -CH₂CH₂OCH₂CH₂-, CH₂CH₂SCH₂CH₂-, 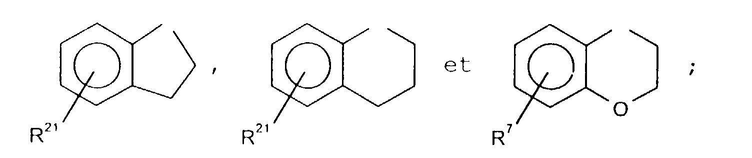
R³ est un groupe phényle, pyridyle ou pyrimidinyle, chacun éventuellement substitué par R¹⁰ ou un groupe phénylméthyle;
R⁴ est H ou un groupe méthyle;
R⁵ est un halogène ou un groupe nitro; cyano; alcoyl en C₁ à C₆; cycloalcoyl en C₅ à C₆; halogénoalcoyl en C₁ à C₆; alcoylthio en C₁ à C₆; halogénoalcoylthio en C₁ à C₆; alcoxy en C₁ à C₆; halogénoalcoxy en C₁ à C₆; cycloalcoyloxy en C₅ à C₆; alcoxyalcoyl en C₂ à C₆; alcoxyalcoxy en C₂ à C₆; alcényle en C₃ à C₆; halogénoalcényle en C₃ à C₆; alcényloxy en C₃ à C₆; alcynyle en C₃ à C₆; halogénoalcynyle en C₃ à C₆; alcynyloxy en C₃ à C₆; alcoylsulfonyle en C₁ à C₆; halogénoalcoylsulfonyle en C₁ à C₆; phényle ou phénylthio, chacun éventuellement substitué par R²⁶; phénylméthyle, phénoxyméthyle, phénéthyle ou styryle, chacun éventuellement substitué par R²⁶ sur le cycle phényle; phénoxy éventuellement substitué par R²⁷; benzyloxy éventuellement substitué par R³⁰ sur le cycle phényle; -OC(=O)NHR²⁸; -C(=O)OR²⁸; ou -OC(=O)R²⁸;
R⁶, R⁷, R¹², R¹³, R²⁴, R²⁶ et R³⁴ sont indépendamment un ou deux halogènes; un groupe nitro; alcoyl en C₁ à C₄; trifluorométhyle; méthylthio; ou alcoxy en C₁ à C₄;
R⁸, R¹⁴, R²⁰, R⁴⁰ et R³⁸ sont indépendamment H ou un groupe alcoyl en C₁ à C₄;
R⁹ est un groupe alcoyl en C₁ à C₁₈; ou phényle éventuellement substitué par R⁷;
R¹⁰, R²⁵ et R³³ sont indépendamment un ou deux substituants choisis dans l'ensemble constitué par les halogènes et les groupes nitro, cyano, alcoyl en C₁ à C₄, trifluorométhyle, alcoylthio en C₁ à C₄, alcoxy en C₁ à C₄ et trifluorométhoxy;
R¹¹ et R³⁶ sont indépendamment un groupe alcoyl en C₁ à C₆; ou phényle éventuellement substitué par R¹²;
R¹⁵ est H; un groupe alcoyl en C₁ à C₈ éventuellement substitué par un groupe alcoxy en C₁ à C₂; cycloalcoyl en C₃ à C₆; alcényle en C₃ à C₈; alcynyle en C₃ à C₈; phényle éventuellement substitué par R¹³; benzyle éventuellement substitué par R¹³ sur le cycle phényle et par R²⁰ sur le carbone benzylique; ou pyridyle éventuellement substitué par R¹³;
R¹⁶ est H; un groupe alcoyl en C₁ à C₁₇ éventuellement substitué par R³¹; alcényle en C₂ à C₁₇ éventuellement substitué par R³²; alcynyle en C₂ à C₇; cycloalcoyl en C₃ à C₈; cycloalcényle en C₅ à C₆; alcoylcycloalcoyl en C₆ à C₇; cycloalcoylalcoyl en C₄ à C₈; phényle éventuellement substitué par R³³; naphtalényle, furannyle, thiényle, benzoyle, ou pyridyle, chacun éventuellement substitué par R³⁴; ou alcoxycarbonyle en C₂ à C₅;
R¹⁷ et R¹⁸ sont indépendamment un groupe alcoyl en C₁ à C₁₈ éventuellement substitué par R²³; alcényle en C₂ à C₁₀ éventuellement substitué par R³²; alcynyle en C₃ à C₈; cycloalcoyl en C₃ à C₁₂; cycloalcényle en C₅ à C₆; alcoylcycloalcoyl en C₆ à C₇; cycloalcoylalcoyl en C₆ à C₇; ou phényle, naphtalényle, ou thiényle, chacun éventuellement substitué par R³⁴;
R¹⁹ est H; un groupe alcoyl en C₁ à C₁₀; cycloalcoyl en C₅ à C₆; ou phényle éventuellement substitué par R³⁴; ou bien
R¹⁹ et R²⁰ peuvent, pris ensemble, former des structures choisies dans l'ensemble constitué par -CH₂(CH₂)₂CH₂-, -CH₂(CH₂)₃CH₂-, -CH₂(CH₂)₄CH₂-, -CH₂CH₂OCH₂CH₂-, -CH₂CH(Me)CH₂CH(Me)CH₂-, et -CH₂CH(Me)OCH(Me)CH₂-;
R²¹ est un ou deux halogènes; un groupe nitro; alcoyl en C₁ à C₄; trifluorométhyle; méthylthio; ou alcoxy en C₁ à C₄; ou bien phénoxy éventuellement substitué par R²⁶;
R²² est un groupe cyano; nitro; alcoylthio en C₁ à C₁₉; alcoylsulfinyle en C₁ à C₁₉; halogénoalcoxy en C₁ à C₁₉; cycloalcoyloxy en C₅ à C₆; alcényloxy en C₃ à C₁₉; alcynyloxy en C₃ à C₁₉; alcoylsulfonyle en C₁ à C₁₉; alcoxycarbonyle en C₂ à C₁₉; hydroxy; hydroxycarbonyle; R²⁸C(=O)O; R²⁸OC(=O)O; R²⁸R⁴⁰NC(=O)O; R³⁹R⁴⁰N; (alcoxy en C₁ à C₄)₂P(=E)O; R¹¹SO₃; R⁴⁰R¹⁴R³⁸N⁺; phényle, phénylthio, phénoxy, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydro-pyrannyle; cycloalcoyl en C₃ à C₆; 2-tétrahydropyrannyloxy; ou C(=Q)R⁴⁰;
E est O ou S;
Q est O ou N-T-W;
T est O; NR³⁷; ou une liaison directe;
W est H; un groupe alcoyl en C₁ à C₈, alcényle en C₃ à C₈, phénylméthyle éventuellement substitué par R⁷ sur le cycle phényle et R¹⁴ sur le carbone benzylique; phényle ou pyridyle, chacun éventuellement substitué par R⁷; C(=O)R²⁸; C(=O)OR^{28;} ou C(=O)NR²⁸R¹⁴;
R²³ est 1 à 3 halogènes; un groupe alcoxy en C₁ à C₁₂; alcoylthio en C₁ à C₁₂; phényle ou naphtalényle, chacun éventuellement substitué par R³⁴; ou phénoxyméthyle éventuellement substitué par R³⁴ sur le cycle phényle;
R²⁷ est un ou deux halogènes; un groupe nitro; cyano; alcoyl en C₁ à C₆; halogénoalcoyl en C₁ à C₆; alcoxy en C₁ à C₆; halogénoalcoxy en C₁ à C₆; alcoylsulfonyle en C₁ à C₄; alcoxyalcoyl en C₂ à C₆; alcoylthio en C₁ à C₄; cycloalcoyl en C₅ à C₆; cycloalcoyloxy en C₅ à C₆; alcényle en C₂ à C₆; halogénoalcényle en C₂ à C₆; alcynyle en C₂ à C₆; hydroxycarbonyle; alcoxycarbonyle en C₂ à C₄; ou phénoxy éventuellement substitué par R²⁴;
R²⁸ est un groupe alcoyl en C₁ à C₈; phényle ou pyridyle, chacun éventuellement substitué par R³⁰;
R³⁰ est un ou deux substituants choisis dans l'ensemble constitué par les halogènes, les groupes nitro, cyano, alcoyl en C₁ à C₄, trifluorométhyle, alcoxy en C₁ à C₄ et trifluorométhoxy; ou phénoxy éventuellement substitué par R²⁶;
R³¹ est 1 à 3 halogènes; un groupe alcoxy en C₁ à C₁₈; allyloxy; alcoylthio en C₁ à C₁₈; phényle, phénoxy, benzyloxy, ou phénylthio, chacun éventuellement substitué par R³⁴ sur le cycle phényle; acétyle; ou alcoxycarbonyle en C₂ à C₅;
R³² est 1 à 3 halogènes; ou un groupe alcoxy en C₁ à C₄;
R³⁵ est un groupe cyano; nitro; alcoylthio en C₁ à C₁₇; alcoylsulfinyle en C₁ à C₁₇; halogénoalcoxy en C₁ à C₁₇; cycloalcoyloxy en C₅ à C₆; halogénoalcényle en C₂ à C₁₇; alcényloxy en C₃ à C₁₇; halogénoalcynyle en C₃ à C₁₇; alcynyloxy en C₃ à C₁₇; alcoylsulfonyle en C₁ à C₁₇; alcoxycarbonyle en C₂ à C₁₇; hydroxy; hydroxycarbonyle; R²⁸C(=O)O; R²⁸OC(=O)O; R²⁸R⁴⁰NC(=O)O; R⁴⁰R³⁹N; (alcoxy en C₁ à C₄)₂P(=E)O; R¹¹SO₃; R⁴⁰R¹⁴R³⁸N⁺; phényle, phénoxy, phénylthio, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydropyrannyle; 2-tétrahydropyrannyloxy; alcoxy en C₁ à C₁₇; alcoxyalcoxy en C₂ à C₁₇; alcynyle en C₃ à C₁₇; cycloalcoyl en C₃ à C₆; ou halogénoalcoxyalcoxy en C₂ à C₁₇;
R³⁷ est H; un groupe alcoyl en C₁ à C₆; ou phényle éventuellement substitué par R⁷;
R³⁹ est un groupe alcoyl en C₁ à C₁₉; alcoylcarbonyle en C₂ à C₁₉; alcoxycarbonyle en C₂ à C₁₉; (R⁹R⁴⁰N)C=O; phényle éventuellement substitué par R²⁵; ou phénoxycarbonyle éventuellement substitué par R⁷;
R⁴¹ est un groupe cyano; nitro; alcoylthio en C₁ à C₁₇; alcoylsulfinyle en C₁ à C₁₇; halogénoalcoxy en C₁ à C₁₇; cycloalcoyloxy en C₅ à C₆; alcényloxy en C₃ à C₁₇; alcynyloxy en C₃ à C₁₇; alcoylsulfonyle en C₁ à C₁₇; alcoxycarbonyle en C₂ à C₁₇; hydroxy; hydroxycarbonyle; R²⁸C(=O)O; R²⁸OC(=O)O; R²⁸R⁴⁰NC(=O)O; R⁴⁰R³⁹N; (alcoxy en C₁ à C₄)₂P(=E)O; R¹¹SO₃; R⁴⁰R¹⁴R³⁸N⁺; phényle, phénoxy, phénylsulfonyle, phénylsulfinyle, pyridyle ou pyridyloxy, chacun éventuellement substitué par R⁷; thiényle, pyrimidinyle, furannyle, naphtalényle, pyrimidinyloxy, naphtalényloxy, chacun éventuellement substitué par R⁷; tétrahydro-pyrannyle; 2-tétrahydropyrannyloxy; alcoxy en C₁ à C₁₇; alcoxyalcoxy en C₂ à C₁₇; cycloalcoyl en C₃ à C₆; ou 1 à 3 halogènes;
R⁴² est un groupe cyano; nitro; alcoylthio en C₁ à C₁₇; alcoylsulfinyle en C₁ à C₁₇; halogénoalcoxy en C₁ à C₁₇; cycloalcoyloxy en C₅ à C₆; alcényloxy en C₃ à C₁₇; halogénoalcynyle en C₃ à C₁₇; alcynyloxy en C₃ à C₁₇; alcoylsulfonyle en C₁ à C₁₇; alcoxycarbonyle en C₂ à C₁₇; hydroxy; hydroxycarbonyle; R²⁸C(=O)O; R²⁸OC(=O)O; R²⁸R⁴⁰NC(=O)O; R⁴⁰R³⁹N; (alcoxy en C₁ à C₄)₂P(=E)O; R¹¹SO₃; R⁴⁰R¹⁴R³⁸N⁺; phényle, phénoxy, phénylthio, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydropyrannyle; 2-tétrahydropyrannyloxy; alcoxy en C₁ à C₁₇; alcoxyalcoxy en C₂ à C₁₇; alcynyle en C₃ à C₁₇; cycloalcoyl en C₃ à C₆; ou 1 à 3 halogènes;
du moment que :
i) quand R² est un cycle hétérocyclique ou phényle substitué, alors seul H ou F peut être un substituant sur le ou les atomes de carbone du cycle hétérocyclique ou phényle adjacent au carbone portant le cycle oxazolidinone;
ii) quand R³ est un cycle hétérocyclique ou phényle disubstitué par deux groupes alcoyl ou alcoxy, ou par un groupe alcoyl et un groupe alcoxy, alors au moins l'un des groupes alcoyl et alcoxy est un groupe méthyle ou méthoxy; et
iii) le nombre total d'atome de carbones dans chacun de R², R¹⁶, R¹⁷ et R¹⁸ est inférieur ou égal à 20.

2. Un composé à action fongicide de formule I : 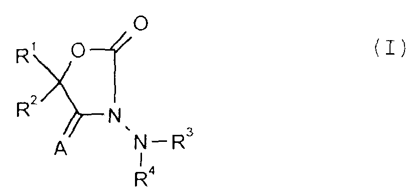 dans laquelle :
A est S ou N-OG;
G est H; un groupe alcoyl en C₁ à C₆; benzyle éventuellement substitué par R³⁴ sur le cycle phényle; C(=O) (alcoyl en C₁ à C₄); C(=O)(alcoxy en C₁ à C₄); ou C(=O)NHR³⁶;
R¹ est un groupe alcoyl en C₁ à C₄; halogénoalcoyl en C₁ à C₄; cycloalcoyl en C₃ à C₆; alcényle en C₂ à C₄; alcoxycarbonyle en C₂ à C₄; ou phénylméthyle éventuellement substitué par R⁶ sur le cycle phényle et par R⁸ sur le carbone benzylique;
R² est un groupe alcoyl en C₁ à C₆; cycloalcoyl en C₅ à C₇; alcényle en C₂ à C₆; cycloalcényle en C₅ à C₇; phényle éventuellement substitué par R⁵ et R⁷; 2-naphtalényle; thiényle éventuellement substitué par R⁵ et R⁷; furyle éventuellement substitué par R⁷; ou pyridyle éventuellement substitué par R⁷; ou bien phénoxy ou phénylthio, chacun éventuellement substitué par R⁷; ou encore
R¹ et R² peuvent, pris ensemble, former des structures choisies dans l'ensemble constitué par -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 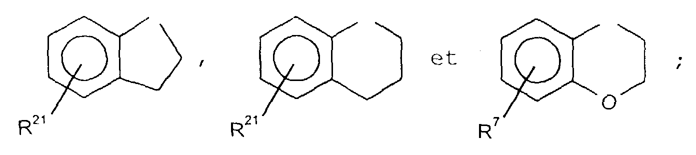
R³ est un groupe phényle, pyridyle ou pyrimidinyle, chacun éventuellement substitué par R¹⁰; ou phénylméthyle;
R⁴ est H ou un groupe méthyle;
R⁵ est un halogène ou un groupe nitro; cyano; alcoyl en C₁ à C₆; cycloalcoyl en C₅ à C₆; halogénoalcoyl en C₁ à C₆; alcoylthio en C₁ à C₆; halogénoalcoylthio en C₁ à C₆; alcoxy en C₁ à C₆; halogénoalcoxy en C₁ à C₆; cycloalcoyloxy en C₅ à C₆; alcoxyalcoyl en C₂ à C₆; alcoxyalcoxy en C₂ à C₆; alcényle en C₃ à C₆; halogénoalcényle en C₃ à C₆; alcényloxy en C₃ à C₆; alcynyle en C₃ à C₆; halogénoalcynyle en C₃ à C₆; alcynyloxy en C₃ à C₆; alcoylsulfonyle en C₁ à C₆; halogénoalcoylsulfonyle en C₁ à C₆; phényle ou phénylthio, chacun éventuellement substitué par R²⁴; phénylméthyle, phénoxyméthyle, phénéthyle ou styryle, chacun éventuellement substitué par R²⁴ sur le cycle phényle; phénoxy éventuellement substitué par R²⁷; et alcoxycarbonyle en C₂ à C₆;
R⁶, R⁷, R²⁴, R²⁶ et R³⁴ sont indépendamment un ou deux halogènes; un groupe nitro; alcoyl en C₁ à C₄; trifluorométhyle; méthylthio; ou alcoxy en C₁ à C₄;
R⁸ est H ou un groupe alcoyl en C₁ à C₄;
R¹⁰ est un ou deux substituants choisis dans l'ensemble constitué par les halogènes et les groupes nitro, cyano, alcoyl en C₁ à C₄, trifluorométhyle, alcoylthio en C₁ à C₄, alcoxy en C₁ à C₄ et trifluorométhoxy;
R²¹ est un halogène; un groupe nitro; alcoyl en C₁ à C₄; trifluorométhyle; méthylthio; ou alcoxy en C₁ à C₄; ou bien phénoxy éventuellement substitué par R²⁶;
R²⁷ est un ou deux halogènes; un groupe nitro; cyano; alcoyl en C₁ à C₆; halogénoalcoyl en C₁ à C₆; alcoxy en C₁ à C₆; halogénoalcoxy en C₁ à C₆; alcoylsulfonyle en C₁ à C₄; alcoxyalcoyl en C₂ à C₆; alcoylthio en C₁ à C₄; cycloalcoyl en C₅ à C₆; cycloalcoyloxy en C₅ à C₆; alcényle en C₂ à C₆; halogénoalcényle en C₂ à C₆; alcynyle en C₂ à C₆; alcoxycarbonyle en C₂ à C₄; ou phénoxy; et
R³⁶ est un groupe alcoyl en C₁ à C₆; ou phényle éventuellement substitué par R¹².

3. Un composé à action fongicide selon la revendication 1, dans lequel :
J est H; OH; C(=O)R¹⁶; C(=O)OR¹⁷; C(=O)NHR¹⁹;
R¹ est un groupe alcoyl en C₁ à C₄; halogénoalcoyl en C₁ à C₄; ou vinyle;
R² est un groupe alcoyl en C₁ à C₂₀; alcoxyalcoyl en C₂ à C₂₀; halogénoalkyle en C₂ à C₂₀; alcoyl en C₃ à C₈ substitué par un groupe phénoxy ou phénylthio, chacun éventuellement substitué par R³⁰; cycloalcoyl en C₅ à C₇; alcényle en C₂ à C₂₀; cycloalcényle en C₅ à C₇; phényle éventuellement substitué par R⁵ et R⁷; 2-naphtalényle; thiényle éventuellement substitué par R⁵ et R⁷; furyle éventuellement substitué par R⁷; ou pyridyle éventuellement substitué par R⁵ et R⁷;
R³ est un groupe phényle éventuellement substitué par R¹⁰;
R¹⁶ est H; un groupe alcoyl en C₁ à C₆; ou phényle éventuellement substitué par R³⁴;
R¹⁷ est un groupe alcoyl en C₁ à C₆;
R¹⁹ est un groupe alcoyl en C₁ à C₆; ou phényle éventuellement substitué par R³⁴;
du moment que lorsque R² est un groupe phényle et R⁵ n'est pas F, alors R⁵ est fixé en position para-par rapport au cycle oxazolidinone.

4. Un composé à action fongicide selon la revendication 3, dans lequel :
J est H; OH ou C(=O)R¹⁶;
R¹ est un groupe alcoyl en C₁ à C₂ ou vinyle;
R² est un groupe alcoyl en C₁ à C₁₂; cycloalcoyl en C₅ à C₇; phényle éventuellement substitué par R⁵ et R⁷; thiényle éventuellement substitué par R⁷; ou pyridyle éventuellement substitué par R⁵ et R⁷;
R³ est un groupe phényle éventuellement substitué par F; Cl; ou méthyle;
R⁴ est H;
R⁵ est un halogène; un groupe nitro; alcoyl en C₁ à C₆; halogénoalcoyl en C₁ à C₃; méthylthio; alcoxy en C₁ à C₆; halogénoalcoxy en C₁ à C₂; cycloalcoyloxy en C₅ à C₆; phénoxy éventuellement substitué par R²⁷; phénylthio substitué par R²⁴; phénoxyméthyle éventuellement substitué par R²⁴ sur le cycle phényle; benzyloxy éventuellement substitué par R³⁰ sur le cycle phényle ou -OC(=O)NHR²⁸;
R⁷ et R²⁴ sont indépendamment F; un groupe alcoyl en C₁ à C₂; méthylthio ou alcoxy en C₁ à C₂;
R¹⁶ est un qroupe alcoyl en C₁ à C₆;
R¹⁹ est un groupe phényle éventuellement substitué par R³⁴;
R²⁷ est un ou deux halogènes; un groupe cyano; alcoyl en C₁ à C₄; trifluorométhyle; alcoxy en C₁ à C₄; trifluorométhyle; alcoxy en C₁ à C₄; halogénoalcoxy en C₁ à C₄; alcoylthio en C₁ à C₄; cycloalcoyloxy en C₅ à C₆; ou allyle;
R³⁰ est un ou deux halogènes, un groupe cyano, alcoyl en C₁ à C₄, trifluorométhyle, alcoxy en C₁ à C₄ ou trifluorométhoxy; et
R³⁴ est un ou deux halogènes; un groupe nitro; alcoyl en C₁ à C₂; ou alcoxy en C₁ à C₂.

5. Un composé à activité fongicide selon la revendication 4, dans lequel :
J est H;
R¹ est un groupe méthyle;
R² est un groupe alcoyl en C₁ à C₁₂; phényle éventuellement substitué par R⁵ et R⁷; pyridyle éventuellement substitué par R⁵ et R⁷; et
R⁵ est F; Cl; Br; un groupe alcoyl en C₁ à C₆; trifluorométhyle; alcoxy en C₁ à C₆; trifluorométhoxy; 2,2,2-trifluoroéthoxy; cycloalcoyloxy en C₅ à C₆; méthylthio; phénoxy éventuellement substitué par R²⁷; phénylthio éventuellement substitué par R²⁴; benzyloxy éventuellement substitué par R³⁰ sur le cycle phényle; ou -OC(=O)R²⁸.

6. Un composé à action fongicide selon la revendication 5, choisi dans l'ensemble constitué par :
la 5-(2,4-difluorophényl)-5-méthyl-3-(phénylamino)-4-thioxo-2-oxazolidinone;
la 4-imino-5-méthyl-(4-phénoxyphényl)-3-(phénylamino)-2-oxazolidinone;
le bromhydrate de 4-imino-5-méthyl-(4-phénoxyphényl)-3-(phénylamino)-2-oxazolidinone; et
la 5-(2,4-difluorophényl) -4 - imino - 5 - méthyl -3-(phénylamino)-2-oxazolidinone.

7. Une composition à action fongicide comprenant les composés selon l'une quelconque des revendications 1 à 6.

8. Un procédé pour maîtriser les maladies des plantes, qui consiste à appliquer sur lesdites plantes un composé à action fongicide de formule I : 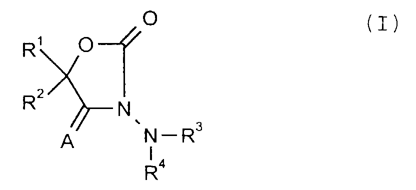 dans laquelle :
A est S ou N-J;
J est R¹⁵; C(=O)R¹⁶; C(=O)OR¹⁷; C(=O)SR¹⁸; C(=O)NR¹⁹R²⁰; P(=O)(alcoyl en C₁ à C₄)₂; ou OG;
G est H; un groupe alcoyl en C₁ à C₆; benzyle éventuellement substitué par R³⁴ sur le cycle phényle; C(=O) (alcoyl en C₁ à C₄); C(=O)(alcoxy en C₁ à C₄); ou C(=O)NHR³⁶;
R¹ est un groupe alcoyl en C₁ à C₄; halogénoalcoyl en C₁ à C₄; cycloalcoyl en C₃ à C₆; alcényle en C₂ à C₄; alcoxycarbonyle en C₂ à C₄; ou phénylméthyle éventuellement substitué par R⁶ sur le cycle phényle et par R⁸ sur le carbone benzylique;
R² est un groupe alcoyl en C₁ à C₂₀ éventuellement substitué par R²²; alcoxyalcoyl en C₂ à C₂₀ éventuellement substitué par R³⁵; alcényle en C₂ à C₂₀ éventuellement substitué par R⁴²; alcynyle en C₂ à C₂₀ éventuellement substitué par R⁴¹; (CH₂CH₂OCH₂CH₂)CH-; (CH₂CH₂SCH₂CH₂)CH-; (CH₂CH₂SO₂CH₂CH₂)CH-; cycloalcoyl en C₅ à C₇; cycloalcényle en C₅ à C₇; phényle éventuellement substitué par R⁵ et R⁷; 2-naphtalényle; thiényle éventuellement substitué par R⁵ et R⁷; furyle éventuellement substitué par R⁷; ou pyridyle éventuellement substitué par R⁵ et R⁷; ou bien
R¹ et R² peuvent, pris ensemble, former des structures choisies dans l'ensemble constitué par -CH₂(CH₂)₂CH₂-, -CH₂(CH₂)₃CH₂-, -CH₂(CH₂)₄CH₂-, CH₂CH₂OCH₂CH₂-, -CH₂CH₂SCH₂CH₂-, 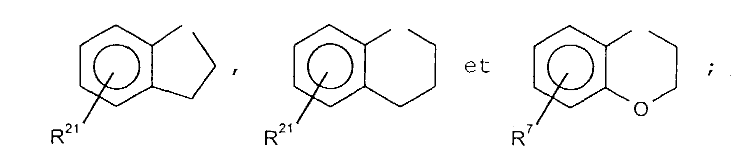
R³ est un groupe phényle, pyridyle ou pyrimidinyle, chacun éventuellement substitué par R¹⁰ ou un groupe phénylméthyle;
R⁴ est H ou un groupe méthyle;
R⁵ est un halogène ou un groupe nitro; cyano; alcoyl en C₁ à C₆; cycloalcoyl en C₅ à C₆; halogénoalcoyl en C₁ à C₆; alcoylthio en C₁ à C₆; halogénoalcoylthio en C₁ à C₆; alcoxy en C₁ à C₆; halogénoalcoxy en C₁ à C₆; cycloalcoyloxy en C₅ à C₆; alcoxyalcoyl en C₂ à C₆; alcoxyalcoxy en C₂ à C₆; alcényle en C₃ à C₆; halogénoalcényle en C₃ à C₆; alcényloxy en C₃ à C₆; alcynyle en C₃ à C₆; halogénoalcynyle en C₃ à C₆; alcynyloxy en C₃ à C₆; alcoylsulfonyle en C₁ à C₆; halogénoalcoylsulfonyle en C₁ à C₆; phényle ou phénylthio, chacun éventuellement substitué par R²⁶; phénylméthyle, phénoxyméthyle, phénéthyle ou styryle, chacun éventuellement substitué par R²⁶ sur le cycle phényle; phénoxy éventuellement substitué par R²⁷; benzyloxy éventuellement substitué par R³⁰ sur le cycle phényle; -OC(=O)NHR²⁸; -C(=O)OR²⁸; ou -OC(=O)R²⁸;
R⁶, R⁷, R¹², R¹³, R²⁴, R²⁶ et R³⁴ sont indépendamment un ou deux halogènes; un groupe nitro; alcoyl en C₁ à C₄; trifluorométhyle; méthylthio; ou alcoxy en C₁ à C₄;
R⁸, R¹⁴, R²⁰, R⁴⁰ et R³⁸ sont indépendamment H ou un groupe alcoyl en C₁ à C₄;
R⁹ est un groupe alcoyl en C₁ à C₁₈; ou phényle éventuellement substitué par R⁷;
R¹⁰, R²⁵ et R³³ sont indépendamment un ou deux substituants choisis dans l'ensemble constitué par les halogènes et les groupes nitro, cyano, alcoyl en C₁ à C₄, trifluorométhyle, alcoylthio en C₁ à C₄, alcoxy en C₁ à C₄ et trifluorométhoxy;
R¹¹ et R³⁶ sont indépendamment un groupe alcoyl en C₁ à C₆; ou phényle éventuellement substitué par R¹²;
R¹⁵ est H; un groupe alcoyl en C₁ à C₈ éventuellement substitué par un groupe alcoxy en C₁ à C₂; cycloalcoyl en C₃ à C₆; alcényle en C₃ à C₈; alcynyle en C₃ à C₈; phényle éventuellement substitué par R¹³; benzyle éventuellement substitué par R¹³ sur le cycle phényle et par R²⁰ sur le carbone benzylique; ou pyridyle éventuellement substitué par R¹³;
R¹⁶ est H; un groupe alcoyl en C₁ à C₁₇ éventuellement substitué par R³¹; alcényle en C₂ à C₁₇ éventuellement substitué par R³²; alcynyle en C₂ à C₇; cycloalcoyl en C₃ à C₈; cycloalcényle en C₅ à C₆; alcoylcycloalcoyl en C₆ à C₇; cycloalcoylalcoyl en C₄ à C₈; phényle éventuellement substitué par R³³; naphtalényle, furannyle, thiényle, benzoyle, ou pyridyle, chacun éventuellement substitué par R³⁴; ou alcoxycarbonyle en C₂ à C₅;
R¹⁷ et R¹⁸ sont indépendamment un groupe alcoyl en C₁ à C₁₈ éventuellement substitué par R²³; alcényle en C₂ à C₁₀ éventuellement substitué par R³²; alcynyle en C₃ à C₈; cycloalcoyl en C₃ à C₁₂; cycloalcényle en C₅ à C₆; alcoylcycloalcoyl en C₆ à C₇; cycloalcoylalcoyl en C₆ à C₇; ou phényle, naphtalényle, ou thiényle, chacun éventuellement substitué par R³⁴;
R¹⁹ est H; un groupe alcoyl en C₁ à C₁₀; cycloalcoyl en C₅ à C₆; ou phényle éventuellement substitué par R³⁴; ou bien
R¹⁹ et R²⁰ peuvent, pris ensemble, former des structures choisies dans l'ensemble constitué par -CH₂(CH₂)₂CH₂-, -CH₂(CH₂)₃CH₂-, -CH₂(CH₂)₄CH₂-, -CH₂CH₂OCH₂CH₂, -CH₂CH(Me)CH₂CH(Me)CH₂-, et -CH₂CH(Me)OCH(Me)CH₂-;
R²¹ est un ou deux halogènes; un groupe nitro; alcoyl en C₁ à C₄; trifluorométhyle; méthylthio; ou alcoxy en C₁ à C₄; ou bien phénoxy éventuellement substitué par R²⁶;
R²² est un groupe cyano; nitro; alcoylthio en C₁ à C₁₉; alcoylsulfinyle en C₁ à C₁₉; halogénoalcoxy en C₁ à C₁₉; cycloalcoyloxy en C₅ à C₆; alcényloxy en C₃ à C₁₉; alcynyloxy en C₃ à C₁₉; alcoylsulfonyle en C₁ à C₁₉; alcoxycarbonyle en C₂ à C₁₉; hydroxy; hydroxycarbonyle; R²⁸C(=O)O; R²⁸OC(=O)O; R²⁸R⁴⁰NC(=O)O; R³⁹R⁴⁰N; (alcoxy en C₁ à C₄)₂P(=E)O; R¹¹SO₃; R⁴⁰R¹⁴R³⁸N⁺; phényle, phénylthio, phénoxy, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydropyrannyle; cycloalcoyl en C₃ à C₆; 2-tétrahydropyrannyloxy; ou C(=Q)R⁴⁰;
E est O ou S;
Q est O ou N-T-W;
T est O; NR³⁷; ou une liaison directe;
W est H; un groupe alcoyl en C₁ à C₈, alcényle en C₃ à C₈, phénylméthyle éventuellement substitué par R⁷ sur le cycle phényle et R¹⁴ sur le carbone benzylique; phényle ou pyridyle, chacun éventuellement substitué par R⁷; C(=O)R²⁸; C(=O)OR²⁸; ou C(=O)NR²⁸R¹⁴;
R²³ est 1 à 3 halogènes; un groupe alcoxy en C₁ à C₁₂; alcoylthio en C₁ à C₁₂; phényle ou naphtalényle, chacun éventuellement substitué par R³⁴; ou phénoxyméthyle éventuellement substitué par R³⁴ sur le cycle phényle;
R²⁷ est un ou deux halogènes; un groupe nitro; cyano; alcoyl en C₁ à C₆; halogénoalcoyl en C₁ à C₆; alcoxy en C₁ à C₆; halogénoalcoxy en C₁ à C₆; alcoylsulfonyle en C₁ à C₄; alcoxyalcoyl en C₂ à C₆; alcoylthio en C₁ à C₄; cycloalcoyl en C₅ à C₆; cycloalcoyloxy en C₅ à C₆; alcényle en C₂ à C₆; halogénoalcényle en C₂ à C₆; alcynyle en C₂ à C₆; hydroxycarbonyle; alcoxycarbonyle en C₂ à C₄; ou phénoxy éventuellement substitué par R²⁴;
R²⁸ est un groupe alcoyl en C₁ à C₈; phényle ou pyridyle, chacun éventuellement substitué par R³⁰;
R³⁰ est un ou deux substituants choisis dans l'ensemble constitué par les halogènes, les groupes nitro, cyano, alcoyl en C₁ à C₄, trifluorométhyle, alcoxy en C₁ à C₄ et trifluorométhoxy; ou phénoxy éventuellement substitué par R²⁶;
R³¹ est 1 à 3 halogènes; un groupe alcoxy en C₁ à C₁₈; allyloxy; alcoylthio en C₁ à C₁₈; phényle, phénoxy, benzyloxy, ou phénylthio, chacun éventuellement substitué par R³⁴ sur le cycle phényle; acétyle; ou alcoxycarbonyle en C₂ à C₅;
R³² est 1 à 3 halogènes; ou un groupe alcoxy en C₁ à C₄;
R³⁵ est un groupe cyano; nitro; alcoylthio en C₁ à C₁₇; alcoylsulfinyle en C₁ à C₁₇; halogénoalcoxy en C₁ à C₁₇; cycloalcoyloxy en C₅ à C₆; halogénoalcényle en C₂ à C₁₇; alcényloxy en C₃ à C₁₇; halogénoalcynyle en C₃ à C₁₇; alcynyloxy en C₃ à C₁₇; alcoylsulfonyle en C₁ à C₁₇; alcoxycarbonyle en C₂ à C₁₇; hydroxy; hydroxycarbonyle; R²⁸C(=O)O; R²⁸OC(=O)O; R²⁸R⁴⁰NC(=O)O; R⁴⁰R³⁹N; (alcoxy en C₁ à C₄)₂P(=E)O; R¹¹SO₃; R⁴⁰R¹⁴R³⁸N⁺; phényle, phénoxy, phénylthio, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydropyrannyle; 2-tétrahydropyrannyloxy; alcoxy en C₁ à C₁₇; alcoxyalcoxy en C₂ à C₁₇; alcynyle en C₃ à C₁₇; cycloalcoyl en C₃ à C₆; ou halogénoalcoxyalcoxy en C₂ à C₁₇;
R³⁷ est H; un groupe alcoyl en C₁ à C₆; ou phényle éventuellement substitué par R⁷;
R³⁹ est un groupe alcoyl en C₁ à C₁₉; alcoylcarbonyle en C₂ à C₁₉; alcoxycarbonyle en C₂ à C₁₉; (R⁹R⁴⁰N)C=O; phényle éventuellement substitué par R²⁵; ou phénoxycarbonyle éventuellement substitué par R⁷;
R⁴¹ est un groupe cyano; nitro; alcoylthio en C₁ à C₁₇; alcoylsulfinyle en C₁ à C₁₇; halogénoalcoxy en C₁ à C₁₇; cycloalcoyloxy en C₅ à C₆; alcényloxy en C₃ à C₁₇; alcynyloxy en C₃ à C₁₇; alcoylsulfonyle en C₁ à C₁₇; alcoxycarbonyle en C₂ à C₁₇; hydroxy; hydroxycarbonyle; R²⁸C(=O)O; R²⁸OC(=O)O; R²⁸R⁴⁰NC(=O)O; R⁴⁰R³⁹N; (alcoxy en C₁ à C₄)₂P(=E)O; R¹¹SO₃; R⁴⁰R¹⁴R³⁸N⁺; phényle, phénoxy, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydropyrannyle; 2-tétrahydropyrannyloxy; alcoxy en C₁ à C₁₇; alcoxyalcoxy en C₂ à C₁₇; cycloalcoyl en C₃ à C₆; ou 1 à 3 halogènes;
R⁴² est un groupe cyano; nitro; alcoylthio en C₁ à C₁₇; alcoylsulfinyle en C₁ à C₁₇; halogénoalcoxy en C₁ à C₁₇; cycloalcoyloxy en C₅ à C₆; alcényloxy en C₃ à C₁₇; halogénoalcynyle en C₃ à C₁₇; alcynyloxy en C₃ à C₁₇; alcoylsulfonyle en C₁ à C₁₇; alcoxycarbonyle en C₂ à C₁₇; hydroxy; hydroxycarbonyle; R²⁸C(=O)O; R²⁸OC(=O)O; R²⁸R⁴⁰NC(=O)O; R⁴⁰R³⁹N; (alcoxy en C₁ à C₄)₂P(=E)O; R¹¹SO₃; R⁴⁰R¹⁴R³⁸N⁺; phényle, phénoxy, phénylthio, phénylsulfonyle, phénylsulfinyle, pyridyle ou pyridyloxy, chacun éventuellement substitué par R³⁰; thiényle, pyrimidinyle, furannyle, naphtalényle, pyrimidinyloxy, naphtalényloxy, chacun éventuellement substitué par R⁷; tétrahydropyrannyle; 2-tétrahydropyrannyloxy; alcoxy en C₁ à C₁₇; alcoxyalcoxy en C₂ à C₁₇; alcynyle en C₃ à C₁₇; cycloalcoyl en C₃ à C₆; ou 1 à 3 halogènes;
du moment que :
i) quand R² est un cycle hétérocyclique ou phényle substitué, alors seul H ou F peut être un substituant sur le ou les atomes de carbone du cycle hétérocyclique ou phényle adjacent au carbone portant le cycle oxazolidinone;
ii) quand R³ est un cycle hétérocyclique ou phényle disubstitué par deux groupes alcoyl ou alcoxy, ou par un groupe alcoyl et un groupe alcoxy, alors au moins l'un des groupes alcoyl et alcoxy est un groupe méthyle ou méthoxy; et
iii) le nombre total d'atome de carbones dans chacun de R², R¹⁶, R¹⁷ et R¹⁸ est inférieur ou égal à 20.

9. Un procédé pour maîtriser les maladies des plantes, qui consiste à appliquer sur lesdites plantes un composé à action fongicide de formule I : 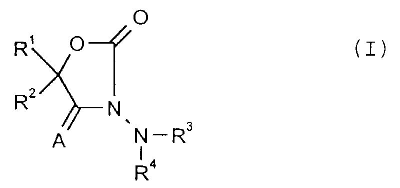 dans laquelle :
A est S ou N-OG;
G est H; un groupe alcoyl en C₁ à C₆; benzyle éventuellement substitué par R³⁴ sur le cycle phényle; C(=O)(alcoyl en C₁ à C₄); C(=O)(alcoxy en C₁ à C₄); ou C(=O)NHR³⁶;
R¹ est un groupe alcoyl en C₁ à C₄; halogénoalcoyl en C₁ à C₄; cycloalcoyl en C₃ à C₆; alcényle en C₂ à C₄; alcoxycarbonyle en C₂ à C₄; ou phénylméthyle éventuellement substitué par R⁶ sur le cycle phényle et par R⁸ sur le carbone benzylique;
R² est un groupe alcoyl en C₁ à C₆; cycloalcoyl en C₅ à C₇; alcényle en C₂ à C₆; cycloalcényle en C₅ à C₇; phényle éventuellement substitué par R⁵ et R⁷; 2-naphtalényle; thiényle éventuellement substitué par R⁵ et R⁷; furyle éventuellement substitué par R⁷; ou pyridyle éventuellement substitué par R⁷; ou bien phénoxy ou phénylthio, chacun éventuellement substitué par R⁷; ou encore
R¹ et R² peuvent, pris ensemble, former des structures choisies dans l'ensemble constitué par -CH₂(CH₂)₂CH₂-, -CH₂(CH₂)₃CH₂-, -CH₂(CH₂)₄CH₂-, -CH₂CH₂OCH₂CH₂-, -CH₂CH₂SCH₂CH₂-, 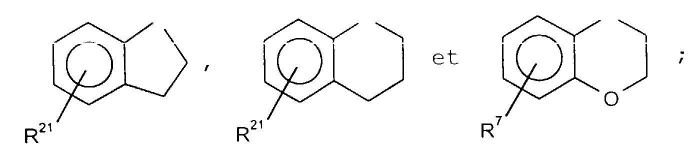
R³ est un groupe phényle, pyridyle ou pyrimidinyle, chacun éventuellement substitué par R¹⁰; ou phénylméthyle;
R⁴ est H ou un groupe méthyle;
R⁵ est un halogène ou un groupe nitro; cyano; alcoyl en C₁ à C₆; cycloalcoyl en C₅ à C₆; halogénoalcoyl en C₁ à C₆; alcoylthio en C₁ à C₆; halogénoalcoylthio en C₁ à C₆; alcoxy en C₁ à C₆; halogénoalcoxy en C₁ à C₆; cycloalcoyloxy en C₅ à C₆; alcoxyalcoyl en C₂ à C₆; alcoxyalcoxy en C₂ à C₆; alcényle en C₃ à C₆; halogénoalcényle en C₃ à C₆; alcényloxy en C₃ à C₆; alcynyle en C₃ à C₆; halogénoalcynyle en C₃ à C₆; alcynyloxy en C₃ à C₆; alcoylsulfonyle en C₁ à C₆; halogénoalcoylsulfonyle en C₁ à C₆; phényle ou phénylthio, chacun éventuellement substitué par R²⁴; phénylméthyle, phénoxyméthyle, phénéthyle ou styryle, chacun éventuellement substitué par R²⁴ sur le cycle phényle; phénoxy éventuellement substitué par R²⁷; et alcoxycarbonyle en C₂ à C₆;
R⁶, R⁷, R²⁴, R²⁶ et R³⁴ sont indépendamment un ou deux halogènes; un groupe nitro; alcoyl en C₁ à C₄; trifluorométhyle; méthylthio; ou alcoxy en C₁ à C₄;
R⁸ est H ou un groupe alcoyl en C₁ à C₄;
R¹⁰ est un ou deux substituants choisis dans l'ensemble constitué par les halogènes et les groupes nitro, cyano, alcoyl en C₁ à C₄, trifluorométhyle, alcoylthio en C₁ à C₄, alcoxy en C₁ à C₄ et trifluorométhoxy;
R²¹ est un halogène; un groupe nitro; alcoyl en C₁ à C₄; trifluorométhyle; méthylthio; ou alcoxy en C₁ à C₄; ou bien phénoxy éventuellement substitué par R²⁶;
R²⁷ est un ou deux halogènes; un groupe nitro; cyano; alcoyl en C₁ à C₆; halogénoalcoyl en C₁ à C₆; alcoxy en C₁ à C₆; halogénoalcoxy en C₁ à C₆; alcoylsulfonyle en C₁ à C₄; alcoxyalcoyl en C₂ à C₆; alcoylthio en C₁ à C₄; cycloalcoyl en C₅ à C₆; cycloalcoyloxy en C₅ à C₆; alcényle en C₂ à C₆; halogénoalcényle en C₂ à C₆; alcynyle en C₂ à C₆; alcoxycarbonyle en C₂ à C₄; ou phénoxy; et
R³⁶ est un groupe alcoyl en C₁ à C₆; ou phényle éventuellement substitué par R¹².

10. Un procédé selon la revendication 8, dans lequel :
J est H; OH; C(=O)R¹⁶; C(=O)OR¹⁷; C(=O)NHR¹⁹;
R¹ est un groupe alcoyl en C₁ à C₄; halogénoalcoyl en C₁ à C₄; ou vinyle;
R² est un groupe alcoyl en C₁ à C₂₀; alcoxyalcoyl en C₂ à C₂₀; halogénoalcoyl en C₂ à C₂₀; alcoyl en C₃ à C₈ substitué par un groupe phénoxy ou phénylthio, chacun éventuellement substitué par R³⁰; cycloalcoyl en C₅ à C₇; alcényle en C₂ à C₂₀; cycloalcényle en C₅ à C₇; phényle éventuellement substitué par R⁵ et R⁷; 2-naphtalényle; thiényle éventuellement substitué par R⁵ et R⁷; furyle éventuellement substitué par R⁷; ou pyridyle éventuellement substitué par R⁵ et R⁷;
R³ est un groupe phényle éventuellement substitué par R¹⁰;
R¹⁶ est H; un groupe alcoyl en C₁ à C₆; ou phényle éventuellement substitué par R³⁴;
R¹⁷ est un groupe alcoyl en C₁ à C₆;
R¹⁹ est un groupe alcoyl en C₁ à C₆; ou phényle éventuellement substitué par R³⁴;
du moment que lorsque R² est un groupe phényle et R⁵ n'est pas F, alors R⁵ est fixé position para-par rapport au cycle oxazolidinone.

11. Le procédé selon la revendication 8, dans lequel :
J est H; OH ou C(=O)R¹⁶;
R¹ est un groupe alcoyl en C₁ à C₂ ou vinyle;
R² est un groupe alcoyl en C₁ à C₁₂; cycloalcoyl en C₅ à C₇; phényle éventuellement substitué par R⁵ et R⁷; thiényle éventuellement substitué par R⁷; ou pyridyle éventuellement substitué par R⁵ et R⁷;
R³ est un groupe phényle éventuellement substitué par F; Cl; ou méthyle;
R⁴ est H;
R⁵ est un halogène; un groupe nitro; alcoyl en C₁ à C₆; halogénoalcoyl en C₁ à C₃; méthylthio; alcoxy en C₁ à C₆; halogénoalcoxy en C₁ à C₂; cycloalcoyloxy en C₅ à C₆; phénoxy éventuellement substitué par R²⁷; phénylthio substitué par R²⁴; phénoxyméthyle éventuellement substitué par R²⁴ sur le cycle phényle; benzyloxy éventuellement substitué par R³⁰ sur le cycle phényle; -OC(=O)NHR²⁸;
R⁷ et R²⁴ sont indépendamment F; un groupe alcoyl en C₁ à C₂; méthylthio; ou alcoxy en C₁ à C₂;
R¹⁶ est un groupe alcoyl en C₁ à C₆;
R¹⁹ est un groupe phényle éventuellement substitué par R³⁴;
R²⁷ est un ou deux halogènes; un groupe cyano; alcoyl en C₁ à C₄; trifluorométhyle; alcoxy en C₁ à C₄; trifluorométhyle; alcoxy en C₁ à C₄; halogénoalcoxy en C₁ à C₄; alcoylthio en C₁ à C₄; cycloalcoyloxy en C₅ à C₆; ou allyle;
R³⁰ est un ou deux halogènes, un groupe cyano, alcoyl en C₁ à C₄, trifluorométhyle, alcoxy en C₁ à C₄ ou trifluorométhoxy; et
R³⁴ est un ou deux halogènes; un groupe nitro; alcoyl en C₁ à C₂; ou alcoxy en C₁ à C₂.

12. Un procédé selon la revendication 11, dans lequel:
J est H;
R¹ est un groupe méthyle;
R² est un groupe alcoyl en C₁ à C₁₂; phényle éventuellement substitué par R⁵ et R⁷; pyridyle éventuellement substitué par R⁵ et R⁷; et
R⁵ est F; Cl; Br; un groupe alcoyl en C₁ à C₆; trifluorométhyle; alcoxy en C₁ à C₆; trifluorométhoxy; 2,2,2-trifluoroéthoxy; cycloalcoyloxy en C₅ à C₆; méthylthio; phénoxy éventuellement substitué par R²⁷; phénylthio éventuellement substitué par R²⁴; benzyloxy éventuellement substitué par R³⁰ sur le cycle phényle; ou -OC(=O)R²⁸.

13. Un procédé selon la revendication 8, dans lequel ledit composé est choisi dans l'ensemble consistant en:
la 5-(2,4-difluorophényl)-5-méthyl-3-(phénylamino)-4-thioxo-2-oxazolidinone;
la 4-imino-5-méthyl- (4-phénoxyphényl) - 3-(phénylamino) - 2-oxazolidinone;
le bromhydrate de 4-imino-5-méthyl-(4-phénoxyphényl)-3-(phénylamino)-2-oxazolidinone; et
la 5-(2,4-difluorophényl)-4-imino-5-méthyl-3-(phénylamino)-2-oxazolidinone.

14. Un procédé pour maîtriser les maladies des plantes, qui consiste à appliquer sur lesdites plantes une composition comprenant un composé à action fongicide selon l'une quelconque des revendications 1 à 6.
